# EUROPEAN PATENT APPLICATION

(11) **EP 4 137 484 A1**
(43) Date of publication of application: **22.02.2023**
(21) Application number: 21789436.9
(22) Date of filing: 13.04.2021
(51) Int. Cl.: C07D 239/48, C07D 223/14, C07D 243/00, C07D 313/06, C07D 487/14, C07D 403/14, C07D 471/14, C07D 519/00, A61K 31/495, A61P 35/00

(54) **TRICYCLIC COMPOUNDS AS EGFR INHIBITORS**

(30) Priority: 14.04.2020 CN 202010292186; 22.08.2020 CN 202010852717; 09.02.2021 CN 202110175424; 24.03.2021 CN 202110312259
(71) Applicant: QILU PHARMACEUTICAL CO., LTD., High Technical Zone Jinan Shandong 250100 (CN)
(72) Inventor: ZHENG, Shansong, Jinan, Shandong 250100 (CN); DENG, Wei, Jinan, Shandong 250100 (CN); CAMPOS, Sebastien Andre, Jinan, Shandong 250100 (CN); YANG, Yingying, Jinan, Shandong 250100 (CN); TIAN, Zhenhua, Jinan, Shandong 250100 (CN); ZHENG, Qingmei, Jinan, Shandong 250100 (CN); WU, Guosheng, Jinan, Shandong 250100 (CN); ZHAO, Zhiwei, Jinan, Shandong 250100 (CN); LI, Leilei, Jinan, Shandong 250100 (CN); FU, Jianmin, Jinan, Shandong 250100 (CN); ZHAO, Shuyong, Jinan, Shandong 250100 (CN)
(74) Representative: Gille Hrabal Partnerschaftsgesellschaft mbB Patentanwälte
(86) International application number: PCT/CN2021/086941
(87) International publication number: WO 2021/208918

(57) **Abstract**

Provided are a class of compounds, represented by formula (I‴), as selective EGFR inhibitors, a pharmaceutical composition containing the compounds, useful intermediates for preparing the compounds, and a method for using the compounds of the present invention to treat cell proliferative diseases, such as cancers.

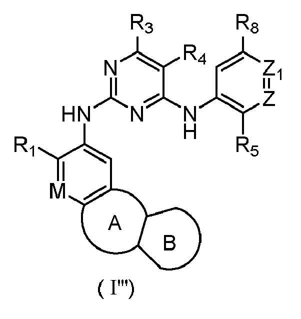

## Description

### CROSS-REFERENCE TO RELATED APPLICATIONS

The present application claims priorities to and benefits of the following previous applications filed to China National Intellectual Property Administration: the Patent Application for Invention with the application No. 202010292186.8 and entitled "FUSED TRICYCLIC COMPOUND AS EGFR INHIBITOR" filed on Apr. 14, 2020, the Patent Application for Invention with the application No. 202010852717.4 and entitled "FUSED TRICYCLIC COMPOUND AS EGFR INHIBITOR" filed on Aug. 22, 2020, the Patent Application for Invention with the application No. 202110175424.1 and entitled "FUSED TRICYCLIC COMPOUND AS EGFR INHIBITOR" filed on Feb. 9, 2021, and the Patent Application for Invention with the application No. 202110312259.X and entitled "FUSED TRICYCLIC COMPOUND AS EGFR INHIBITOR" filed on Mar. 24, 2021; which are incorporated herein by reference in their entirety.

### TECHNICAL FIELD

The present disclosure belongs to the field of pharmaceutical chemistry, and specifically relates to a novel compound as a selective EGFR inhibitor, a pharmaceutical composition containing the compound, a useful intermediate for preparing the compound and a method for the treatment of a cell proliferative disease, for example a cancer by using the compound of the present disclosure.

### BACKGROUND

Lung cancer is the cancer with highest incidence and mortality, which seriously threatens human health and life. Lung cancer is mainly classified into small cell lung cancer (SCLC) and non-small cell lung cancer (NSCLC), of which about 80% are NSCLC.

EGFR i.e., an epidermal growth factor receptor, is widely distributed on the surface of mammalian cells such as epithelial cells, fibroblasts, glial cells and other cells. The EGFR signaling pathway plays an important role in physiological processes such as growth, proliferation and differentiation of cells. EGFR mutation is also one of the most common type of mutation in NSCLC patients, especially in Asian populations, which can occur in 40%-50%. Therefore, EGFR has always been one of the most popular targets in the field of drug development and research.

At present, there are first-, second- and third-generation EGFR inhibitors on the market. The first generation of EGFR inhibitors are reversible targeted drugs, such as Gefitinib, Erlotinib and Icotinib. The second generation of EGFR inhibitors are irreversible targeted drugs, such as Afatinib and Dacomitinib. Although the first- and second-generation targeted drugs have significant curative effect, most patients still suffer from drug resistance after using drugs for 1-2 years. Among patients with EGFR inhibitor resistance, 50% of the drug resistance is associated with T790M mutation. The third-generation EGFR targeting drug omisertinib can overcome the tumor resistance caused by T790M mutation, and brings better survival benefit to more patients with lung cancer. However, the third-generation targeting drug also inevitably generates drug resistance, and the drug resistance is mainly caused by C797S mutation. The mutation of C797S, which is embodied by mutation of cysteine into serine, disrupts the binding of EGFR protein to third-generation targeted drugs, thereby failing to prevent the phosphorylation of the EGFR protein and the activation of downstream signaling pathways. At present, no mature treatment is available for two *cis*-triple mutations, Del19/T790M/C797S and L858R/T790M/C797S, mainly appearing after resistance to Omisertinib, the clinical demand is urgent, and the present disclosure is based on solving this problem.

### SUMMARY

The object of the present disclosure is to provide a class of tricyclic compounds as selective EGFR inhibitors, a pharmaceutical composition containing the compounds, a useful intermediate for preparing the compounds and use of the compounds for the manufacturing of a medicament for the treatment of cancer.

The present disclosure provides a compound represented by formula (I‴), or a stereoisomer, a tautomer or a pharmaceutically acceptable salt, a prodrug, a hydrate, a solvate and an isotopically labeled derivative thereof, 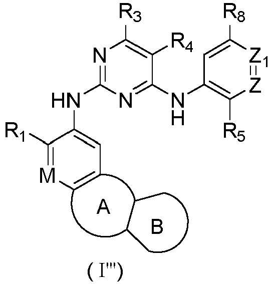 wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₂₋₆ alkynyloxy, C₁₋₆ alkylamino, C₁₋₆ haloalkylamino, C₃₋₆ cycloalkylamino, 3-6 membered heterocycloalkylamino, C₂₋₆ alkenylamino, and C₂₋₆ alkynylamino;
M is selected from N and CRₐ; Rₐ is H, halogen, C₁₋₆ alkyl, C₃₋₆ cycloalkyl, C₁₋₆ heteroalkyl, or C₁₋₆ haloalkyl;
Z is selected from N and CR₆;
Z₁ is selected from N and CR₇;
or, Rₐ cyclizes with R₁ to form a substituted or unsubstituted 5-8 membered heterocyclyl or 5-8 membered carbocyclyl;
ring A is selected from the substituted or unsubstituted 5-8 membered heterocyclyl or 5-8 membered carbocyclyl;
ring B is absent or selected from aryl or 5-6 membered heteroaryl, and 4-8 membered heterocycloalkyl or C₄₋₈ cycloalkyl, which are optionally substituted with one or more R₂;
R₂ is each independently selected from H, halogen, -CN, -C(=O)R_{b}, -C(=O)NR_{b}R_{c}, -S(=O)₂R_{b}, - S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₅₋₆ aryl, C₅₋₆ arylalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, C₂₋₆ alkenylamino, and -(CH₂)ᵣNR_{c}R_{d}, r is optionally selected from 0, 1, 2, and 3; wherein the C₃₋₆ cycloalkyl, the 3-6 membered heterocycloalkyl, the C₅₋₆ aryl, the C₅₋₆ arylalkyl, the C₃₋₆ cycloalkyloxy, the 3-6 membered heterocycloalkyloxy, the C₂₋₆ alkenyloxy, the C₁₋₆ alkylamino, the C₁₋₆ haloalkylamino, the C₃₋₆ cycloalkylamino, the 3-6 membered heterocycloalkylamino or the C₂₋₆ alkenylamino in R₂ is optionally substituted with one or more C₁₋₃ alkyl or C₁₋₃ alkoxy;
R₃ and R₄ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl, 3-8 membered heterocycloalkyl, C₁₋₆ alkylamino, C₁₋₆ haloalkylamino, C₃₋₆ cycloalkylamino, 3-6 membered heterocycloalkylamino, and C₂₋₆ alkenylamino;
or, R₃ cyclizes with R₄ to form aryl, C₄₋₇ cycloalkyl, 5-7 membered heterocycloalkyl, or 5-6 membered heteroaryl;
R₅ is selected from substituted or unsubstituted -NH₂, -C(=O)NR_{b}R_{c}, -S(=O)₂R_{b}, -P(=O)R_{b}R_{c}, - P(=O)R_{b}NR_{c}R_{d}, -P(=O)R_{b}OR_{c}, -P(=O)OR_{b}OR_{c}, -P(=S)R_{b}R_{c}, -P(=S)R_{b}NR_{c}R_{d}, -P(=S)R_{b}OR_{c}, - P(=S)OR_{b}OR_{c}, -S(=O)₂NR_{b}R_{c}, R_{b}S(=O)₂NR_{c}-, -N=S(=O)R_{b}R_{c} or R_{b}N=S(=O)(R_{c})-, -NR_{b}C(O)R_{c}, and R_{c}S(=NR_{b})(=O)NR_{d}-;
R_{b}, R_{c} and R_{d} are each independently selected from H, -CN, C₁₋₃ alkyl, C₁₋₃ haloalkyl, C₃₋₆ cycloalkyl, C₄₋₆ heterocycloalkyl, C₅₋₁₀ aryl, and 5-10 membered heteroaryl;
or, R_{b} and R_{c} cyclizes with atoms to which they are both attached to form 5-6 membered heterocycloalkyl unsubstituted or optionally substituted with one or more C₁₋₃ alkyl or C₁₋₃ alkoxy; R₆, R₇ and R₈ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ alkylamino, C₁₋₆ haloalkylamino, C₃₋₆ cycloalkylamino, 3-6 membered heterocycloalkylamino, and C₂₋₆ alkenylamino;
or, R₅ cyclizes with R₆ to form a 4-7 membered ring containing -P(=O)(R_{b})-, -P(=S)(R_{b})-, - N(R_{b})S(=O)₂-, -S(=O)₂N(R_{b})-, or -S(=O)₂;
or, R₆ cyclizes with R₇ to form C₄₋₆ cycloalkyl, 4-6 membered heterocycloalkyl, aryl, and 5-6 membered heteroaryl;
or, R₇ cyclizes with R₈ to form C₄₋₆ cycloalkyl, 4-6 membered heterocycloalkyl, aryl, and 5-6 membered heteroaryl.

In some embodiments of the present disclosure, R₁ is selected from H, halogen, -CN, C₁₋₆ alkyl, C₁₋₆ alkoxy, C₁₋₆ haloalkyl, and C₁₋₆ haloalkoxy, preferably, R₁ is selected from H, halogen, -CN, C₁₋₆ alkyl, and C₁₋₆ alkoxy.

In some embodiments of the present disclosure, M is selected from N and CRₐ; Rₐ is H, halogen, C₁₋₃ alkyl, C₃₋₆ cycloalkyl or C₁₋₃ haloalkyl, preferably, M is selected from N and CH.

In some embodiments of the present disclosure, ring A is selected from a substituted or unsubstituted 5-8 membered carbocyclyl and a 5-8 membered heterocyclyl containing 1 or 2 heteroatoms selected from O, S and N; in some embodiments, ring A may comprise a double bond; in some embodiments, 1 or 2 ring atoms on ring A may be optionally replaced with -C(=O), -N(=O), -S(=O), and -S(=O)₂, ring A may also be optionally substituted with one or more Rₓ groups, wherein the Rₓ is selected from H, -OH, -CN, -NH₂, halogen, C₁₋₆ alkylcarbonyl, C₁₋₆ alkyl, C₁₋₆ haloalkyl, C₃₋₈ cycloalkyl, 3-8 membered heterocycloalkyl, C₃₋₈ cycloalkyl-C₁₋₆ alkyl-, 3-8 membered heterocycloalkyl-C₁₋₆ alkyl-, aryl-C₁₋₆ alkyl-, C₅₋₁₃ spirocyclyl, and 5-13 membered spiroheterocyclyl; wherein the C₃₋₈ cycloalkyl, the 3-8 membered heterocycloalkyl, the C₃₋₈ cycloalkyl-C₁₋₆ alkyl-, the 3-8 membered heterocycloalkyl-C₁₋₆ alkyl-, the aryl-C₁₋₆ alkyl-, the C₅₋₁₃ spirocyclyl, and the 5-13 membered spirocyclyl membered spiroheterocyclyl are optionally substituted with one or more R_{y}; the R_{y} is selected from H, halogen, C₁₋₆ alkyl, C₁₋₆ haloalkyl, C₁₋₆ alkoxy, C₃₋₈ cycloalkyl, 4-8 membered heterocycloalkyl, C₃₋₈ cycloalkyl-C₁₋₆ alkyl-, 4-8 membered heterocycloalkyl-C₁₋₆ alkyl-, 5-10 membered aryl, and 5-10 membered heteroaryl.

In some embodiments of the present disclosure, ring B is aryl or 5-6 membered heteroaryl, optionally substituted with one or more R₂; the aryl and the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, pyridazinyl, or triazinyl.

In some embodiments of the present disclosure, R₂ is each independently selected from H, halogen, -CN, -C(=O)R_{b}, -S(=O)₂R_{b}, -S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₅₋₆ aryl, C₅₋₆ arylalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, C₂₋₆ alkenylamino, and -(CH₂)ᵣNR_{c}R_{d}, wherein r is optionally selected from 0, 1, 2, and 3; wherein the 3-6 membered heterocycloalkyl or the C₅₋₆ arylalkyl in R₂ is optionally substituted with one or more C₁₋₃ alkyl or C₁₋₃ alkoxy.

In some embodiments of the present disclosure, R₃ and R₄ are each independently selected from H, halogen, -CN, C₁₋₆ alkyl, C₁₋₆ alkoxy, C₁₋₆ haloalkyl, C₁₋₆ haloalkoxy, and C₃₋₆ cycloalkyl; or, R₃ cyclizes with R₄ to form phenyl, C₄₋₇ cycloalkyl, and 5-7 membered heterocycloalkyl or 5-6 membered heteroaryl containing 1 or 2 heteroatoms selected from O, S, and N; preferably, the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, pyridazinyl, or triazinyl. In some embodiments of the present disclosure, R₅ is selected from substituted or unsubstituted - NH₂, -C(=O)NR_{b}R_{c}, -S(=O)₂R_{b}, -P(=O)R_{b}R_{c}, -P(=O)R_{b}NR_{c}R_{d}, -P(=O)R_{b}OR_{c}, -P(=O)OR_{b}OR_{c}, - P(=S)R_{b}R_{c}, -P(=S)R_{b}NR_{c}R_{d}, -P(=S)R_{b}OR_{c}, -P(=S)OR_{b}OR_{c}, -S(=O)₂NR_{b}R_{c}, R_{b}S(=O)₂NR_{c}-, - N=S(=O)R_{b}R_{c}, R_{b}N=S(=O)(R_{c})-, -NR_{b}C(O)R_{c}; or, R₅ cyclizes with R₆ to form a 4-7 membered ring containing -P(=O)(R_{b})-, -P(=S)(R_{b})-, -N(R_{b})S(=O)₂-, -S(=O)₂N(R_{b})- or -S(=O)₂.

In some embodiments of the present disclosure, R_{b}, R_{c} and R_{d} are each independently selected from H, C₁₋₃ alkyl, C₁₋₃ haloalkyl, and C₃₋₆ cycloalkyl; or, R_{b} and R_{c} cyclizes with atoms to which they are both attached to form 5-6 membered heterocycloalkyl unsubstituted or optionally substituted with one or more C₁₋₃ alkyl or C₁₋₃ alkoxy.

In some embodiments of the present disclosure, R₆ and R₇ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ alkylamino, C₁₋₆ haloalkylamino, C₃₋₆ cycloalkylamino, 3-6 membered heterocycloalkylamino, and C₂₋₆ alkenylamino; or, R₆ cyclizes with R₇ to form C₄₋₆ cycloalkyl, 4-6 membered heterocycloalkyl, phenyl, and 5-6 membered heteroaryl; preferably, the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, pyridazinyl, or triazinyl.

In some embodiments of the present disclosure, R₈ is H.

The present disclosure provides a compound represented by formula (I"), or a stereoisomer, a tautomer or a pharmaceutically acceptable salt, a prodrug, a hydrate, a solvate and an isotopically labeled derivative thereof, 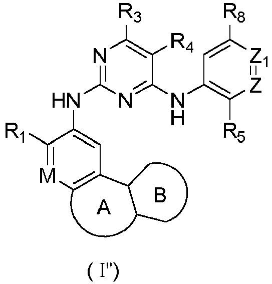 wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₂₋₆ alkynyloxy, C₁₋₆ alkylamino, C₁₋₆ haloalkylamino, C₃₋₆ cycloalkylamino, 3-6 membered heterocycloalkylamino, C₂₋₆ alkenylamino, and C₂₋₆ alkynylamino;
M is selected from N and CRₐ; Rₐ is H, halogen, C₁₋₃ alkyl, C₃₋₆ cycloalkyl or C₁₋₃ haloalkyl;
Z is selected from N and CR₆;
Z₁ is selected from N and CR₇;
or, Rₐ cyclizes with R₁ to form a substituted or unsubstituted 5-8 membered heterocyclyl; ring A is selected from the substituted or unsubstituted 5-8 membered heterocyclyl or 5-8 membered carbocyclyl;
ring B is aryl or 5-6 membered heteroaryl, optionally substituted with one or more R₂; the aryl and the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, pyridazinyl, or triazinyl;
R₂ is each independently selected from H, halogen, -CN, -C(=O)R_{b}, -S(=O)₂R_{b}, -S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₅₋₆ aryl, C₅₋₆ arylalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, C₂₋₆ alkenylamino, and -(CH₂)ᵣNR_{c}R_{d}, wherein r is optionally selected from 0, 1, 2, and 3;
R₃ and R₄ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl, 3-8 membered heterocycloalkyl, C₁₋₆ alkylamino, C₁₋₆ haloalkylamino, C₃₋₆ cycloalkylamino, 3-6 membered heterocycloalkylamino, and C₂₋₆ alkenylamino;
or, R₃ cyclizes with R₄ to form aryl, C₄₋₇ cycloalkyl, 5-7 membered heterocycloalkyl, or 5-6 membered heteroaryl;
R₅ is selected from substituted or unsubstituted -NH₂, -C(=O)NR_{b}R_{c}, -S(=O)₂R_{b}, -P(=O)R_{b}R_{c}, - P(=O)R_{b}NR_{c}R_{d}, -P(=O)R_{b}OR_{c}, -P(=O)OR_{b}OR_{c}, -P(=S)R_{b}R_{c}, -P(=S)R_{b}NR_{c}R_{d}, -P(=S)R_{b}OR_{c}, - P(=S)OR_{b}OR_{c}, -S(=O)₂NR_{b}R_{c}, R_{b}S(=O)₂NR_{c}-, -N=S(=O)R_{b}R_{c} or R_{b}N=S(=O)(R_{c})-, and - NR_{b}C(O)R_{c};
R_{b}, R_{c} and R_{d} are each independently selected from H, -CN, C₁₋₃ alkyl, C₁₋₃ haloalkyl, C₃₋₆ cycloalkyl, C₄₋₆ heterocycloalkyl, C₅₋₁₀ aryl, and 5-10 membered heteroaryl;
or, R_{b} and R_{c} cyclizes with atoms to which they are both attached to form 5-6 membered heterocycloalkyl;
R₆, R₇ and R₈ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ alkylamino, C₁₋₆ haloalkylamino, C₃₋₆ cycloalkylamino, 3-6 membered heterocycloalkylamino, and C₂₋₆ alkenylamino;
or, R₅ cyclizes with R₆ to form a 4-7 membered ring containing -P(=O)(R_{b})-, -P(=S)(R_{b})-, - N(R_{b})S(=O)₂-, -S(=O)₂N(R_{b})-, or -S(=O)₂;
or, R₆ cyclizes with R₇ to form C₄₋₆ cycloalkyl, 4-6 membered heterocycloalkyl, aryl, and 5-6 membered heteroaryl;
or, R₇ cyclizes with R₈ to form C₄₋₆ cycloalkyl, 4-6 membered heterocycloalkyl, aryl, and 5-6 membered heteroaryl.

The present disclosure provides a compound represented by formula (I'), or a stereoisomer, a tautomer or a pharmaceutically acceptable salt, a prodrug, a hydrate, a solvate and an isotopically labeled derivative thereof, 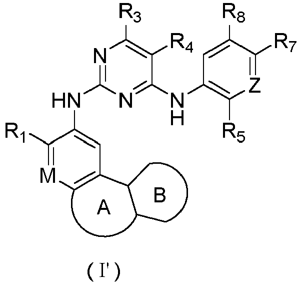 wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₂₋₆ alkynyloxy, C₁₋₆ alkylamino, C₁₋₆ haloalkylamino, C₃₋₆ cycloalkylamino, 3-6 membered heterocycloalkylamino, C₂₋₆ alkenylamino, and C₂₋₆ alkynylamino;
M is selected from N and CRₐ; Rₐ is H, halogen, C₁₋₃ alkyl, C₃₋₆ cycloalkyl or C₁₋₃ haloalkyl;
Z is selected from N and CR₆;
or, Rₐ cyclizes with R₁ to form a substituted or unsubstituted 5-8 membered heterocyclyl; ring A is selected from the substituted or unsubstituted 5-8 membered heterocyclyl or 5-8 membered carbocyclyl;
ring B is aryl or 5-6 membered heteroaryl, optionally substituted with one or more R₂; the aryl and the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl;
R₂ is each independently selected from H, halogen, -CN, -C(=O)R_{b}, -S(=O)₂R_{b}, -S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₅₋₆ aryl, C₅₋₆ arylalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, and C₂₋₆ alkenylamino;
R₃ and R₄ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl, 3-8 membered heterocycloalkyl, C₁₋₆ alkylamino, C₁₋₆ haloalkylamino, C₃₋₆ cycloalkylamino, 3-6 membered heterocycloalkylamino, and C₂₋₆ alkenylamino;
or, R₃ cyclizes with R₄ to form aryl, C₄₋₇ cycloalkyl, 5-7 membered heterocycloalkyl, or 5-6 membered heteroaryl;
R₅ is selected from substituted or unsubstituted -NH₂, -C(=O)NR_{b}R_{c}, -S(=O)₂R_{b}, -P(=O)R_{b}R_{c}, - P(=O)R_{b}NR_{c}R_{d}, -P(=O)R_{b}OR_{c}, -P(=O)OR_{b}OR_{c}, -P(=S)R_{b}R_{c}, -P(=S)R_{b}NR_{c}R_{d}, -P(=S)R_{b}OR_{c}, - P(=S)OR_{b}OR_{c}, -S(=O)₂NR_{b}R_{c}, R_{b}S(=O)₂NR_{c}-, -N=S(=O)R_{b}R_{c} and R_{b}N=S(=O)(R_{c})-;
R_{b}, R_{c} and R_{d} are each independently selected from H, -CN, C₁₋₃ alkyl, C₁₋₃ haloalkyl, C₃₋₆ cycloalkyl, C₄₋₆ heterocycloalkyl, C₅₋₁₀ aryl, and 5-10 membered heteroaryl;
R₆, R₇ and R₈ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ alkylamino, C₁₋₆ haloalkylamino, C₃₋₆ cycloalkylamino, 3-6 membered heterocycloalkylamino, and C₂₋₆ alkenylamino;
or, R₅ cyclizes with R₆ to form a 4-7 membered ring containing -P(=O)R_{b}-;
or, R₆ cyclizes with R₇ to form C₄₋₆ cycloalkyl, 4-6 membered heterocycloalkyl, aryl, and 5-6 membered heteroaryl;
or, R₇ cyclizes with R₈ to form C₄₋₆ cycloalkyl, 4-6 membered heterocycloalkyl, aryl, and 5-6 membered heteroaryl.

The present disclosure provides a compound represented by formula (I), or a stereoisomer, a tautomer or a pharmaceutically acceptable salt, a prodrug, a hydrate, a solvate and an isotopically labeled derivative thereof, 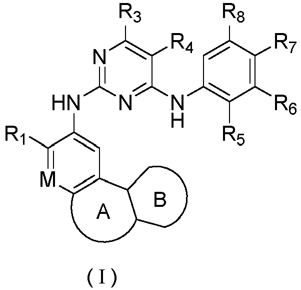 wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, and C₂₋₆ alkenylamino;
M is selected from N and CRₐ;
Rₐ is H, halogen, C₁₋₃ alkyl, C₃₋₆ cycloalkyl or C₁₋₃ haloalkyl;
or, Rₐ cyclizes with R₁ to form a substituted or unsubstituted 5-8 membered heterocyclyl;
ring A is selected from substituted or unsubstituted 4-8 membered heterocyclyl and 5-8 membered carbocyclyl;
ring B is aryl or 5-6 membered heteroaryl, optionally substituted with one or more R₂; the aryl and the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl;
R₂ is each independently selected from H, halogen, -CN, -C(=O)R_{b}, -S(=O)₂R_{b}, -S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy and C₂₋₆ alkenyloxy, C₁₋₆ alkylamino, C₁₋₆ haloalkylamino, C₃₋₆ cycloalkylamino, 3-6 membered heterocycloalkylamino, and C₂₋₆ alkenylamino;
R₃ and R₄ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl, 3-8 membered heterocycloalkyl, C₁₋₆ alkylamino, C₁₋₆ haloalkylamino, C₃₋₆ cycloalkylamino, 3-6 membered heterocycloalkylamino, and C₂₋₆ alkenylamino;
or, R₃ cyclizes with R₄ to form aryl, C₄₋₇ cycloalkyl, 5-7 membered heterocycloalkyl, or 5-6 membered heteroaryl;
R₅ is selected from substituted or unsubstituted -NH₂, -C(=O)NR_{b}R_{c}, -S(=O)₂R_{b}, -P(=O)R_{b}R_{c}, - P(=O)R_{b}NR_{c}R_{d}, -S(=O)₂NR_{b}R_{c}, R_{b}S(=O)₂NR_{c}-, -N=S(=O)R_{b}R_{c}, and R_{b}N=S(=O)(R_{c})-;
R_{b}, R_{c} and R_{d} are each independently selected from H, -CN, C₁₋₃ alkyl, C₁₋₃ haloalkyl, C₃₋₆ cycloalkyl, C₄₋₆ heterocycloalkyl, C₅₋₁₀ aryl, and 5-10 membered heteroaryl;
R₆, R₇ and R₈ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ alkylamino, C₁₋₆ haloalkylamino, C₃₋₆ cycloalkylamino, 3-6 membered heterocycloalkylamino, and C₂₋₆ alkenylamino;
or, R₅ cyclizes with R₆ to form a 4-7 membered ring containing -P(=O)R_{b}-;
or, R₆ cyclizes with R₇ to form C₄₋₆ cycloalkyl, 4-6 membered heterocycloalkyl, aryl, and 5-6 membered heteroaryl;
or, R₇ cyclizes with R₈ to form C₄₋₆ cycloalkyl, 4-6 membered heterocycloalkyl, aryl, and 5-6 membered heteroaryl.

In the compounds of formula (I‴), formula (I"), formula (I') or formula (I) of the present disclosure, R₁ is selected from H, halogen, -CN, C₁₋₃ alkyl, C₁₋₃ alkoxy, C₁₋₃ haloalkyl, and C₁₋₃ haloalkoxy; M is selected from N and CH;
ring A is selected from the substituted or unsubstituted 5-8 membered heterocyclyl or 5-8 membered carbocyclyl;
ring B is 5-6 membered heteroaryl, optionally substituted with one or more R₂; the 5-6 membered heteroaryl is pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl;
R₂ is each independently selected from H, C₁₋₄ alkyl, C₁₋₄ haloalkyl, -(CH₂)ᵣNR_{c}R_{d}, 3-6 membered heterocycloalkyl, and C₅₋₆ arylalkyl, wherein r is optionally selected from 0, 1, 2, and 3, and the 3-6 membered heterocycloalkyl and the C₅₋₆ arylalkyl are optionally substituted with one or more C₁₋₃ alkyl or C₁₋₃ alkoxy;
R₃ and R₄ are each independently selected from H, halogen, C₁₋₄ alkyl, C₁₋₄ halogenated alkyl, and C₃₋₆ cycloalkyl;
or, R₃ cyclizes with R₄ to form 5-6 membered heteroaryl; the 5-6 membered heteroaryl is pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl;
R₅ is selected from -C(=O)NR_{b}R_{c}, -P(=O)R_{b}R_{c}, -P(=S)R_{b}R_{c}, -S(=O)₂NR_{b}R_{c}, R_{b}S(=O)₂NR_{c}-, and - NR_{b}C(O)R_{c};
R_{b}, R_{c} and R_{d} are each independently selected from H, C₁₋₃ alkyl, C₁₋₃ alkoxy, and C₃₋₆ cycloalkyl; or, R_{b} and R_{c} cyclizes with atoms to which they are both attached to form 5-6 membered heterocycloalkyl unsubstituted or optionally substituted with one or more C₁₋₃ alkyl or C₁₋₃ alkoxy; or, R₅ cyclizes with R₆ to form a 4-7 membered ring containing -P(=O)(R_{b})-, -P(=S)(R_{b})-, - N(R_{b})S(=O)₂-, -S(=O)₂N(R_{b})-, or -S(=O)₂;
R₆, R₇ and R₈ are each independently selected from H, halogen, and C₁₋₃ alkyl;
or, R₈ is selected from H, R₆ cyclizes with R₇ to form 5-6 membered heteroaryl; the 5-6 membered heteroaryl is pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl.

In the compounds of formula (I‴), formula (I"), formula (I') or formula (I) of the present disclosure, R₁ is selected from H, halogen, -CN, C₁₋₃ alkyl, C₁₋₃ alkoxy, C₁₋₃ haloalkyl, and C₁₋₃ haloalkoxy; preferably, the R₁ is selected from H, methyl, ethyl, n-propyl, isopropyl, n-butyl, methoxy, ethoxy, n-propoxy, isopropoxy, n-butoxy, trifluoromethyl, trifluoromethoxy, trichloromethyl, trichloromethoxy, and 2,2,2-trifluoroethoxy; and more preferably, the R₁ is selected from methoxy. In the compounds of formula (I‴), formula (I"), formula (I') or formula (I) of the present disclosure, M is selected from N and CH, preferably M is selected from CH.

In the compounds of formula (I‴), formula (I"), formula (I') or formula (I) of the present disclosure, R₂ is selected from H, methyl, ethyl, isopropyl, difluoromethyl, trifluoromethyl, - CH₂CH₂N(CH₃)CH₃,

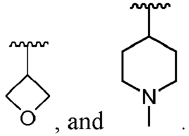

In the compounds of formula (I‴), formula (I"), formula (I') or formula (I) of the present disclosure, R₂ is selected from H, methyl, ethyl, isopropyl, difluoromethyl, and trifluoromethyl; preferably, the R₂ is selected from methyl.

In the compounds of formula (I‴), formula (I"), formula (I') or formula (I) of the present disclosure, R₃ and R₄ are each independently selected from H, F, Cl, Br, CN, methyl, ethyl, isopropyl, methoxy, ethoxy, isopropoxy, difluoromethyl, trifluoromethyl, 2,2,2-trifluoroethyl, and cyclopropyl; preferably, R₃ and R₄ are each independently selected from H, F, Cl, Br, methyl, difluoromethyl, trifluoromethyl, and cyclopropyl; preferably, R₃ is selected from H, and R₄ is selected from Cl, Br, methyl, and trifluoromethyl.

In the compounds of formula (I‴), formula (I"), formula (I') or formula (I) of the present disclosure, R₃ is selected from H, and R₄ is selected from H, F, Cl, Br, methyl, ethyl, difluoromethyl, trifluoromethyl, and cyclopropyl.

In the compounds of formula (I‴), formula (I"), formula (I') or formula (I) of the present disclosure, R₃ is selected from H, and R₄ is selected from Cl, Br, and methyl.

In the compounds of formula (I‴), formula (I"), formula (I') or formula (I) of the present disclosure, R₃ is selected from H, and R₄ is selected from Br.

In the compounds of formula (I‴), formula (I"), formula (I') or formula (I) of the present disclosure, R₃ cyclizes with R₄ to form a thiophene ring and a pyrrole ring, wherein the thiophene ring and the pyrrole ring may be optionally substituted with C₁₋₄ alkyl.

In the compounds of formula (I‴), formula (I"), formula (I') or formula (I) of the present disclosure, R₅ is selected from

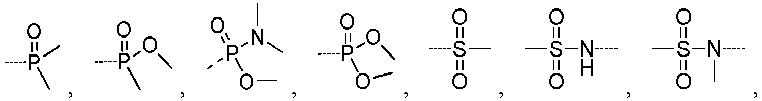

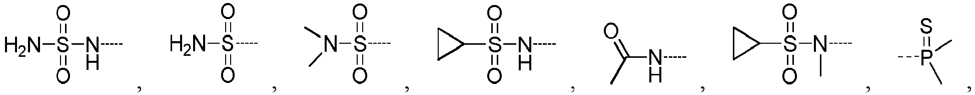

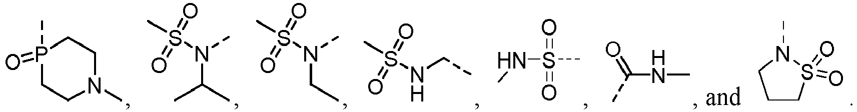

In the compounds of formula (I‴), formula (I"), formula (I') or formula (I) of the present disclosure, R₅ is selected from 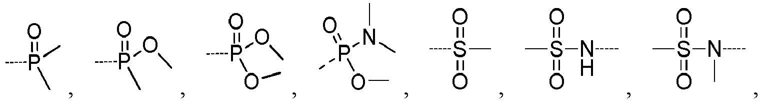 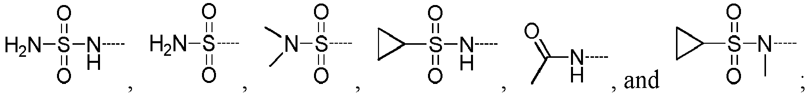 in the compounds of formula (I‴), formula (I"), formula (I') or formula (I) of the present disclosure, R₅ is selected from 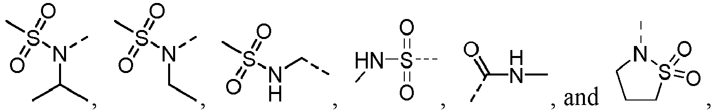 preferably, R₅ is selected from 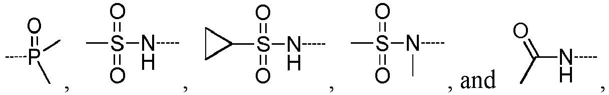 preferably, the R₅ is selected from

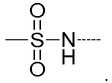

In the compounds of formula (I‴), formula (I"), formula (I') or formula (I) of the present disclosure, R₅ cyclizes with R₆ to form

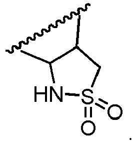

In the compounds of formula (I‴), formula (I"), formula (I') or formula (I) of the present disclosure, R₆, R₇ and R₈ are each independently selected from H, methyl, and halogen; preferably, each independently selected from H and methyl.

In the compounds of formula (I‴), formula (I"), formula (I') or formula (I) of the present disclosure, R₆, R₇ and R₈ are selected from H.

In the compounds of formula (I‴), formula (I"), formula (I') or formula (I) of the present disclosure, R₆ and R₈ are selected from H, and R₇ is selected from F.

In the compounds of formula (I‴), formula (I"), formula (I') or formula (I) of the present disclosure, R₆ cyclizes with R₇ independently or R₇ cyclizes with R₈ independently to form cyclobutane, cyclopentane, a tetrahydropyrrole ring, a tetrahydrofuran ring, a tetrahydropyrane ring, a thiophene ring, an imidazole ring, a pyrazole ring, a pyrrole ring, an oxazole ring, a thiazole ring, an isoxazole ring, a piperazine ring, an isothiazole ring, a benzene ring, a pyridine ring, a piperidine ring, a pyrimidine ring, a pyridazine ring, and a pyrazine ring; preferably, R₆ cyclizes with R₇ independently or R₇ cyclizes with R₈ independently to form a cyclobutane, a pyridine ring, and a pyrazine ring; and more preferably, R₆ cyclizes with R₇ to form a pyrazine ring.

In the compounds of formula (I‴), formula (I"), formula (I') or formula (I) of the present disclosure, structural unit 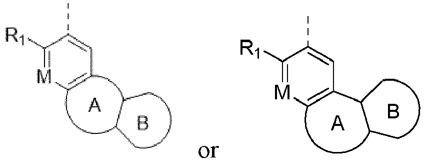 is selected from 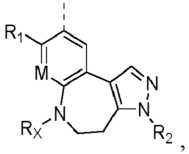 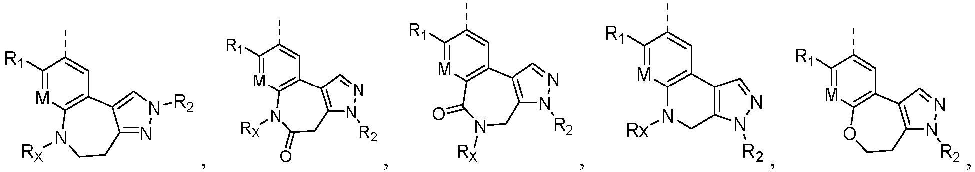 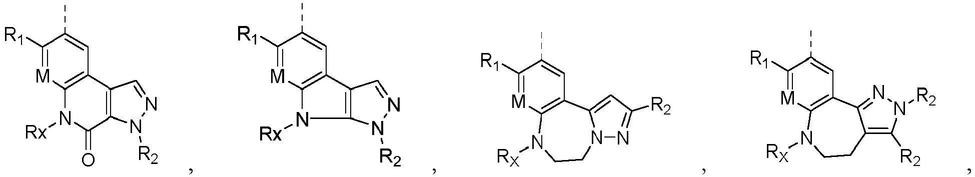 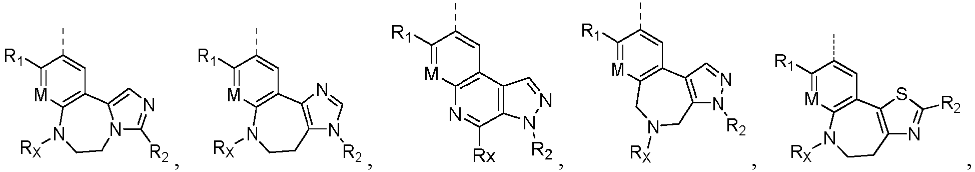 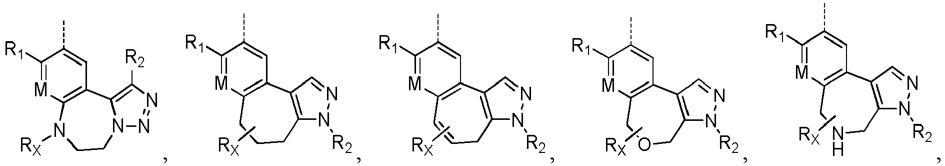 and 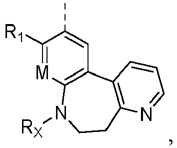 and R₁, M and R₂ are as defined above; Rₓ is selected from H, -OH, -CN, -NH₂, halogen, C₁₋₆ alkylcarbonyl, C₁₋₆ alkyl, C₁₋₆ haloalkyl, C₃₋₈ cycloalkyl, 3-8 membered heterocycloalkyl, C₃₋₈ cycloalkyl-C₁₋₆ alkyl-, 3-8 membered heterocycloalkyl-C₁₋₆ alkyl-, aryl-C₁₋₆ alkyl-, C₅₋₁₃ spirocyclyl, and 5-13 membered spiroheterocyclyl; wherein the C₃₋₈ cycloalkyl, 3-8 membered heterocycloalkyl, C₃₋₈ cycloalkyl-C₁₋₆ alkyl-, 3-8 membered heterocycloalkyl-C₁₋₆ alkyl-, aryl-C₁₋₆ alkyl-, C₅₋₁₃ spirocyclyl, and 5-13 membered spirocyclyl membered spiroheterocyclyl are optionally substituted with one or more R_{y}; the R_{y} is selected from H, halogen, C₁₋₆ alkyl, C₁₋₆ haloalkyl, C₁₋₆ alkoxy, C₃₋₈ cycloalkyl, 4-8 membered heterocycloalkyl, C₃₋₈ cycloalkyl-C₁₋₆ alkyl-, 4-8 membered heterocycloalkyl-C₁₋₆ alkyl-, 5-10 membered aryl, and 5-10 membered heteroaryl.

When Rₓ is directly attached to N atom, Rₓ is not -OH, -NH₂, and halogen.

In the compounds of formula (I‴), formula (I"), formula (I') or formula (I) of the present disclosure, Rₓ is selected from H, -OH, -CN, -NH₂, halogen, C₁₋₆ alkylcarbonyl, C₁₋₆ alkyl, C₁₋₆ haloalkyl, C₃₋₈ cycloalkyl, 3-8 membered heterocycloalkyl, C₃₋₈ cycloalkyl-C₁₋₆ alkyl-, 3-8 membered heterocycloalkyl-C₁₋₆ alkyl-; wherein the C₃₋₈ cycloalkyl, 3-8 membered heterocycloalkyl, C₃₋₈ cycloalkyl-C₁₋₆ alkyl-, 3-8 membered heterocycloalkyl-C₁₋₆ alkyl-, and aryl-C₁₋₆ alkyl- are optionally substituted with one or more R_{y}; the R_{y} is selected from H, halogen, C₁₋₆ alkyl, C₁₋₆ haloalkyl, C₁₋₆ alkoxy, C₃₋₈ cycloalkyl, 4-8 membered heterocycloalkyl, C₃₋₈ cycloalkyl-C₁₋₆ alkyl-, 4-8 membered heterocycloalkyl-C₁₋₆ alkyl-, 5-10 membered aryl, and 5-10 membered heteroaryl.

In the compounds of formula (I‴), formula (I"), formula (I') or formula (I) of the present disclosure, Rₓ is selected from H, -OH, -CN, -NH₂, halogen, C₁₋₆ alkylcarbonyl, C₁₋₆ alkyl, C₁₋₆ haloalkyl, C₃₋₆ cycloalkyl, 3-6 membered heterocycloalkyl, C₃₋₆ cycloalkyl-C₁₋₄ alkyl-, 3-6 membered heterocycloalkyl-C₁₋₄ alkyl-, aryl-C₁₋₆ alkyl-, and 7-11 membered spiroheterocyclyl, wherein the C₃₋₆ cycloalkyl, 3-6 membered heterocycloalkyl, C₃₋₆ cycloalkyl-C₁₋₄ alkyl-, 3-6 membered heterocycloalkyl-C₁₋₄ alkyl-, aryl-C₁₋₆ alkyl-, and spiroheterocyclyl are optionally substituted with one or more R_{y}; R_{y} is selected from H, halogen, C₁₋₄ alkyl, C₁₋₄ haloalkyl, C₁₋₄ alkoxy, and C₃₋₆ cycloalkyl-C₁₋₆ alkyl-; preferably, R_{y} is selected from H, F, methyl, ethyl, isopropyl, methoxy, 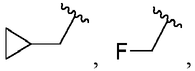 and FCH₂CH₂-.

In the compounds of formula (I‴), formula (I"), formula (I') or formula (I) of the present disclosure, Rₓ is selected from H, -OH, -CN, -NH₂, F, methyl, ethyl, isopropyl, trifluoroethyl, methylcarbonyl, 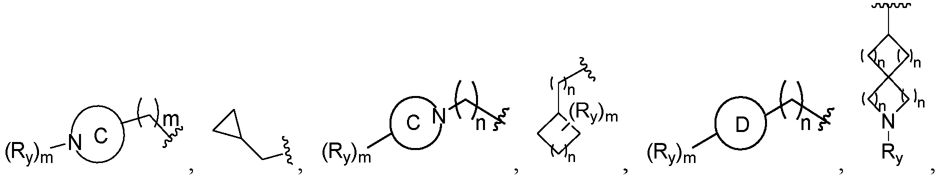 and 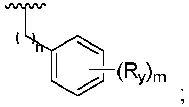 ring C is 4-8 membered heterocycloalkyl, ring D is 4-8 membered heterocycloalkyl containing oxygen; m and n are independently 0, 1, 2, or 3; R_{y} is as defined above. In the compounds of formula (I‴), formula (I"), formula (I') or formula (I) of the present disclosure, Rₓ is selected from H, -OH, -CN, -NH₂, methyl, ethyl, isopropyl, methylcarbonyl, 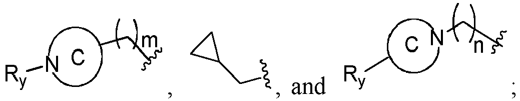 ring C is 4-8 membered heterocycloalkyl; m and n are independently 0, 1, 2, or 3.

In the compounds of formula (I‴), formula (I"), formula (I') or formula (I) of the present disclosure, Rₓ is selected from H, -OH, -CN, -NH₂, F, methyl, ethyl, isopropyl, trifluoroethyl, methylcarbonyl, 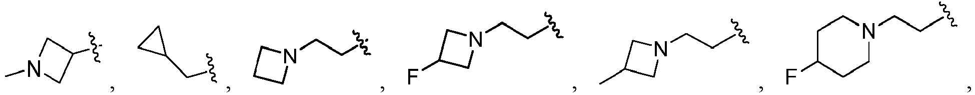 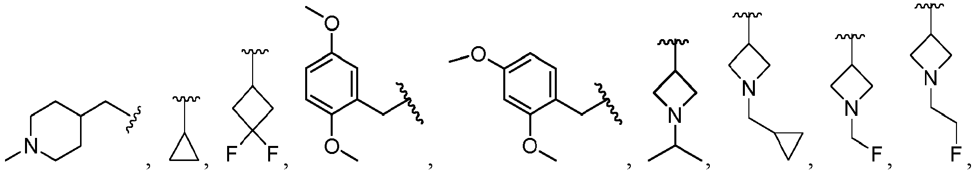 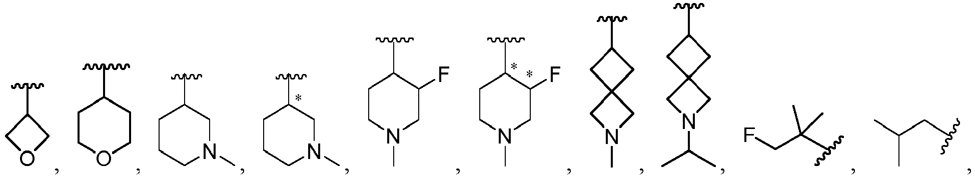 and

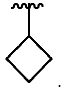

In the compounds of formula (I‴), formula (I"), formula (I') or formula (I) of the present disclosure, Rₓ is selected from H, methyl, ethyl, isopropyl,

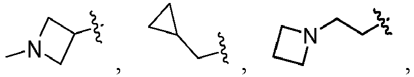

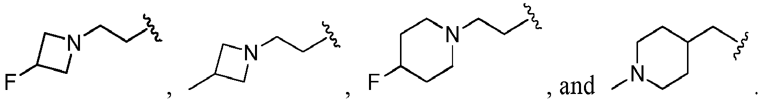

In the compounds of formula (I‴), formula (I"), formula (I') or formula (I) of the present disclosure, Rₓ is selected from H, methyl, ethyl, isopropyl,

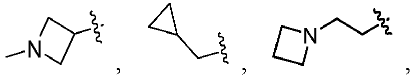

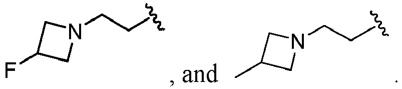

In the compounds of formula (I‴), formula (I"), formula (I') or formula (I) of the present disclosure, Rₓ is selected from H, methyl, ethyl, and isopropyl.

In the compounds of formula (I‴), formula (I"), formula (I') or formula (I) of the present disclosure, Rₓ is selected from methyl and isopropyl.

In the compounds of formula (I‴), formula (I"), formula (I') or formula (I) of the present disclosure, structural unit 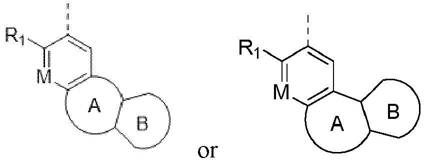 is selected from 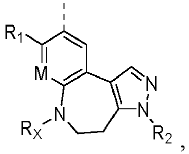 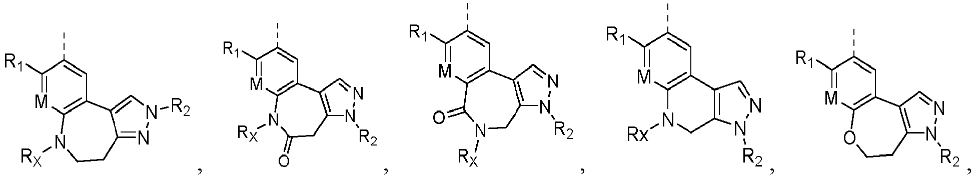 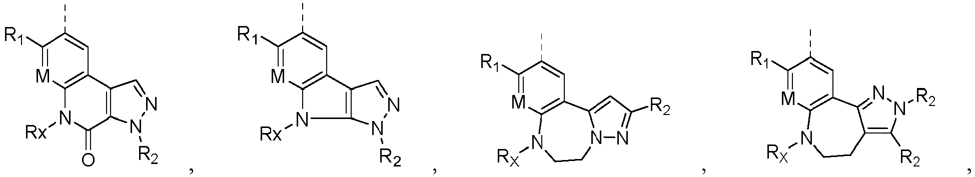 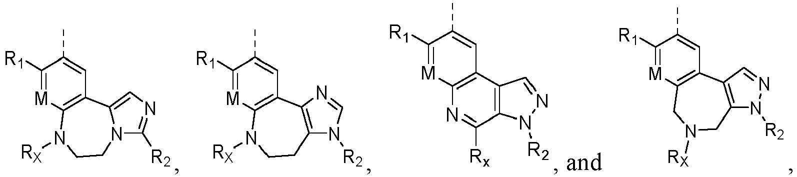 R₁, M, Rₓ, and R₂ are as defined above.

In the compounds of formula (I‴), formula (I"), formula (I') or formula (I) of the present disclosure, structural unit 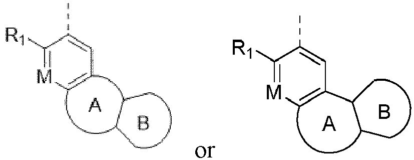 is selected from 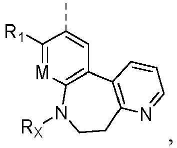 and R₁, M, and Rₓ are as defined above.

In the compounds of formula (I‴), formula (I"), formula (I') or formula (I) of the present disclosure, structural unit 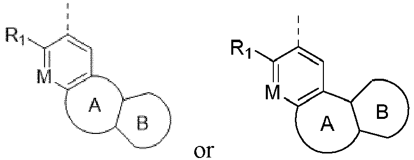 is selected from 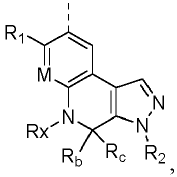 and R₁, M, Rₓ, R_{b}, R_{c}, and R₂ are as defined above, preferably, the structural unit 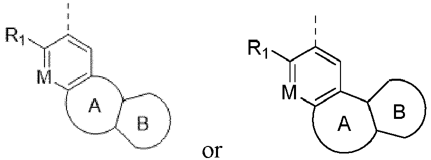 is selected from 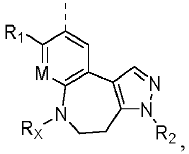 wherein M, R₁, R₂, and Rₓ are as defined above.

Preferably, the compounds or the stereoisomers, the tautomers or a pharmaceutically acceptable salts, the prodrugs, the hydrates, the solvates and the isotopically labeled derivatives thereof described above are selected from 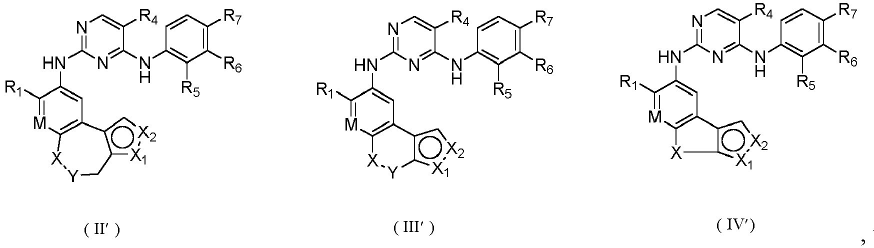 wherein,
X and Y are each independently selected from -C(=O)-, -C=C-, -NRₓ-, -O-, -CR₉R₁₀-, -S(=O)-, and -S(=O)₂-;
X₁ and X₂ are each independently selected from N and NR₂;
R₉ and R₁₀ are each independently selected from H, halogen, C₁₋₆ alkyl, C₁₋₆ alkoxy, C₁₋₆ haloalkyl, C₁₋₆ haloalkoxy, C₃₋₈ cycloalkyl, and 3-8 membered heterocycloalkyl;
R₁, R₂, R₄, R₅, R₆, R₇, Rₓ, and M are as defined above.

Preferably, the compounds or the stereoisomers, the tautomers or a pharmaceutically acceptable salts, the prodrugs, the hydrates, the solvates and the isotopically labeled derivatives thereof described above are selected from 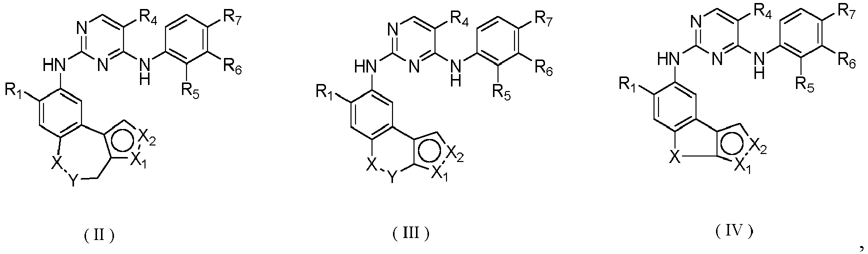 wherein,
X and Y are each independently selected from -C(=O)-, -NRₓ-, -O-, -CR₉R₁₀-, -S(=O)-, and - S(=O)₂-;
X₁ and X₂ are each independently selected from N and NR₂;
R₉ and R₁₀ are each independently selected from H, halogen, C₁₋₆ alkyl, C₁₋₆ alkoxy, C₁₋₆ haloalkyl, C₁₋₆ haloalkoxy, C₃₋₈ cycloalkyl, and 3-8 membered heterocycloalkyl;
R₁, R₂, R₄, R₅, R₆, R₇, and Rₓ are as defined above.

Preferably, the compounds or the stereoisomers, the tautomers or a pharmaceutically acceptable salts, the prodrugs, the hydrates, the solvates and the isotopically labeled derivatives thereof described above are selected from 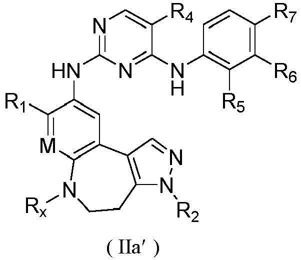 wherein, R₁, R₂, R₄, R₅, R₆, R₇, Rₓ, and M are as defined above.

Preferably, the compounds or the stereoisomers, the tautomers or a pharmaceutically acceptable salts, the prodrugs, the hydrates, the solvates and the isotopically labeled derivatives thereof described above are selected from 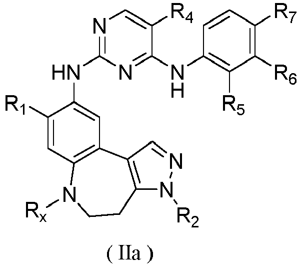 wherein, R₁, R₂, R₄, R₅, R₆, R₇, and Rₓ are as defined above.

Further preferably, the compounds or the stereoisomers, the tautomers or a pharmaceutically acceptable salts, the prodrugs, the hydrates, the solvates and the isotopically labeled derivatives thereof described above are selected from, 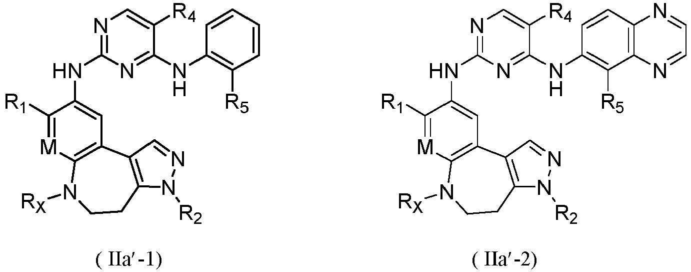 wherein, R₁, R₂, R₄, R₅, Rₓ and M are as defined above.

Further preferably, the compounds or the stereoisomers, the tautomers or a pharmaceutically acceptable salts, the prodrugs, the hydrates, the solvates and the isotopically labeled derivatives thereof described above are selected from, 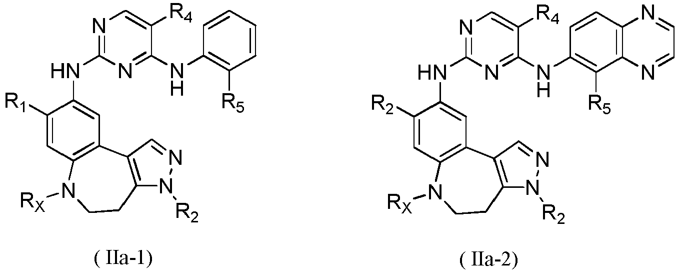 wherein, R₁, R₂, R₄, R₅, and Rₓ are as defined above.

Further preferably, the compounds or the stereoisomers, the tautomers or a pharmaceutically acceptable salts, the prodrugs, the hydrates, the solvates and the isotopically labeled derivatives thereof described above are selected from 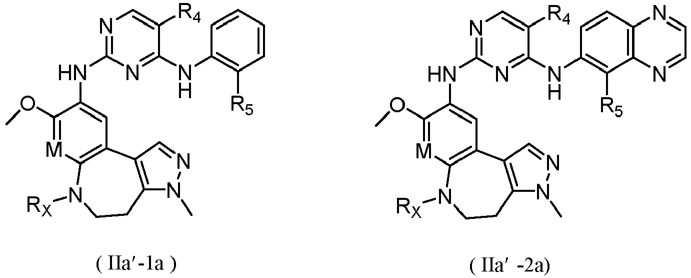 wherein, R₄, R₅, Rₓ, and M are as defined above.

Further preferably, the compounds or the stereoisomers, the tautomers or a pharmaceutically acceptable salts, the prodrugs, the hydrates, the solvates and the isotopically labeled derivatives thereof described above are selected from 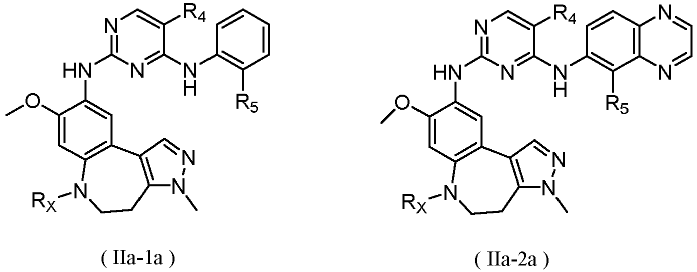 wherein, R₄, R₅, and Rₓ are as defined above.

Further preferably, the compounds or the stereoisomers, the tautomers or a pharmaceutically acceptable salts, the prodrugs, the hydrates, the solvates and the isotopically labeled derivatives thereof described above are selected from 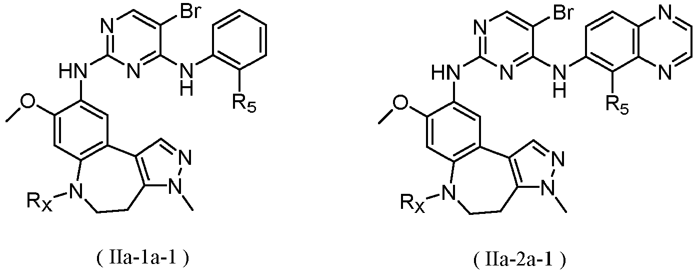 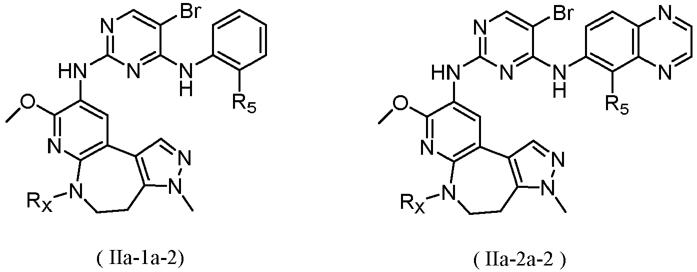 wherein, R₅ and Rₓ are as defined above.

In some embodiments of the present disclosure, the compound of formula (I‴), or the stereoisomer, the tautomer, the pharmaceutically acceptable salt, the prodrug, the hydrate, the solvate, and the isotopically labeled derivative thereof is selected from a compound of formula (I‴-A) 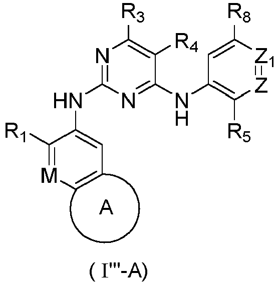 wherein, R₁, M, R₃, R₄, R₅, R₈, ring A, Z, and Z₁ are as defined above.

In some embodiments of the present disclosure, the compound of formula (I‴), or the stereoisomer, the tautomer, the pharmaceutically acceptable salt, the prodrug, the hydrate, the solvate, and the isotopically labeled derivative thereof is selected from a compound of formula (I‴-B), 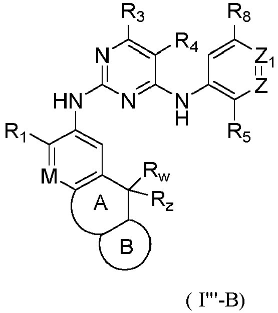 wherein, R₁, M, R₃, R₄, R₅, R₈ ring A, ring B, Z, and Z₁ are as defined above;
R_{w} and R_{z} are each independently selected from H, -OH, -CN, -NH₂, halogen, C₁₋₆ alkyl, C₁₋₆ heteroalkyl, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₂₋₆ alkynyloxy, C₁₋₆ alkylamino, C₁₋₆ haloalkylamino, C₃₋₆ cycloalkylamino, 3-6 membered heterocycloalkylamino, C₂₋₆ alkenylamino, and C₂₋₆ alkynylamino.

Most preferably, the compounds, or the stereoisomers, the tautomers or a pharmaceutically acceptable salts, the prodrugs, hydrates, the solvates, and the isotopically labeled derivatives thereof include, but are not limited to:

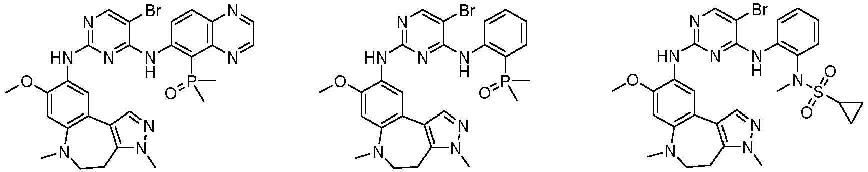

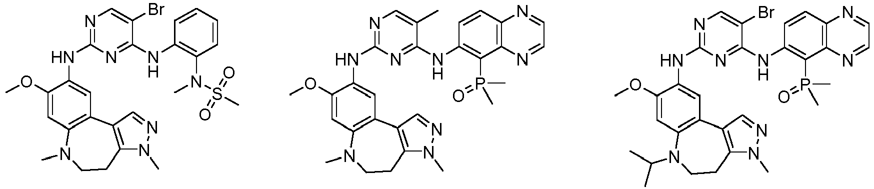

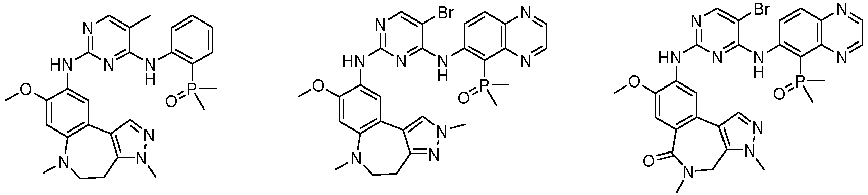

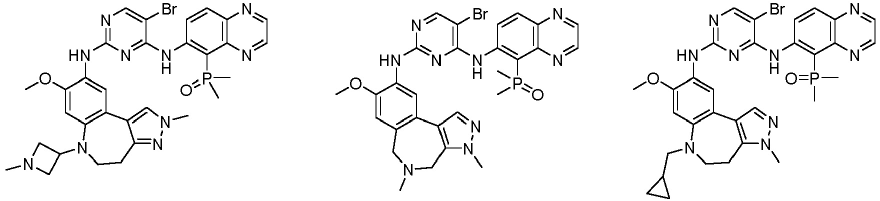

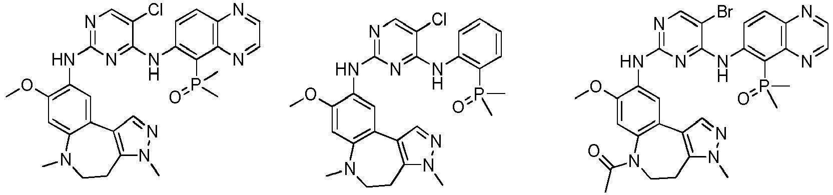

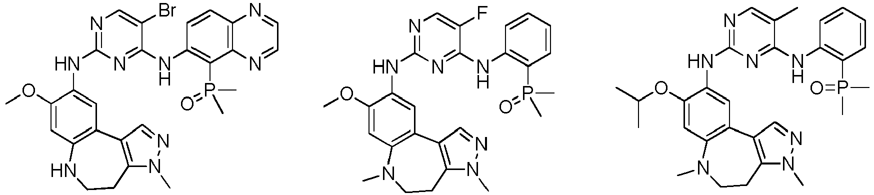

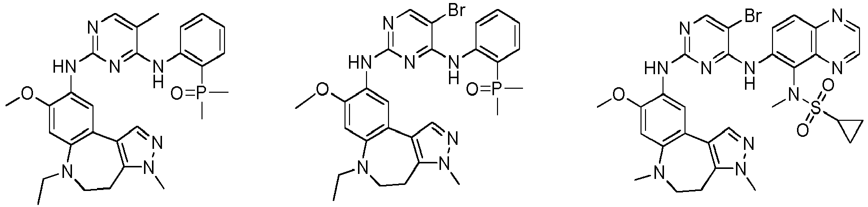

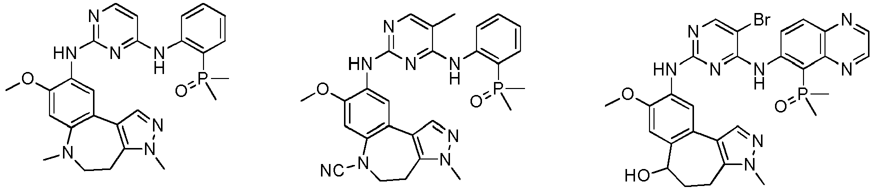

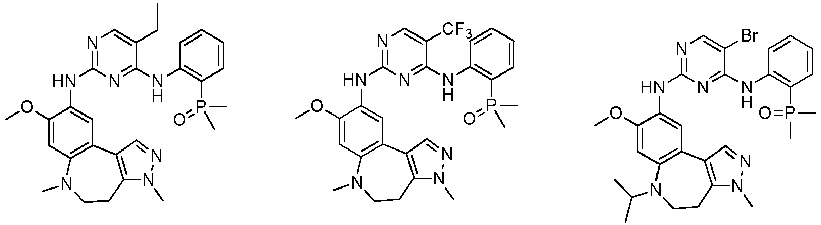

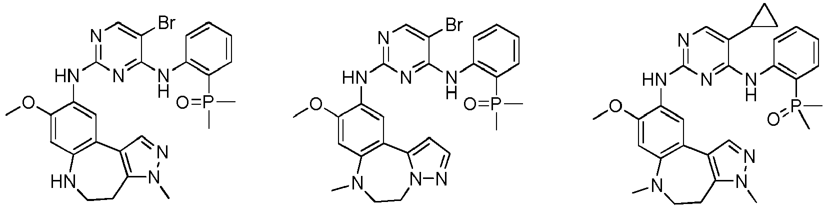

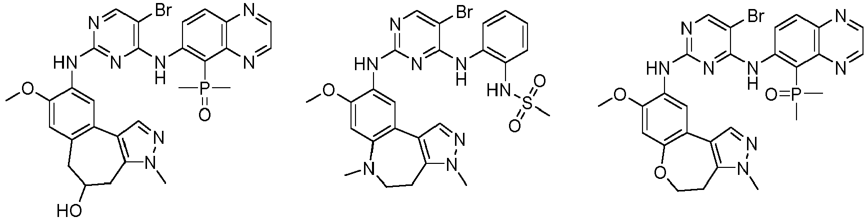

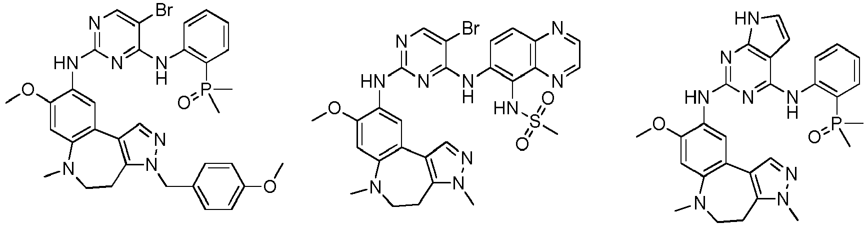

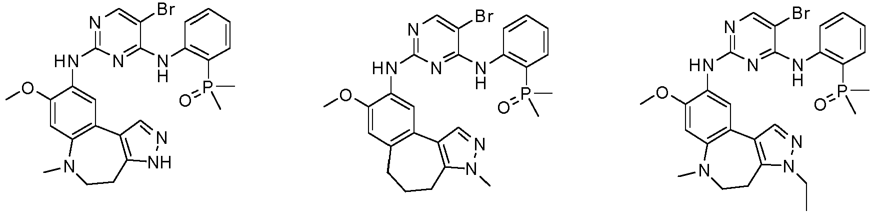

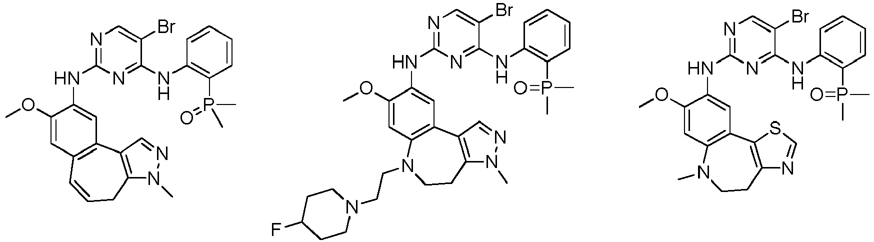

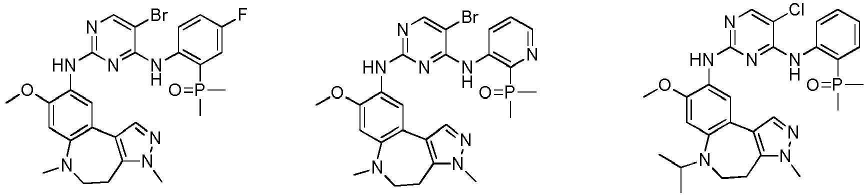

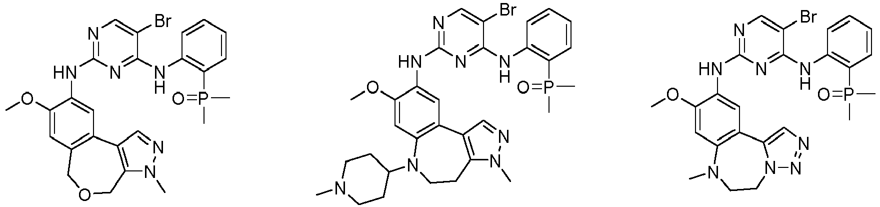

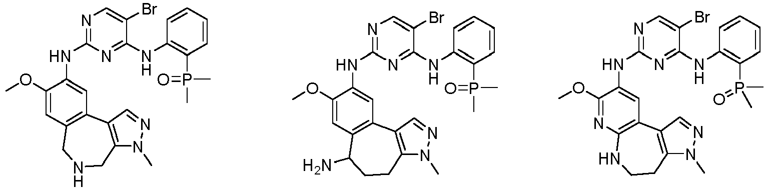

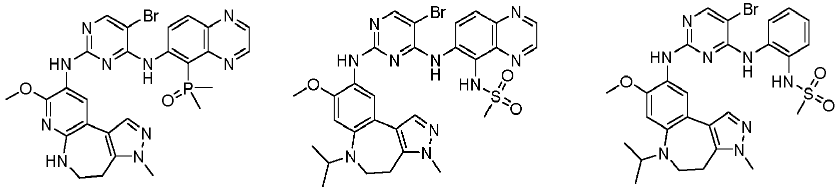

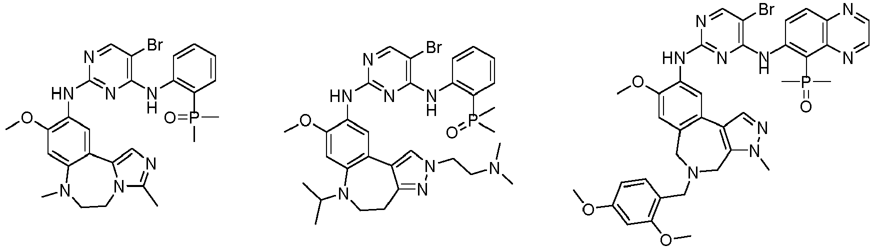

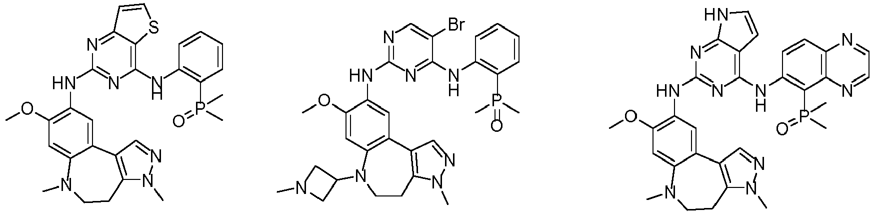

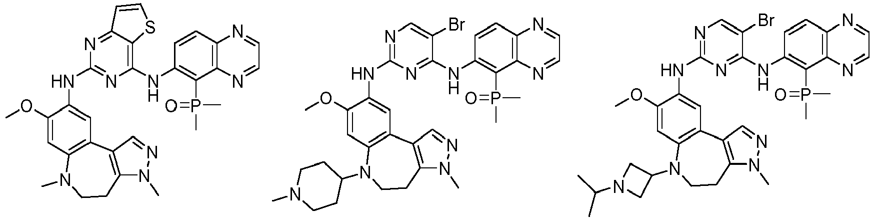

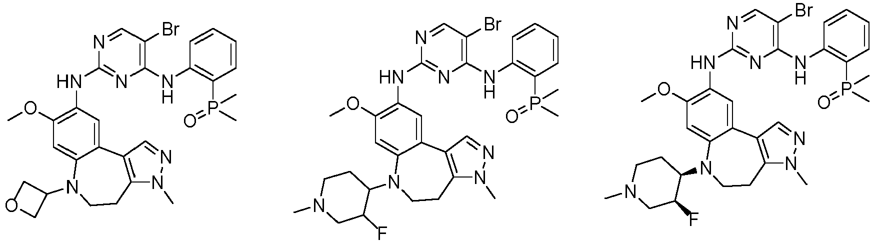

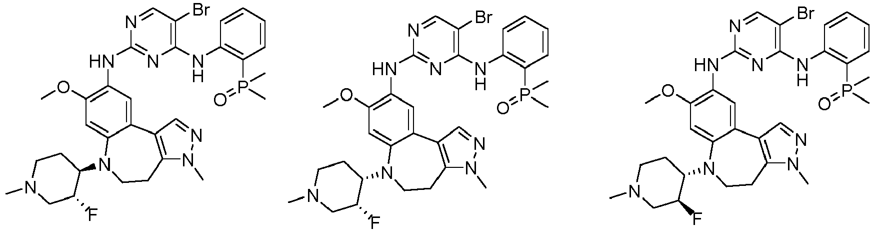

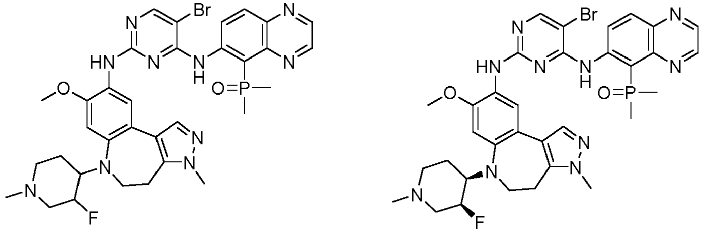

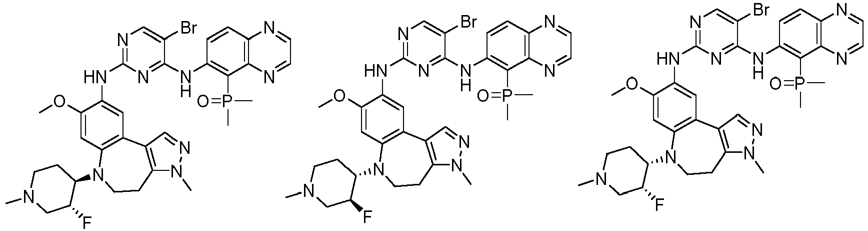

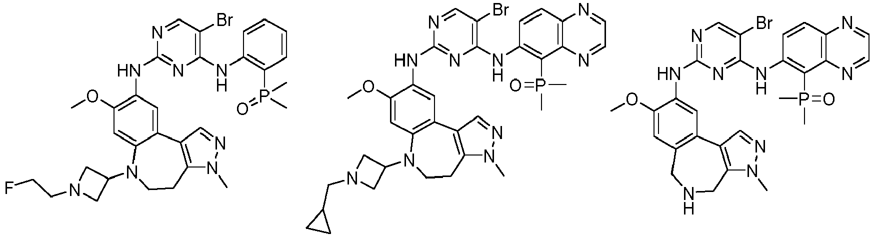

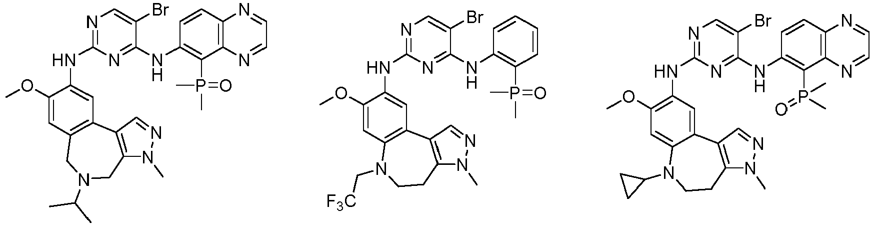

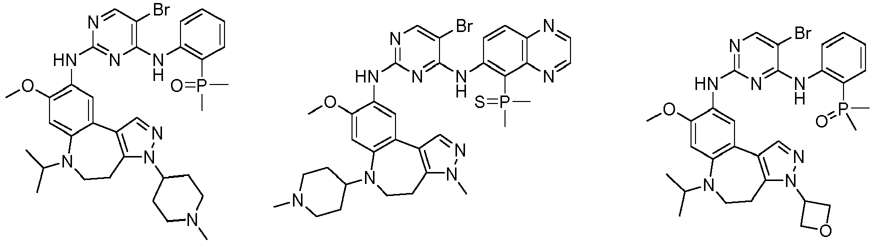

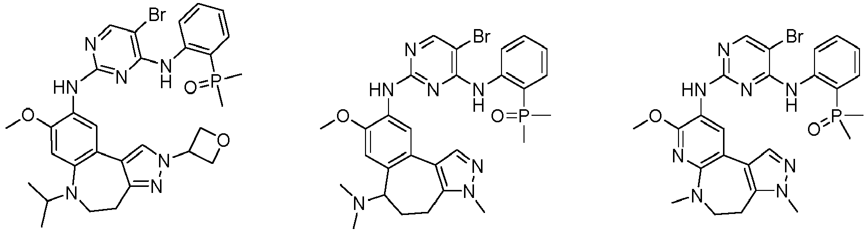

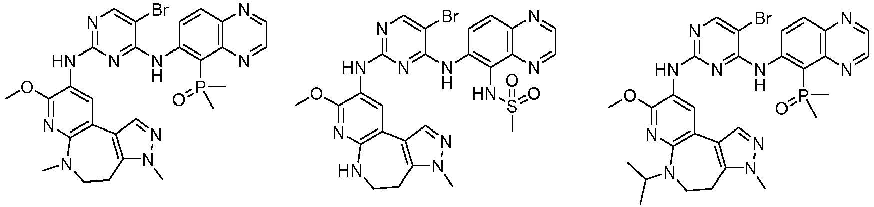

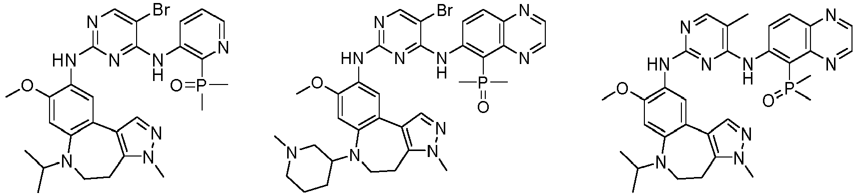

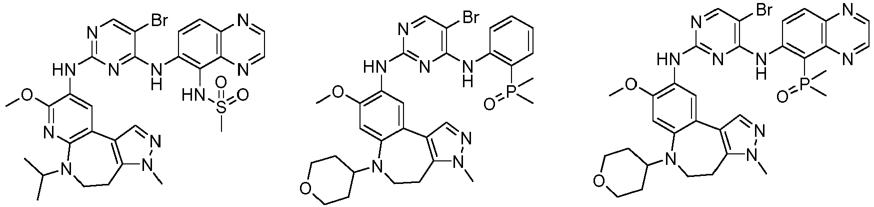

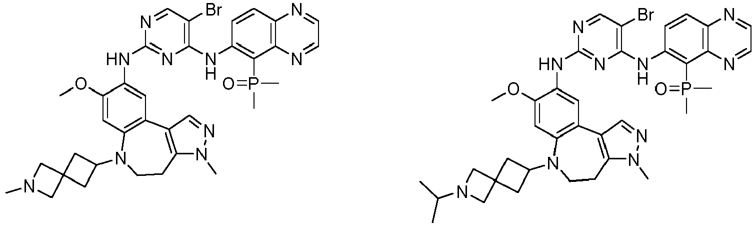

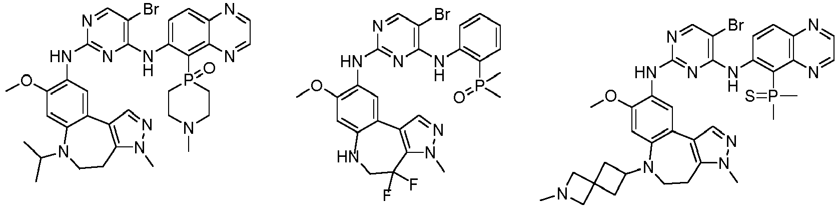

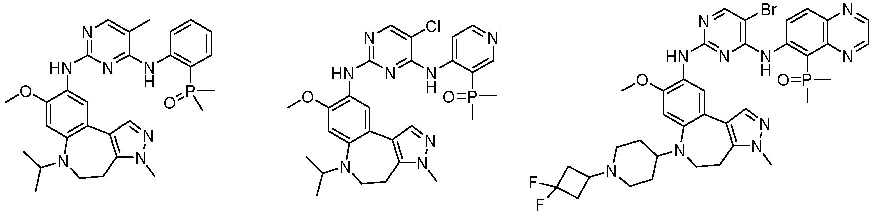

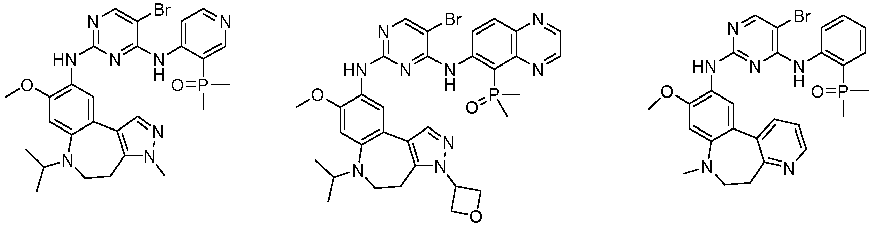

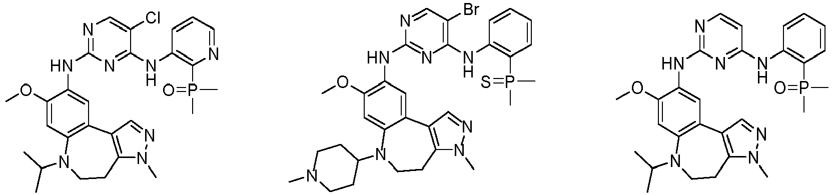

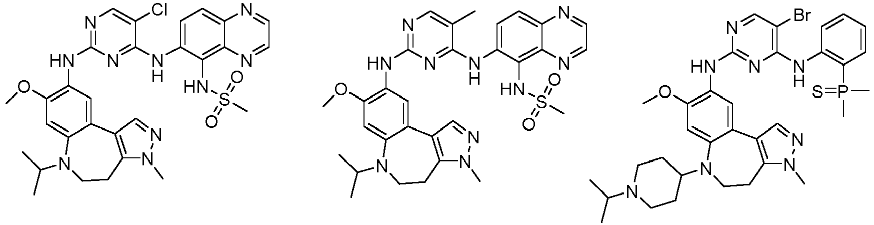

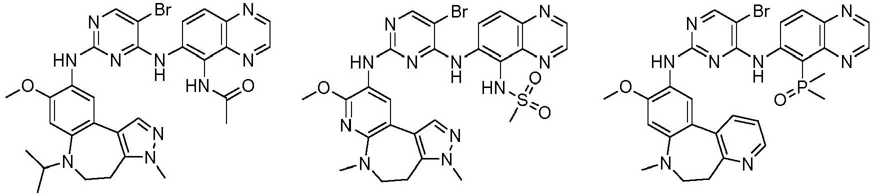

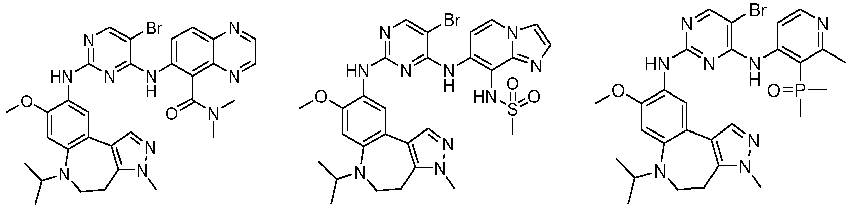

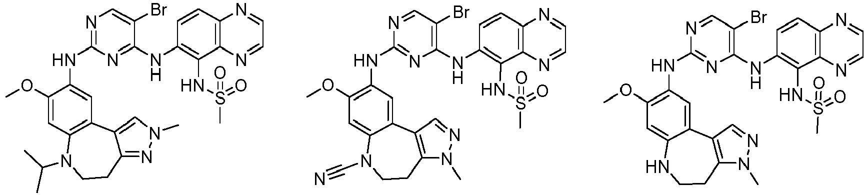

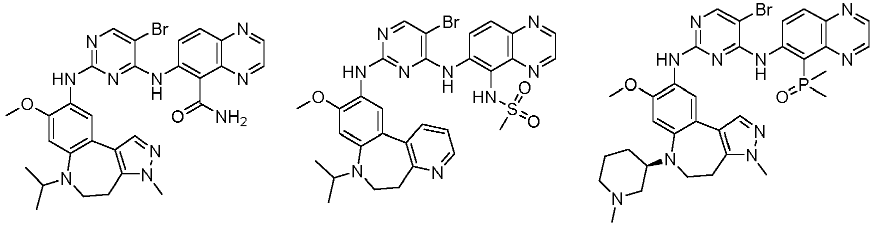

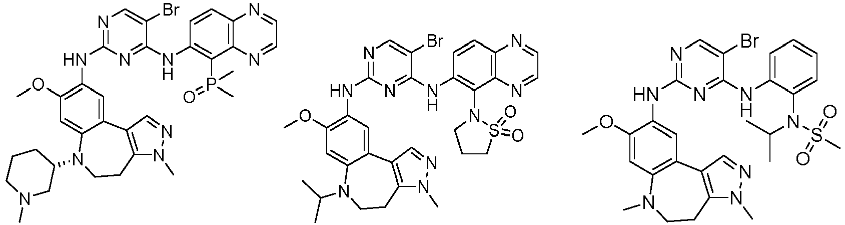

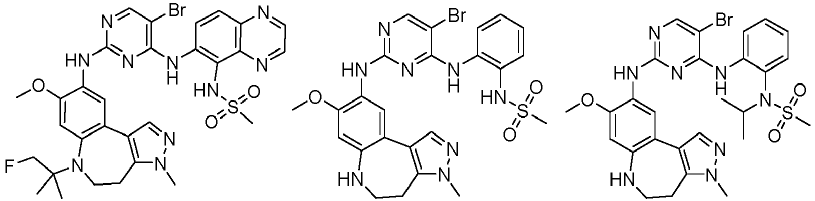

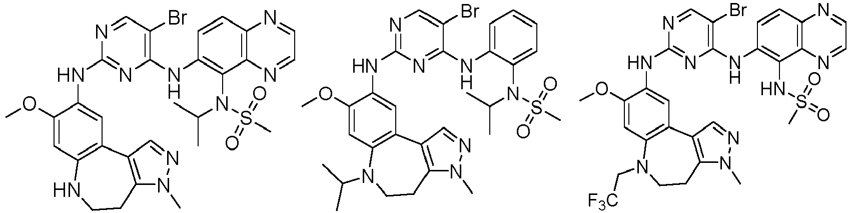

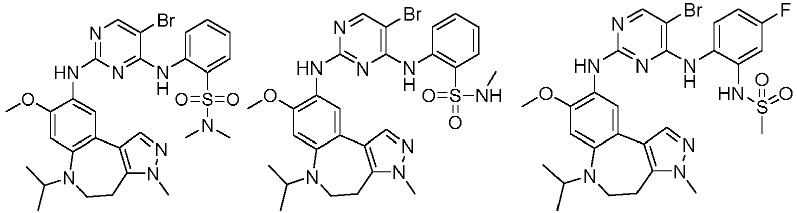

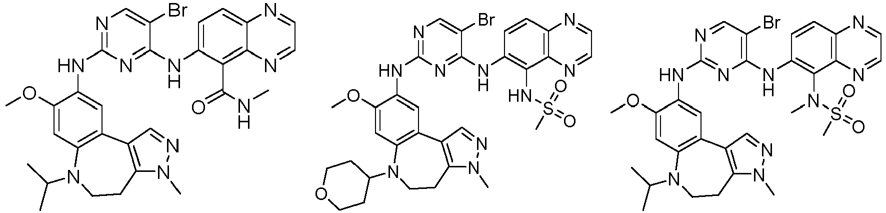

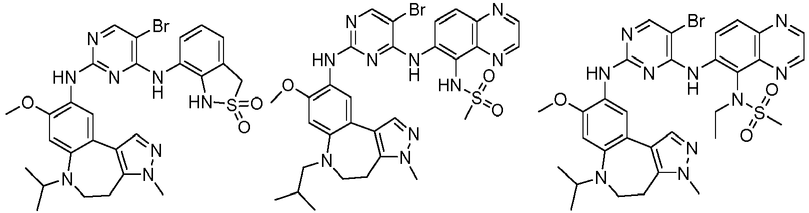

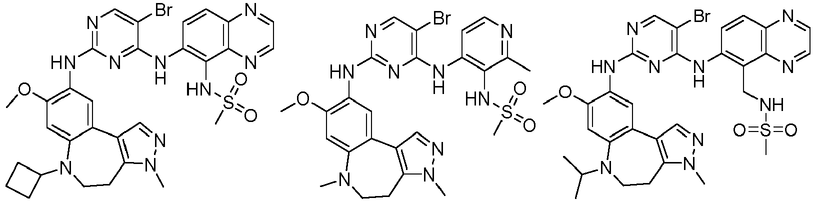

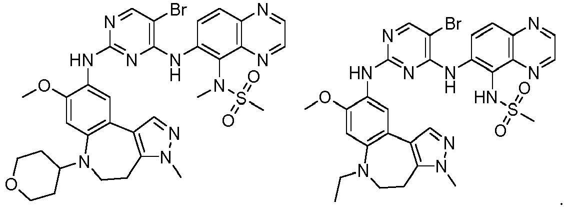

The present disclosure also provides a pharmaceutical composition comprising a therapeutically effective amount of the compound or the pharmaceutically acceptable salt thereof described above and a pharmaceutically acceptable carrier, diluent, and excipient. The pharmaceutical compositions provided by the present disclosure can be formulated for specific routes of administration, such as oral administration, parenteral administration, and rectal administration. Specifically, the routes of administration include oral administration in forms of, e.g., tablets, capsules (including sustained release or timed release formulations), pills, powders, granules, elixirs, tinctures, suspensions (including nanosuspensions, microsuspensions, spray-dried dispersions), syrups, and emulsions; sublingual administration; administration in forms of taking orally; parenteral administration, e.g., by subcutaneous, intravenous, intramuscular, or intrasternal injection, or infusion techniques (e.g., as sterile injectable aqueous solution or nonaqueous solutions or suspensions); nasal administration including administration to the nasal mucosa, e.g., by inhalation spray; topical administration in the form of, e.g., creams or ointments; or rectal administration in the form of, e.g., suppositories. They may be administered alone, but will generally be administered with the pharmaceutical carrier selected according to the chosen route of administration and standard pharmaceutical practice.

"Pharmaceutically acceptable carriers" refer to vehicles generally accepted in the art for delivering biologically active agents to animals, particularly mammals, and includes, for example, adjuvants, excipients such as diluents, preservatives, fillers, flow modifiers, disintegrants, wetting agents, emulsifiers, suspending agents, sweeteners, flavoring agents, fragrances, antibacterial agents, antifungal agents, lubricants and dispersing agents, depending on the mode of administration and the nature of the dosage form. Pharmaceutically acceptable carriers are formulated within the purview of one of ordinary skill in the art based on a number of factors, which include, but are not limited to: the type and nature of the formulated active agents, the subject to which the composition containing the agents is to be administered, the intended route of administration of the composition, and the targeted therapeutic indication. Pharmaceutically acceptable carriers include both aqueous and non-aqueous media and a variety of solid and semi-solid dosage forms. Such carriers include many different ingredients and additives in addition to the active agents, and such additional ingredients included in the formulation for a variety of reasons (e.g., stabilizing the active agents and adhesives) are well known to those of ordinary skill in the art.

As a general guideline, when used for the indicated effect, the daily oral dosage of each active ingredient is in a range of between about 0.001-5000 mg daily, or about 1-500 mg, or about 1-250 mg, or about 1-150 mg, or about 0.5-100 mg, or about 1-50 mg; the most preferred intravenous dosage during a constant rate infusion is in a range of about 0.01-10 mg/kg/minute. The compounds of the present disclosure may be administered in a single daily dose, or the total daily dose may be administered in divided doses of 2, 3 or 4 times daily.

The dosage regimen for the compounds of the present disclosure will, of course, vary depending upon known factors such as the pharmacodynamic characteristics of the specific agent and its mode and route of administration, the species, age, gender, health, medical condition and body weight of the subject, the nature and extent of the symptoms, the type of concurrent therapy, the frequency of therapy, the route of administration, the renal and hepatic function of the patient, and the desired effect. A therapeutically effective dose of the compound, the pharmaceutical composition, or a combination thereof will depend upon the species, body weight, age, and individual condition of the subject, the treated condition or disease, or the severity thereof. A physician, clinician or veterinarian of ordinary skill can readily determine the effective amount of each active ingredient which is required to prevent, treat or inhibit the condition or disease progression.

The present disclosure also provides use of the compound or the pharmaceutically acceptable salt thereof descibed above or the pharmaceutical composition for the manufacturing of a medicament for the treatment of a tumor.

EGFR (an epidermal growth factor receptor) is widely distributed on the surface of mammalian cells such as epithelial cells, fibroblasts, and glial cells. The EGFR signaling pathway plays an important role in physiological processes such as growth, proliferation and differentiation of cells. EGFR mutation is also one of the most common types of mutations in NSCLC patients, and can occur in 40%-50% of the Asian population in particular, thus in some embodiments, the compounds of the present disclosure can be used to treat cancers with high EGFR expression. The above cancers include lymphoma, non-Hodgkin's lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular cancer, gastric cancer, gastrointestinal stromal tumor (GIST), acute myelogenous leukemia (AML), cholangiocarcinoma, renal cancer, thyroid cancer, anaplastic large cell lymphoma, mesothelioma, multiple myeloma, and melanoma.

The present disclosure also provides a method for treating cancer comprising administering to a patient a therapeutically effective amount of the compound of formula (I‴), formula (I"), formula (I') or formula (I) or the pharmaceutically acceptable salt thereof described above or the pharmaceutical composition described above. The above cancers include lymphoma, non-Hodgkin's lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular cancer, gastric cancer, gastrointestinal stromal tumor (GIST), acute myelogenous leukemia (AML), cholangiocarcinoma, renal cancer, thyroid cancer, anaplastic large cell lymphoma, mesothelioma, multiple myeloma, and melanoma.

The compound of formula (I‴), formula (I"), formula (I') or formula (I) or the pharmaceutically acceptable salt thereof or the pharmaceutical composition provided by the present disclosure is used for treating cancers including lymphoma, non-Hodgkin's lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular cancer, gastric cancer, gastrointestinal stromal tumor (GIST), acute myelogenous leukemia (AML), cholangiocarcinoma, renal cancer, thyroid cancer, anaplastic large cell lymphoma, mesothelioma, multiple myeloma, and melanoma.

In some embodiments of the present disclosure, the cancer is lung cancer.

The present disclosure also provides an intermediate compound represented by formula (V), and the intermediate compound or the pharmaceutically acceptable salt thereof is selected from: 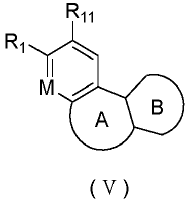
wherein, R₁₁ is -NH₂ or -NO₂;
the structural unit 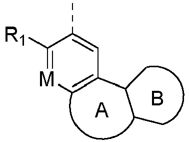 is as defined above;
further preferably, the intermediate compound of formula (V) or the pharmaceutically acceptable salt thereof is selected from 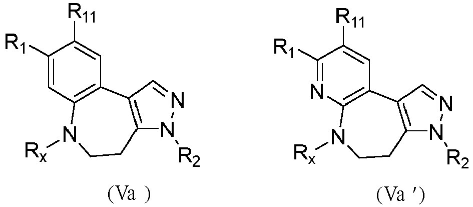
wherein, R₁, R₂, R₁₁, and Rₓ are as defined above.

Further preferably, the intermediate compound represented by formula (V) or the pharmaceutically acceptable salt thereof is selected from 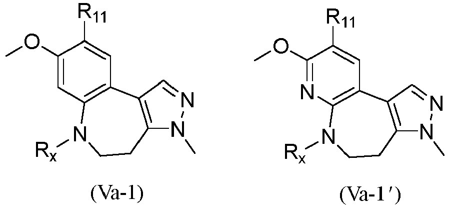 wherein, R₁₁ and Rₓ are as defined above.

### Technical Effects

The compound of the present disclosure has good inhibition effect on EGFR (L858R/T790M/C797S) kinase, and weak inhibition effect on wild-type EGFR kinase, which indicates that the compound of the present disclosure has relatively good kinase activity and selectivity.

The compound of the present disclosure has good inhibition effect on cell proliferation of a Ba/F3 Del19/T790M/C797S EGFR triple-mutation cell line and a Ba/F3 L858R/T790M/C797S EGFR triple-mutation cell line, and relatively weak inhibition effect on EGFR wild-type cell line A431, which indicates that the compounds of the present disclosure has good cell viability and selectivity.

### BRIEF DESCRIPTION OF THE DRAWINGS

FIG. 1 is a curve diagram of tumor growth (mm³) for each group of animals in the *in vivo* efficacy study experiment.
FIG. 2 is a curve diagram of body weight (g) for each group of animals in the *in vivo* efficacy study experiment.

### Description and definition

Unless otherwise stated, the following terms and phrases used herein are intended to have the following meanings. A particular term or phrase, unless otherwise specifically defined, should not be considered as uncertain or unclear, but should be construed according to its common meaning. The term "pharmaceutically acceptable" refers to those compounds, materials, compositions and/or dosage forms which are, within the scope of sound medical judgment, suitable for being in contact with the tissues of human beings and animals without excessive toxicity, irritation, allergic response, or other problems or complications, and are commensurate with a reasonable benefit/risk ratio.

The term "pharmaceutically acceptable salts" refers to derivatives of the compounds of the present disclosure which are prepared with relatively nontoxic acids or bases. These salts may be prepared during synthesis, separation, purification of the compounds, or by reacting the free form of the purified compounds with a suitable acid or base. When the compounds contain relatively acidic functional groups, the compounds can be reacted with alkali metals, alkaline earth metal hydroxides or organic amines to obtain base addition salts, including cations based on alkali metals and alkaline earth metals, such as sodium, lithium, potassium, calcium, and magnesium, as well as non-toxic ammonium, quaternary ammonium, and amine cations. When the compounds contain relatively basic functional groups, the compounds are reacted with organic acids or inorganic acids to obtain acid addition salts.

The compounds provided herein also include prodrug forms which mean compounds that are rapidly converted *in vivo* to the parent compound of the formula, and that are converted to the compounds of the present disclosure by chemical or biochemical means in an *in vivo* or *in vitro* environment, e.g., by hydrolysis in blood.

The compounds of the present disclosure can exist in non-solvated forms and solvated forms, including hydrated forms. In general, the solvated forms are equivalent to non-solvated forms and are intended to be encompassed within the scope of the present disclosure.

The compounds of the present disclosure have geometric isomers and stereoisomers, such as *cis-trans* isomers, enantiomers, diastereoisomers, and racemic and other mixtures thereof, all of which are within the scope of the present disclosure.

The term "enantiomer" refers to stereoisomers that are mirror images of each other.

The term "diastereoisomer" refers to stereoisomers whose molecules have two or more chiral centers and are not mirror images of each other.

The term *"cis-trans* isomer" refers to a configuration results from the inability of a single bond of a ring carbon atom or a double bond in a molecule to rotate freely.

Unless otherwise stated, the absolute configuration of a stereogenic center is represented by a wedged solid bond 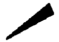 and a wedged dashed bond 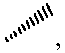 and the relative configuration of a stereogenic center is represented by a straight solid bond 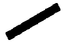 and a straight dashed bond 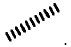 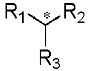 represents that the carbon atom is a chiral carbon atom, and the structure represents the optically pure compound of which the spatial configuration of the carbon atom is (R) configuration or (5) configuration and the mixture thereof.

Stereoisomers of the compounds of the present disclosure may be prepared by chiral synthesis or chiral reagents or other conventional techniques. For example, one enantiomer of a compound of the present disclosure may be prepared by asymmetric catalysis techniques or by chiral auxiliary derivatization techniques, or by chiral resolution techniques, i.e., a compound with a single spatial configuration is obtained from a mixture, or by using chiral starting materials. The separation of optically pure compounds in the present disclosure is usually accomplished using preparative chromatography and employing chiral chromatographic columns to achieve the separation of chiral compounds.

The term "optically pure" or "enantiomerically enriched" means that the isomer or enantiomer is present in an amount of greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

The absolute spatial configuration of the compound can be confirmed by means of a method which is conventional in the art. For example, the absolute configuration of a compound can also be confirmed by a monocrystal X-ray diffraction, the chiral structure of starting materials and the reaction mechanism of asymmetric synthesis. The compounds marked with "absolute configuration not determined" herein are usually resolved from the racemate into the individual isomers by chiral preparative HPLC or SFC, followed by characterization and testing.

For example, the following formula represents compounds 117A and 117B resolve from compound 84 as enantiomers of each other; however, the absolute spatial configurations of compounds 117A and 117B are not determined.

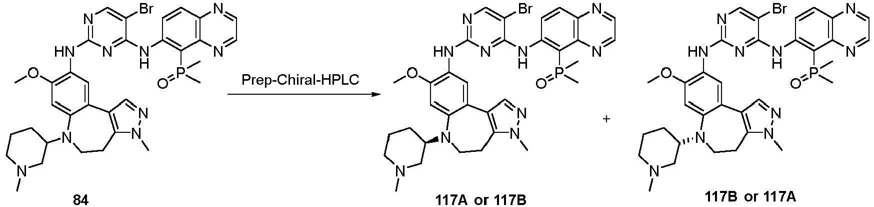

The present disclosure also comprises isotopically-labeled compounds including isotopes of hydrogen, carbon, nitrogen, oxygen, phosphorus, sulfur, fluorine, and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl. The compounds of the present disclosure containing the above isotopes and/or other isotopes of other atoms are within the scope of this disclosure.

The term "pharmaceutically acceptable carriers" refer to vehicles generally accepted in the art for delivering biologically active agents to animals, particularly mammals, and includes, for example, adjuvants, excipients such as diluents, preservatives, fillers, flow modifiers, disintegrants, wetting agents, emulsifiers, suspending agents, sweeteners, flavoring agents, fragrances, antibacterial agents, antifungal agents, lubricants and dispersing agents, depending on the mode of administration and the nature of the dosage form. Pharmaceutically acceptable carriers are formulated within the purview of one of ordinary skill in the art based on a number of factors, which include, but are not limited to: the type and nature of the formulated active agents, the subject to which the composition containing the agents is to be administered, the intended route of administration of the composition, and the targeted therapeutic indication. Pharmaceutically acceptable carriers include both aqueous and non-aqueous media and a variety of solid and semi-solid dosage forms. Such carriers include many different ingredients and additives in addition to the active agents, and such additional ingredients included in the formulation for a variety of reasons (e.g., stabilizing the active agents and adhesives) are well known to those of ordinary skill in the art.

The term "excipient" generally refers to a carrier, diluent, and/or medium necessary to formulate an effective pharmaceutical composition.

The term "effective prophylactic or therapeutic amount of" refers to a sufficient amount of the compound of the present disclosure or the pharmaceutically acceptable salt thereof, to treat a disorder at a reasonable benefit/risk ratio applicable to any medical treatment and/or prevention. It shall be appreciated, however, that the total daily amount of the compound of formula I or the pharmaceutically acceptable salt thereof and the composition of the present disclosure will be determined by an attending physician within the scope of sound medical judgment. For any particular patient, the particular therapeutically effective dose level will depend upon a variety of factors including the disorder being treated and the severity of the disorder, the activity of a particular compound employed, the particular composition employed, the age, body weight, general health, gender, and diet of the patient, the time of administration, route of administration and excretion rate of the particular compound employed, the duration of the treatment, the drugs used in combination or simultaneously with the particular compound employed, and similar factors well known in the medical arts. For example, it is known in the art to start a dose of a compound at a level below that required to achieve a desired therapeutic effect and then gradually increase the dose until the desired therapeutic effect is achieved. In general, the dosage of the compound of formula I or the pharmaceutically acceptable salt thereof of the present disclosure for mammals, especially humans, can be from about 0.001 to about 1000 mg/kg bw/day, such as from about 0.01 to about 100 mg/kg bw/day, and from about 0.01 to about 10 mg/kg bw/day.

The term "optionally substituted" means that an atom can be substituted with a substituent or not. Unless otherwise specified, the type and number of the substituent may be arbitrary as long as being chemically achievable. For example, the term "optionally substituted with one or more R₂" means that the atom may be substituted with one or more R₂ or not.

When any variable (e.g., R₂) occurs more than once in the constitution or structure of a compound, the variable is independently defined in each case. For example, if a group is substituted with 0-2 R₂, the group can be optionally substituted with up to two R₂, and the definition of R₂ in each case is independent.

When a linking group has a number of 0, such as -O(CH₂)ₙCH₃, n = 0 indicates that the linking group is a single bond, i.e., -OCH₃.

When a bond of a substituent can be cross-linked to two atoms on a ring, the substituent can be bonded to any atom on the ring. For example, the structural unit 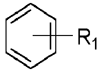 represents that the substituent R₁ may be substituted at any position on the phenyl ring.

When the substituents listed are not indicated by which atom they are attached to the compounds included in the general chemical structural formula but not specifically mentioned, such substituents may be bonded through any atom thereof. For example, pyrazole as a substituent means that any one of carbon atoms on the pyrazole ring is attached to a substituted group; 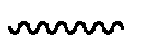 or 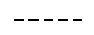 being present in the structure indicates that the atom is a bonding atom, for example, 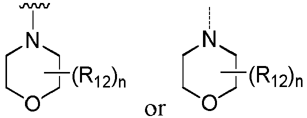 both indicates that N atoms in a morpholine ring are bonding atoms.

Unless otherwise specified, a "ring" refers to a saturated, partially saturated or unsaturated monocyclic and polycyclic ring including spirocyclic, bicyclic or bridged ring. Representative "rings" include substituted or unsubstituted cycloalkyl, heterocycloalkyl, cycloalkenyl, heterocycloalkenyl, cycloalkynyl, heterocycloalkynyl, aryl, or heteroaryl. The term "hetero" represents a substituted or unsubstituted heteroatom as well as an oxidized form of a heteroatom, wherein the heteroatom is generally selected from N, O, and S, and the oxidized form of the heteroatom generally includes NO, SO, and S(O)₂, and nitrogen atoms may be substituted, i.e., NR (R is H or other substituent as defined herein); the number of atoms on the ring is generally defined as the number of ring members, e.g., "3-6 membered heterocycloalkyl" refers to a ring of 3-6 atoms arranged around, each ring optionally containing 1-3 heteroatoms, i.e., N, O, S, NO, SO, S(O)₂, or NR, each ring optionally substituted with R, and the R being a group as defined herein.

Unless otherwise specified, the term "carbocyclic ring" or "carbocyclyl" means a stable cyclic structure composed of carbon atoms and which may be monocyclic, bicyclic or tricyclic, and which may be saturated, partially unsaturated or unsaturated (aromatic). Any of the above carbocyclic rings may be fused to one or more aromatic rings and aromatic heterocyclic rings to form polycyclic rings such as bicyclic and tricyclic rings. For example, in the structural unit 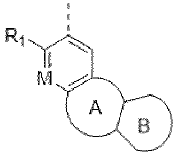 or 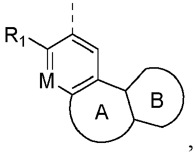 ring A can be a 5-8 membered carbocyclyl, examples of which include, but are not limited to, 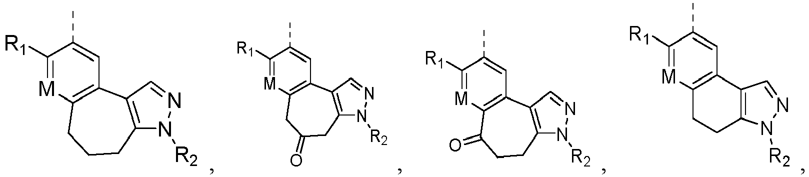 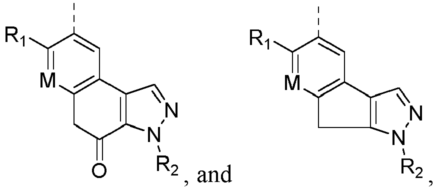 and R₁, R₂, Rₓ, and M are groups as defined herein.

Unless otherwise specified, the term "heterocyclic ring" or "heterocyclyl" means a stable monocyclic, bicyclic, or tricyclic ring containing heteroatom or heteroatom group, these rings may be saturated, partially unsaturated, or unsaturated (aromatic), and contain carbon atoms and 1, 2, 3 heteroatoms independently selected from N, O, S, NO, SO, S(O)₂, and NR, wherein any of the above heterocyclic rings can be fused to one or more aromatic rings and aromatic heterocyclic rings to form polycyclic rings such as bicyclic and tricyclic rings. For example, in the structural unit 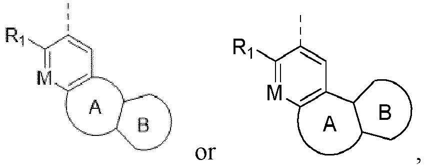 ring A can be a 5-8 membered heterocyclyl, examples of which include, but are not limited to, 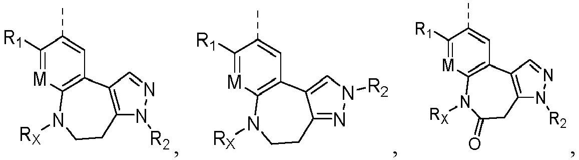 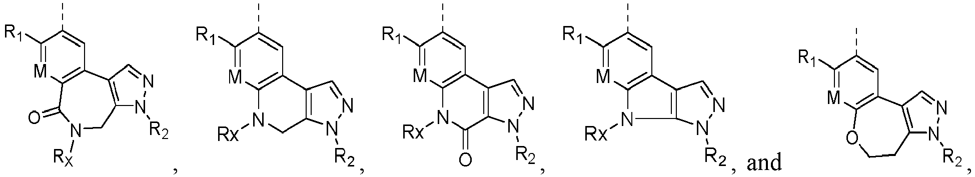 and R₁, R₂, Rₓ, and M are groups as defined herein.

Unless otherwise specified, the term "aryl" refers to an unsaturated and usually aromatic hydrocarbyl that may be a single ring or multiple rings fused together, preferably C₅₋₁₀ aryl, more preferably C₅₋₈ aryl, and most preferably monocyclic C₅₋₆ aryl; examples of aryl include, but are not limited to, phenyl and naphthyl.

Unless otherwise specified, the term "heteroaryl" means a stable monocyclic or polycyclic aromatic hydrocarbon containing at least one heteroatom (N, O, S, NO, SO, S(O)₂ or NR), preferably 5- or 6-membered monocyclic heteroaryl. Examples of heteroaryl include, but are not limited to, pyrrolyl, pyrazolyl, imidazolyl, pyrazinyl, oxazolyl, isoxazolyl, thiazolyl, furanyl, thienyl, pyridyl, and pyrimidinyl.

Unless otherwise specified, the term "alkyl" refers to a linear or branched saturated hydrocarbyl, preferably C₁₋₆ alkyl, and more preferably C₁₋₃ alkyl; examples of alkyl include, but are not limited to, methyl, ethyl, n-propyl, isopropyl, butyl, isobutyl, pentyl, isopentyl, neopentyl, n-hexyl and the like.

Unless otherwise specified, the term "heteroalkyl" refers to an alkyl group in which one or more carbon atoms are substituted with a heteroatom selected from B, O, N and S, wherein the nitrogen and sulfur atoms are optionally oxidized, the nitrogen heteroatom is optionally quaternized, including but not limited to "alkoxy", "alkylamino", "alkylthio", and the like; examples of "heteroalkyl" include, but are not limited to, -OCH₃, -OCH₂CH₃, -OCH₂CH₂CH₃, -OCH(CH₃)₂, - N(CH₃)₂, -CH₂-CH₂-O-CH₃, -CH₂-CH₂-NH-CH₃, -CH₂-CH₂-N(CH₃)-CH₃, -CH₂-S-CH₂-CH₃, - CH₂-CH₂, -S(O)-CH₃, -S(O)₂-CH₃, -CH₂-CH₂-S(O)₂-CH₃, and the like.

Unless otherwise specified, "alkenyl" refers to an alkyl group having one or more carbon-carbon double bonds, preferably C₂₋₈ alkenyl, and examples of alkenyl include, but are not limited to, ethenyl, propenyl, butenyl, pentenyl, hexenyl, and the like.

Unless otherwise specified, "alkynyl" refers to an alkyl group having one or more carbon-carbon triple bonds, preferably C₂₋₈ alkynyl, and examples of alkynyl include, but are not limited to, ethynyl, propynyl, butynyl, pentynyl, and the like.

Unless otherwise specified, the term "halogen" refers to a fluorine, chlorine, bromine or iodine atom.

Unless otherwise specified, the term "haloalkyl" refers to an alkyl group having one or more hydrogen atoms substituted with a halogen atom, preferably C₁₋₆ haloalkyl, and more preferably C₁₋₃ haloalkyl; and examples of haloalkyl include, but are not limited to, monofluoromethyl, difluoromethyl, trifluoromethyl, trichloromethyl, tribromomethyl, 2,2,2-trifluoroethyl, 2,2,2 trichloroethyl, and the like.

Unless otherwise specified, the term "alkoxy" refers to an alkyl group attached through an oxygen bridge, i.e., a group resulting from the substitution of a hydrogen atom in a hydroxyl group with an alkyl group, preferably C₁₋₆ alkoxy, and more preferably C₁₋₃ alkoxy. Examples of alkoxy include, but are not limited to, methoxy, ethoxy, n-propoxy, isopropoxy, n-butoxy, sec-butoxy, *tert*-butoxy, n-pentoxy, neopentoxy, and n-hexoxy.

Unless otherwise specified, the term "cycloalkyloxy" refers to a cycloalkyl group attached through an oxygen bridge, i.e., a group resulting from the substitution of a hydrogen atom in a hydroxyl group with a cycloalkyl group. The cycloalkyloxy is preferably 3-7 membered, 4-7 membered, or 5-7 membered cycloalkyloxy. Examples of cycloalkyloxy include, but are not limited to, cyclopropyloxy, cyclobutyloxy, cyclopentyloxy, and cyclohexyloxy.

Unless otherwise specified, the term "haloalkoxy" refers to an alkoxy group in which one or more hydrogen atoms are substituted with a halogen atom. Examples of haloalkoxy include, but are not limited to, trifluoromethoxy, trichloromethoxy, 2,2,2-trifluoroethoxy, and 2,2,2-trichloroethoxy. Unless otherwise specified, "cycloalkyl" refers to a saturated monocyclic or polycyclic hydrocarbyl. The cycloalkyl is preferably 3-8 membered monocyclic alkyl, and more preferably 3-6 membered monocyclic alkyl, and examples of these monocyclic alkyl include, but are not limited to, cyclopropyl, cyclobutyl, cyclopentyl, cyclohexyl, cycloheptyl, and cyclooctyl.

Unless otherwise specified, "heterocycloalkyl" refers to mono-heterocycloalkyl and polyheterocycloalkyl containing a specified number of heteroatoms on the ring, wherein the heteroatoms are generally selected from N, O, S, NO, SO, S(O)₂, and NR. The heterocycloalkyl is preferably 3-8 membered monocyclic heterocycloalkyl, and more preferably 3-6 membered monocyclic heterocycloalkyl, and examples of these monocyclic heterocycloalkyl include, but are not limited to, oxiranyl, tetrahydropyrrolyl, piperidyl, piperazinyl, morpholinyl, tetrahydrofuranyl, tetrahydrothienyl, tetrahydropyranyl, 1,3-dioxolane, 1,4-dioxane, and the like.

Unless otherwise specified, "spirocyclyl" refers to a bicyclic or polycyclic hydrocarbyl sharing a carbon atom between two monocyclic rings. The spirocyclyl is preferably 5-13 membered spiro ring group, 6-12 membered spirocyclyl, or 7-11 membered spirocyclyl, the 6-12 membered spirocyclyl means a hydrocarbon group consisting of 6-12 atoms in the spirocyclic skeletal structure, and examples of spirocyclyl include, but are not limited to, spiro[2.2]pentyl, spiro[2.3]hexyl, spiro[2.4]heptyl, spiro[2.5]octyl, spiro[2.6]nonyl, spiro[3.3]heptyl, spiro[3.4]octyl, spiro[3.5]nonyl, spiro[3.6]decyl, spiro[4.4]nonyl, spiro[4.5]decyl, spiro[4.6]undecyl, spiro[5.5]undecyl, spiro[5.6]dodecyl, spiro[6.6]tridecyl, and spiro[6.7]tetradecyl.

Unless otherwise specified, "spiroheterocyclyl" refers to a spirocyclic group in which one or more carbon atoms in the spirocyclic skeletal structure are substituted with a heteroatom selected from N, O, and S. The spiroheterocyclyl is preferably 5-13 membered spiroheterocyclyl, 6-12 membered spiroheterocyclyl, or 7-11 membered spiroheterocyclyl. Examples of spiroheterocyclyl include, but are not limited to, 2-oxa-7-azaspiro[5.3]nonan-7-yl, 2-oxa-7-azaspiro[4.4]nonan-7-yl, 2-oxa-6-azaspiro[3.3]heptan-6-yl, 2-oxa-8-azaspiro[4.5]decan-8-yl, 1,4,9-triazaspiro[5.5]undecan-9-yl, 3-oxa-9-azaspiro[5.5]undecan-9-yl, 2,6-diazaspiro[3.3]heptan-2-yl, 2,7-diazaspiro[5.3]nonan-7-yl, 2,7-dioxaspiro[5.3]nonyl, 3,9-diazaspiro[5.5]undecan-3-yl, 1-oxa-4,9-diazaspiro[5.5]undecan-9-yl, 1-oxa-4,8-diazaspiro[5.4]decan-8-yl, 3-azaspiro[5.5]undecan-3-yl, 7-azaspiro[3.5]decan-7-yl, 1-oxa-4,9-diazaspiro[5.5]undecan-4-yl, 6-oxa-2,9-diazaspiro[4.5]decan-9-yl, 9-oxa-2,6-diazaspiro[4.5]decan-6-yl, 3-azaspiro[5.5]undecan-3-yl, and 4-oxa-1,9-diazaspiro[5.5]undecan-9-yl.

The term "4-7 membered ring containing P(=O)R_{b}" refers to a group having the structure 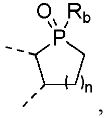 wherein n is 0, 1, 2, or 3; R_{b} is a group as defined herein, the 4-7 membered ring containing -P(=S)(R_{b})-, -N(R_{b})S(=O)₂-, -S(=O)₂N(R_{b})- or -S(=O)₂ represents the structure 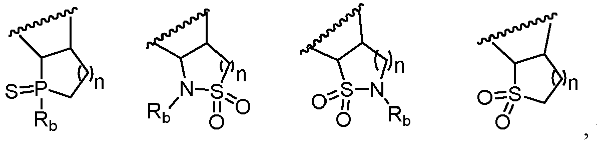 wherein n is 0, 1, 2, or 3; R_{b} is a group as defined herein.

It is specifically stated that all combinations of a substituent and/or a variant thereof is permissible only if the combinations can result in a stable compound.

In the examples of the present disclosure, the title compound is named after conversion by the compound structure by Chemdraw. If the name of the compound is inconsistent with the structure of the compound, the name of the compound can be determined by assisting of integrating related information and a reaction route; and the given structural formula of the compound shall prevail if the name of the compound cannot be confirmed by other methods.

The preparation methods of some compounds in the present disclosure refer to the preparation methods of the similar compounds described above. It will be understood by those skilled in the art that the feed ratio of the reactants, the reaction solvent, the reaction temperature and the like may be appropriately adjusted depending on the reactants when the preparation method cited herein is used or referred to.

The compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. Preferred embodiments include, but are not limited to, the examples of the present disclosure.

Abbreviations and their corresponding chemical names used in the examples of the present disclosure are as follows:

| **Abbreviations** | **Description** |
|---|---|
| Bpin-Bpin | Bis(pinacolato)diboron |
| Pd(dppf)Cl₂ | [1,1'-bis(diphenylphosphino)ferrocene]dichloropalladium(II) |
| NIS | *N*-iodosuccinimide |
| NBS | *N*-bromosuccinimide |
| Pd/C | Palladium on carbon |
| DPPA | Diphenylphosphoryl azide |
| Boc₂O | Di-*tert*-butyl dicarbonate |
| MeI | Iodomethane |
| HCl-dioxane | A solution of hydrochloric acid in dioxane |
| K₂CO₃ | Potassium carbonate |
| DMF | *N,N*-dimethylformamide |
| Fe | Iron |
| NH₄Cl | Ammonium chloride |
| CH₃NH₂ | Methylamine |
| NaH | Sodium hydride |
| LiOH | Lithium hydroxide |
| BH₃-Me₂S | Borane-dimethyl sulfide solution |
| PtO₂ | Platinum dioxide |
| Ac₂O | Acetic anhydride |
| BBr₃ | Boron tribromide |
| Xphos Pd G₃ | Methanesulfonato(2-dicyclohexylphosphino-2',4',6'-triisopropyl-1,1'-biphenyl)(2'-amino-1,1'-biphenyl-2-yl)palladium(II) |
| Xphos | 2-Dicyclohexylphosphino-2,4,6-triisopropylbiphenyl |
| NH₂Boc | *Tert*-butyl carbamate |
| BrCN | Cyanogen bromide |
| PPA | Polyphosphoric acid |
| THP | Tetrahydropyrane |
| Et₃SiH | Triethylsilane |
| MeONa | Sodium methoxide |
| Br₄C | Tetrabromomethane |
| TosMIC | Tosylmethyl isocyanide |
| TBAB | Tetrabutylammonium bromide |
| TBSCl | *Tert*-butyldimethylsilyl chloride |
| TBAF | Tetrabutylammonium fluoride |
| SEM-Cl | 2-(trimethylsilyl)ethoxymethyl chloride |
| PMB-Cl | *p*-methoxybenzyl chloride |

### DETAILED DESCRIPTION

The compound structure of the present disclosure is determined by nuclear magnetic resonance (NMR) and/or liquid chromatography-mass spectrometry (LC-MS). The NMR chemical shift (δ) is given in parts per million (ppm). NMR determination is performed using NMR spectrometers (Bruker AVANCE III HD 400 and Bruker AVANCE III HD 300), with deuterated dimethyl sulfoxide (DMSO-*d₆*), deuterated methanol (CD₃OD) and deuterated chloroform (CDCl₃) as solvents and tetramethylsilane (TMS) as an internal standard.

The LC-MS determination is conducted by using a SHIMADZU LCMS-2020 mass spectrometer (with electrospray ionization as an ion source). The HPLC determination is conducted by using SHIMADZU LC-20 AP _{XR} and SPD-M20A high pressure liquid chromatography.

A Xinnuo Chemical GF254 (Yantai) silica gel plate of specifications 0.15 mm to 0.20 mm is adopted for thin layer chromatography (TLC) analysis, and a Yuchen Chemical silica gel of 200-300 mesh is generally utilized as a carrier in column chromatography.

Starting materials in the Examples of the present disclosure are known and commercially available, or may be synthesized by using or according to methods known in the art.

### Example 1:

### Preparation of intermediate 1A

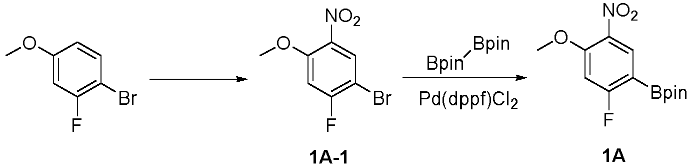

### Compound 1A-1:

3-Fluoro-4-bromoanisole (20 g, 98 mmol) was dissolved in concentrated sulfuric acid (80 mL) at 0 °C. Subsequently, potassium nitrate (9.86 g, 98 mmol) was added in batches to the reaction liquid, and the reaction system was successively stirred at that temperature for 30 min. After the starting material disappeared as detected by TLC, the reaction liquid was slowly poured into ice water (500 g) to quench the reaction. The mixture was extracted with ethyl acetate (200 mL × 3), the organic phases were combined, washed with saturated brine (200 mL × 3), then dried over anhydrous sodium sulfate, filtered, and concentrated under reduced pressure, and the resulting residue was purified by silica gel column chromatography (eluent: petroleum ether/ethyl acetate = 5/1) to obtain compound **1A-1** (11.2 g).

¹H NMR (300 MHz, CDCl₃) δ 8.19 (d, *J=* 7.2 Hz, 1H), 6.91 (d, *J=* 9.9 Hz, 1H), 3.99 (s, 3H).

### Intermediate 1A:

Compound **1A-1** (3 g, 12 mmol) was dissolved in 1,4-dioxane (20 mL) at room temperature under nitrogen atmosphere. Subsequently, bis(pinacolato)diboron (3.3 g, 13 mmol), potassium acetate (2.4 g, 24 mmol) and [1,1'-bis(diphenylphosphino)ferrocene]dichloropalladium(II) (0.88 g, 1.2 mmol) were successively added to the reaction liquid. The reaction system was heated to 80 °C and successively stirred for 16 h. After the starting material disappeared as detected by TLC, the reaction liquid was cooled to room temperature and quenched by addition of water (100 mL). The mixture was extracted with ethyl acetate (100 mL × 3), the organic phases were combined, washed with saturated brine (100 mL × 3) firstly, then dried over anhydrous sodium sulfate, filtered, and finally concentrated under reduced pressure, and the resulting residue was purified by silica gel column chromatography (eluent: petroleum ether/ethyl acetate = 5/1) to obtain compound 1A (1.5 g).

¹H NMR (300 MHz, CDCl₃) δ 8.36 (d, *J* = 6.0 Hz, 1H), 6.76 (d, *J* = 10.5 Hz, 1H), 4.00 (s, 3H), 1.37 (s, 12H).

### Preparation of intermediate 1B

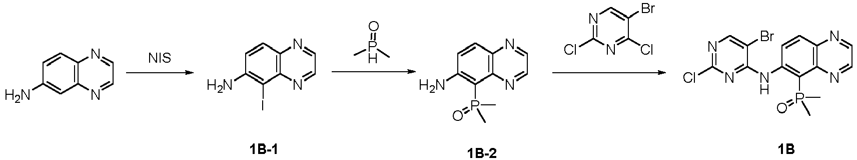

### Compound 1B-1:

6-Aminoquinoxaline (59 g, 406.4 mmol) was dissolved in *N*,*N*-dimethylformamide (600 mL), and N-iodosuccinimide (100.6 g, 447.1 mmol) was added in batches at room temperature. The reaction system was stirred at room temperature for 1 h. After the starting material disappeared as detected by LCMS, the reaction liquid was quenched by addition of water (3000 mL). The mixture was extracted with ethyl acetate (1000 mL × 3), the organic phases were combined, washed with saturated brine (500 mL × 3), then dried over anhydrous sodium sulfate, filtered, and finally concentrated under reduced pressure, and the resulting residue was purified by silica gel column chromatography (eluent: petroleum ether/ethyl acetate = 1/2) to obtain compound **1B-1** (50 g).

MS (ESI) M/Z: 272.0 [M + H]⁺.

### Compound 1B-2:

Compound **1B-1** (40 g, 147.6 mmol) was dissolved in *N*,*N*-dimethylformamide (400 mL) at room temperature under nitrogen atmosphere. Subsequently, dimethylphosphine oxide (17.3 g, 221.4 mmol), palladium acetate (3.3 g, 14.7 mmol), 4,5-bis-diphenylphosphino-9,9-dimethylxanthene (12.8 g, 22.1 mmol) and *N,N-*diisopropylethylamine (38.1 g, 295.1 mmol) were added to the above reaction liquid successively. The reaction liquid was heated to 120 °C and successively stirred for 16 h. After the starting material disappeared as detected by LCMS, the reaction liquid was cooled to room temperature and filtered, and the filter cake was washed with ethanol (100 mL × 3); the filtrate was concentrated under reduced pressure, and the resulting residue was purified by silica gel column chromatography (eluent: dichloromethane/methanol = 10/1) to obtain compound **1B-2** (30 g).

MS (ESI) M/Z: 222.0 [M + H]⁺.

### Intermediate 1B:

Compound **1B-2** (5 g, 22.6 mmol) was dissolved in *N*,*N*-dimethylformamide (100 mL) at room temperature under nitrogen atmosphere. Sodium hydride (60%, 1.99 g, 49.8 mmol) was then added in batches to the above reaction liquid at 0 °C, and the reaction liquid was successively stirred at that temperature for 30 min; 2,4-dichloro-5-bromopyrimidine (6.18 g, 27.1 mmol) was then added to the reaction liquid at 0 °C and the reaction liquid was warmed to room temperature and successively stirred for 2 h. After the starting material disappeared as detected by LCMS, the reaction liquid was quenched by addition of saturated aqueous ammonium chloride (600 mL). The mixture was extracted with ethyl acetate (200 mL × 3), the organic phases were combined, washed with saturated brine (500 mL), then dried over anhydrous sodium sulfate, filtered, and finally concentrated under reduced pressure, and the resulting residue was purified by silica gel column chromatography (eluent: ethyl acetate) to obtain compound **1B** (3 g).

### Preparation of intermediate 1C

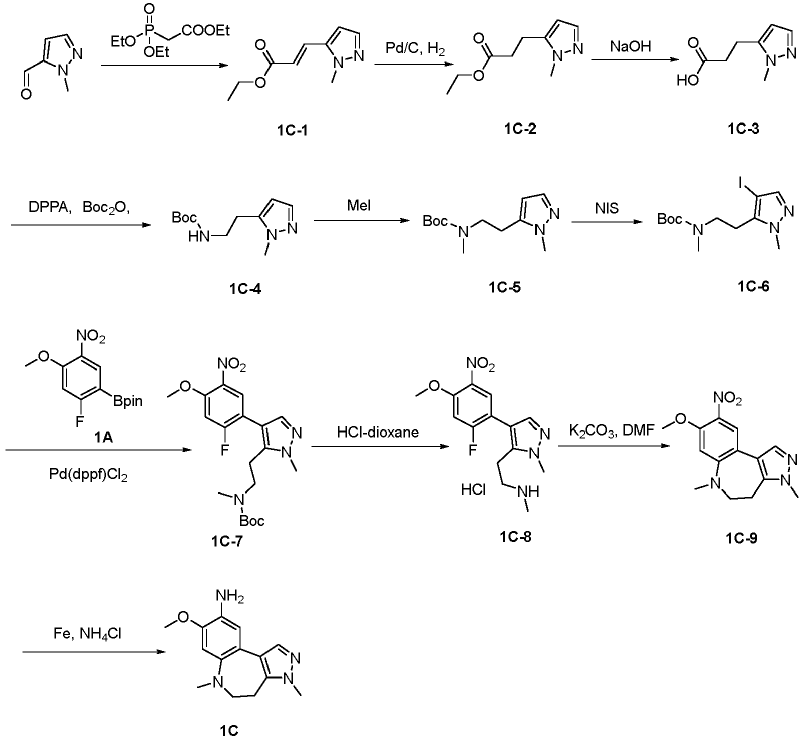

### Compound 1C-1:

Triethyl phosphoryl acetate (61.1 g, 273 mmol) was dissolved in tetrahydrofuran (120 mL). Subsequently, sodium hydride (60%, 10.9 g, 273 mmol) was added to the reaction liquid under nitrogen atmosphere at 0 °C, and the reaction liquid was successively stirred at that temperature for 30 min. To the reaction liquid was added a solution of 1-methyl-1*H*-pyrazole-5-carbaldehyde (20 g, 182 mmol) in tetrahydrofuran (100 mL). The reaction system was warmed to room temperature and successively stirred for 1.5 h. After the starting material disappeared as detected by LCMS, the reaction liquid was quenched by addition of saturated aqueous ammonium chloride (100 mL). The mixture was extracted with ethyl acetate (300 mL × 3), and the organic phases were combined, washed with saturated brine (100 mL × 3), then dried over anhydrous sodium sulfate, filtered, and concentrated under reduced pressure. The resulting residue was purified by silica gel column chromatography (eluent: petroleum ether/ethyl acetate = 1/1) to obtain compound **1C-1** (33 g).

MS (ESI) M/Z: 181.2 [M + H]⁺.

### Compound 1C-2:

Compound **1C-1** (33 g, 183.1 mmol) was dissolved in ethanol (500 mL). Wet palladium on carbon (10%, 6.6 g) was added to the reaction liquid. After the reaction system was purged with hydrogen 3 times, the reaction liquid was stirred at room temperature for 1 h. After the starting material disappeared as detected by LCMS, the reaction liquid was filtered through celite, the filter cake was washed with ethyl acetate (30 mL × 3), and the resulting filtrate was concentrated under reduced pressure to obtain compound **1C-2** (33 g).

MS (ESI) M/Z: 183.1 [M + H]⁺.

### Compound 1C-3:

Compound **1C-2** (32 g, 175.6 mmol) was dissolved in ethanol (320 mL). Subsequently, sodium hydroxide (1 N, 352 mL) was added to the reaction liquid. The reaction system was heated to 70 °C and successively stirred for 1 h. After the starting material disappeared as detected by LCMS, the reaction liquid was cooled to room temperature and concentrated under reduced pressure to remove ethanol. The aqueous phase was adjusted to pH = 3 with diluted hydrochloric acid (1 N) and with ethyl acetate (100 mL × 3), and the organic phases were combined, washed with saturated brine (100 mL × 3), dried over anhydrous sodium sulfate, filtered, and concentrated under reduced pressure to obtain compound **1C-3** (25 g).

MS (ESI) M/Z: 155.1 [M + H]⁺.

### Compound 1C-4:

Compound **1C-3** (5 g, 32.4 mmol) and diphenylphosphoryl azide (abbreviated as DPPA, 9.8 g, 35.7 mmol) were dissolved in tert-butanol (80 mL) under nitrogen atmosphere. Subsequently, triethylamine (13.1 g, 129.7 mmol) and di-tert-butyl dicarbonate (21.2 g, 97.3 mmol) were added to the reaction liquid, and the reaction liquid was successively stirred at room temperature for 1 h. The reaction system was warmed to 80°C and successively stirred for 16 h. After the starting material disappeared as detected by LCMS, the reaction liquid was cooled to room temperature and quenched by addition of saturated sodium bicarbonate (100 mL). The mixture was extracted with ethyl acetate (200 mL × 3), and the organic phases were combined, washed with saturated brine (100 mL × 3), then dried over anhydrous sodium sulfate, filtered, and concentrated under reduced pressure. The resulting residue was purified by silica gel column chromatography (eluent: petroleum ether/ethyl acetate = 1/1) to obtain compound **1C-4** (5.8 g).

MS (ESI) M/Z: 226.1 [M + H]⁺.

### Compound 1C-5:

Compound **1C-4** (500 mg, 2.22 mmol) was dissolved in *N,N*-dimethylformamide (4 mL) at 0 °C under nitrogen atmosphere. Subsequently, sodium hydride (60%, 69 mg, 1.7 mmol) was added to the above reaction liquid. The reaction liquid was successively stirred at 0 °C for 30 min. Subsequently, iodomethane (329 mg, 2.3 mmol) was added to the reaction liquid. The reaction system was warmed to room temperature and successively stirred for 2 h. After the starting material disappeared as detected by LCMS, the reaction liquid was quenched by addition of saturated aqueous ammonium chloride (20 mL). The mixture was extracted with ethyl acetate (30 mL × 3), the organic phases were combined, washed with saturated brine (50 mL × 3) firstly, then dried over anhydrous sodium sulfate, filtered, and finally concentrated under reduced pressure, and the resulting residue was purified by silica gel column chromatography (eluent: dichloromethane/methanol = 15/1) to obtain compound **1C-5** (450 mg).

MS (ESI, m/z): 240.1 [M + H]⁺.

### Compound 1C-6:

Compound **1C-5** (400 mg, 1.7 mmol) was dissolved in acetonitrile (2 mL). Subsequently, N-iodosuccinimide (451 mg, 2.0 mmol) was added to the reaction liquid at 0 °C. The reaction system was warmed to room temperature and successively stirred for 2 h. After the starting material disappeared as detected by LCMS, the reaction liquid was concentrated under reduced pressure, and the resulting residue was purified by silica gel column chromatography (eluent: petroleum ether/ethyl acetate = 1/1) to obtain compound **1C-6** (600 mg).

MS (ESI, m/z): 366.0 [M + H]⁺.

### Compound 1C-7:

Compound **1C-6** (664 mg, 1.8 mmol) and compound **1A** (450 mg, 1.5 mmol) were dissolved in a mixed solvent of dioxane (6 mL) and water (1.2 mL) under nitrogen atmosphere. Subsequently, [1,1'-bis(diphenylphosphino)ferrocene]dichloropalladium(II) (124 mg, 0.15 mmol) and sodium carbonate (321 mg, 3.0 mmol) were added to the reaction liquid. The reaction system was heated to 80 °C and successively stirred for 2 h. After the starting material disappeared as detected by LCMS, the reaction liquid was cooled to room temperature and quenched by addition of water (20 mL). The mixture was extracted with ethyl acetate (30 mL × 3), and the organic phases were combined, washed with saturated brine (30 mL × 3), then dried over anhydrous sodium sulfate, filtered, and concentrated under reduced pressure. The resulting residue was purified by silica gel column chromatography (eluent: petroleum ether/ethyl acetate = 1/2) to obtain compound **1C-7** (440 mg).

MS (ESI) M/Z: 409.2 [M + H]⁺.

### Compound 1C-8:

Compound **1C-7** (430 mg, 1.1 mmol) was dissolved in a solution of hydrochloric acid in dioxane (4 M, 4 mL), and the mixture was successively stirred at room temperature for 1 h. After the starting material disappeared as detected by LCMS, the reaction liquid was concentrated under reduced pressure to obtain compound **1C-8** (400 mg).

MS (ESI) M/Z: 309.2 [M + H]⁺.

### Compound 1C-9:

Compound **1C-8** (100 mg, 0.3 mmol) and potassium carbonate (120 mg, 0.9 mmol) were dissolved in *N,N*-dimethylformamide (2 mL). The reaction system was heated to 100 °C and successively stirred for 1 h. After the starting material disappeared as detected by LCMS, the reaction liquid was cooled to room temperature and purified directly using a reverse phase C18 column. The purification conditions were as follows: a chromatography column: 40 g of C18 reverse phase column; mobile phase: water (containing 10 mM ammonium bicarbonate) and acetonitrile; flow rate: 35 mL/min; gradient: acetonitrile elevating from 40% to 80% in 30 min; detection wavelength: 254 nm. The product was collected and lyophilized under reduced pressure to obtain compound **1C-9** (50 mg).

MS (ESI) M/Z: 289.2 [M + H]⁺.

### Intermediate 1C:

Compound **1C-9** (50 mg, 0.17 mmol) was dissolved in a mixed solvent of ethanol (1 mL) and water (0.2 mL). Subsequently, iron powder (48 mg, 0.9 mmol) and ammonium chloride (14 mg, 0.26 mmol) were added to the above reaction liquid, and the reaction system was heated to 80 °C and successively stirred for 2 h. After the starting material disappeared as detected by LCMS, the reaction liquid was cooled to room temperature and filtered, the filter cake was washed with ethanol (10 mL × 5), and the filtrate was concentrated under reduced pressure to obtain intermediate compound **1C** (50 mg).

MS (ESI) M/Z: 259.2 [M + H]⁺.

### Preparation of compound 1

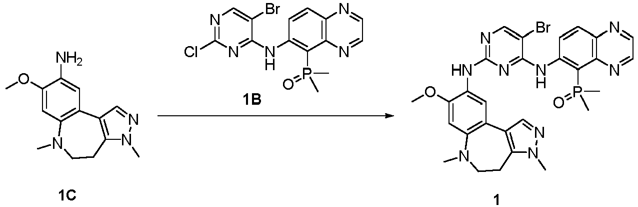

Compound **1C** (40 mg, 0.16 mmol) and compound **1B** (64 mg, 0.16 mmol) were dissolved in *N-*methylpyrrolidinone (2 mL). Subsequently, methanesulfonic acid (45 mg, 0.47 mmol) was added to the above reaction liquid. The reaction liquid was heated to 100 °C and successively stirred for 3 h. After the starting material disappeared as detected by LCMS, the reaction liquid was cooled to room temperature and purified using a reverse phase C18 column. Purification conditions: a chromatography column: 40 g of C18 reverse phase column; mobile phase: water (containing 0.1% formic acid) and acetonitrile; flow rate: 35 mL/min; gradient: acetonitrile elevating from 10% to 50% in 20 min; detection wavelength: 254 nm. The product was collected and lyophilized under reduced pressure to obtain compound **1** (20 mg).

MS (ESI, m/z): 634.2, 636.2 [M + H]⁺.

¹H NMR (300 MHz, CD₃OD) δ 8.82 (d, *J* = 2.1 Hz, 1H), 8.76-8.67 (m, 2H), 8.25 (s, 1H), 8.09 (s, 1H), 7.52 (d, *J=* 9.6 Hz, 1H), 6.89 (s, 1H), 6.66 (s, 1H), 3.92 (s, 3H), 3.69 (s, 3H), 3.30 (br s, 2H), 3.06 (br s, 5H), 2.17 (d, *J=* 14.4 Hz, 6H).

### Example 2:

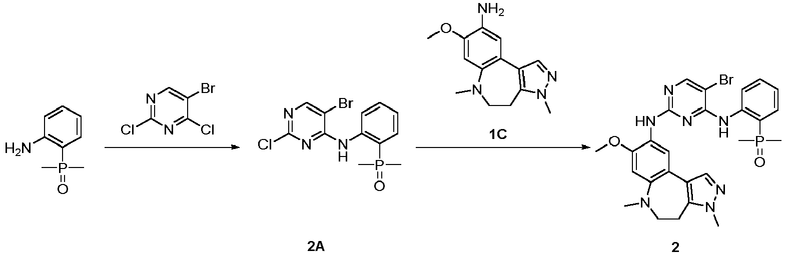

### Compound 2A:

5-Bromo-2,4-dichloropyrimidine (2 g, 8.8 mmol) was dissolved in *N*,*N*-dimethylformamide (30 mL), and anhydrous potassium carbonate (3.64 g, 26.3 mmol) and 2-(dimethylphosphinoxy)aniline (1.48 g, 8.8 mmol) were added successively. The reaction liquid was heated to 60 °C and successively stirred for 12 h. After the starting material disappeared as detected by LCMS, the reaction liquid was cooled to room temperature and quenched by addition of water (150 mL). The mixture was extracted with ethyl acetate (100 mL × 3), the organic phases were combined, washed with saturated brine (80 mL × 3) firstly, then dried over anhydrous sodium sulfate, filtered, and concentrated under reduced pressure, and the resulting residue was purified by silica gel column chromatography (eluent: dichloromethane/methanol = 10/1) to obtain compound **2A** (2.96 g).

MS (ESI) M/Z: 360.0, 362.0 [M + H]⁺.

### Compound 2:

Compound **2** (13 mg) was prepared by replacing the starting material with compound **2A** (100 mg, 0.28 mmol) according to the method for the preparation of compound **1** from compound **1B** and compound **1C** in Example 1.

MS (ESI, m/z): 582.3, 584.3 [M + H]⁺.

¹H NMR (400 MHz, DMSO-*d₆*) δ 10.96 (s, 1H), 8.38 (s, 1H), 8.26 (s, 1H), 8.16 (s, 1H), 7.71 (s, 1H), 7.57 (s, 1H), 7.50-7.45 (m, 1H), 7.08-6.99 (m, 2H), 6.58 (s, 1H), 3.77 (s, 3H), 3.75 (s, 3H), 3.19 (t, *J=* 5.2 Hz, 2H), 3.03-3.00 (m, 5H), 1.75 (d, *J=* 13.6 Hz, 6H).

### Example 3:

### Preparation of intermediate 3A

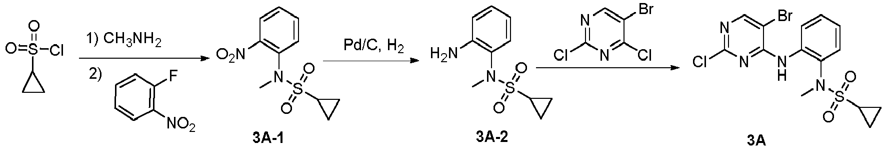

### Compound 3A-1:

A solution of methylamine in tetrahydrofuran (2 M, 106.7 mL, 213.4 mmol) and triethylamine (10.8 g, 106.7 mmol) were dissolved in dichloromethane (80 mL). Subsequently, cyclopropylsulfonyl chloride (10 g, 71.1 mmol) was added dropwise to the reaction liquid at 0 °C. The reaction system was warmed to room temperature and successively stirred for 24 h. After the starting material disappeared as detected by TLC, the reaction liquid was concentrated under reduced pressure. The resulting residue was dissolved in acetonitrile (100 mL). Subsequently, cesium carbonate (31.1 g, 95.4 mmol) and o-nitrofluorobenzene (15.5 g, 95.4 mmol) were added to the reaction liquid, and the reaction liquid was successively stirred at room temperature for 16 h. After the starting material disappeared as detected by LCMS, the reaction liquid was quenched by addition of water (100 mL). The mixture was extracted with ethyl acetate (100 mL × 3), and the organic phases were combined, washed with saturated brine (100 mL × 3) firstly, then dried over anhydrous sodium sulfate, filtered, and finally concentrated under reduced pressure. The resulting residue was purified by silica gel column chromatography (eluent: petroleum ether/ethyl acetate = 1/1) to obtain compound **3A-1** (14 g).

MS (ESI) M/Z: 257.0 [M + H]⁺.

### Compound 3A-2:

Compound **3A-1** (14 g, 55 mmol) was dissolved in a mixed solvent of ethanol (50 mL) and ethyl acetate (50 mL). Subsequently, wet palladium on carbon (10%, 600 mg) was added to the reaction liquid. After the reaction system was purged with hydrogen (1 atm) 3 times, the reaction liquid was stirred at room temperature for 16 h. After the starting material disappeared as detected by LCMS, the reaction liquid was filtered through celite, the filter cake was washed with ethyl acetate (30 mL × 3), and the resulting filtrate was concentrated under reduced pressure to obtain compound **3A-2** (12 g).

MS (ESI) M/Z: 227.1 [M + H]⁺.

### Intermediate 3A:

Compound **3A** (9 g) was prepared by replacing the starting material with compound **3A-2** (12 g, 53 mmol) according to the method for the preparation of compound **2A** in Example 2.

MS (ESI, m/z): 416.9, 418.9 [M + H]⁺.

### Preparation of compound 3

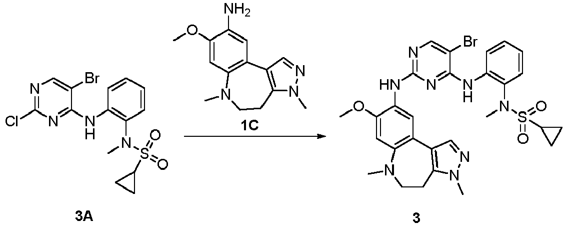

Compound **3** (96 mg) was prepared by replacing the starting material with compound **3A** (80 mg, 0.3 mmol) according to the method for the preparation of compound **1** from compound **1B** and compound **1C** in Example 1.

MS (ESI, m/z): 638.9, 640.9 [M + H]⁺.

¹H NMR (300 MHz, DMSO-*d₆*) δ 8.30-8.13 (m, 4H), 7.72 (s, 1H), 7.60-7.55 (m, 2H), 7.06-7.01 (m, 2H), 6.60 (s, 1H), 3.77 (s, 3H), 3.75 (s, 3H), 3.22-3.19 (m, 5H), 3.04-3.01 (m, 5H), 2.89-2.81 (m, 1H), 1.07-1.05 (m, 2H), 0.88 (s, 2H).

### Example 4:

### Preparation of intermediate 4A

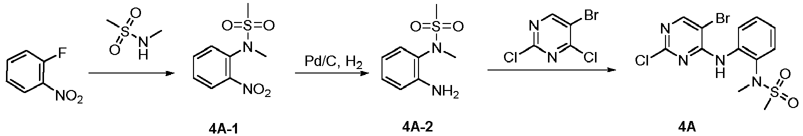

### Compound 4A-1:

Methylsulfonylmethylamine (34.8 g, 319 mmol) and o-fluoronitrobenzene (30 g, 212.6 mmol) were dissolved in acetonitrile (400 mL). Subsequently, cesium carbonate (138.6 g, 425 mmol) was added to the above reaction liquid, and the reaction liquid was successively stirred at room temperature for 16 h. After the starting material disappeared as detected by LCMS, the reaction liquid was quenched by addition of water (300 mL). The mixture was extracted with ethyl acetate (300 mL × 3), and the organic phases were combined, washed with saturated brine (150 mL × 3) firstly, then dried over anhydrous sodium sulfate, filtered, and finally concentrated under reduced pressure. The resulting residue was purified by silica gel column chromatography (eluent: petroleum ether/ethyl acetate = 2/1) to obtain compound **4A-1** (30 g).

¹H NMR (400 MHz, DMSO-*d₆*) δ 7.97-7.95 (m, 1H), 7.87-7.75 (m, 2H), 7.67-7.58 (m, 1H), 3.29 (s, 3H), 3.06 (s, 3H).

### Compound 4A-2:

Compound **4A-2** (12 g) was prepared by replacing the starting material with compound **4A-1** (15 g, 65 mmol) according to the method for the preparation of compound **3A-2** from compound **3A-1** in Example 3.

MS (ESI, m/z): 201.0 [M + H]⁺.

### Intermediate 4A:

Intermediate compound **4A** (3 g) was prepared by replacing the starting material with compound **4A-2** (5 g, 25 mmol) according to the method for the preparation of compound **2A** in Example 2. MS (ESI, m/z): 390.9, 392.9 [M + H]⁺.

### Preparation of compound 4

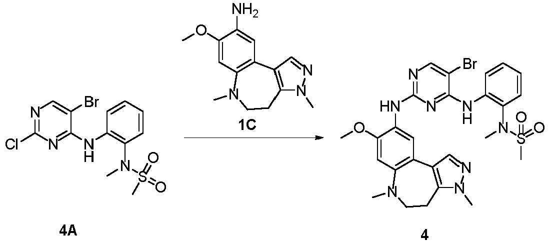

Compound **4** (25 mg) was prepared by replacing the starting material with compound **4A** (152 mg, 0.4 mmol) according to the method for the preparation of compound **1** from compound **1B** and compound **1C** in Example 1.

MS (ESI, m/z): 613.1, 615.1 [M + H]⁺.

¹H NMR (300 MHz, DMSO-*d₆*) δ 8.29-8.25 (m, 3H), 8.19 (s, 1H), 7.72 (s, 1H), 7.55-7.53 (m, 2H), 7.07-6.93 (m, 2H), 6.60 (s, 1H), 3.77 (s, 3H), 3.76 (s, 3H), 3.22-3.19 (m, 5H), 3.11 (s, 3H), 3.04-3.01 (m, 5H).

### Example 5:

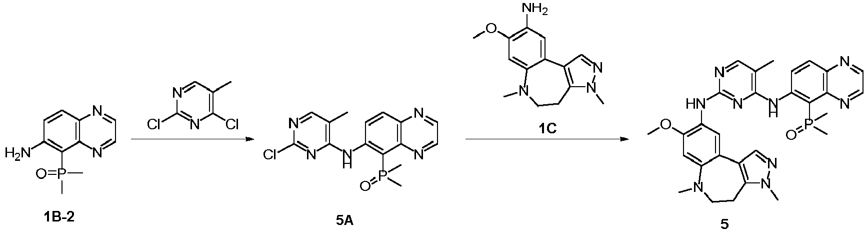

### Intermediate 5A:

Compound **5A** (4.2 g) was prepared by replacing the starting material with 2,4-dichloro-5-methylpyrimidine (5.53 g, 34 mmol) according to the method for the preparation of compound **1B** from compound **1B-2** in Example 1.

MS (ESI, m/z): 348.3, 350.3 [M + H]⁺.

### Compound 5:

Compound **5** (53 mg) was prepared by replacing the starting material with compound **5A** (80 mg, 0.23 mmol) according to the method for the preparation of compound 1 from compound **1B** and compound **1C** in Example 1.

MS (ESI, m/z): 570.3 [M + H]⁺.

¹H NMR (400 MHz, CDCl₃) δ 12.56 (s, 1H), 9.15 (dd, *J* = 9.6, 4.4 Hz, 1H), 8.67-8.66 (m, 2H), 8.31-8.23 (m, 2H), 7.87 (s, 1H), 7.59 (d, *J* = 9.2 Hz, 1H), 7.23 (s, 1H), 6.57 (s, 1H), 3.90 (s, 3H), 3.75 (s, 3H), 3.33 (t, *J=* 5.6 Hz, 2H), 3.08-3.05 (m, 5H), 2.28 (s, 3H), 2.13 (s, 3H), 2.09 (s, 3H).

### Example 6:

### Preparation of intermediate 6A

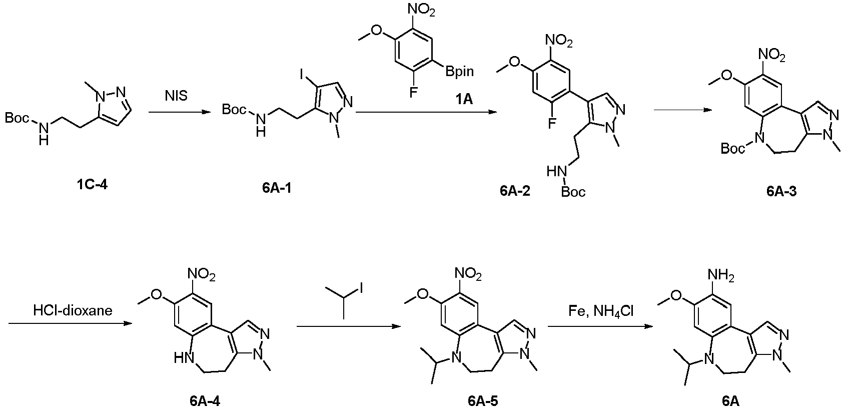

### Compound 6A-1:

Compound **6A-1** (3.57 g) was prepared by replacing the starting material with compound **1C-4** (3.5 g, 15.5 mmol) according to the method for the preparation of compound **1C-6** from compound **1C-5** in Example 1.

MS (ESI, m/z): 352.0 [M + H]⁺.

### Compound 6A-2:

Compound **6A-2** (2.8 g) was prepared by replacing the starting material with compound **6A-1** (3.4 g, 9.7 mmol) according to the method for the preparation of compound **1C-7** from compound **1C-6** in Example 1.

MS (ESI, m/z): 395.3 [M + H]⁺.

### Compound 6A-3:

Compound **6A-3** (1.2 g) was prepared by replacing the starting material with compound **6A-2** (2.7 g, 6.8 mmol) according to the method for the preparation of compound **1C-9** from compound **1C-8** in Example 1.

MS (ESI, m/z): 375.2 [M + H]⁺.

### Compound 6A-4:

Compound **6A-4** (0.84 g) was prepared by replacing the starting material with compound **6A-3** (1.2 g, 3.3 mmol) according to the method for the preparation of compound **1C-8** from compound **1C-**7 in Example 1.

MS (ESI, m/z): 275.0 [M + H]⁺.

### Compound 6A-5:

Compound **6A-4** (300 mg, 1.1 mmol) and cesium carbonate (1.07 g, 3.3 mmol) were dissolved in *N,N*-dimethylformamide (6 mL). Subsequently, 2-iodopropane (1.86 g, 10.9 mmol) was added to the above reaction liquid. The reaction system was heated to 80 °C and successively stirred for 16 h. After the starting material disappeared as detected by LCMS, the reaction liquid was cooled to room temperature and quenched by addition of water (30 mL). The mixture was extracted with ethyl acetate (50 mL × 3), and the organic phases were combined, washed with saturated brine (50 mL × 3) firstly, then dried over anhydrous sodium sulfate, filtered, and finally concentrated under reduced pressure. The resulting residue was purified by silica gel column chromatography (eluent: dichloromethane/methanol = 10/1) to obtain compound **6A-5** (75mg).

MS (ESI, m/z): 317.2 [M + H]⁺.

### Intermediate 6A:

Intermediate compound **6A** (48 mg) was prepared by replacing the starting material with compound **6A-5** (75 mg, 0.24 mmol) according to the method for the preparation of compound **1C** from compound **1C-9** in Example 1.

MS (ESI, m/z): 287.2 [M + H]⁺.

### Preparation of compound 6

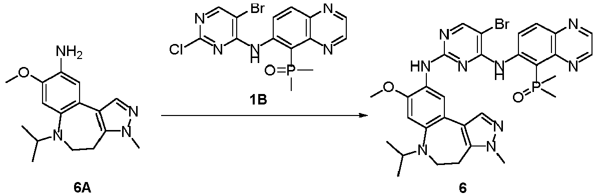

Compound **6** (19 mg) was prepared by replacing the starting material with compound **6A** (48 mg, 0.17 mmol) according to the method for the preparation of compound **1** from compound **1B** and compound **1C** in Example 1.

MS (ESI, m/z): 662.3, 664.3 [M + H]⁺.

¹H NMR (400 MHz, CDCl₃) δ 12.47 (s, 1H), 8.88 (dd, *J* = 9.2, 4.0 Hz, 1H), 8.69 (d, *J* = 2.0 Hz, 1H), 8.67 (d, *J* = 2.0 Hz, 1H), 8.37 (s, 1H), 8.27 (s, 1H), 7.63 (s, 1H), 7.35 (s, 1H), 7.00 (s, 1H), 6.56 (s, 1H), 3.88 (s, 4H), 3.70 (s, 3H), 3.35-3.27 (m, 2H), 3.00-2.91 (m, 2H), 2.15 (s, 3H), 2.12 (s, 3H), 1.31 (d, *J* = 6.4 Hz, 6H).

### Example 7:

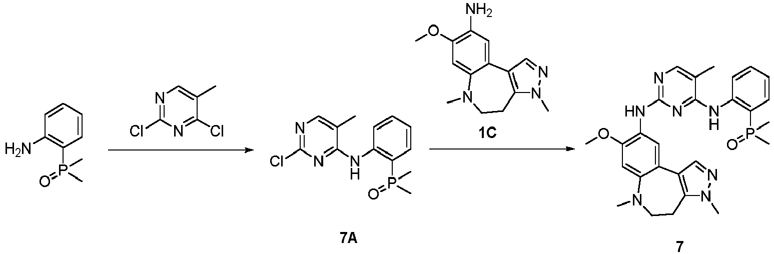

### Intermediate 7A:

Compound **7A** (300 mg) was prepared by replacing the starting material with 2,4-dichloro-5-methylpyrimidine (578 mg, 3.55 mmol) and 2-(dimethylphosphoryl)aniline (300 mg, 2.96 mmol) according to the method for the preparation of compound **1B** from compound **1B-2** in Example 1.

MS (ESI, m/z): 296.1, 298.1 [M + H]⁺.

### Compound 7:

Formate of compound **7** (111 mg) was prepared by replacing the starting material with compound **7A** (80 mg, 0.27 mmol) according to the method for the preparation of compound **1** from compound **1B** and compound **1C** in Example 1.

MS (ESI, m/z): 518.2 [M + H]⁺.

¹H NMR (400 MHz, CD₃OD) δ 8.48 (dd, *J* = 8.4, 4.4 Hz, 1H), 8.32 (s, 1H), 7.91 (s, 1H), 7.73 (s, 1H), 7.55-7.49 (m, 1H), 7.38 (s, 1H), 7.20-7.16 (m, 1H), 7.10-7.06 (m, 1H), 6.66 (s, 1H), 3.87 (s, 3H), 3.80 (s, 3H), 3.27 (t, *J* = 5.6 Hz, 2H), 3.06-3.03 (m, 5H), 2.17 (s, 3H), 1.89 (s, 3H), 1.85 (s, 3H).

### Example 8:

### Preparation of intermediate 8A

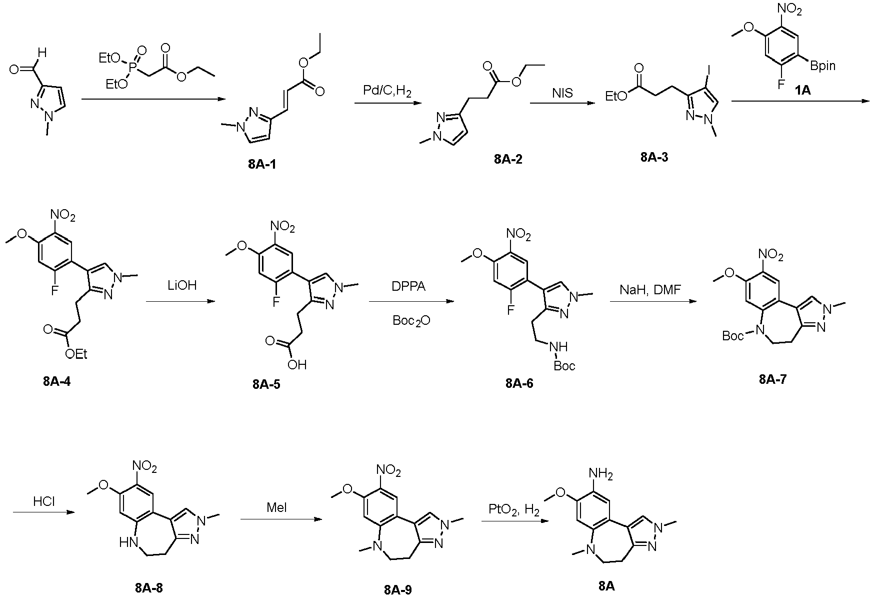

### Compound 8A-1:

Compound **8A-1** (16 g) was prepared by replacing the starting material with 1-methyl-1H-pyrazole-3-carbaldehyde (10 g, 90.8 mmol) according to the method for the preparation of compound **1C-1** in Example 1.

MS (ESI, m/z): 181.2 [M + H]⁺.

### Compound 8A-2:

Compound **8A-2** (16 g) was prepared by replacing the starting material with compound **8A-1** (16.8 g, 93.2 mmol) according to the method for the preparation of compound **1C-2** from compound **1C-1** in Example 1.

MS (ESI, m/z): 183.1 [M + H]⁺.

### Compound 8A-3:

Compound **8A-2** (3 g, 16.5 mmol) was dissolved in hexafluoroisopropanol (20 mL). Subsequently, N-iodosuccinimide (7.4 g, 32.9 mmol) was added to the above reaction liquid, and the reaction liquid was successively stirred at room temperature for 1 h. After the starting material disappeared as detected by LCMS, the reaction liquid was concentrated under reduced pressure. The resulting residue was dissolved in water (100 mL), the mixture was extracted with dichloromethane (3 × 100 mL), and the organic phases were combined, washed with saturated brine (150 mL × 3), then dried over anhydrous sodium sulfate, filtered and finally concentrated under reduced pressure. The resulting residue was purified by silica gel column chromatography (eluent: dichloromethane/methanol = 40/1) to obtain compound **8A-3** (4.5 g).

MS (ESI, m/z): 309.1 [M + H]⁺.

### Compound 8A-4:

Compound **8A-4** (470 mg) was prepared by replacing the starting material with compound **8A-3** (750 mg, 2.4 mmol) according to the method for the preparation of compound **1C-7** from compound **1C-6** in Example 1.

MS (ESI, m/z): 352.2 [M + H]⁺.

### Compound 8A-5:

Compound **8A-4** (380 mg, 1.1 mmol) was dissolved in a mixed solvent of tetrahydrofuran (10 mL) and water (2 mL). Subsequently, lithium hydroxide (130 mg, 5.4 mmol) was added to the above reaction liquid, and the reaction liquid was successively stirred at room temperature for 2 h. After the starting material disappeared as detected by LCMS, the reaction liquid was added with water (20 mL) for dilution and adjusted to pH = 2 with 1 N hydrochloric acid. The mixture was extracted with ethyl acetate (50 mL × 3), and the organic phases were combined, washed with saturated brine (50 mL × 3) firstly, then dried over anhydrous sodium sulfate, filtered, and finally concentrated under reduced pressure to obtain compound **8A-5** (330 mg).

MS (ESI, m/z): 324.1 [M + H]⁺.

### Compound 8A-6:

Compound **8A-6** (260 mg) was prepared by replacing the starting material with compound **8A-5** (350 mg, 1.1 mmol) according to the method for the preparation of compound **1C-4** from compound **1C-3** in Example 1.

MS (ESI, m/z): 395.1 [M + H]⁺.

### Compound 8A-7:

Compound **8A-6** (260 mg, 0.66 mmol) was dissolved in *N,N*-dimethylformamide (10 mL). Subsequently, sodium hydride (60%, 40 mg, 1 mmol) was added to the above reaction liquid at 0 °C. The reaction system was warmed to room temperature and successively stirred for 2 h. After the starting material disappeared as detected by LCMS, the reaction liquid was quenched by addition of water (30 mL). The mixture was extracted with ethyl acetate (50 mL × 3), and the organic phases were combined, washed with saturated brine (50 mL × 3) firstly, then dried over anhydrous sodium sulfate, filtered, and finally concentrated under reduced pressure. The resulting residue was purified by silica gel column chromatography (eluent: petroleum ether/ethyl acetate = 1/1) to obtain compound **8A-7** (230 mg).

MS (ESI, m/z): 375.2 [M + H]⁺.

### Compound 8A-8:

Compound **8A-8** (160 mg) was prepared by replacing the starting material with compound **8A-7** (240 mg, 0.64 mmol) according to the method for the preparation of compound **1C-8** from compound **1C-7** in Example 1.

MS (ESI, m/z): 275.1 [M + H]⁺.

### Compound 8A-9:

Compound **8A-8** (240 mg, 0.88 mmol) was dissolved in *N,N*-dimethylformamide (10 mL). Subsequently, sodium hydride (60%, 350 mg, 8.75 mmol) was added to the reaction liquid at 0 °C, and the reaction liquid was successively stirred at that temperature for 30 min, and added with iodomethane (1.2 g, 8.5 mmol). The reaction system was warmed to room temperature and successively stirred for 1 h. After the starting material disappeared as detected by LCMS, the reaction liquid was quenched by addition of water (30 mL). The mixture was extracted with ethyl acetate (50 mL × 3), and the organic phases were combined, washed with saturated brine (50 mL × 3) firstly, then dried over anhydrous sodium sulfate, filtered, and finally concentrated under reduced pressure. The resulting residue was purified by silica gel column chromatography (eluent: dichloromethane/methanol = 20/1) to obtain compound **8A-9** (140 mg).

MS (ESI, m/z): 289.1 [M + H]⁺.

### Intermediate 8A:

Compound **8A-9** (70 mg, 0.24 mmol) was dissolved in ethanol (5 mL). Subsequently, platinum dioxide (20 mg) was added to the reaction liquid. After the reaction system was purged with hydrogen 3 times, the reaction liquid was stirred at room temperature for 16 h. After the starting material disappeared as detected by LCMS, the reaction liquid was filtered through celite, the filter cake was washed with ethyl acetate (10 mL × 3), and the resulting filtrate was concentrated under reduced pressure to obtain compound **8A** (60 mg).

MS (ESI) M/Z: 259.1 [M + H]⁺.

### Preparation of compound 8

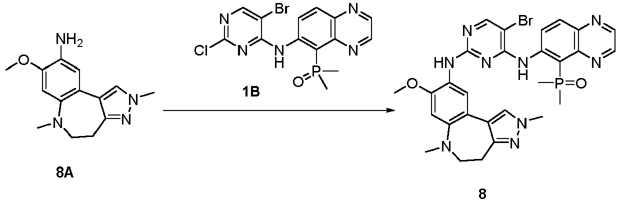

Compound **8** (37 mg) was prepared by replacing the starting material with compound **8A** (70 mg, 0.17 mmol) according to the method for the preparation of compound **1** from compound **1B** and compound **1C** in Example 1.

MS (ESI, m/z): 634.2, 636.2 [M + H]⁺.

¹H NMR (300 MHz, DMSO-*d₆*) δ 12.67 (s, 1H), 8.83-8.79 (m, 3H), 8.36 (s, 1H), 8.25 (s, 1H), 7.77 (s, 1H), 7.65 (s, 1H), 7.56 (s, 1H), 6.59 (s, 1H), 3.79 (s, 3H), 3.57 (s, 3H), 3.22-3.18 (m, 2H), 3.01 (s, 3H), 2.98-2.94 (m, 2H), 2.04 (s, 3H), 1.99 (s, 3H).

### Example 9:

### Preparation of intermediate 9A

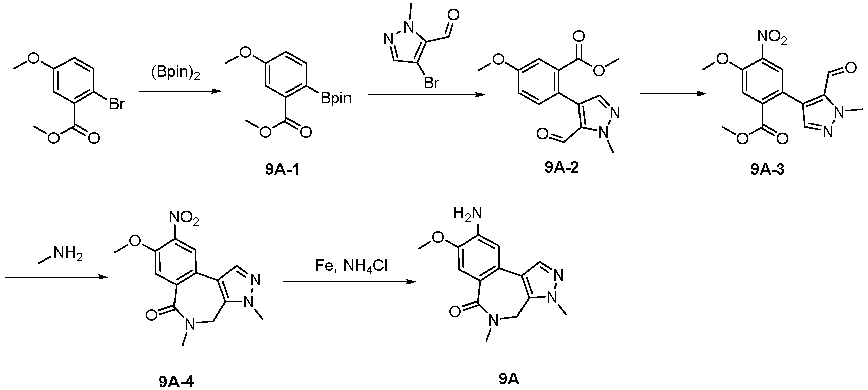

### Compound 9A-1:

Compound **9A-1** (10 g) was prepared by replacing the starting material with methyl 2-bromo-5-methoxybenzoate (10 g, 40.8 mmol) according to the method for the preparation of compound **1A** from compound **1A-1** in Example 1.

MS (ESI, m/z): 293.1 [M + H]⁺.

### Compound 9A-2:

Compound **9A-2** (4.7 g) was prepared by replacing the starting material with compound **9A-1** (10 g, 34.2 mmol) and 4-bromo-1-methyl-pyrazole-5-carbaldehyde (6.47 g, 34.2 mmol) according to the method for the preparation of compound **1C-7** from compound **1C-6** in Example 1.

MS (ESI, m/z): 275.1 [M + H]⁺.

### Compound 9A-3:

Compound **9A-2** (2.4 g, 8.75 mmol) was dissolved in concentrated sulfuric acid (25 mL) at 0 °C. Subsequently, potassium nitrate (973 mg, 9.63 mmol) was added to the reaction liquid in batches, and the reaction liquid was successively stirred at that temperature for 10 min. After the starting material disappeared as detected by TLC, the reaction liquid was slowly poured into ice water (100 g) to quench the reaction. The mixture was extracted with ethyl acetate (100 mL × 3), and the organic phases were combined, washed with saturated brine (100 mL × 3) firstly, then dried over anhydrous sodium sulfate, filtered, and finally concentrated under reduced pressure. The resulting residue was purified by silica gel column chromatography (eluent: petroleum ether/ethyl acetate = 1/2) to obtain compound **9A-3** (650 mg).

MS (ESI, m/z): 320.1 [M + H]⁺.

### Compound 9A-4:

Compound **9A-3** (1.4 g, 4.4 mmol) and a solution of methylamine in tetrahydrofuran (2 M, 8.77 mL, 17.54 mmol) were dissolved in methanol (10 mL). Subsequently, glacial acetic acid (276 mg, 4.6 mmol) was added to the above reaction liquid, and the reaction liquid was successively stirred at room temperature for 16 h. Sodium borohydride (332 mg, 8.77 mmol) was added to the above reaction liquid, and the reaction liquid was successively stirred at room temperature for 2 h. After the starting material disappeared as detected by LCMS, the reaction liquid was quenched by addition of water (50 mL). The mixture was extracted with ethyl acetate (50 mL × 3), and the organic phases were combined, washed with saturated brine (50 mL × 3) firstly, then dried over anhydrous sodium sulfate, filtered, and finally concentrated under reduced pressure. The resulting residue was purified by silica gel column chromatography (eluent: dichloromethane/methanol = 10/1) to obtain compound **9A-4** (540 mg).

MS (ESI, m/z): 303.1 [M + H]⁺.

### Intermediate 9A:

Compound **9A** (80 mg) was prepared by replacing the starting material with compound **9A-4** (100 mg, 0.33 mmol) according to the method for the preparation of compound **1C** from compound **1C-9** in Example 1.

MS (ESI, m/z): 273.1 [M + H]⁺.

### Preparation of compound 9

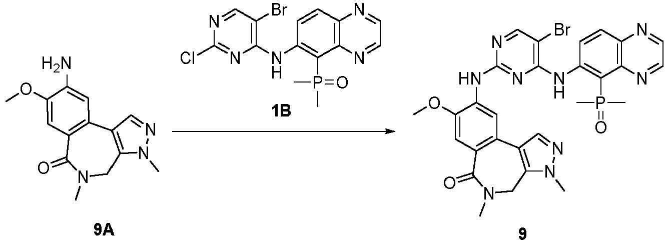

### Compound 9:

Compound **9** (25 mg) was prepared by replacing the starting material with compound **9A** (60 mg, 0.22 mmol) according to the method for the preparation of compound **1** from compound **1B** and compound **1C** in Example 1.

MS (ESI, m/z): 648.1, 650.1 [M + H]⁺.

¹H NMR (400 MHz, CDCl₃) δ 12.65 (s, 1H), 8.88 (dd, *J=* 9.2, 3.6 Hz, 1H), 8.74 (s, 2H), 8.43 (s, 1H), 8.34 (s, 1H), 7.85 (s, 1H), 7.57 (s, 2H), 6.98 (s, 1H), 4.34 (s, 2H), 3.99 (s, 3H), 3.93 (s, 3H), 3.26 (s, 3H), 2.17 (s, 3H), 2.13 (s, 3H).

### Example 10:

### Preparation of intermediate 10A

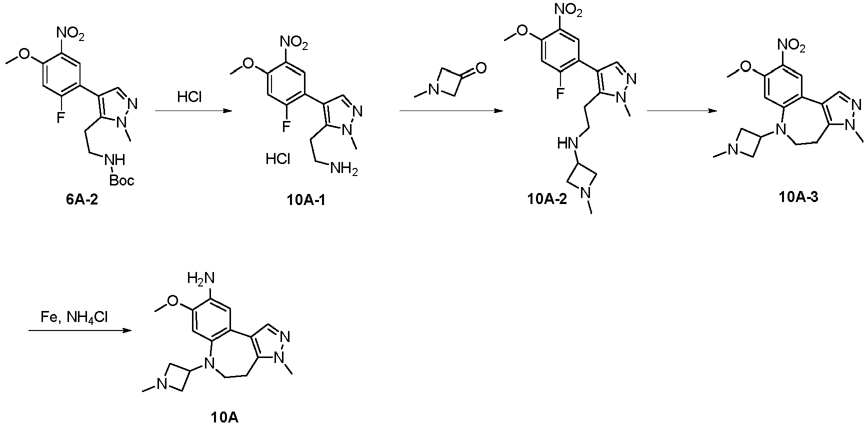

### Compound 10A-1:

Compound **10A-1** (260 mg) was prepared by replacing the starting material with compound **6A-2** (300 mg, 0.76 mmol) according to the method for the preparation of compound **1C-8** from compound **1C-7** in Example 1.

MS (ESI, m/z): 295.2 [M + H]⁺.

### Compound 10A-2:

Compound **10A-1** (370 mg, 1.12 mmol) and 1-methyl-3-azetidinone (95 mg, 1.12 mmol) were dissolved in dichloromethane (7 mL). Subsequently, triethylamine (453 mg, 4.48 mmol) and tetraisopropyl titanate (636 mg, 2.24 mmol) were added to the reaction liquid, and the reaction liquid was successively stirred at room temperature for 1 h. Sodium borohydride (85 mg, 2.24 mmol) was added to the above reaction liquid, and the reaction liquid was successively stirred at room temperature for 1 h. After the starting material disappeared as detected by LCMS, the reaction liquid was purified using a reverse phase C18 column. The purification conditions were as follows: a chromatography column: 40 g of C18 reverse phase column; mobile phase: water (containing 10 mM ammonium bicarbonate) and acetonitrile; flow rate: 35 mL/min; gradient: acetonitrile elevating from 5% to 40% in 20 min; detection wavelength: 254 nm. The product was collected and lyophilized under reduced pressure to obtain compound **10A-2** (70 mg).

MS (ESI) M/Z: 364.2 [M + H]⁺.

### Compound 10A-3:

Compound **10A-3** (50 mg) was prepared by replacing the starting material with compound **10A-2** (70 mg, 0.19 mmol) according to the method for the preparation of compound **1C-9** from compound **1C-8** in Example 1.

MS (ESI, m/z): 344.1 [M + H]⁺.

### Intermediate 10A:

Compound **10A** (50 mg) was prepared by replacing the starting material with compound **10A-3** (50 mg, 0.15mmol) according to the method for the preparation of compound **1C** from compound **1C-9** in Example 1.

MS (ESI, m/z): 314.2 [M + H]⁺.

### Preparation of compound 10

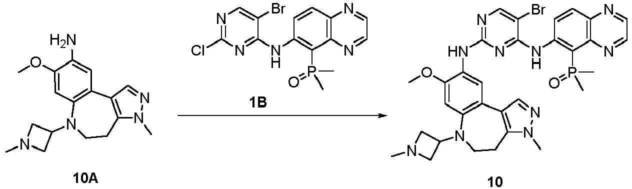

Compound **10** (8 mg) was prepared by replacing the starting material with compound **10A** (50 mg, 0.12 mmol) according to the method for the preparation of compound **1** from compound **1B** and compound **1C** in Example 1.

MS (ESI, m/z): 689.3, 691.3 [M + H]⁺.

¹H NMR (400 MHz, CD₃OD) δ 8.82 (d, *J* = 2.0 Hz, 1H), 8.73 (d, *J* = 2.0 Hz, 1H), 8.66-8.63 (m, 1H), 8.52 (br s, 1H), 8.25 (s, 1H), 8.18 (s, 1H), 7.47 (d, *J* = 9.6 Hz, 1H), 6.84 (s, 1H), 6.24 (s, 1H), 4.57-4.54 (m, 1H), 4.32-4.28 (m, 2H), 3.89 (s, 3H), 3.85-3.82 (m, 2H), 3.68 (s, 3H), 3.31-3.30 (m, 2H), 3.13-3.10 (m, 2H), 2.80 (s, 3H), 2.17 (s, 3H), 2.13 (s, 3H).

### Example 11:

### Preparation of intermediate 11A

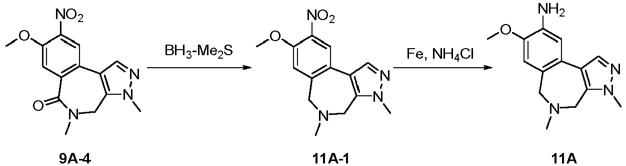

### Compound 11A-1:

Compound **9A-4** (100 mg, 0.33 mmol) was dissolved in tetrahydrofuran (2 mL) under nitrogen atmosphere. Subsequently, borane-dimethyl sulfide solution (251 mg, 3.31 mmol) was added to the above reaction liquid, and the reaction liquid was successively stirred at room temperature for 16 h. After the starting material disappeared as detected by LCMS, the reaction liquid was quenched by addition of methanol (5 mL). The mixture was heated to 60 °C, successively stirred for 1 h, and cooled to 0 °C; a white solid was precipitated, filtered and dried to obtain compound **11A-1** (60 mg).

MS (ESI, m/z): 289.1 [M + H]⁺.

### Intermediate 11A:

Compound **11A** (100 mg) was prepared by replacing the starting material with compound **11A-1** (125 mg, 0.43 mmol) according to the method for the preparation of compound **1C** from compound **1C-9** in Example 1.

MS (ESI, m/z): 259.1 [M + H]⁺.

### Preparation of compound 11

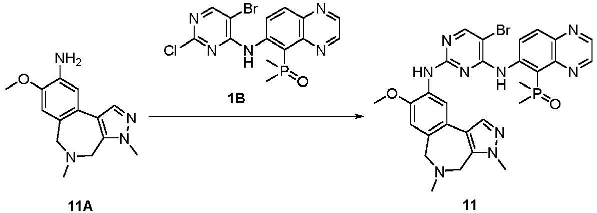

Compound **11** (20 mg) was prepared by replacing the starting material with compound **11A** (90 mg, 0.35 mmol) according to the method for the preparation of compound **1** from compound **1B** and compound **1C** in Example 1.

MS (ESI, m/z): 634.2, 636.2 [M + H]⁺.

¹H NMR (300 MHz, CDCl₃) δ 12.55 (s, 1H), 8.88 (dd, *J* = 9.6, 4.2 Hz, 1H), 8.74-8.72 (m, 2H), 8.52 (s, 1H), 8.34 (s, 1H), 7.64 (d, *J* = 9.3 Hz, 1H), 7.56 (s, 1H), 7.00 (s, 1H), 6.74 (s, 1H), 4.16 (s, 2H), 3.94 (s, 3H), 3.93 (s, 2H), 3.75 (s, 3H), 2.55 (s, 3H), 2.19 (s, 3H), 2.14 (s, 3H).

### Example 12:

### Preparation of intermediate 12A

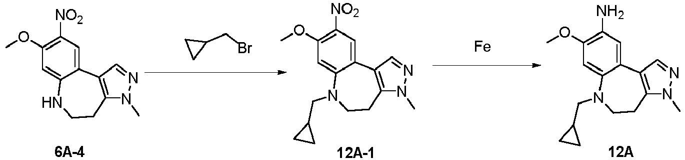

### Compound 12A-1:

Compound **12A-1** (160 mg) was prepared by replacing the starting material with compound **6A-4** (150 mg, 0.55 mmol) and (bromomethyl)cyclopropane (736 mg, 5.45 mmol) according to the method for the preparation of compound **8A-9** from compound **8A-8** in Example 8.

MS (ESI, m/z): 329.2 [M + H]⁺.

### Intermediate 12A:

Compound **12A** (70 mg) was prepared by replacing the starting material with compound **12A-1** (80 mg, 0.24 mmol) according to the method for the preparation of compound **1C** from intermediate **1C-9** in Example 1.

MS (ESI, m/z): 299.1 [M + H]⁺.

### Preparation of compound 12

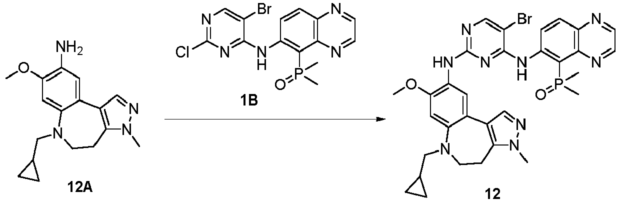

Compound **12** (30 mg) was prepared by replacing the starting material with compound **12A** (70 mg, 0.24 mmol) according to the method for the preparation of compound **1** from compound **1B** and compound **1C** in Example 1.

MS (ESI, m/z): 674.3, 676.3 [M + H]⁺.

¹H NMR (300 MHz, CDCl₃) δ 12.51 (s, 1H), 8.89 (dd, *J* = 9.6, 3.9 Hz, 1H), 8.71-8.68 (m, 2H), 8.37 (s, 1H), 8.27 (s, 1H), 7.66-7.63 (m, 1H), 7.36 (s, 1H), 7.04 (s, 1H), 6.56 (s, 1H), 3.90 (s, 3H), 3.74 (s, 3H), 3.48-3.45 (m, 2H), 3.17 (d, *J* = 6.3 Hz, 2H), 3.07-3.04 (m, 2H), 2.17 (s, 3H), 2.12 (s, 3H), 1.13-1.07 (m, 1H), 0.67-0.61 (m, 2H), 0.30-0.27 (m, 2H).

### Example 13:

### Preparation of intermediate 13A

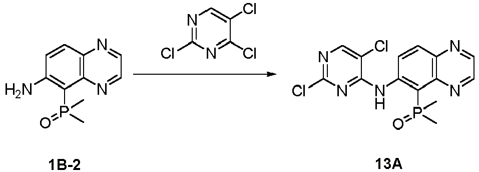

Compound **13A** (4.5 g) was prepared by replacing the starting material with 2,4,5-trichloropyrimidine (5.37 g, 29.3 mmol) according to the method for the preparation of compound **1B** from compound **1B-2** in Example 1.

MS (ESI, m/z): 367.9, 369.9 [M + H]⁺.

### Preparation of compound 13

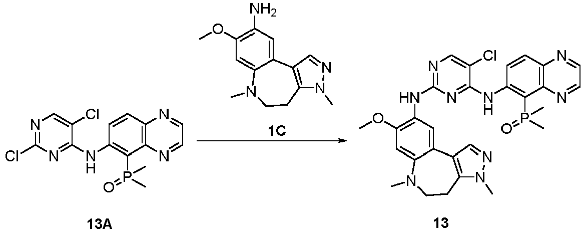

Compound **13** (73 mg) was prepared by replacing the starting material with compound **13A** (100 mg, 0.27 mmol) according to the method for the preparation of compound **1** from compound **1B** and compound **1C** in Example 1.

MS (ESI, m/z): 590.3, 592.3 [M + H]⁺.

¹H NMR (300 MHz, DMSO-*d₆*) δ 12.84 (s, 1H), 8.96 (s, 1H), 8.82 (dd, *J* = 11.4, 1.8 Hz, 2H), 8.42 (s, 1H), 8.19 (s, 1H), 7.72 (s, 1H), 7.59 (s, 1H), 7.52 (s, 1H), 6.65 (s, 1H), 3.80 (s, 3H), 3.73 (s, 3H), 3.24-3.20 (m, 2H), 3.04 (br s, 5H), 2.04 (s, 3H), 1.99 (s, 3H).

### Example 14:

### Preparation of compound 14

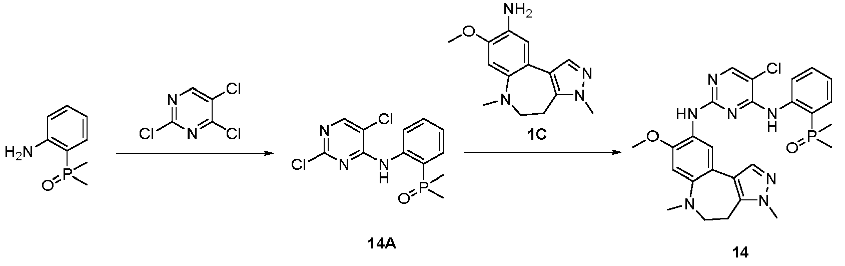

### Compound 14A

Compound **14A** (14.4 g) was prepared by replacing the starting material with 2,4,5-trichloropyrimidine (13 g, 71 mmol) and 2-(dimethylphosphoryl)aniline (10 g, 59 mmol) according to the method for the preparation of compound **2A** in Example 2.

MS (ESI, m/z): 316.0, 318.0 [M + H]⁺.

### Compound 14:

Compound **14** (80 mg) was prepared by replacing the starting material with compound **14A** (80 mg, 0.25 mmol) according to the method for the preparation of compound **1** from compound **1B** and compound **1C** in Example 1.

MS (ESI, m/z): 538.3, 540.3 [M + H]⁺.

¹H NMR (300 MHz, CDCl₃) δ 10.88 (s, 1H), 8.62 (dd, *J* = 8.4, 4.5 Hz, 1H), 8.43 (s, 1H), 8.13 (s, 1H), 7.38 (s, 1H), 7.32-7.17 (m, 3H), 7.02-6.97 (m, 1H), 6.57 (s, 1H), 3.92 (s, 3H), 3.84 (s, 3H), 3.32-3.28 (m, 2H), 3.06 (br s, 5H), 1.88 (s, 3H), 1.84 (s, 3H).

### Example 15:

### Preparation of intermediate 15A

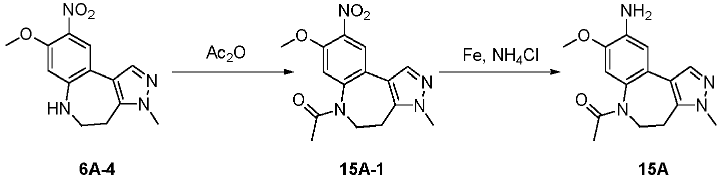

### Compound 15A-1:

Compound **6A-4** (100 mg, 0.37 mmol) was dissolved in acetic anhydride (1.5 mL). The reaction liquid was heated to 140 °C and successively stirred for 1 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound **15A-1** (90 mg).

MS (ESI, m/z): 317.2 [M + H]⁺.

### Intermediate 15A:

Compound **15A** (66 mg) was prepared by replacing the starting material with compound **15A-1** (80 mg, 0.25 mmol) according to the method for the preparation of compound **1C** from intermediate **1C-9** in Example 1.

MS (ESI, m/z): 287.1 [M + H]⁺.

### Preparation of compound 15

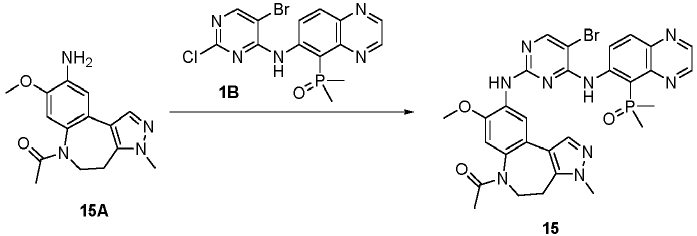

Compound **15** (17 mg) was prepared by replacing the starting material with compound **15A** (50 mg, 0.17 mmol) according to the method for the preparation of compound **1** from compound **1B** and compound **1C** in Example 1.

MS (ESI, m/z): 662.2, 664.2 [M + H]⁺.

¹H NMR (300 MHz, CDCl₃) δ 12.62 (s, 1H), 8.86 (dd, *J* = 9.6, 4.2 Hz, 1H), 8.75-8.73 (m, 2H), 8.61 (s, 1H), 8.36 (s, 1H), 7.70-7.63 (m, 2H), 6.99 (s, 1H), 6.73 (s, 1H), 4.85 (dd, *J* = 13.2, 5.7 Hz, 1H), 3.94 (s, 3H), 3.71 (s, 3H), 3.53-3.41 (m, 1H), 3.13-3.03 (m, 1H), 2.94-2.89 (m, 1H), 2.21-2.14 (m, 6H), 1.99 (s, 3H).

### Example 16:

### Preparation of intermediate 16A

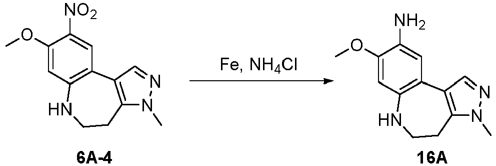

Compound **16A** (70 mg) was prepared by replacing the starting material with compound **6A-4** (100 mg, 0.25 mmol) according to the method for the preparation of compound **1C** from intermediate **1C-9** in Example 1.

MS (ESI, m/z): 245.2 [M + H]⁺.

### Preparation of compound 16

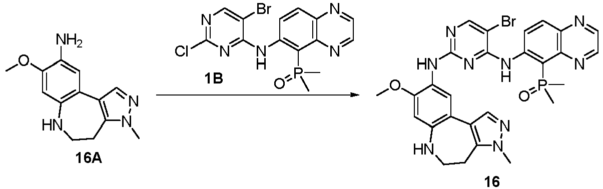

Compound **16** (13 mg) was prepared by replacing the starting material with compound **16A** (60 mg, 0.25 mmol) according to the method for the preparation of compound **1** from compound **1B** and compound **1C** in Example 1.

MS (ESI, m/z): 620.2, 622.2 [M + H]⁺.

¹H NMR (300 MHz, DMSO-*d₆*) δ 12.65 (s, 1H), 8.84-8.79 (m, 3H), 8.34 (s, 1H), 8.24 (s, 1H), 7.63 (s, 2H), 7.53 (s, 1H), 6.58 (s, 1H), 5.93 (s, 1H), 3.71 (s, 3H), 3.70 (s, 3H), 3.28-3.23 (m, 2H), 2.98-2.95 (m, 2H), 2.03 (s, 3H), 1.99 (s, 3H).

### Example 17:

### Preparation of compound 17

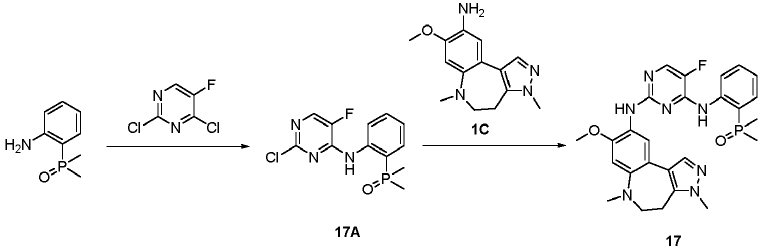

### Compound 17A:

Compound **17A** (1.9 g) was prepared by replacing the starting material with 2,4-dichloro-5-fluoropyrimidine (2 g, 11.98 mmol) and 2-(dimethylphosphoryl)aniline (2.03 g, 11.98 mmol) according to the method for the preparation of compound **2A** in Example 2.

MS (ESI, m/z): 300.0, 302.0 [M + H]⁺.

### Compound 17:

Compound **17** (21 mg) was prepared by replacing the starting material with compound **17A** (60 mg, 0.2 mmol) according to the method for the preparation of compound **1** from compound **1B** and compound **1C** in Example 1.

MS (ESI, m/z): 522.3 [M + H]⁺.

¹H NMR (300 MHz, CDCl₃) δ 11.41 (s, 1H), 8.82-8.76 (m, 1H), 8.44 (s, 1H), 8.00 (s, 1H), 7.56 (s, 1H), 7.34-7.20 (s, 3H), 7.00 (s, 1H), 6.59 (s, 1H), 3.92 (s, 3H), 3.84 (s, 3H), 3.32-3.27 (m, 2H), 3.07 (br s, 5H), 1.86 (m, 6H).

¹⁹F NMR (282 MHz, CDCl₃) δ -164.46.

### Example 18:

### Preparation of intermediate 18A

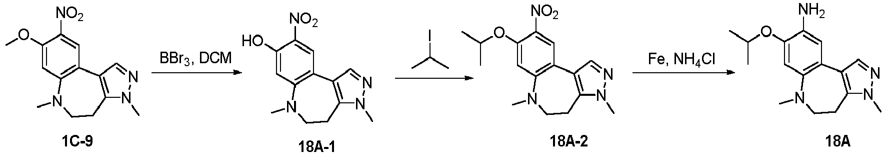

### Compound 18A-1

Compound **1C-9** (100 mg, 0.35 mmol) was dissolved in dichloromethane (2 mL) under nitrogen atmosphere at 0 °C. Subsequently, boron tribromide (174 mg, 0.69 mmol) was added to the above reaction liquid. The reaction system was warmed to room temperature and successively stirred for 16 h. After the starting material disappeared as detected by LCMS, the reaction liquid was quenched by addition of water (10 mL). The mixture was extracted with ethyl acetate (10 mL × 3), and the organic phases were combined, washed with saturated brine (30 mL × 3) firstly, then dried over anhydrous sodium sulfate, filtered, and finally concentrated under reduced pressure. The resulting residue was purified by silica gel column chromatography (eluent: dichloromethane/methanol = 20/1) to obtain compound **8A-1** (50 mg).

MS (ESI, m/z): 275.1 [M + H]⁺.

### Compound 18A-2:

Compound **18A-1** (75 mg, 0.27 mmol) and potassium carbonate (113 mg, 0.82 mmol) were dissolved in *N,N*-dimethylformamide (20 mL). 2-Iodopropane (139 mg, 0.82 mmol) was added to the reaction liquid at 0 °C. The reaction system was heated to 60 °C and successively stirred for 16 h. After the starting material disappeared as detected by LCMS, the reaction liquid was cooled to room temperature and quenched by addition of water (10 mL). The mixture was extracted with ethyl acetate (20 mL × 3), and the organic phases were combined, washed with saturated brine (30 mL × 3) firstly, then dried over anhydrous sodium sulfate, filtered, and finally concentrated under reduced pressure. The resulting residue was purified by silica gel column chromatography (eluent: dichloromethane/methanol = 20/1) to obtain compound **18A-2** (70 mg).

MS (ESI, m/z): 317.1 [M + H]⁺.

### Intermediate 18A:

Compound **18A** (55 mg) was prepared by replacing the starting material with compound **18A-2** (70 mg, 0.22 mmol) according to the method for the preparation of compound **1C** from intermediate **1C-9** in Example 1.

MS (ESI, m/z): 287.1 [M + H]⁺.

### Preparation of compound 18

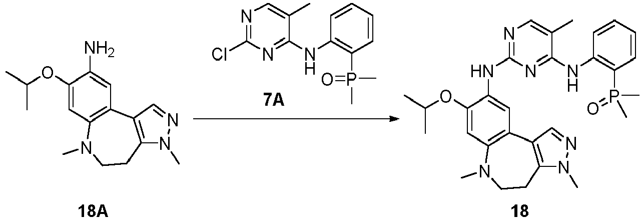

Compound **18** (20 mg) was prepared by replacing the starting material with compound **18A** (50 mg, 0.18 mmol) and compound **7A** (52 mg, 0.18 mmol) according to the method for the preparation of compound **1** from compound **1B** and compound **1C** in Example 1.

MS (ESI, m/z): 546.0 [M + H]⁺.

¹H NMR (300 MHz, CDCl₃) δ 10.49 (s, 1H), 8.74 (dd, *J* = 8.7, 4.5 Hz, 1H), 8.48 (s, 1H), 7.92 (s, 1H), 7.52 (s, 1H), 7.37 (s, 1H), 7.26-7.19 (m, 1H), 7.16-7.10 (m, 1H), 6.96-6.90 (m, 1H), 6.59 (s, 1H), 4.62-4.54 (m, 1H), 3.83 (s, 3H), 3.29 (t, *J=* 5.4 Hz, 2H), 3.04 (t, *J=* 5.7 Hz, 5H), 2.22 (s, 3H), 1.87 (s, 3H), 1.83 (s, 3H), 1.41 (s, 3H), 1.39 (s, 3H).

### Example 19:

### Preparation of intermediate 19A

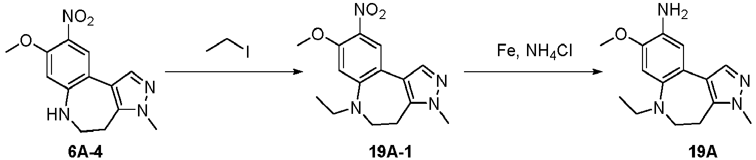

### Compound 19A-1:

Compound **19A-1** (150 mg) was prepared by replacing the starting material with compound **6A-4** (200 mg, 0.73 mmol) and iodoethane (341 mg, 2.19 mmol) according to the method for the preparation of compound **8A-9** from compound **8A-8** in Example 8.

MS (ESI, m/z): 303.2 [M + H]⁺.

### Intermediate 19A:

Compound **19A** (100 mg) was prepared by replacing the starting material with compound **19A-1** (150 mg, 0.5 mmol) according to the method for the preparation of compound **1C** from intermediate **1C-9** in Example 1.

MS (ESI, m/z): 273.1 [M + H]⁺.

### Preparation of compound 19

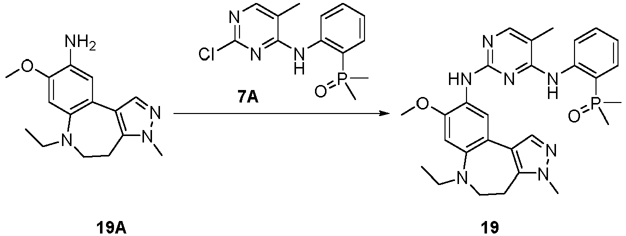

Compound **19** (33 mg) was prepared by replacing the starting material with compound **19A** (60 mg, 0.22 mmol) and compound **7A** (65 mg, 0.22 mmol) according to the method for the preparation of compound **1** from compound **1B** and compound **1C** in Example 1.

MS (ESI, m/z): 532.2 [M + H]⁺.

¹H NMR (300 MHz, CDCl₃) δ 10.49 (s, 1H), 8.74 (dd, *J=* 8.7, 4.5 Hz, 1H), 8.45 (s, 1H), 7.92 (s, 1H), 7.50 (s, 1H), 7.44 (s, 1H), 7.25-7.14 (m, 1H), 6.97-6.92 (m, 2H), 6.57 (s, 1H), 3.89 (s, 3H), 3.83 (s, 3H), 3.39-3.29 (m, 4H), 3.01-2.98 (m, 2H), 2.22 (s, 3H), 1.87 (s, 3H), 1.82 (s, 3H), 1.31 (t, *J* = 6.9 Hz, 3H).

### Example 20:

### Preparation of compound 20

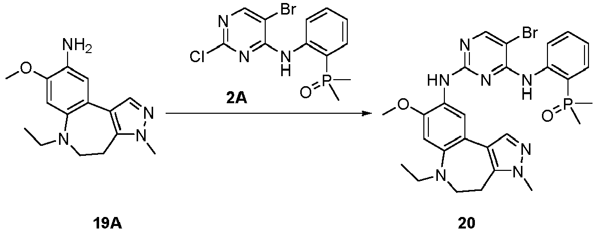

Compound 20 (33 mg) was prepared by replacing the starting material with compound **19A** (60 mg, 0.22 mmol) and compound **2A** (79 mg, 0.22 mmol) according to the method for the preparation of compound **1** from compound **1B** and compound **1C** in Example 1.

MS (ESI, m/z): 596.2, 598.2 [M + H]⁺.

¹H NMR (300 MHz, CDCl₃) δ 10.61 (s, 1H), 8.50 (dd, *J* = 8.6, 4.5 Hz, 1H), 8.41 (s, 1H), 8.23 (s, 1H), 7.33-7.20 (m, 4H), 7.03-6.97 (m, 1H), 6.57 (s, 1H), 3.89 (s, 3H), 3.83 (s, 3H), 3.39-3.32 (m, 4H), 3.01-2.97 (m, 2H), 1.88 (s, 3H), 1.84 (s, 3H), 1.31 (t, *J* = 7.2 Hz, 3H).

### Example 21:

### Preparation of intermediate 21A

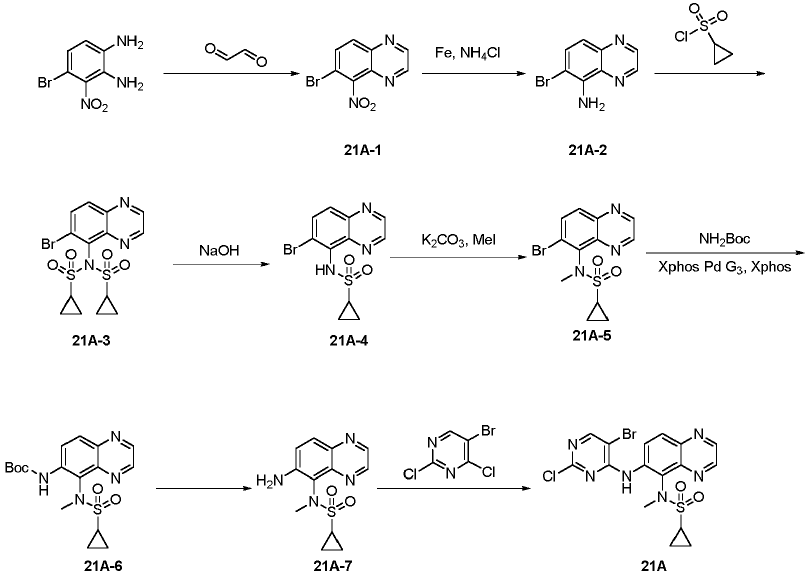

### Compound 21A-1:

4-Bromo-3-nitro-1,2-phenylenediamine (10 g, 43 mmol) and aqueous glyoxal (40%, 15.01 g, 103 mmol) were dissolved in water (400 mL). The reaction system was heated to 100 °C and successively stirred for 4 h. After the starting material disappeared as detected by TLC, the reaction liquid was cooled to room temperature. The precipitated solid was filtered, washed with water (20 mL × 3), and dried to obtain crude compound **21A-1** (13.4 g).

¹H NMR (300 MHz, DMSO-*d₆*) δ 8.16 (d, *J* = 1.8 Hz, 1H), 9.11 (d, *J* = 2.1 Hz, 1H), 8.30 (d, *J* = 5.4 Hz, 2H).

### Compound 21A-2:

Compound **21A-2** (5.3 g) was prepared by replacing the starting material with compound **21A-1** (11 g, 43.3 mmol) according to the method for the preparation of compound **1C** from intermediate **1C-9** in Example 1.

MS (ESI, m/z): 223.8, 225.8 [M + H]⁺.

### Compound 21A-3:

Compound **21A-2** (3.7 g, 17 mmol) was dissolved in *N*,*N*-dimethylformamide (50 mL) under nitrogen atmosphere. Subsequently, sodium hydride (60%, 2.6 g, 66 mmol) was added to the reaction liquid at 0 °C, and the reaction liquid was successively stirred for 30 min, and added dropwise with cyclopropylsulfonyl chloride (7 g, 50 mmol). The reaction system was warmed to room temperature and successively stirred for 2 h. After the starting material disappeared as detected by LCMS, the reaction liquid was quenched by addition of saturated aqueous ammonium chloride (50 mL). The mixture was extracted with ethyl acetate (100 mL × 3), and the organic phases were combined, washed with saturated brine (80 mL × 3) firstly, then dried over anhydrous sodium sulfate, filtered, and finally concentrated under reduced pressure; the resulting residue was purified by silica gel column chromatography (eluent: petroleum ether/ethyl acetate = 1/1) to obtain compound **21A-3** (1.5 g).

MS (ESI, m/z): 431.8, 433.8 [M + H]⁺.

### Compound 21A-4:

Compound **21A-3** (2.2 g, 5.1 mmol) was dissolved in methanol (25 mL), followed by addition of sodium hydroxide (8.1 g, 102 mmol) to the reaction liquid. The reaction system was heated to 90 °C and successively stirred for 2 h. After the starting material disappeared as detected by LCMS, the reaction liquid was concentrated under reduced pressure, added with water (50 mL) for dilution and adjusted to pH = 6 with concentrated hydrochloric acid. The mixture was extracted with chloroform/isopropanol (3/1, 80 mL × 3), and the organic phases were combined, washed with saturated brine (80 mL × 3) firstly, then dried over anhydrous sodium sulfate, filtered, and finally concentrated under reduced pressure; the resulting residue was purified by silica gel column chromatography (eluent: petroleum ether/ethyl acetate = 1/1) to obtain compound **21A-4** (0.94 g). MS (ESI, m/z): 327.9, 329.9 [M + H]⁺.

### Compound 21A-5:

Compound **21A-4** (930 mg, 2.8 mmol) and anhydrous potassium carbonate (789 mg, 5.7 mmol) were dissolved in *N*,*N*-dimethylformamide (10 mL). Subsequently, iodomethane (603 mg, 4.3 mmol) was added to the above reaction liquid at 0 °C. The reaction system was warmed to room temperature and successively stirred for 2 h. After the starting material disappeared as detected by LCMS, the reaction liquid was quenched by addition of water (50 mL). The mixture was extracted with ethyl acetate (80 mL × 3), and the organic phases were combined, washed with saturated brine (80 mL × 3) firstly, then dried over anhydrous sodium sulfate, filtered, and finally concentrated under reduced pressure; the resulting residue was purified by silica gel column chromatography (eluent: petroleum ether/ethyl acetate = 1/1) to obtain compound **21A-5** (930 mg).

MS (ESI, m/z): 341.9, 343.9 [M + H]⁺.

### Compound 21A-6:

Compound **21A-5** (950 mg, 2.8 mmol), *tert*-butyl carbamate (488 mg, 4.2 mmol), cesium carbonate (1.8 g, 5.6 mmol), Methanesulfonato(2-dicyclohexylphosphino-2',4',6'-triisopropyl-1,1'-biphenyl)(2'-amino-1,1'-biphenyl-2-yl)palladium(II) (235 mg, 0.28 mmol), and 2-dicyclohexylphosphonium-2,4,6-triisopropylbiphenyl (132 mg, 0.28 mmol) were dissolved in 1,4-dioxane (10 mL) under nitrogen atmosphere. The reaction system was heated to 90 °C and successively stirred for 4 h. After the starting material disappeared as detected by LCMS, the reaction liquid was cooled to room temperature and concentrated under reduced pressure. The resulting residue was purified by silica gel column chromatography (eluent: petroleum ether/ethyl acetate = 1/1) to obtain compound **21A-6** (810 mg).

MS (ESI, m/z): 379.0 [M + H]⁺.

### Compound 21A-7:

Compound **21A-6** (810 mg, 2.1 mmol) was dissolved in a solution of hydrogen chloride in ethyl acetate (4 M, 10 mL), and the reaction liquid was successively stirred at room temperature for 2 h. After the starting material disappeared as detected by LCMS, the reaction liquid was concentrated under reduced pressure, added with water (10 mL) for dilution and adjusted to pH = 8 with saturated aqueous sodium bicarbonate solution. The mixture was extracted with ethyl acetate (50 mL × 3), and the organic phases were combined, washed with saturated brine (80 mL × 3) firstly, then dried over anhydrous sodium sulfate, filtered, and finally concentrated under reduced pressure to obtain crude compound **21A-7** (660 mg).

MS (ESI, m/z): 279.2 [M + H]⁺.

### Intermediate 21A:

Compound **21A** (700 mg) was prepared by replacing the starting material with compound **21A-7** (620 mg, 2.2 mmol) according to the method for the preparation of compound **1B** from compound **1B-2** in Example 1.

MS (ESI, m/z): 468.9, 470.9 [M + H]⁺.

### Preparation of compound 21

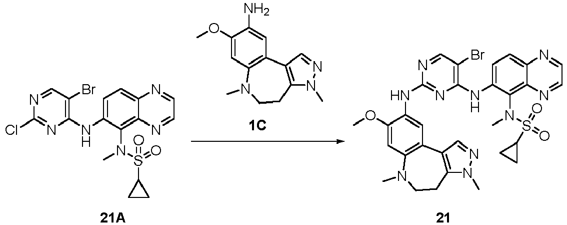

Compound **21** (29 mg) was prepared by replacing the starting material with compound **21A** (110 mg, 0.23 mmol) according to the method for the preparation of compound **1** from compound **1B** and compound **1C** in Example 1.

MS (ESI, m/z): 691.3, 693.3 [M + H]⁺.

¹H NMR (400 MHz, CDCl₃) δ 9.35 (s, 1H), 8.85 (d, *J* = 1.6 Hz, 1H), 8.79 (d, *J* = 1.6 Hz, 1H), 8.43 (d, *J* = 9.2 Hz, 1H), 8.23-7.93 (m, 2H), 7.58 (d, *J* = 9.2 Hz, 1H), 7.02 (s, 1H), 3.93 (s, 3H), 3.74 (s, 3H), 3.61-3.46 (m, 4H), 3.19-2.98 (m, 4H), 2.93-2.89 (m, 2H), 2.79-2.71 (m, 1H), 1.37-1.29 (m, 1H), 1.17-1.08 (m, 1H), 0.83-0.75 (m, 1H), 0.68-0.58 (m, 1H).

### Example 22:

### Preparation of compound 22

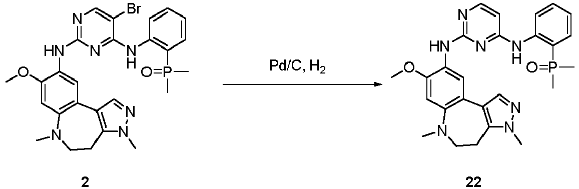

Compound 2 (70 mg, 0.12 mmol) was dissolved in methanol (6 mL). Palladium on carbon (10%, 25 mg) was added to the reaction liquid. After being purged with hydrogen 3 times, the reaction system was successively stirred at room temperature for 3 h. After the starting material disappeared as detected by LCMS, palladium on carbon was filtered off, the filtrate was concentrated under reduced pressure, and the resulting residue was purified by silica gel column chromatography (eluent: dichloromethane/methanol = 10/1) to obtain compound **22** (27 mg).

MS (ESI, m/z): 504.3 [M + H]⁺.

¹H NMR (300 MHz, CDCl₃) δ 10.91 (s, 1H), 8.65-8.44 (m, 2H), 8.30 (s, 1H), 7.96 (d, *J=* 6.3 Hz, 1H), 7.54 (s, 1H), 7.25-7.17 (m, 1H), 7.13-7.08 (m, 1H), 6.98-6.92 (m, 1H), 6.58 (s, 1H), 6.16 (d, *J=* 6.0 Hz, 1H), 3.91 (s, 3H), 3.84 (s, 3H), 3.33-3.29 (m, 2H), 3.07 (s, 3H), 3.06-3.04 (m, 2H), 1.86 (s, 3H), 1.82 (s, 3H).

### Example 23:

### Preparation of intermediate 23A

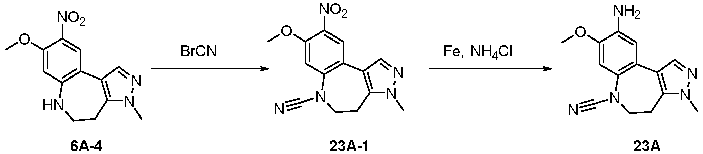

### Compound 23A-1:

Compound **6A-4** (100 mg, 0.36 mmol) was dissolved in *N,N*-dimethylformamide (3 mL) under nitrogen atmosphere. Subsequently, sodium hydride (60%, 22 mg, 0.5 mmol) was added to the reaction liquid at 0 °C, and the reaction liquid was successively stirred for 30 min, and added with cyanogen bromide (386 mg, 3.6 mmol). The reaction system was warmed to room temperature and successively stirred for 3 h. After the starting material disappeared as detected by LCMS, the reaction liquid was purified using a reverse phase C18 column. The purification conditions: a chromatography column: 40 g of C18 reverse phase column; mobile phase: water (containing 10 mM ammonium bicarbonate) and acetonitrile; flow rate: 35 mL/min; gradient: acetonitrile elevating from 10% to 80% in 20 min; detection wavelength: 254 nm. The product was collected and lyophilized under reduced pressure to obtain compound **23A-1** (65 mg).

MS (ESI, m/z): 300.2 [M + H]⁺.

### Intermediate 23A:

Compound **23A** (50 mg) was prepared by replacing the starting material with compound **23A-1** (55 mg, 0.18 mmol) according to the method for the preparation of compound **1C** from intermediate **1C-9** in Example 1.

MS (ESI, m/z): 270.2 [M + H]⁺.

### Preparation of compound 23:

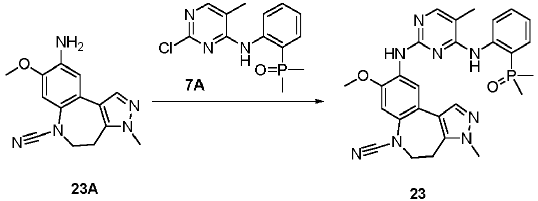

Compound **23A** (50 mg, 0.18 mmol), compound **7A** (38 mg, 0.13 mmol), cesium carbonate (121 mg, 0.37 mmol), tris(dibenzylideneacetone)dipalladium (0) (17 mg, 0.019 mmol) and 4,5-bis(diphenylphosphino)-9,9-dimethylxanthene (11 mg, 0.019 mmol) were dissolved in 1,4-dioxane (1 mL) under nitrogen atmosphere. The reaction system was heated to 90 °C and stirred successively for 4 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound 23 (17 mg).

MS (ESI, m/z): 529.3 [M + H]⁺.

¹H NMR (300 MHz, CDCl₃) δ 10.51 (s, 1H), 8.78 (s, 1H), 8.69-8.65 (m, 1H), 7.99 (s, 1H), 7.42 (s, 2H), 7.33-7.26 (m, 2H), 7.05-7.00 (m, 1H), 6.91 (s, 1H), 3.95 (s, 3H), 3.86 (s, 3H), 3.85-3.81 (m, 2H), 3.23 (t, *J* = 5.7 Hz, 2H), 2.24 (s, 3H), 1.89 (s, 3H), 1.84 (s, 3H).

### Example 24:

### Preparation of intermediate 24A

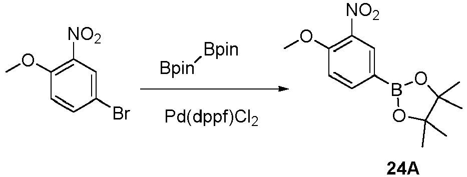

Compound **24A** (19.7 g) was prepared by replacing the starting material with 4-bromo-2-nitroanisole (20 g, 86.16 mmol) according to the method for the preparation of compound **1A** from intermediate **1A-1** in Example 1.

¹H NMR (300 MHz, CDCl₃) δ 8.27 (d, *J* = 1.5 Hz, 1H), 7.96 (dd, *J* = 8.4, 1.8 Hz, 1H), 7.08 (d, *J* = 8.4 Hz, 1H), 4.00 (s, 3H), 1.36 (s, 12H).

### Preparation of intermediate 24B

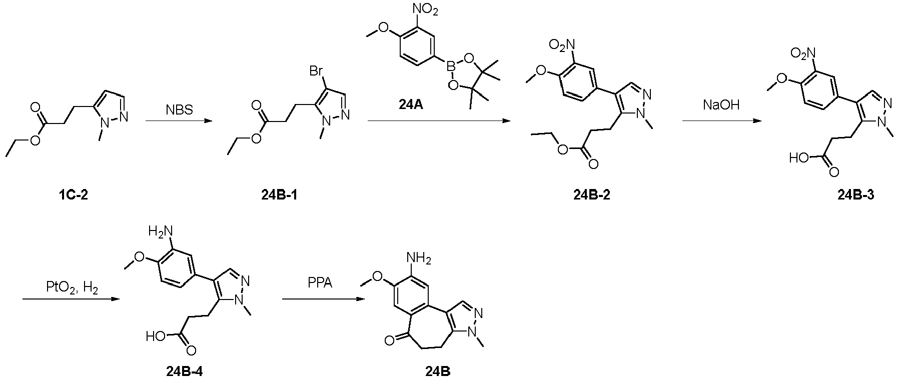

### Compound 24B-1:

Compound **1C-2** (6 g, 32.9 mmol) was dissolved in dichloromethane (100 mL). Subsequently, *N-*bromosuccinimide (8.8 g, 49.4 mmol) was added to the above reaction liquid at 0 °C. The reaction system was warmed to room temperature and successively stirred for 1 h. After the starting material disappeared as detected by LCMS, the reaction liquid was poured into water (100 mL) to quench the reaction. The mixture was extracted with dichloromethane (100 mL × 3), and the organic phases were combined, washed with saturated brine (80 mL × 3) firstly, then dried over anhydrous sodium sulfate, filtered, and concentrated under reduced pressure; the resulting residue was purified by silica gel column chromatography (eluent: petroleum ether/ethyl acetate = 1/1) to obtain compound **24B-1** (8.5 g).

MS (ESI) M/Z: 261.1, 263.1 [M + H]⁺.

### Compound 24B-2:

Compound **24B-2** (2 g) was prepared by replacing the starting material with compound **24B-1** (2 g, 7.6 mmol) and compound **24A** (2.57 g, 9.2 mmol) according to the method for the preparation of compound **1C-7** from compound **1C-6** in Example 1.

MS (ESI, m/z): 334.1 [M + H]⁺.

### Compound 24B-3:

Compound **24B-2** (2 g, 6.0 mmol) was dissolved in ethanol (20 mL). Subsequently, an aqueous solution of sodium hydroxide (2 N, 9 mL) was added to the reaction liquid. The reaction system was successively stirred at room temperature for 1 h. After the starting material disappeared as detected by LCMS, the reaction liquid was concentrated under reduced pressure and adjusted to pH = 2 with 1 N aqueous hydrochloric acid solution. The precipitated solid was filtered and dried to obtain compound **24B-3** (1.5 g).

MS (ESI, m/z): 306.2 [M + H]⁺.

### Compound 24B-4:

Compound **24B-3** (1.1 g, 3.6 mmol) was dissolved in a mixed solvent of ethanol (25 mL) and tetrahydrofuran (25 mL). Subsequently, platinum dioxide (221 mg, 0.97 mmol) was added to the reaction liquid. After the reaction system was purged with hydrogen 3 times, the reaction liquid was stirred at room temperature for 2 h. After the starting material disappeared as detected by LCMS, the reaction liquid was filtered through celite, the filter cake was washed with dichloromethane (30 mL × 3), and the resulting filtrate was concentrated under reduced pressure to obtain compound **24B-4** (0.95 g).

MS (ESI, m/z): 276.2 [M + H]⁺.

### Intermediate 24B:

Compound **24B-4** (1 g, 3.6 mmol) was dissolved in polyphosphoric acid (15 mL). The reaction system was heated to 100 °C and successively stirred for 48 h. After the starting material disappeared as detected by LCMS, the reaction liquid was poured into ice water (80 g). The mixture was extracted with ethyl acetate (100 mL × 3), the organic phases were combined, washed with saturated brine (80 mL × 3) firstly, then dried over anhydrous sodium sulfate, filtered, and concentrated under reduced pressure, and the resulting residue was purified by silica gel column chromatography (eluent: dichloromethane/methanol = 10/1) to obtain compound **24B** (0.65 g).

MS (ESI) M/Z: 258.2 [M + H]⁺.

### Preparation of compound 24

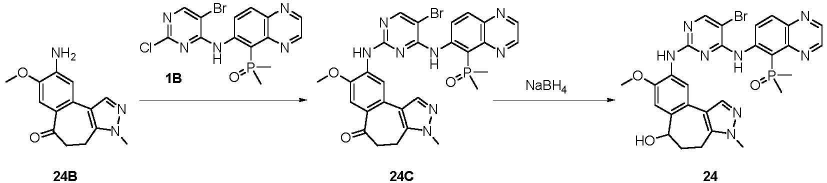

### Compound 24C:

Compound **24C** (200 mg) was prepared by replacing the starting material with compound **24B** (250 mg, 0.97 mmol) according to the method for the preparation of compound **1** from compound **1B** and compound **1C** in Example 1.

MS (ESI, m/z): 633.3, 635.2 [M + H]⁺.

### Compound 24:

Compound **24C** (100 mg, 0.16 mmol) was dissolved in methanol (2 mL). Sodium borohydride (12 mg, 0.3 mmol) was added to the reaction liquid at 0 °C. The reaction system was warmed to room temperature and successively stirred for 2 h. After the starting material disappeared as detected by LCMS, the reaction liquid was added with saturated aqueous ammonium chloride (10 mL). The mixture was extracted with ethyl acetate (20 mL × 3), the organic phases were combined, washed with saturated brine (20 mL × 3) firstly, then dried over anhydrous sodium sulfate, filtered, and concentrated under reduced pressure, and the resulting residue was purified by silica gel column chromatography (eluent: dichloromethane/methanol = 10/1) to obtain compound **24** (60 mg).

MS (ESI) M/Z: 634.9, 636.9 [M + H]⁺.

¹H NMR (300 MHz, CDCl₃) δ 12.68 (s, 1H), 8.87 (dd, *J* = 9.6, 4.2 Hz, 1H), 8.74-8.72 (m, 2H), 8.43 (s, 1H), 8.30 (s, 1H), 7.60 (s, 2H), 7.05 (s, 1H), 7.01 (s, 1H), 4.90 (d, *J* = 8.1 Hz, 1H), 3.95 (s, 3H), 3.73 (s, 3H), 3.09-2.98 (m, 2H), 2.47-2.28 (m, 2H), 2.18 (s, 3H), 2.14 (s, 3H).

### Example 25:

### Preparation of compound 25

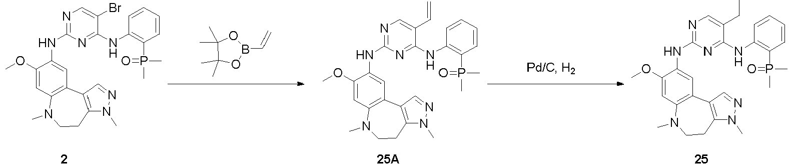

### Compound 25A:

Compound **2** (120 mg, 0.21 mmol), vinyl borate pinacol ester (63 mg, 0.41 mmol), palladium acetate (5 mg, 0.02 mmol), 2-bicyclohexylphosphino-2',6'-dimeoxybiphenyl (13 mg, 0.03 mmol) and potassium phosphate (131 mg, 0.62 mmol) were dissolved in a mixed solvent of 1,4-dioxane (3 mL) and water (0.6 mL) under nitrogen atmosphere. The reaction system was heated to 85°C and successively stirred for 4 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound **25A** (70 mg).

MS (ESI, m/z): 530.3 [M + H]⁺.

### Compound 25:

Compound **25A** (70 mg, 0.13 mmol) was dissolved in ethanol (5 mL). Wet palladium on carbon (10%, 50 mg) was added to the reaction liquid. After the reaction system was purged with hydrogen 3 times, the reaction liquid was stirred at room temperature for 2 h. After the starting material disappeared as detected by LCMS, the reaction liquid was filtered through celite, the filter cake was washed with ethanol (5 mL × 3), and the resulting filtrate was concentrated under reduced pressure. The resulting residue was purified by silica gel column chromatography (eluent: dichloromethane/methanol = 10/1) to obtain compound **25** (53 mg).

MS (ESI, m/z): 532.3 [M + H]⁺.

¹H NMR (300 MHz, CDCl₃) δ 10.82 (s, 1H), 8.94 (s, 1H), 8.64 (dd, *J* = 8.1, 4.5 Hz, 1H), 8.17 (s, 1H), 7.80 (s, 1H), 7.42 (s, 1H), 7.25-7.18 (m, 1H), 7.08-6.96 (m, 1H), 6.96-6.90 (m, 1H), 6.58 (s, 1H), 3.89 (s, 3H), 3.83 (s, 3H), 3.32-3.29 (m, 2H), 3.07-3.04 (m, 5H), 2.63 (q, *J* = 7.5 Hz, 2H), 1.86 (s, 3H), 1.82 (s, 3H), 1.30 (t, *J=* 7.5 Hz, 3H).

### Example 26:

### Preparation of compound 26

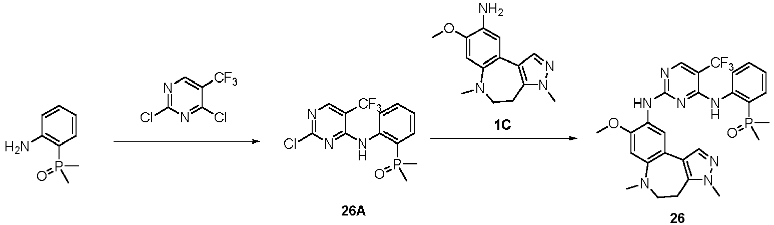

### Compound 26A:

2,4-Dichloro-5-trifluoromethylpyrimidine (2 g, 9.2 mmol) was dissolved in *N,N-*dimethylformamide (20 mL), and anhydrous potassium carbonate (1.5 g, 11.1 mmol) and 2-(dimethylphosphinoxy)aniline (1.6 g, 9.2 mmol) were added successively. The reaction liquid was heated to 80 °C and successively stirred for 12 h. After the starting material disappeared as detected by LCMS, the reaction liquid was cooled to room temperature and quenched by addition of water (150 mL). The mixture was extracted with ethyl acetate (100 mL × 3), the organic phases were combined, washed with saturated brine (80 mL × 3) firstly, then dried over anhydrous sodium sulfate, filtered, and concentrated under reduced pressure, and the resulting residue was purified by silica gel column chromatography (eluent: dichloromethane/methanol = 10/1) to obtain compound **26A** (0.35 g).

MS (ESI) M/Z: 350.1, 352.1 [M + H]⁺.

### Compound 26:

Compound **26** (41 mg) was prepared by replacing the starting material with compound **26A** (108 mg, 0.31 mmol) according to the method for the preparation of compound **1** from compound **1B** and compound **1C** in Example 1.

MS (ESI, m/z): 572.3 [M + H]⁺.

¹H NMR (300 MHz, CDCl₃) δ 10.35 (s, 1H), 8.40-8.32 (m, 3H), 7.49 (s, 1H), 7.37-7.29 (m, 1H), 7.17-6.98 (m, 2H), 6.56 (s, 1H), 3.97 (s, 3H), 3.83 (s, 3H), 3.31-3.27 (m, 2H), 3.05-3.02 (m, 5H), 1.88 (s, 3H), 1.83 (s, 3H). ¹⁹F NMR (282 MHz, CDCl₃) δ -60.82.

### Example 27:

### Preparation of compound 27

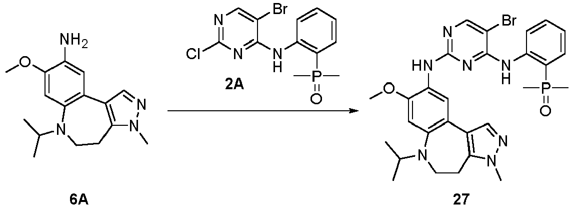

Compound **6A** (80 mg, 0.28 mmol) and compound **2A** (101 mg, 0.28 mmol) were dissolved in *N-*methylpyrrolidinone (2 mL). Subsequently, methanesulfonic acid (81 mg, 0.84 mmol) was added to the above reaction liquid. The reaction system was heated to 95 °C and successively stirred for 16 h. After the starting material disappeared as detected by LCMS, the reaction liquid was cooled to room temperature and purified using a reverse phase C18 column. Purification conditions: a chromatography column: 80 g of C18 reverse phase column; mobile phase: water (containing 0.1% formic acid) and acetonitrile; flow rate: 50 mL/min; gradient: acetonitrile elevating from 15% to 55% in 25 min; detection wavelength: 254 nm. The product was collected and concentrated under reduced pressure to obtain compound **27** (60 mg).

MS (ESI, m/z): 610.2, 612.2 [M + H]⁺.

¹H NMR (300 MHz, DMSO-*d₆*) δ 10.93 (s, 1H), 8.39 (s, 1H), 8.22 (s, 1H), 8.16 (s, 1H), 7.72 (s, 1H), 7.56 (s, 1H), 7.52-7.44 (m, 1H), 7.07-6.99 (m, 2H), 6.57 (s, 1H), 3.98-3.89 (m, 1H), 3.75 (br s, 6H), 3.22-3.18 (m, 2H), 2.96-2.92 (m, 2H), 1.78 (s, 3H), 1.73 (s, 3H), 1.29 (s, 3H), 1.27 (s, 3H).

### Example 28:

### Preparation of compound 28

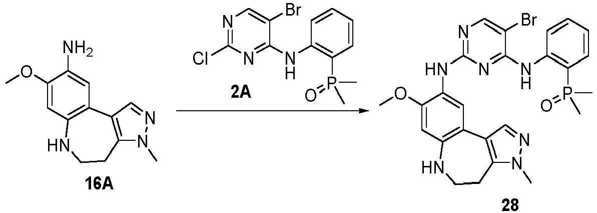

Compound 28 (15 mg) was prepared by replacing the starting material with compound **2A** (162 mg, 0.45 mmol) and compound **16A** (110 mg, 0.45 mmol) according to the method for the preparation of compound **1** from compound **1B** and compound **1C** in Example 1.

MS (ESI, m/z): 568.2, 570.2 [M + H]⁺.

¹H NMR (300 MHz, DMSO-*d₆*) δ 10.95 (s, 1H), 8.40 (s, 1H), 8.18 (s, 1H), 8.14 (s, 1H), 7.63 (s, 1H), 7.61 (s, 1H), 7.51-7.44 (m, 1H), 7.07-6.99 (m, 2H), 6.53 (s, 1H), 5.87 (s,1H), 3.73 (s, 3H), 3.68 (s, 3H), 3.26-3.24 (m, 2H), 2.98-2.94 (m, 2H), 1.77 (s, 3H), 1.73 (s, 3H).

### Example 29:

### Preparation of intermediate 29A

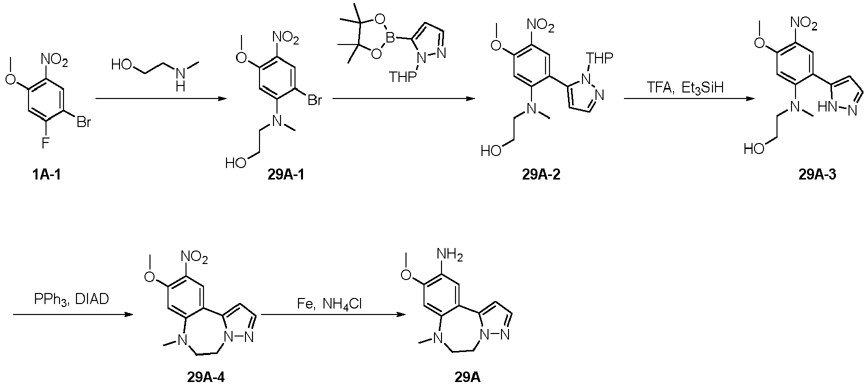

### Compound 29A-1:

Compound **1A-1** (1 g, 4 mmol), potassium carbonate (1.11 g, 8 mmol) and N-methylethanolamine (0.45 g, 6 mmol) were dissolved in N,N-dimethylformamide (20 mL). The reaction system was heated to 80 °C and successively stirred for 2 h. After the starting material disappeared as detected by LCMS, the reaction liquid was cooled to room temperature and quenched by addition of water (100 mL). The mixture was extracted with ethyl acetate (80 mL × 3), the organic phases were combined, washed with saturated brine (80 mL × 3) firstly, then dried over anhydrous sodium sulfate, filtered, and concentrated under reduced pressure, and the resulting residue was purified by silica gel column chromatography (eluent: dichloromethane/methanol = 20/1) to obtain compound **29A-1** (1 g).

MS (ESI) M/Z: 305.0, 307.0 [M + H]⁺.

### Compound 29A-2:

Compound **29A-2** (0.15 g) was prepared by replacing the starting material with compound **29A-1** (1 g, 3.3 mmol) and 1-(2-tetrahydropyranyl)-1*H*-pyrazole-5-boronic acid pinacol ester (1.37 g, 4.92 mmol) according to the method for the preparation of compound **1C-7** from compound **1C-6** in Example 1.

MS (ESI, m/z): 377.3 [M + H]⁺.

### Compound 29A-3:

Compound **29A-2** (300 mg, 0.8 mmol) was dissolved in dichloromethane (3 mL). Subsequently, trifluoroacetic acid (1 mL) and triethylsilane (185 mg, 1.6 mmol) were added to the reaction liquid, and the reaction liquid was successively stirred at room temperature for 5 h. After the starting material disappeared as detected by LCMS, the reaction liquid was concentrated under reduced pressure. The resulting residue was purified by silica gel column chromatography (eluent: dichloromethane/methanol = 20/1) to obtain compound **29A-3** (90 mg).

MS (ESI) M/Z: 292.9 [M + H]⁺.

### Compound 29A-4:

Triphenylphosphine (129 mg, 0.5 mmol) was dissolved in tetrahydrofuran (2 mL) under nitrogen atmosphere. Diethyl azodicarboxylate (97 mg, 0.56 mmol) was added to the reaction liquid at 0 °C, and the reaction liquid was successively stirred at 0 °C for 1 h (to yield a milky turbid liquid). Compound **29A-3** (90 mg, 0.31 mmol) was added to the above reaction liquid, and the reaction liquid was successively stirred at room temperature for 4 h. After the starting material disappeared as detected by LCMS, the reaction liquid was concentrated under reduced pressure, and the resulting residue was purified by silica gel column chromatography (eluent: dichloromethane/methanol = 20/1) to obtain compound **29A-4** (35 mg).

MS (ESI) M/Z: 275.1 [M + H]⁺.

### Intermediate 29A:

Compound **29A** (20 mg) was prepared by replacing the starting material with compound **29A-4** (35 mg, 0.13 mmol) according to the method for the preparation of compound **1C** from intermediate **1C-9** in Example 1.

MS (ESI, m/z): 245.2 [M + H]⁺.

### Preparation of compound 29

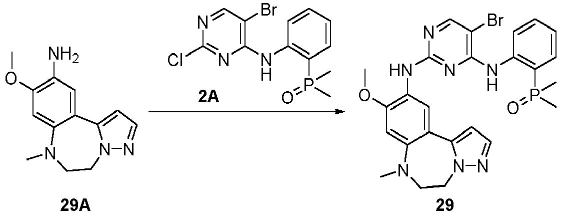

Compound **29** (7.6 mg) was prepared by replacing the starting material with compound **2A** (51.66 mg, 0.14 mmol) and compound **29A** (35 mg, 0.14 mmol) according to the method for the preparation of compound **1** from compound **1B** and compound **1C** in Example 1.

MS (ESI, m/z): 568.2, 570.2 [M + H]⁺.

¹H NMR (300 MHz, CDCl₃) δ 10.58 (s, 1H), 8.39 (s, 1H), 8.34 (s, 1H), 8.23 (s, 1H), 7.39 (s, 1H), 7.30 (s, 1H), 7.23 (s, 1H), 7.01 (s, 2H), 6.56 (s, 1H), 6.04 (s, 1H), 4.46-4.42 (m, 2H), 3.95 (s, 3H), 3.63-3.59 (m, 2H), 2.98 (s, 3H), 1.87 (s, 3H), 1.83 (s, 3H).

### Example 30:

### Preparation of compound 30

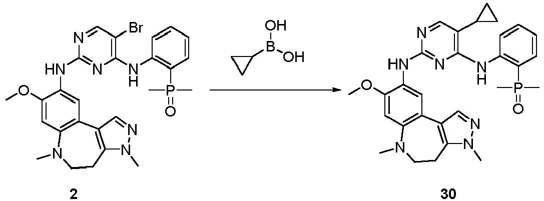

Compound **2** (300 mg, 0.52 mmol), cyclopropylboronic acid (133 mg, 1.55 mmol), potassium phosphate (328 mg, 1.55 mmol), palladium acetate (12 mg, 0.05 mmol) and tricyclohexylphosphine (14 mg, 0.05 mmol) were dissolved in a mixed solvent of toluene (2 mL) and water (0.4 mL) under nitrogen atmosphere. The reaction system was heated to 100 °C and successively stirred for 24 h. After the starting material disappeared as detected by LCMS, the reaction liquid was added with water (10 mL). The mixture was extracted with ethyl acetate (30 mL × 3), the organic phases were combined, washed with saturated brine (20 mL × 3) firstly, then dried over anhydrous sodium sulfate, filtered, and concentrated under reduced pressure, and the resulting residue was purified by silica gel column chromatography (eluent: dichloromethane/methanol = 10/1) to obtain compound **30** (91 mg).

MS (ESI) M/Z: 544.3 [M + H]⁺.

¹H NMR (300 MHz, CDCl₃) δ 10.43 (s, 1H), 8.72-8.67 (m, 1H), 8.52 (s, 1H), 7.95 (s, 1H), 7.38 (s, 1H), 7.33-7.30 (m, 1H), 7.27-7.15 (m, 2H), 7.00-6.93 (m, 1H), 6.56 (s, 1H), 3.91 (s, 3H), 3.83 (s, 3H), 3.31-3.27 (m, 2H), 3.06-3.02 (m, 5H), 1.87 (s, 3H), 1.83 (s, 3H), 1.77-1.70 (m, 1H), 1.10-0.99 (m, 2H), 0.67-0.55 (m, 2H).

### Example 31:

### Preparation of intermediate 31A

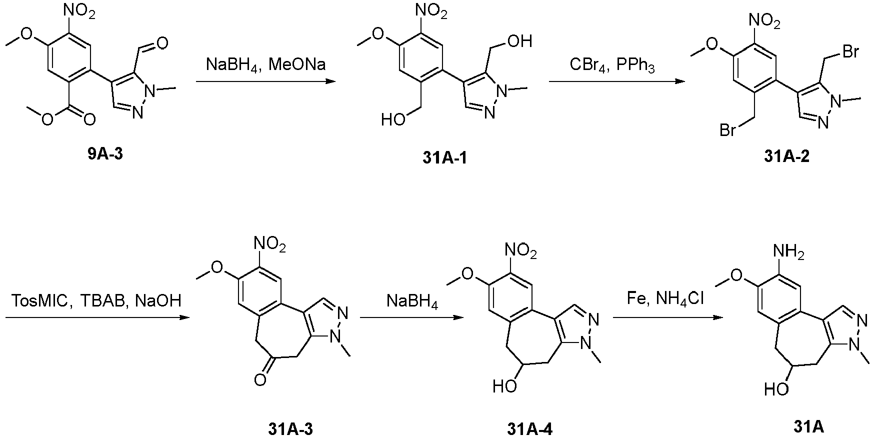

### Compound 31A-1:

Compound **9A-3** (600 mg, 1.88 mmol) and sodium methoxide (5 mg, 0.09 mmol) were dissolved in methanol (12 mL). Subsequently, sodium borohydride (284 mg, 7.52 mmol) was added to the reaction liquid, and the reaction liquid was successively stirred at room temperature for 16 h. After the starting material disappeared as detected by LCMS, the reaction liquid was filtered, the filtrate was concentrated under reduced pressure, and the resulting residue was purified by silica gel column chromatography (eluent: dichloromethane/methanol = 10/1) to obtain compound **31A-1** (550 mg).

MS (ESI) M/Z: 294.1 [M + H]⁺.

### Compound 31A-2:

Compound **31A-1** (850 mg, 2.9 mmol) and tetrabromomethane (3.84 g, 11.59 mmol) were dissolved in tetrahydrofuran (10 mL) under nitrogen atmosphere. Subsequently, triphenylphosphine (3.04 g, 11.59 mmol) was added to the reaction liquid, and the reaction liquid was successively stirred at room temperature for 5 h. After the starting material disappeared as detected by LCMS, the reaction liquid was concentrated under reduced pressure. The resulting residue was purified by silica gel column chromatography (eluent: ethyl acetate) to obtain compound **31A-2** (130 mg).

MS (ESI) M/Z: 418.0, 420.0, 422.0 [M + H]⁺.

### Compound 31A-3:

Compound **31A-2** (880 mg, 2.1 mmol), tetrabutylammonium bromide (162 mg, 0.5 mmol) and tosylmethyl isocyanide (451 mg, 2.3 mmol) were dissolved in dichloromethane (24 mL). Subsequently, a solution of sodium hydroxide (428 mg, 10.71 mmol) in water (6 mL) was added to the reaction liquid at 0 °C. The reaction system was warmed to room temperature and successively stirred for 16 h. After the starting material disappeared as detected by LCMS, the two phases were separated and the aqueous phase was extracted with dichloromethane (20 mL). The organic phases were combined and added with aqueous hydrochloric acid solution (7 mL, 37%), and the reaction liquid was successively stirred at room temperature for 3 h. The two phases were separated, and the organic phase was washed successively with water (40 mL), saturated sodium bicarbonate (40 mL) and brine (40 mL), then dried over anhydrous sodium sulfate, filtered and concentrated under reduced pressure; the resulting residue was purified by silica gel column chromatography (eluent: ethyl acetate) to obtain compound **31A-3** (360 mg).

MS (ESI) M/Z: 288.1 [M + H]⁺.

### Compound 31A-4:

Compound **31A-3** (100 mg, 0.35 mmol) was dissolved in methanol (4 mL). Subsequently, sodium borohydride (20 mg, 0.5 mmol) was added to the reaction liquid at 0 °C. The reaction system was warmed to room temperature and successively stirred for 1 h. After the starting material disappeared as detected by LCMS, the reaction liquid was quenched by addition of water (10 mL). The mixture was extracted with ethyl acetate (30 mL × 3), and the organic phases were combined, washed with saturated brine (20 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **31A-4** (85 mg). MS (ESI) M/Z: 290.1 [M + H]⁺.

### Intermediate 31A:

Compound **31A** (65 mg) was prepared by replacing the starting material with compound **31A-4** (85 mg, 0.29 mmol) according to the method for the preparation of compound **1C** from intermediate **1C-9** in Example 1.

MS (ESI, m/z): 260.1 [M + H]⁺.

### Preparation of compound 31

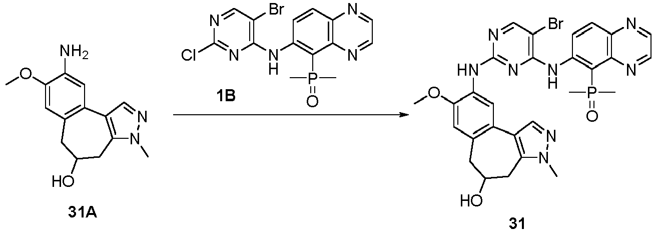

Compound **31** (30 mg) was prepared by replacing the starting material with compound **31A** (65 mg, 0.25 mmol) according to the method for the preparation of compound **1** from compound **1B** and compound **1C** in Example 1.

MS (ESI, m/z): 635.1, 637.1 [M + H]⁺.

¹H NMR (300 MHz, CDCl₃) δ 12.97 (s, 1H), 8.87-8.70 (m, 3H), 8.43 (s, 1H), 8.21 (s, 1H), 8.14 (s, 1H), 7.59 (d, *J* = 9.6 Hz, 1H), 7.10 (s, 1H), 6.80 (s, 1H), 4.49-4.47 (m, 1H), 3.92 (s, 3H), 3.77 (s, 3H), 3.24-2.95 (m, 5H), 2.17 (s, 3H), 2.13 (s, 3H).

### Example 32:

### Preparation of intermediate 32A

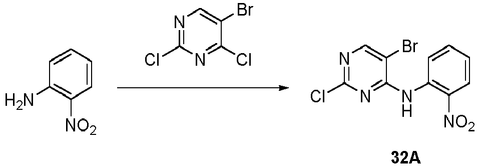

Compound **32A** (3.2 g) was prepared by replacing the starting material with 2-nitroaniline (5.0 g, 36.2 mmol) according to the method for the preparation of compound **1B** from compound **1B-2** in Example 1.

MS (ESI, m/z): 328.8, 330.8 [M + H]⁺.

### Preparation of compound 32

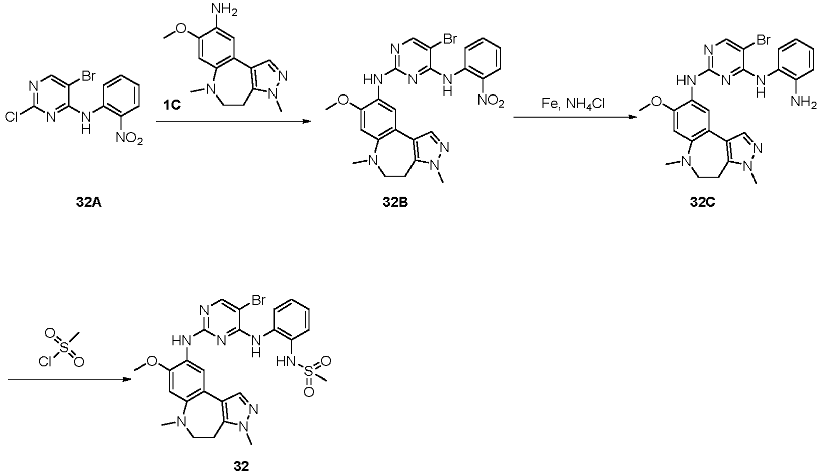

### Compound 32B:

Compound **32B** (270 mg) was prepared by replacing the starting material with compound **32A** (200 mg, 0.61 mmol) according to the method for the preparation of compound **1** from compound **1B** and compound **1C** in Example 1.

MS (ESI, m/z): 551.0, 553.0 [M + H]⁺.

### Compound 32C:

Compound **32C** (80 mg) was prepared by replacing the starting material with compound **32B** (130 mg, 0.24 mmol) according to the method for the preparation of compound **1C** from intermediate **1C-9** in Example 1.

MS (ESI, m/z): 521.2, 523.2 [M + H]⁺.

### Compound 32:

Compound **32C** (40 mg, 0.08 mmol) was dissolved in pyridine (1.5 mL) at 0 °C. Subsequently, methanesulfonyl chloride (27 mg, 0.24 mmol) was added to the reaction liquid. The reaction system was warmed to room temperature and successively stirred for 3 h. After the starting material disappeared as detected by LCMS, the reaction liquid was purified using reverse phase C18 column. Purification conditions: a chromatography column: 80 g of C18 reverse phase column; mobile phase: water (containing 0.1% formic acid) and acetonitrile; flow rate: 50 mL/min; gradient: acetonitrile elevating from 5% to 40% in 30 min; detection wavelength: 254 nm. The product was collected and lyophilized under reduced pressure to obtain compound **32** (21 mg).

MS (ESI, m/z): 599.0, 601.0 [M + H]⁺.

¹H NMR (300 MHz, CDCl₃) δ 8.17 (s, 1H), 8.13 (s, 1H), 7.68-7.59 (m, 1H), 7.49 (s, 1H), 7.39 (d, *J* = 7.5 Hz, 1H), 7.17-7.12 (m, 3H), 6.52 (s, 1H), 3.89 (s, 3H), 3.85 (s, 3H), 3.27 (s, 2H), 3.04 (br s, 5H), 2.91 (s, 3H).

### Example 33:

### Preparation of intermediate 33A

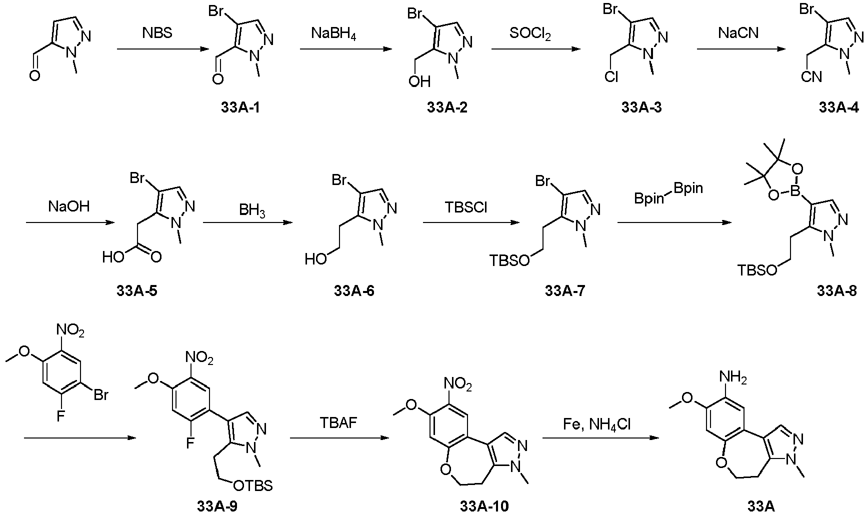

### Compound 33A-1:

Compound **33A-1** (80 g) was prepared by replacing the starting material with 1-methyl-1*H-*pyrazole-5-carbaldehyde (59.7 g, 542 mmol) according to the method for the preparation of compound **24B-1** from intermediate **1C-2** in Example 24.

¹H NMR (300 MHz, CDCl₃) δ 9.91 (s, 1H), 7.54 (s, 1H), 4.18 (s, 3H).

### Compound 33A-2:

Compound **33A-1** (60 g, 317.4 mmol) was dissolved in ethanol (500 mL) at 0°C. Subsequently, sodium borohydride (6 g, 158.7 mmol) was added to the reaction liquid in batches. The reaction system was warmed to room temperature and successively stirred for 1 h. After the starting material disappeared as detected by LCMS, the reaction liquid was concentrated under reduced pressure. The mixture was added with water (300 mL) for dilution and adjusted to pH = 6 with concentrated hydrochloric acid. The mixture was extracted with ethyl acetate (300 mL × 3), and the organic phases were combined, washed with saturated brine (200 mL × 3) firstly, then dried over anhydrous sodium sulfate, filtered, and concentrated under reduced pressure to obtain compound **33A-2** (60 g).

MS (ESI) M/Z: 191.2, 193.2 [M + H]⁺.

### Compound 33A-3:

Compound **33A-2** (60 g, 314 mmol) was dissolved in dichloromethane (500 mL) at 0 °C. Subsequently, thionyl chloride (18.68 g, 157 mmol) was added dropwise to the reaction liquid. The reaction system was warmed to room temperature and successively stirred for 16 h. After the starting material disappeared as detected by LCMS, the reaction liquid was concentrated under reduced pressure. The mixture was added with dichloromethane (300 mL) for dilution, and slowly added dropwise to a saturated aqueous sodium bicarbonate solution (300 mL). The mixture was extracted with dichloromethane (300 mL × 2), and the organic phases were combined, washed with saturated brine (300 mL × 3) firstly, then dried over anhydrous sodium sulfate, filtered, and concentrated under reduced pressure to obtain compound **33A-3** (62 g).

MS (ESI) M/Z: 209.0, 211.0 [M + H]⁺.

### Compound 33A-4:

Compound **33A-3** (62 g, 296 mmol) and sodium cyanide (17.41 g, 355 mmol) were dissolved in dimethyl sulfoxide (300 mL). The reaction system was heated to 50 °C and successively stirred for 16 h. After the starting material disappeared as detected by LCMS, the reaction liquid was poured to saturated sodium bicarbonate (500 mL). The mixture was extracted with ethyl acetate (300 mL × 2), and the organic phases were combined, washed with saturated brine (300 mL × 3) firstly, then dried over anhydrous sodium sulfate, filtered, and concentrated under reduced pressure. The resulting residue was purified by silica gel column chromatography (eluent: petroleum ether/ethyl acetate = 1/1) to obtain compound **33A-4** (15 g).

MS (ESI) M/Z: 200.0, 202.0 [M + H]⁺.

### Compound 33A-5:

Compound **33A-4** (5 g, 25 mmol) and sodium hydroxide (10 g, 250 mmol) were dissolved in water (100 mL). The reaction system was heated to 100 °C and successively stirred for 16 h. After the starting material disappeared as detected by LCMS, the reaction liquid was cooled to room temperature and adjusted to pH = 1 with concentrated hydrochloric acid. The mixture was extracted with ethyl acetate (200 mL × 2), and the organic phases were combined, washed with saturated brine (150 mL × 3) firstly, then dried over anhydrous sodium sulfate, filtered, and concentrated under reduced pressure to obtain compound **33A-5** (5 g).

MS (ESI) M/Z: 218.7, 220.7 [M + H]⁺.

### Compound 33A-6:

Compound **33A-5** (1 g, 4.6 mmol) was dissolved in tetrahydrofuran (10 mL) under nitrogen atmosphere. Subsequently, a solution of borane in tetrahydrofuran (1 M, 10 mL) was added to the reaction liquid. The reaction system was heated to 40 °C and successively stirred for 16 h. After the starting material disappeared as detected by LCMS, the reaction liquid was cooled to room temperature and slowly added to methanol (40 mL), heated to 60 °C and successively stirred for 30 min. The reaction liquid was cooled to room temperature and then concentrated under reduced pressure, and the resulting residue was purified by silica gel column chromatography (eluent: dichloromethane/methanol = 10/1) to obtain compound **33A-6** (0.8 g).

MS (ESI) M/Z: 205.0, 207.0 [M + H]⁺.

### Compound 33A-7:

Compound **33A-6** (300 mg, 1.46 mmol,) and imidazole (120 mg, 1.76 mmol) were dissolved in N,N-dimethylformamide (3 mL). Subsequently, tert-butyldimethylsilyl chloride (265 mg, 1.76 mmol) was added to the reaction liquid at 0 °C. The reaction system was warmed to room temperature and successively stirred for 2 h. After the starting material disappeared as detected by LCMS, the reaction liquid was quenched by addition of water (10 mL). The mixture was extracted with ethyl acetate (20 mL × 2), and the organic phases were combined, washed with saturated brine (10 mL × 3) firstly, then dried over anhydrous sodium sulfate, filtered, and concentrated under reduced pressure. The resulting residue was purified by silica gel column chromatography (eluent: petroleum ether/methyl tert-butyl ether = 2/1) to obtain compound **33A-7** (140 mg).

MS (ESI) M/Z: 319.1, 321.1 [M + H]⁺.

### Compound 33A-8:

Compound **33A-7** (330 mg, 1.03 mmol) was dissolved in tetrahydrofuran (5 mL) under nitrogen atmosphere. Subsequently, n-Butyllithium (2.5 M, 0.6 mL, 1.5 mmol) was added to the reaction liquid at -78 °C, and the reaction liquid was successively stirred at that temperature for 1 h. Isopropoxyboronic acid pinacol ester (230 mg, 1.2 mmol) was added to the reaction liquid, and the reaction liquid was successively stirred at that temperature for 1 h. After the starting material disappeared as detected by LCMS, the reaction liquid was quenched by addition of saturated aqueous ammonium chloride (10 mL). The mixture was extracted with ethyl acetate (20 mL × 2), and the organic phases were combined, washed with saturated brine (10 mL × 3) firstly, then dried over anhydrous sodium sulfate, filtered, and concentrated under reduced pressure. The resulting residue was purified by silica gel column chromatography (eluent: petroleum ether/methyl *tert-*butyl ether = 1/1) to obtain compound **33A-8** (300 mg).

MS (ESI) M/Z: 367.2 [M + H]⁺.

### Compound 33A-9:

Compound **33A-9** (100 mg) was prepared by replacing the starting material with compound **33A-8** (100 mg, 0.273 mmol) and compound **1A-1** (55 mg, 0.22 mmol) according to the method for the preparation of compound **1C-7** from compound **1C-6** in Example 1.

MS (ESI, m/z): 410.2 [M + H]⁺.

### Compound 33A-10:

Compound **33A-9** (100 mg, 0.24 mmol) was dissolved in tetrahydrofuran (2 mL). Subsequently, a solution of tetrabutylammonium fluoride in tetrahydrofuran (1 M, 1 mL) was added to the reaction liquid, and the reaction liquid was successively stirred at room temperature for 5 h. After the starting material disappeared as detected by LCMS, the reaction liquid was concentrated under reduced pressure, and the resulting residue was purified by silica gel column chromatography (eluent: dichloromethane/methanol = 10/1) to obtain compound **33A-10** (40 mg).

MS (ESI) M/Z: 276.1 [M+H]⁺.

### Intermediate 33A:

Compound **33A** (30 mg) was prepared by replacing the starting material with compound **33A-10** (40 mg, 0.15 mmol) according to the method for the preparation of compound **1C** from intermediate **1C-9** in Example 1.

MS (ESI, m/z): 246.1 [M + H]⁺.

### Preparation of compound 33

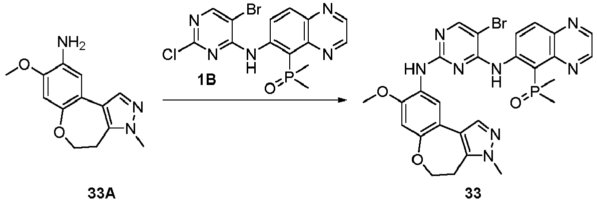

Compound **33** (20 mg) was prepared by replacing the starting material with compound **33A** (25 mg, 0.1 mmol) according to the method for the preparation of compound **1** from compound **1B** and compound **1C** in Example 1.

MS (ESI, m/z): 621.1, 623.1 [M + H]⁺.

¹H NMR (300 MHz, CDCl₃) δ 12.66 (s, 1H), 8.91-8.86 (m, 1H), 8.73-8.70 (m, 2H), 8.34 (s, 1H), 8.29 (s, 1H), 7.72 (d, *J* = 9.3 Hz, 1H), 7.58 (s, 1H), 6.99 (s, 1H), 6.63 (s, 1H), 4.40-4.36 (m, 2H), 3.88 (s, 3H), 3.73 (s, 3H), 3.19-3.15 (m, 2H), 2.18 (s, 3H), 2.13 (s, 3H).

### Example 34:

### Preparation of intermediate 34A

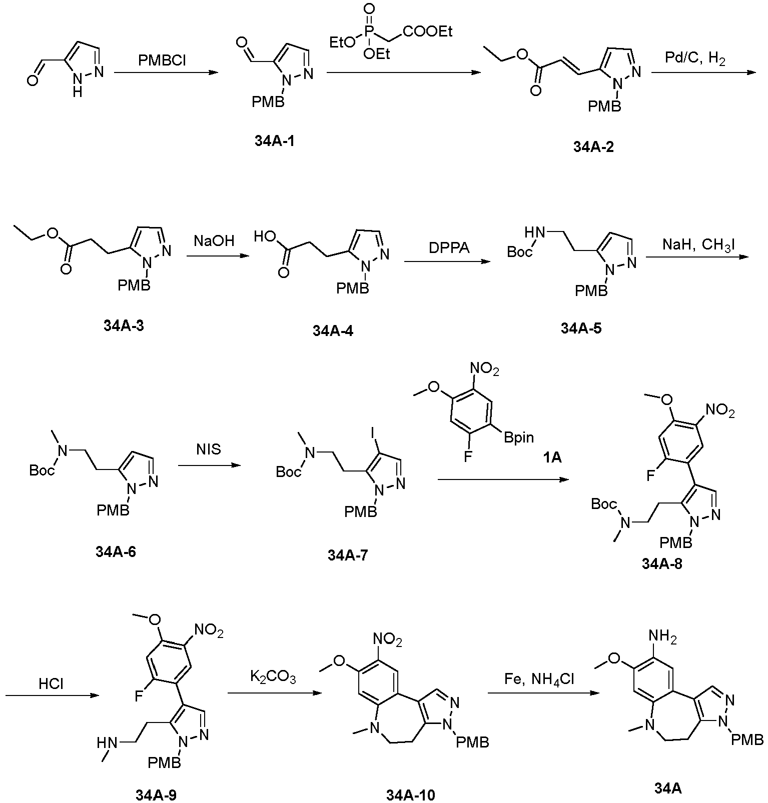

### Compound 34A-1:

1*H*-pyrazole-5-carbaldehyde (3.7 g, 38 mmol) and potassium phosphate (16 g, 77 mmol) were dissolved in *N,N*-dimethylformamide (50 mL). Subsequently, p-methoxybenzyl chloride (PNM-Cl, 6 g, 38 mmol) was added to the reaction liquid. The reaction system was heated to 80 °C and successively stirred for 3 h. After the starting material disappeared as detected by LCMS, the reaction liquid was poured into water (150 mL). The mixture was extracted with ethyl acetate (100 mL × 2), and the organic phases were combined, washed with saturated brine (100 mL × 3) firstly, then dried over anhydrous sodium sulfate, filtered, and concentrated under reduced pressure. The resulting residue was purified by silica gel column chromatography (eluent: petroleum ether/ethyl acetate = 1/1) to obtain compound **34A-1** (4 g).

MS (ESI) M/Z: 217.2 [M + H]⁺.

### Compound 34A-2:

Compound **34A-2** (2.6 g) was prepared by replacing the starting material with compound **34A-1** (2 g, 9 mmol) according to the method for the preparation of compound **1C-1** in Example 1.

MS (ESI, m/z): 287.1 [M + H]⁺.

### Compound 34A-3:

Compound **34A-3** (2.4 g) was prepared by replacing the starting material with compound **34A-2** (2.6 g, 9 mmol) according to the method for the preparation of compound **1C-2** from compound **1C-1** in Example 1.

MS (ESI, m/z): 289.1 [M + H]⁺.

### Compound 34A-4:

Compound **34A-4** (2 g) was prepared by replacing the starting material with compound **34A-3** (2.6 g, 9 mmol) according to the method for the preparation of compound **1C-3** from compound **1C-2** in Example 1.

MS (ESI, m/z): 259.1 [M - H]⁻.

### Compound 34A-5:

Compound **34A-5** (1.8 g) was prepared by replacing the starting material with compound **34A-4** (2 g, 7.7 mmol) according to the method for the preparation of compound **1C-4** from compound **1C-**3 in Example 1.

MS (ESI, m/z): 332.2 [M + H]⁺.

### Compound 34A-6:

Compound **34A-6** (1.6 g) was prepared by replacing the starting material with compound **34A-5** (1.8 g, 5.4 mmol) according to the method for the preparation of compound **1C-5** from compound **1C-4** in Example 1.

MS (ESI, m/z): 346.3 [M + H]⁺.

### Compound 34A-7:

Compound **34A-7** (2.1 g) was prepared by replacing the starting material with compound **34A-6** (2 g, 5.8 mmol) according to the method for the preparation of compound **1C-6** from compound **1C-5** in Example 1.

MS (ESI, m/z): 472.0 [M + H]⁺.

### Compound 34A-8:

Compound **34A-8** (1.9 g) was prepared by replacing the starting material with compound **34A-7** (1.3 g, 4.2 mmol) according to the method for the preparation of compound **1C-7** from compound **1C-6** in Example 1.

MS (ESI, m/z): 515.3 [M + H]⁺.

### Compound 34A-9:

Compound **34A-9** (1 g) was prepared by replacing the starting material with compound **34A-8** (1.9 g, 3.7 mmol) according to the method for the preparation of compound **1C-8** from compound **1C-**7 in Example 1.

MS (ESI, m/z): 415.2 [M + H]⁺.

### Compound 34A-10:

Compound **34A-10** (0.8 g) was prepared by replacing the starting material with compound **34A-9** (1 g, 2.4 mmol) according to the method for the preparation of compound **1C-9** from compound **1C-8** in Example 1.

MS (ESI, m/z): 395.3 [M + H]⁺.

### Compound 34A:

Compound **34A** (200 mg) was prepared by replacing the starting material with compound **34A-10** (300 mg, 0.76 mmol) according to the method for the preparation of compound **1C** from compound **1C-9** in Example 1.

MS (ESI, m/z): 365.2 [M + H]⁺.

### Preparation of compound 34

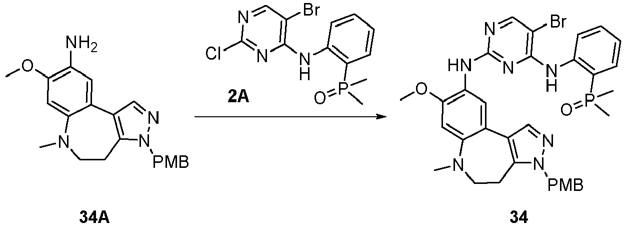

Compound **34** (200 mg) was prepared by replacing the starting material with compound **34A** (202 mg, 0.56 mmol) and compound **2A** (200 mg, 0.56 mmol) according to the method for the preparation of compound **1** from compound **1B** and compound **1C** in Example 1.

MS (ESI, m/z): 688.3, 690.3 [M + H]⁺.

¹H NMR (300 MHz, CDCl₃) δ 10.84 (s, 1H), 8.69-8.65 (m, 1H), 8.31 (s,1H), 8.27 (s, 1H), 7.32-7.27 (m, 2H), 7.20-7.17 (m, 3H), 7.05-7.00 (m, 1H), 6.91-6.88 (m, 2H), 6.61 (s, 1H), 5.16 (s, 2H), 3.97 (s, 3H), 3.87 (s, 3H), 3.39-3.37 (m, 2H), 3.26-3.23 (m, 2H), 3.11 (s, 3H), 1.93 (s, 3H), 1.89 (s, 3H).

### Example 35:

### Preparation of intermediate 35A

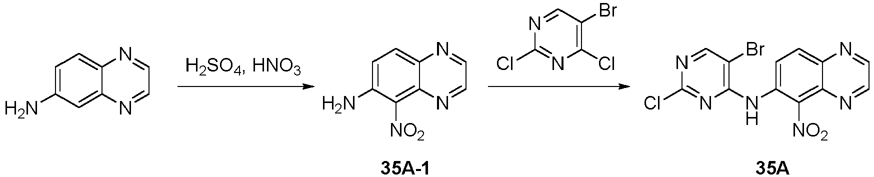

### Compound 35A-1:

6-Aminoquinoxaline (10 g, 68.89 mmol) was dissolved in concentrated sulfuric acid (20 mL). Potassium nitrate (9.054 g, 89.55 mmol) was added in batches to the reaction liquid at 0 °C, and the reaction system was successively stirred at that temperature for 30 min. After the starting material disappeared as detected by LCMS, the reaction liquid was poured into ice water (100 g). The reaction liquid was adjusted to pH = 8 with 1 M aqueous sodium hydroxide solution. The mixture was extracted with ethyl acetate (200 mL × 2), and the organic phases were combined, washed with saturated brine (100 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **35A-1** (2 g).

MS (ESI) M/Z: 191.2 [M + H]⁺.

### Intermediate 35A:

Compound **35A** (40 mg) was prepared by replacing the starting material with compound **35A-1** (50 mg, 0.26 mmol) according to the method for the preparation of compound **1B** from compound **1B-2** in Example 1.

MS (ESI, m/z): 381.0, 383.0 [M + H]⁺.

### Preparation of compound 35

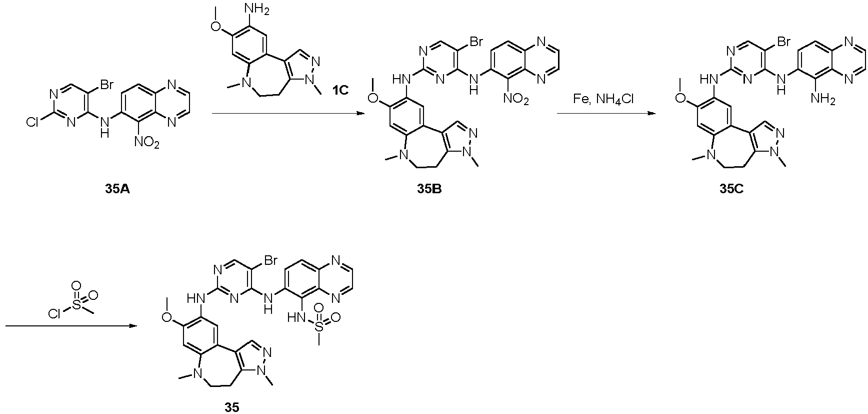

### Compound 35B:

Compound **35B** (130 mg) was prepared by replacing the starting material with compound **35A** (200 mg, 0.52 mmol) according to the method for the preparation of compound **1** from compound **1B** and compound **1C** in Example 1.

MS (ESI, m/z): 603.0, 605.0 [M + H]⁺.

### Compound 35C:

Compound **35C** (76 mg) was prepared by replacing the starting material with compound **35B** (130 mg, 0.22 mmol) according to the method for the preparation of compound **1C** from intermediate **1C-9** in Example 1.

MS (ESI, m/z): 573.1, 575.1 [M + H]⁺.

### Compound 35:

Compound **35** (33 mg) was prepared by replacing the starting material with compound **35C** (65 mg, 0.11 mmol) according to the method for the preparation of compound **32** from intermediate **32C** in Example 32.

MS (ESI, m/z): 650.9, 652.9 [M + H]⁺.

¹H NMR (300 MHz, CDCl₃) δ 9.13 (s, 1H), 8.91-8.69 (m, 2H), 8.59 (d, *J* = 9.3 Hz, 1H), 8.27 (s, 2H), 7.59 (s, 2H), 7.39 (s, 1H), 6.75 (s, 1H), 6.53 (s, 1H), 3.91 (s, 3H), 3.69 (s, 3H), 3.31 (s, 2H), 3.03 (br s, 5H), 2.96 (s, 3H).

### Example 36:

### Preparation of intermediate 36A

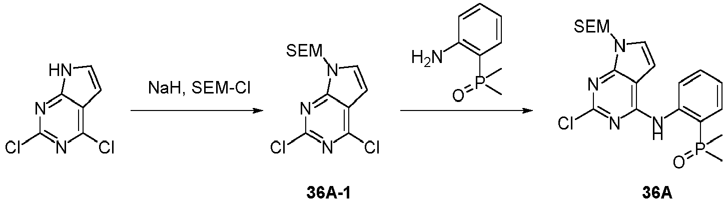

### Compound 36A-1:

2,4-Dichloro-7*H*-pyrrolo[2,3-*d*]pyrimidine (2.5 g, 13.3 mmol) was dissolved in *N,N-*dimethylformamide (25 mL) under nitrogen atmosphere. Subsequently, Sodium hydride (60%, 0.69 g, 17.3 mmol) was added in batches to the reaction liquid at 0 °C, and the reaction liquid was successively stirred at that temperature for 30 min. 2-(Trimethylsilyl)ethoxymethyl chloride (2.88 g, 17.3 mmol) was added to the reaction liquid. The reaction system was warmed to room temperature and successively stirred for 1 h. After the starting material disappeared as detected by LCMS, the reaction liquid was quenched by addition of water (100 mL). The mixture was extracted with ethyl acetate (100 mL × 2), and the organic phases were combined, washed with saturated brine (100 mL × 3) firstly, then dried over anhydrous sodium sulfate, filtered, and concentrated under reduced pressure. The resulting residue was purified by silica gel column chromatography (eluent: petroleum ether/ethyl acetate = 5/1) to obtain compound **36A-1** (3.9 g).

MS (ESI) M/Z: 318.1, 320.1 [M + H]⁺.

### Intermediate 36A:

Compound **36A** (1.1 g) was prepared by replacing the starting material with compound **36A-1** (1.1 mg, 3.55 mmol) and 2-(dimethylphosphoryl)aniline (0.5 g, 2.96 mmol) according to the method for the preparation of compound **1B** from compound **1B-2** in Example 1.

MS (ESI, m/z): 451.2, 452.2 [M + H]⁺.

### Preparation of compound 36

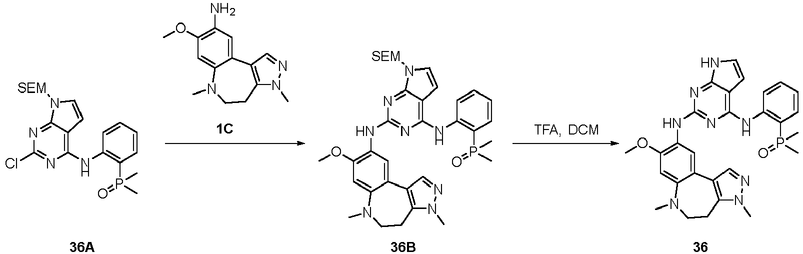

### Compound 36B:

Compound **36A** (200 mg, 0.44 mmol), compound **1C** (126 mg, 0.49 mmol), tris(dibenzylideneacetone)dipalladium (41 mg, 0.044 mmol), 2-dicyclohexylphosphonium-2',4',6'-triisopropylbiphenyl (32 mg, 0.07 mmol), and potassium carbonate (123 mg, 0.89 mmol) were dissolved in tert-butanol (8 mL) under nitrogen atmosphere. The reaction system was heated to 110 °C and successively stirred for 2 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound **36B** (260 mg).

MS (ESI) M/Z: 673.4 [M + H]⁺.

### Compound 36:

Compound **36B** (260 mg, 0.39 mmol) was dissolved in dichloromethane (5 mL). Subsequently, trifluoroacetic acid (1.5 mL) was added to the above reaction liquid, and the reaction liquid was successively stirred at room temperature for 1.5 h. After the starting material disappeared as detected by LCMS, the reaction liquid was concentrated under reduced pressure. Methanol (5 mL) and potassium carbonate (300 mg) were added to the residue, and the reaction liquid was successively stirred at room temperature for 30 min. The reaction liquid was filtered, and the filtrate was concentrated under reduced pressure. The resulting residue was purified by silica gel column chromatography (eluent: dichloromethane/methanol = 10/1) to obtain compound 36 (45 mg). MS (ESI) M/Z: 543.3 [M + H]⁺.

¹H NMR (400 MHz, CDCl₃) δ 11.26 (s, 1H), 9.69 (s, 1H), 8.89 (dd, *J* = 8.4, 4.4 Hz, 1H), 8.50 (s, 1H), 7.62 (s, 1H), 7.41 (s, 1H), 7.30-7.14 (m, 2H), 7.00-6.85 (m, 1H), 6.79 (d, *J=* 3.6 Hz, 1H), 6.56 (d, *J* = 4.0 Hz, 2H), 3.90 (s, 3H), 3.82 (s, 3H), 3.29 (t, *J* = 5.6 Hz, 2H), 3.04 (d, *J* = 5.6 Hz, 2H), 3.00 (s, 3H), 1.86 (s, 3H), 1.82 (s, 3H).

### Example 37:

### Preparation of compound 37

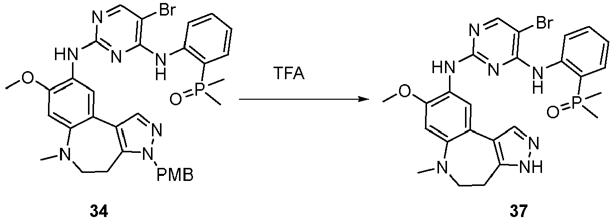

Compound **34** (40 mg, 0.06 mmol) was dissolved in trifluoroacetic acid (1 mL). The reaction system was heated to 100 °C and successively stirred for 6 h. After the starting material disappeared as detected by LCMS, the reaction liquid was cooled to room temperature and concentrated under reduced pressure. The resulting residue was prepared under high pressure (preparation conditions: reverse phase column: XBridge Shield RP18 OBD Column, 30 × 150 mm, 5 □m; mobile phase A: water (0.1% formic acid), and mobile phase B: acetonitrile; flow rate: 25 mL/min; gradient: mobile phase B elevating from 10% to 90% in 28 min; detection wavelength: 254 nm) to obtain compound **37** (10 mg).

MS (ESI) M/Z: 568.0, 570.0 [M + H]⁺.

¹H NMR (300 MHz, CDCl₃) δ 10.71 (s, 1H), 8.45-8.43 (m, 2H), 8.22 (s, 1H), 7.55 (s, 1H), 7.35-7.30 (m, 3H), 7.18-7.11 (m, 1H), 7.02-6.98 (m, 1H), 6.61 (s, 1H), 3.92 (s, 3H), 3.31-3.06 (m, 7H), 1.89 (s, 3H), 1.85 (s, 3H).

### Example 38:

### Preparation of intermediate 38A

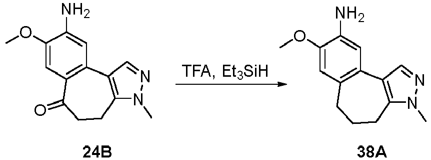

Compound **24B** (100 mg, 0.39 mmol) and triethylsilane (226 mg, 1.9 mmol) were dissolved in trifluoroacetic acid (4 mL). The reaction system was heated to 60 °C and successively stirred for 5 h. After the starting material disappeared as detected by LCMS, the reaction liquid was cooled to room temperature and concentrated under reduced pressure. The resulting residue was purified by a C18 reverse phase column. Purification conditions: a chromatography column: 40 g of C18 reverse phase column; mobile phase: water (containing 0.1% formic acid) and acetonitrile; flow rate: 35 mL/min; gradient: acetonitrile elevating from 5% to 95% in 20 min; detection wavelength: 254 nm. The product was collected and lyophilized under reduced pressure to obtain compound **38A** (90 mg).

MS (ESI, m/z): 244.2 [M + H]⁺.

### Preparation of compound 38

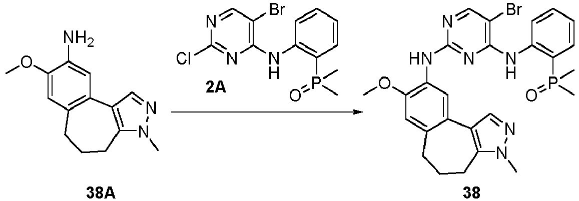

Compound **38** (10 mg) was prepared by replacing the starting material with compound **38A** (90 mg, 0.37 mmol) and compound **2A** (133 mg, 0.37 mmol) according to the method for the preparation of compound **1** from compound **1B** and compound **1C** in Example 1.

MS (ESI, m/z): 567.2, 569.2 [M + H]⁺.

¹H NMR (400 MHz, CDCl₃) δ 10.80 (s, 1H), 8.45-8.42 (m, 1H), 8.31 (s, 1H), 8.18 (s, 1H),7.52 (s, 1H) 7.32-7.28 (m, 1H), 7.18-7.12 (m, 2H), 7.03-6.99 (m, 1H), 6.66 (s, 1H), 3.88 (s, 3H), 3.78 (s, 3H), 2.93-2.89 (m, 2H), 2.81-2.79 (m, 2H), 2.14-2.07 (m, 2H), 1.86 (s, 3H), 1.83 (s, 3H).

### Example 39:

### Preparation of compound 39

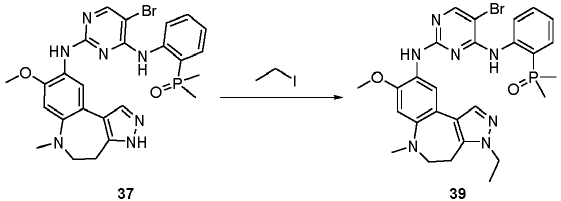

Compound **37** (100 mg, 0.18 mmol) and potassium carbonate (49 mg, 0.35 mmol) were dissolved in *N*,*N-*dimethylformamide (2 mL). Iodothane (41 mg, 0.26 mmol) was added to the reaction liquid. The reaction system was heated to 50 °C and successively stirred for 2 h. After the starting material disappeared as detected by LCMS, the reaction liquid was cooled to room temperature and added with water (10 mL). The mixture was extracted with ethyl acetate (10 mL × 2), and the organic phases were combined, washed with saturated brine (10 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **39** (10 mg).

MS (ESI) M/Z: 595.9, 597.9 [M + H]⁺.

¹H NMR (300 MHz, CDCl₃) δ 10.78 (s, 1H), 8.63 (dd, *J* = 8.4, 4.5 Hz, 1H), 8.30 (s, 1H), 8.22 (s, 1H), 7.26-7.18 (m, 2H), 7.10 (s, 1H), 6.98-6.93 (m, 1H), 6.54 (s, 1H), 3.99 (q, *J* = 7.2 Hz, 2H), 3.89 (s, 3H), 3.30 (br s, 2H), 3.15 (br s, 2H), 3.03 (s, 3H), 1.87 (s, 3H), 1.82 (s, 3H), 1.37 (t, *J=* 7.2 Hz, 3H).

### Example 40:

### Preparation of intermediate 40A

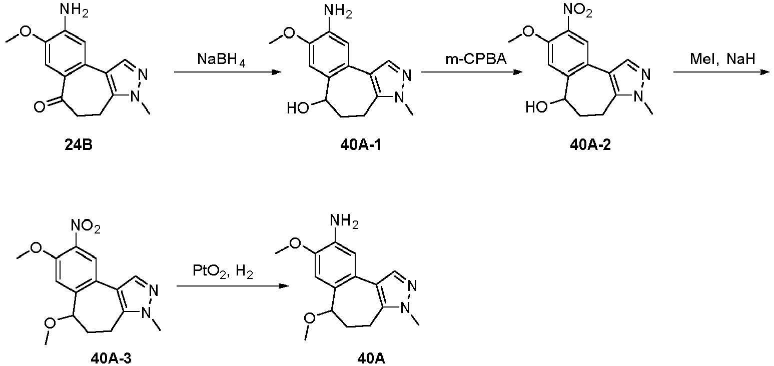

### Compound 40A-1:

Compound **40A-1** (400 mg) was prepared by replacing the starting material with compound **24B** (700 mg, 2.72 mmol) according to the method for the preparation of compound **24** from compound 24C in Example 24.

MS (ESI, m/z): 260.1 [M + H]⁺.

### Compound 40A-2:

Compound **40A-1** (400 mg, 1.54 mmol) was dissolved in 1,2-dichloroethane (8 mL). Subsequently, *m*-chloroperoxybenzoic acid (799 mg, 4.63 mmol) was added to the reaction liquid. The reaction system was heated to 80 °C and successively stirred for 1 h. After the starting material disappeared as detected by LCMS, the reaction liquid was cooled to room temperature and added with water (20 mL). The mixture was extracted with dichloromethane (20 mL × 2), and the organic phases were combined, washed with saturated brine (20 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **40A-2** (160 mg).

MS (ESI, m/z): 290.1 [M + H]⁺.

### Compound 40A-3:

Compound **40A-2** (75 mg, 0.26 mmol) was dissolved in N,N-dimethylformamide (2 mL) under nitrogen atmosphere. Subsequently, Sodium hydride (60%, 31 mg, 0.78 mmol) was added in batches to the reaction liquid at 0 °C, and the reaction liquid was successively stirred at that temperature for 30 min. Iodomethane (110 mg, 0.78 mmol) was added to the reaction liquid. The reaction system was warmed to room temperature and successively stirred for 2 h. After the starting material disappeared as detected by LCMS, the reaction liquid was quenched by addition of water (20 mL). The mixture was extracted with ethyl acetate (20 mL × 2), and the organic phases were combined, washed with saturated brine (20 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **40A-3** (75 mg).

MS (ESI) M/Z: 304.1 [M + H]⁺.

### Intermediate 40A:

Compound **40A** (65 mg) was prepared by replacing the starting material with compound **40A-3** (75 mg, 0.25 mmol) according to the method for the preparation of compound **8A** from compound **8A-9** in Example 8.

MS (ESI, m/z): 274.1 [M + H]⁺.

### Preparation of compound 40

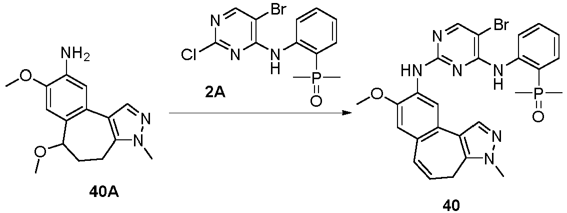

Compound **40** (10 mg) was prepared by replacing the starting material with compound **40A** (65 mg, 0.24 mmol) and compound **2A** (86 mg, 0.24 mmol) according to the method for the preparation of compound **1** from compound **1B** and compound **1C** in Example 1.

MS (ESI, m/z): 564.9, 566.9 [M + H]⁺.

¹H NMR (400 MHz, CDCl₃) δ 10.70 (s, 1H), 8.53-8.34 (m, 2H), 8.23 (s, 1H), 7.76 (s, 1H), 7.34-7.28 (m, 1H), 7.16-7.12 (m, 2H), 7.03-6.99 (m, 1H), 6.74 (s, 1H), 6.51 (d, *J=* 11.2 Hz, 1H), 5.82-5.76 (m, 1H), 3.92 (s, 3H), 3.86 (s, 3H), 3.33 (d, *J=* 6.0 Hz, 2H), 1.87 (s, 3H), 1.84 (s, 3H).

### Example 41:

### Preparation of intermediate 41A

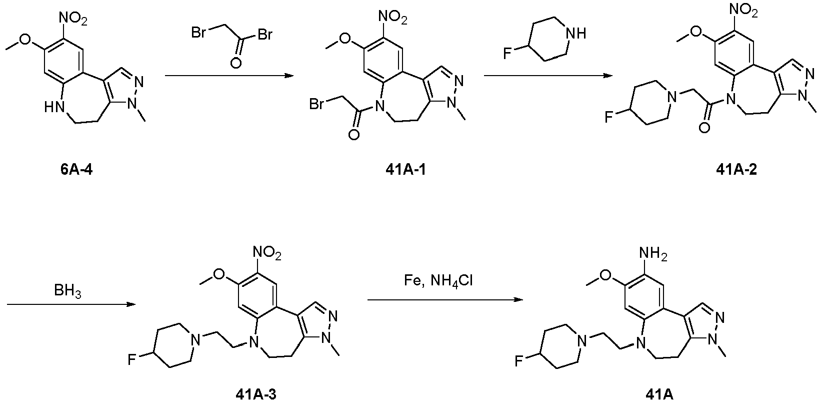

### Compound 41A-1:

Compound **6A-4** (500 mg, 1.8 mmol) and triethylamine (553 mg, 5.5 mmol) were dissolved in dichloromethane (10 mL). Subsequently, bromoacetyl bromide (736 mg, 3.65 mmol) was added to the reaction liquid at 0 °C. The reaction system was warmed to room temperature and successively stirred for 16 h. After the starting material disappeared as detected by LCMS, the reaction liquid was quenched by addition of water (50 mL). The mixture was extracted with ethyl acetate (50 mL × 2), and the organic phases were combined, washed with saturated brine (30 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **41A-1** (720 mg).

MS (ESI) M/Z: 395.0, 397.0 [M + H]⁺.

### Compound 41A-2:

Compound **41A-1** (670 mg, 1.7 mmol) and diisopropylethylamine (657 mg, 5.09 mmol) were dissolved in *N*,*N*-dimethylformamide (15 mL). Subsequently, 4-fluoropiperidine hydrochloride (355 mg, 2.54 mmol) was added to the reaction liquid, and the reaction liquid was successively stirred at room temperature for 16 h. After the starting material disappeared as detected by LCMS, the reaction liquid was quenched by addition of water (50 mL). The mixture was extracted with ethyl acetate (50 mL × 2), and the organic phases were combined, washed with saturated brine (50 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **41A-2** (690 mg).

MS (ESI) M/Z: 417.9 [M + H]⁺.

### Compound 41A-3:

Compound **41A-3** (298 mg) was prepared by replacing the starting material with compound **41A-2** (450 mg, 1.08 mmol) according to the method for the preparation of compound **11A-1** from compound **9A-4** in Example 11.

MS (ESI, m/z): 404.1 [M + H]⁺.

### Intermediate 41A:

Compound **41A** (90 mg) was prepared by replacing the starting material with compound **41A-3** (100 mg, 0.25 mmol) according to the method for the preparation of compound **1C** from compound **1C-9** in Example 1.

MS (ESI, m/z): 374.1 [M + H]⁺.

### Preparation of compound 41

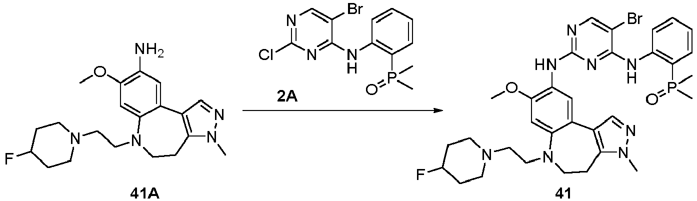

Compound **41** (64 mg) was prepared by replacing the starting material with compound **41A** (90 mg, 0.24 mmol) and compound **2A** (87 mg, 0.24 mmol) according to the method for the preparation of compound **1** from compound **1B** and compound **1C** in Example 1.

MS (ESI, m/z): 697.1, 699.1 [M + H]⁺.

¹H NMR (300 MHz, CDCl₃) δ 10.57 (s, 1H), 8.53-8.39 (m, 2H), 8.24 (s, 1H), 7.39-7.29 (m, 2H), 7.28-7.16 (m, 2H), 7.05-6.94 (m, 1H), 6.59 (s, 1H), 4.88-4.72 (m, 1H), 3.91 (s, 3H), 3.84 (s, 3H), 3.75-3.70 (m, 2H), 3.39-3.35 (m, 2H), 3.10-2.65 (m, 8H), 2.23-1.91 (m, 4H), 1.89 (s, 3H), 1.85 (s, 3H).

### Example 42:

### Preparation of intermediate 42A

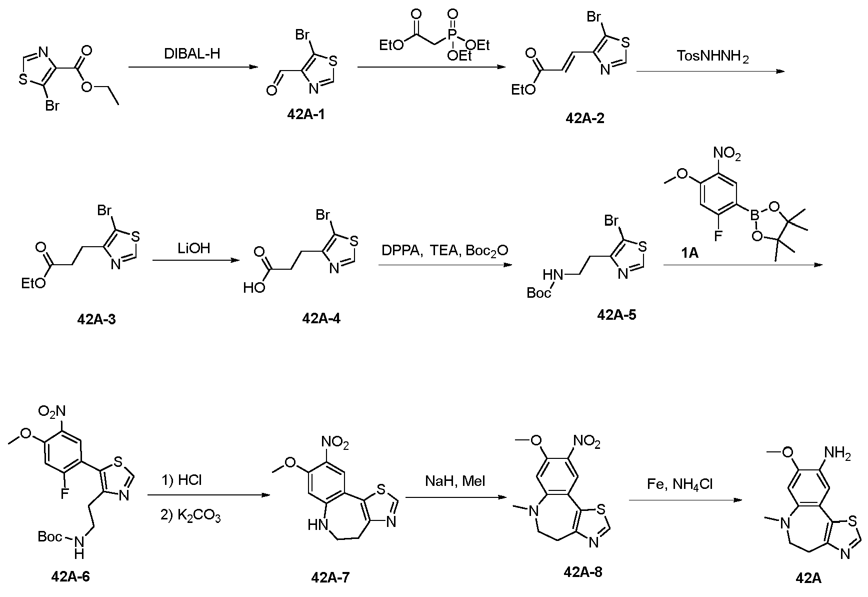

### Compound 42A-1:

*Ethyl* 5-bromothiazole-4-formate (10 g, 42.36 mmol) was dissolved in dichloromethane (80 mL) under nitrogen atmosphere. Subsequently, a solution of diisobutylaluminum hydride in toluene (1.5 M, 56.5 mL, 84.74 mmol) was added to the reaction liquid at -78 °C, and the reaction liquid was successively stirred at that temperature for 3 h. After the starting material disappeared as detected by LCMS, saturated aqueous sodium potassium tartrate (200 mL) and dichloromethane (100 mL) were added to the reaction liquid, and the reaction liquid was successively stirred at room temperature for 1 h. The mixture was extracted with dichloromethane (100 mL × 2), and the organic phases were combined, washed with saturated brine (100 mL × 3) firstly, then dried over anhydrous sodium sulfate, filtered, and concentrated under reduced pressure. The resulting residue was purified by silica gel column chromatography (eluent: petroleum ether/ethyl acetate = 1/1) to obtain compound **42A-1** (6 g).

MS (ESI, m/z): 191.9, 193.9 [M + H]⁺.

### Compound 42A-2:

Compound **42A-2** (7.4 g) was prepared by replacing the starting material with compound **42A-1** (6 g, 31.25 mmol) according to the method for the preparation of compound **1C-1** in Example 1. MS (ESI, m/z): 261.7, 263.7 [M + H]⁺.

### Compound 42A-3:

Compound **42A-2** (7.4 g, 28.23 mmol), sodium acetate (4.63 g, 56.46 mmol) and *p-*toluenesulfonylhydrazide (21.03 g, 112.9 mmol) were dissolved in ethanol (80 mL). The reaction system was heated to 90 °C and successively stirred for 3 h. After the starting material disappeared as detected by LCMS, the reaction liquid was cooled to room temperature and concentrated under reduced pressure. The residue was added with water (100 mL). The mixture was extracted with ethyl acetate (100 mL × 2), and the organic phases were combined, washed with saturated brine (100 mL × 3) firstly, then dried over anhydrous sodium sulfate, filtered, and concentrated under reduced pressure. The resulting residue was purified by silica gel column chromatography (eluent: petroleum ether/ethyl acetate = 5/1) to obtain compound **42A-3** (6.4 g).

MS (ESI) M/Z: 263.9, 265.9 [M + H]⁺.

### Compound 42A-4:

Compound **42A-3** (3.3 g, 12.49 mmol) was dissolved in a mixed solvent of tetrahydrofuran (30 mL) and methanol (10 mL). Lithium hydroxide (1.2 g, 49.97 mmol) and water (10 mL) were added to the reaction liquid. The reaction system was heated to 50 °C and successively stirred for 5 h. After the starting material disappeared as detected by LCMS, the reaction liquid was cooled to room temperature and concentrated under reduced pressure. The reaction liquid was added with water (20 mL) for dilution and adjusted to pH = 4 with 1 M hydrochloric acid. The mixture was extracted with ethyl acetate (100 mL × 2), and the organic phases were combined, washed with saturated brine (50 mL × 3) firstly, then dried over anhydrous sodium sulfate, filtered, and concentrated under reduced pressure to obtain compound **42A-4** (2.5 g).

MS (ESI) M/Z: 234.0, 236.0 [M - H]⁻.

### Compound 42A-5:

Compound **42A-5** (2.5 g) was prepared by replacing the starting material with compound **42A-4** (3 g, 12.7 mmol) according to the method for the preparation of compound **1C-4** from compound **1C-**3 in Example 1.

MS (ESI, m/z): 306.8, 308.8 [M + H]⁺.

### Compound 42A-6:

Compound **42A-6** (2.8 g) was prepared by replacing the starting material with compound **42A-5** (2.5 g, 8.14 mmol) according to the method for the preparation of compound **1C-7** from compound **1C-6** in Example 1.

MS (ESI, m/z): 398.1 [M + H]⁺.

### Compound 42A-7:

Compound **42A-6** (1 g, 2.52 mmol) was dissolved in dichloromethane (10 mL). Subsequently, a solution of hydrogen chloride in ethyl acetate (4 M, 5 mL) was added to the reaction liquid, and the reaction liquid was successively stirred at room temperature for 1 h. After the starting material disappeared as detected by LCMS, the reaction liquid was concentrated under reduced pressure. The resulting residue was dissolved in N,N-dimethylformamide (10 mL), and the reaction liquid was added with potassium carbonate (1.74 g, 12.6 mmol). The reaction system was heated to 90°C and successively stirred for 1 h. After the starting material disappeared as detected by LCMS, the reaction liquid was cooled to room temperature and added with water (50 mL). The mixture was extracted with ethyl acetate (50 mL × 3), and the organic phases were combined, washed with saturated brine (50 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **42A-7** (0.4 g).

MS (ESI) M/Z: 277.9 [M + H]⁺.

### Compound 42A-8:

Compound **42A-8** (117 mg) was prepared by replacing the starting material with compound **42A-7** (150 mg, 0.54 mmol) according to the method for the preparation of compound **8A-9** from compound **8A-8** in Example 8.

MS (ESI, m/z): 292.2 [M + H]⁺.

### Intermediate 42A:

Compound **42A** (92 mg) was prepared by replacing the starting material with compound **42A-8** (117 mg, 0.4 mmol) according to the method for the preparation of compound **1C** from compound **1C-9** in Example 1.

MS (ESI, m/z): 262.3 [M + H]⁺.

### Preparation of compound 42

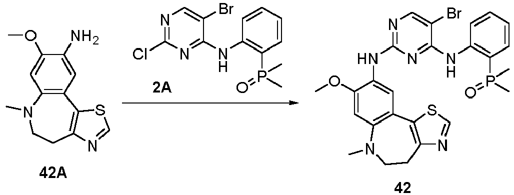

Compound **42** (35 mg) was prepared by replacing the starting material with compound **42A** (70 mg, 0.27 mmol) and compound **2A** (96 mg, 0.27 mmol) according to the method for the preparation of compound 1 from compound **1B** and compound **1C** in Example 1.

MS (ESI, m/z): 585.1, 587.1 [M + H]⁺.

¹H NMR (300 MHz, CDCl₃) δ 10.75 (s, 1H), 8.47 (s, 1H), 8.45-8.40 (m, 1H), 8.37 (s, 1H), 8.22 (s, 1H), 7.55 (s, 1H), 7.26-7.21 (m, 1H), 7.15-7.09 (m, 1H), 6.97-6.92 (m, 1H), 6.54 (s, 1H), 3.93 (s, 3H), 3.36 (br s, 4H), 3.08 (s, 3H), 1.89 (s, 3H), 1.85 (s, 3H).

### Example 43:

### Preparation of intermediate 43A

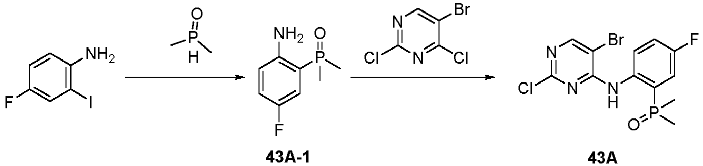

### Compound 43A-1

2-Iodo-4-fluoroaniline (3 g, 12.66 mmol), dimethylphosphine oxide (1.18 g, 15.2 mmol), palladium acetate (142 mg, 0.63 mmol), 4,5-bis(diphenylphosphino)-9,9-dimethylxanthene (549 mg, 0.95 mmol) and diisopropylethylamine (3.27 g, 25.32 mmol) were dissolved in *N*,*N-*dimethylformamide (30 mL) under nitrogen atmosphere. The reaction system was heated to 120 °C and successively stirred for 16 h. After the starting material disappeared as detected by LCMS, the reaction liquid was concentrated under reduced pressure. The resulting residue was purified by silica gel column chromatography (eluent: dichloromethane/methanol = 10/1) to obtain compound **43A-1** (0.65 g). MS (ESI) M/Z: 187.9 [M + H]⁺.

### Intermediate 43A:

Compound **43A** (200 mg) was prepared by replacing the starting material with compound **43A-1** (450 mg, 2.4 mmol) according to the method for the preparation of compound **2A** in Example 2. MS (ESI, m/z): 377.6, 379.6 [M + H]⁺.

### Preparation of compound 43

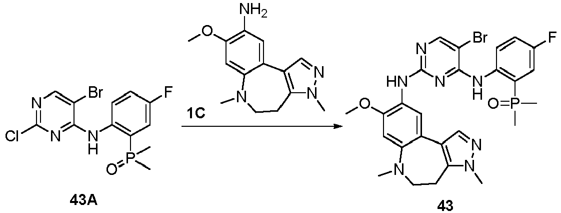

Compound **43** (10 mg) was prepared by replacing the starting material with compound **43A** (33 mg, 0.087 mmol) according to the method for the preparation of compound **1** from compound **1B** and compound **1C** in Example 1.

MS (ESI, m/z): 600.2, 602.2 [M + H]⁺.

¹H NMR (300 MHz, CDCl₃) δ 10.39 (s, 1H), 8.36-8.30 (m, 2H), 8.20 (s, 1H), 7.46 (s, 1H), 7.18 (s, 1H), 7.01-6.93 (m, 1H), 6.77-6.71 (m, 1H), 6.57 (s, 1H), 3.92 (s, 3H), 3.84 (s, 3H), 3.32 (br s, 2H), 3.06 (br s, 5H), 1.89 (s, 3H), 1.85 (s, 3H).

### Example 44:

### Preparation of intermediate 44A

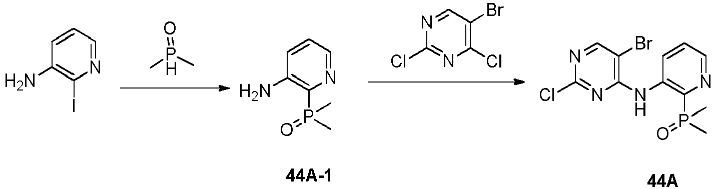

### Compound 44A-1:

Compound **44A-1** (300 mg) was prepared by replacing the starting material with 2-iodo-3-aminopyridine (500 mg, 2.27 mmol) according to the method for the preparation of compound **43A** in Example 43.

MS (ESI, m/z): 171.1 [M + H]⁺.

### Intermediate 44A:

Compound **44A** (730 mg) was prepared by replacing the starting material with compound **44A-1** (800 mg, 4.7 mmol) according to the method for the preparation of compound **2A** in Example 2. MS (ESI, m/z): 360.9, 362.9 [M + H]⁺.

### Preparation of compound 44

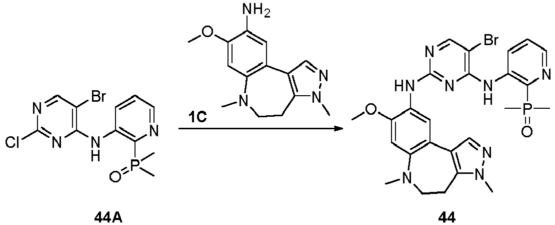

Compound **44** (33 mg) was prepared by replacing the starting material with compound **44A** (80 mg, 0.22 mmol) according to the method for the preparation of compound **1** from compound **1B** and compound **1C** in Example 1.

MS (ESI, m/z): 583.1, 585.1 [M + H]⁺.

¹H NMR (300 MHz, CDCl₃) δ 11.04 (s, 1H), 9.01-8.96 (m, 1H), 8.29 (s, 1H), 8.24 (s, 1H), 8.19-8.17 (m, 1H), 7.48 (s, 1H), 7.19 (s, 1H), 6.96 (s, 1H), 6.58 (s, 1H), 3.91 (s, 3H), 3.85 (s, 3H), 3.32 (br s, 2H), 3.07 (br s, 5H), 1.91 (s, 3H), 1.86 (s, 3H).

### Example 45:

### Preparation of compound 45

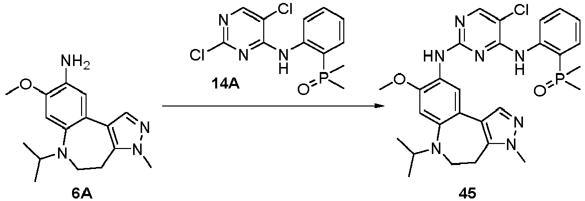

Compound **45** (49 mg) was prepared by replacing the starting material with compound **14A** (60 mg, 0.19 mmol) and compound **6A** (49 mg, 0.19 mmol) according to the method for the preparation of compound **1** from compound **1B** and compound **1C** in Example 1.

MS (ESI, m/z): 566.1, 568.1 [M + H]⁺.

¹H NMR (300 MHz, CDCl₃) δ 10.79 (s, 1H), 8.64-8.59 (m, 1H), 8.42 (s, 1H), 8.13 (s, 1H), 7.38 (s, 1H), 7.31-7.23 (m, 1H), 7.19 (s, 2H), 7.01-6.95 (m, 1H), 6.58 (s, 1H), 3.88 (br s, 4H), 3.82 (s, 3H), 3.30 (br s, 2H), 2.95 (br s, 2H), 1.87 (s, 3H), 1.83 (s, 3H), 1.33 (s, 3H), 1.27 (s, 3H).

### Example 46:

### Preparation of intermediate 46A

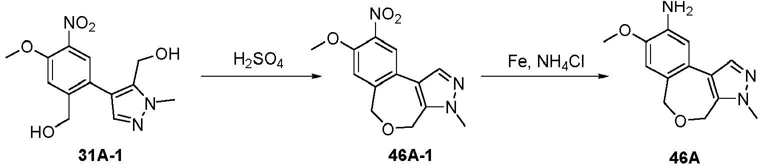

### Compound 46A-1:

Compound **31A-1** (180 mg, 0.61 mmol) was dissolved in 50% aqueous sulfuric acid (5 mL). The reaction system was heated to 85 °C and successively stirred for 4 h. After the starting material disappeared as detected by LCMS, the reaction liquid was cooled to room temperature and adjusted to pH = 8 with 1 M sodium hydroxide. The mixture was extracted with ethyl acetate (30 mL × 3), and the organic phases were combined, washed with saturated brine (30 mL × 3) firstly, then dried over anhydrous sodium sulfate, filtered, and concentrated under reduced pressure. The resulting residue was purified by silica gel column chromatography (eluent: ethyl acetate) to obtain compound **46A-1** (35 mg).

MS (ESI) M/Z: 276.0 [M + H]⁺.

### Intermediate 46A:

Compound **46A** (35 mg) was prepared by replacing the starting material with compound **46A-1** (45 mg, 0.16 mmol) according to the method for the preparation of compound **1C** from compound **1C-**9 in Example 1.

MS (ESI, m/z): 246.1 [M + H]⁺.

### Preparation of compound 46

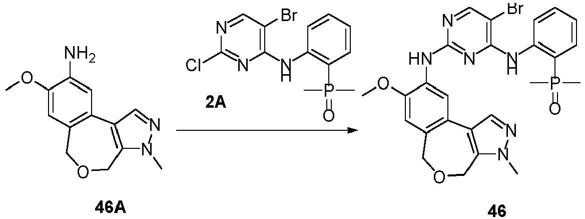

Compound **46** (19 mg) was prepared by replacing the starting material with compound **46A** (35 mg, 0.14 mmol) and compound **2A** (51 mg, 0.14 mmol) according to the method for the preparation of compound **1** from compound **1B** and compound **1C** in Example 1.

MS (ESI, m/z): 569.2, 571.2 [M + H]⁺.

¹H NMR (400 MHz, CDCl₃) δ 10.55 (s, 1H), 8.56 (s, 1H), 8.42 (dd, *J=* 8.4, 4.4 Hz, 1H), 8.26 (s, 1H), 7.57 (s, 1H), 7.37-7.30 (m, 1H), 7.25-7.21 (m, 1H), 7.10 (s, 1H), 7.08-6.99 (m, 1H), 6.71 (s, 1H), 5.04 (s, 2H), 4.64 (s, 2H), 3.90 (s, 3H), 3.74 (s, 3H), 1.88 (s, 3H), 1.85 (s, 3H).

### Example 47:

### Preparation of intermediate 47A

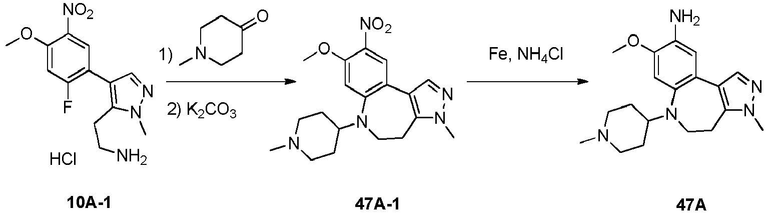

### Compound 47A-1:

Compound **10A-1** (200 mg, 0.61 mmol), N-methyl-4-piperidone (342 mg, 3.03 mmol) and sodium acetate (99 mg, 1.21 mmol) were dissolved in methanol (3 mL), and the reaction liquid was stirred at room temperature for 30 min. The reaction liquid was added with acetic acid (73 mg, 1.21 mmol) and sodium cyanoborohydride (76 mg, 1.21 mmol). The reaction system was successively stirred at room temperature for 16 h. After the starting material disappeared as detected by LCMS, the reaction liquid was concentrated under reduced pressure. The resulting residue was dissolved in *N*,*N*-dimethylformamide (3 mL), and the reaction liquid was added with potassium carbonate (200 mg). The reaction liquid was heated to 110 °C and successively stirred for 3 h. After the starting material disappeared as detected by LCMS, the reaction liquid was cooled to room temperature and poured into water (30 mL). The mixture was extracted with ethyl acetate (50 mL × 3), and the organic phases were combined, washed with saturated brine (50 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **47A-1** (160 mg).

MS (ESI) M/Z: 372.2 [M + H]⁺.

### Intermediate 47A:

Compound **47A** (130 mg) was prepared by replacing the starting material with compound **47A-1** (160 mg, 0.43 mmol) according to the method for the preparation of compound **1C** from compound **1C-9** in Example 1.

MS (ESI, m/z): 342.2 [M + H]⁺.

### Preparation of compound 47

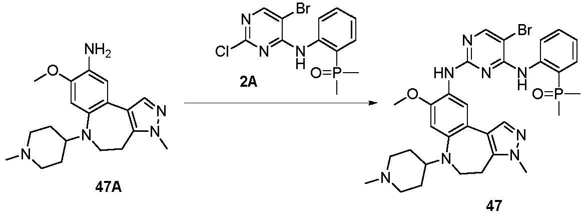

Compound **47** (44 mg) was prepared by replacing the starting material with compound **47A** (130 mg, 0.38 mmol) and compound **2A** (137 mg, 0.38 mmol) according to the method for the preparation of compound **1** from compound **1B** and compound **1C** in Example 1.

MS (ESI, m/z): 665.0, 667.0 [M + H]⁺.

¹H NMR (300 MHz, CDCl₃) δ 10.57 (s, 1H), 8.49-8.45 (m, 2H), 8.24 (s, 1H), 7.35-7.17 (m, 4H), 7.03-6.98 (m, 1H), 6.52 (s, 1H), 3.86 (s, 3H), 3.80 (s, 3H), 3.52-3.45 (m, 1H), 3.43-3.36 (m, 4H), 2.94 (t, *J* = 5.7 Hz, 2H), 2.59 (s, 3H), 2.57-2.50 (m, 2H), 2.31-2.15 (m, 2H), 2.02-1.92 (m, 2H), 1.86 (s, 3H), 1.82 (s, 3H).

### Example 48:

### Preparation of intermediate 48A

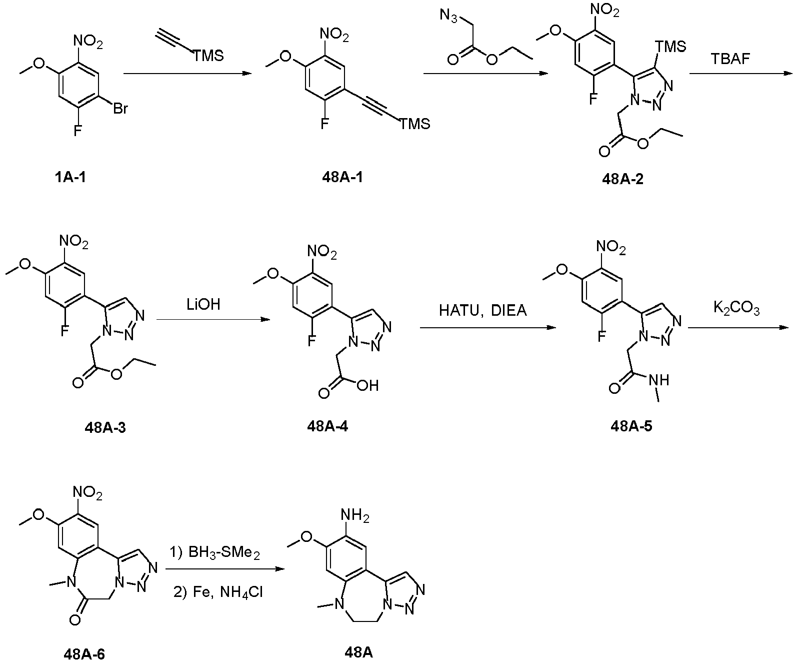

### Compound 48A-1:

Compound **1A-1** (2 g, 8.0 mmol), trimethylsilylacetylene (2.36 g, 24 mmol), tetrakis(triphenylphosphine)palladium (1.85 g, 1.6 mmol) and copper(I) iodide (0.15 g, 0.8 mmol) were dissolved in triethylamine (20 mL) under nitrogen atmosphere. The reaction system was heated to 80 °C and successively stirred for 16 h. After the starting material disappeared as detected by LCMS, the reaction liquid was cooled to room temperature and concentrated under reduced pressure. The resulting residue was purified by silica gel column chromatography (eluent: petroleum ether/ethyl acetate = 15/1) to obtain compound **48A-1** (1.73 g).

¹H NMR (400 MHz, CDCl₃) δ 8.07 (d, *J* = 7.2 Hz, 1H), 6.79 (d, *J* = 10.4 Hz, 1H), 3.97 (s, 3H), 0.26 (s, 9H).

### Compound 48A-2:

Compound **48A-1** (2.6 g, 9.7 mmol) and ethyl azidoacetate (1.26 g, 9.7 mmol) were dissolved in toluene (30 mL). The reaction system was heated to 100°C and successively stirred for 2 days. After the starting material disappeared as detected by LCMS, the reaction liquid was cooled to room temperature and concentrated under reduced pressure. The resulting residue was purified by silica gel column chromatography (eluent: petroleum ether/ethyl acetate = 2/1) to obtain compound **48A-2** (2.8 g).

MS (ESI, *m*/*z):* 397.2 [M + H]⁺.

¹H NMR (300 MHz, CDCl₃) δ 7.95 (d, *J* = 7.5 Hz, 1H), 6.96 (d, *J* = 10.5 Hz, 1H), 5.03 (s, 2H), 4.20 (q, *J=* 7.2 Hz, 2H), 4.07 (s, 3H), 1.25 (t, *J=* 7.2 Hz, 3H), 0.23 (s, 9H).

### Compound 48A-3:

Compound **48A-2** (2.8 g, 7.1 mmol) was dissolved in tetrahydrofuran (20 mL) at 0 °C. Subsequently, tetrabutylammonium fluoride (1.85 g, 7.1 mmol) was added to the reaction liquid. The reaction liquid was warmed to room temperature and successively stirred for 2 h. After the starting material disappeared as detected by LCMS, the reaction liquid was cooled to room temperature and concentrated under reduced pressure. The resulting residue was purified by silica gel column chromatography (eluent: petroleum ether/ethyl acetate = 1/1) to obtain compound **48A-3** (1 g).

MS (ESI, *m*/*z):* 325.1 [M + H]⁺.

### Compound 48A-4:

Compound **48A-4** (200 mg) was prepared by replacing the starting material with compound **48A-3** (300 mg, 0.9 mmol) according to the method for the preparation of compound **42A-4** from compound **42A-3** in Example 42.

MS (ESI, m/z): 297.1 [M + H]⁺.

### Compound 48A-5:

Compound **48A-4** (300 mg, 1.0 mmol) and 2-(7-azabenzotriazole)-*N*,*N*,*N'*,*N'*-tetramethyluronium hexafluorophosphate (501 mg, 1.3 mmol) were dissolved in *N*,*N-*dimethylformamide (5 mL), and the reaction liquid was successively stirred at room temperature for 30 min. Subsequently, methylamine hydrochloride (137 mg, 2.0 mmol) and diisopropylethylamine (524 mg, 4.1 mmol) were added to the reaction liquid, and the reaction liquid was successively stirred at room temperature for 16 h. After the starting material disappeared as detected by LCMS, the reaction liquid was purified using reverse phase C18 column. The purification conditions: a chromatography column: 40 g of C18 reverse phase column; mobile phase: water (containing 10 mM ammonium bicarbonate) and acetonitrile; flow rate: 35 mL/min; gradient: acetonitrile elevating from 20% to 70% in 20 min; detection wavelength 254 nm. The product was collected and lyophilized under reduced pressure to obtain compound **48A-5** (100 mg).

MS (ESI, m/z): 310.1 [M + H]⁺.

### Compound 48A-6:

Compound **48A-5** (100 mg, 0.3 mmol) and potassium carbonate (89 mg, 0.6 mmol) were dissolved in *N*,*N*-dimethylformamide (1 mL). The reaction system was heated to 80 °C and successively stirred for 1 h. After the starting material disappeared as detected by LCMS, the reaction liquid was cooled to room temperature. The reaction liquid was purified by a C18 reverse phase column. The purification conditions: a chromatography column: 40 g of C18 reverse phase column; mobile phase: water (containing 10 mM ammonium bicarbonate) and acetonitrile; flow rate: 35 mL/min; gradient: acetonitrile elevating from 20% to 60% in 20 min; detection wavelength 254 nm. The product was collected and lyophilized under reduced pressure to obtain compound **48A-6** (45 mg). MS (ESI, m/z): 290.1 [M + H]⁺.

### Intermediate 48:

Compound **48A-6** (45 mg, 0.17 mmol) was dissolved in tetrahydrofuran (2 mL). Subsequently, borane dimethyl sulfide (59 mg, 0.78 mmol) was added to the reaction liquid, and the reaction liquid was successively stirred at room temperature for 2 h. After the starting material disappeared as detected by LCMS, the reaction liquid was quenched by slow addition of methanol (1 mL) and concentrated under reduced pressure. The residue was dissolved in ethanol (2 mL) and water (0.4 mL). Subsequently, iron powder (43 mg, 0.78 mmol) and ammonium chloride (13 mg, 0.23 mmol) were added. The reaction system was heated to 80°C and successively stirred for 2 h. After the starting material disappeared as detected by LCMS, the reaction liquid was cooled to room temperature and filtered, and the filtrate was concentrated under reduced pressure to obtain crude compound **48A** (40 mg).

MS (ESI, m/z): 246.2 [M + H]⁺.

### Preparation of compound 48

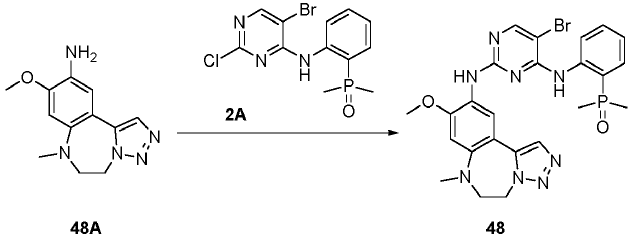

Compound **48** (25 mg) was prepared by replacing the starting material with compound **48A** (40 mg, 0.16 mmol) and compound **2A** (41 mg, 0.1 mmol) according to the method for the preparation of compound **1** from compound **1B** and compound **1C** in Example 1.

MS (ESI, m/z): 568.9, 570.9 [M + H]⁺.

¹H NMR (300 MHz, CDCl₃) δ 10.67 (s, 1H), 8.49 (s, 1H), 8.32 (s, 1H), 8.23 (s, 1H), 7.40-7.33 (m, 1H), 7.55 (m, 1 H), 7.15-7.03 (m, 3H), 6.53 (s, 1H), 4.77-4.67 (m, 2H), 3.97 (s, 3H), 3.60-3.57 (m, 2H), 3.09 (s, 3H), 1.91 (s, 3H), 1.87 (s, 3H).

### Example 49:

### Preparation of intermediate 49A

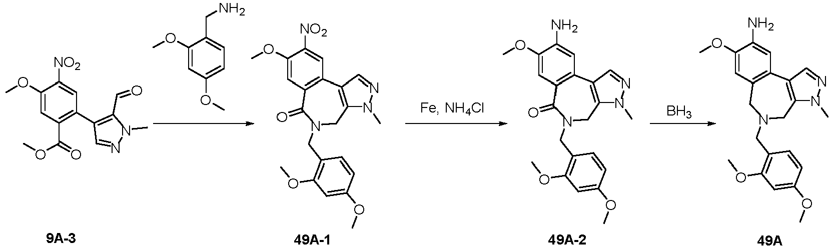

### Compound 49A-1:

Compound **49A-1** (410 mg) was prepared by replacing the starting material with 2,4-dimethoxybenzylamine (786 mg, 4.7 mmol) according to the method for the preparation of compound **9A-4** from compound **9A-3** in Example 9.

MS (ESI, m/z): 439.1 [M + H]⁺.

### Compound 49A-2:

Compound **49A-2** (308 mg) was prepared by replacing the starting material with compound **49A-1** (380 mg, 0.87 mmol) according to the method for the preparation of compound **1C** from compound **1C-9** in Example 1.

MS (ESI, m/z): 409.2 [M + H]⁺.

### Intermediate 49A:

Compound **49A** (212 mg) was prepared by replacing the starting material with compound **49A-1** (250 mg, 0.61 mmol) according to the method for the preparation of compound **11A-1** from compound **9A-4** in Example 11.

MS (ESI, m/z): 395.2 [M + H]⁺.

### Preparation of compound 49

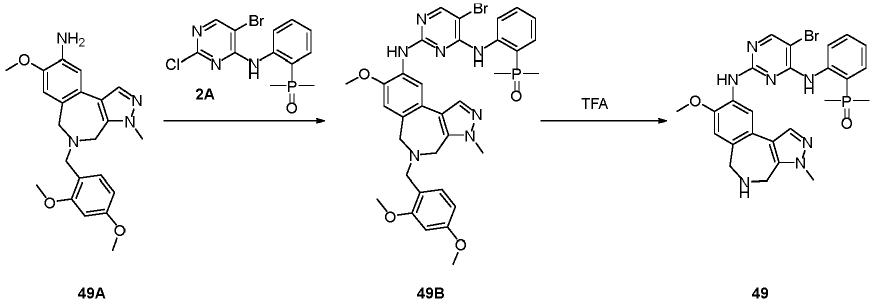

### Compound 49B:

Compound **49B** (117 mg) was prepared by replacing the starting material with compound **49A** (100 mg, 0.25 mmol) and compound **2A** (91 mg, 0.25 mmol) according to the method for the preparation of compound **1** from compound **1B** and compound **1C** in Example 1.

MS (ESI, m/z): 718.1, 720.1 [M + H]⁺.

### Compound 49:

Compound **49** (26 mg) was prepared by replacing the starting material with compound **49B** (100 mg, 0.14 mmol) according to the method for the preparation of compound **37** from compound **34** in Example 37.

MS (ESI, m/z): 568.1.1, 570.1 [M + H]⁺.

¹H NMR (300 MHz, CDCl₃) δ 10.60 (s, 1H), 8.56 (s, 1H), 8.47 (dd, *J* = 8.7, 4.5 Hz, 1H), 8.27 (s, 1H), 7.50 (s, 1H), 7.38-7.31 (m, 1H), 7.25 (d, *J=* 8.1 Hz, 1H), 7.19 (s, 1H), 7.09-7.04 (m, 1H), 6.70 (s, 1H), 4.27 (s, 2H), 3.97 (s, 2H), 3.92 (s, 3H), 3.79 (s, 3H), 1.90 (s, 3H), 1.85 (s, 3H).

### Example 50:

### Preparation of intermediate 50A

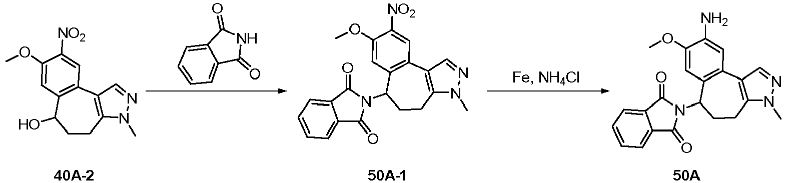

### Compound 50A-1:

Compound **40A-2** (150 mg, 0.52 mmol) and triphenylphosphine (204 mg, 0.78 mmol) were dissolved in tetrahydrofuran (5 mL) under nitrogen atmosphere. Subsequently, diethyl azodicarboxylate (135 mg, 0.78 mmol) was added to the reaction liquid at 0 °C. The reaction system was warmed to room temperature and successively stirred for 16 h. After the starting material disappeared as detected by LCMS, the reaction liquid was concentrated under reduced pressure. The resulting residue was purified by silica gel column chromatography (eluent: ethyl acetate) to obtain compound **50A-1** (70 mg).

MS (ESI, *m*/*z):* 419.0 [M + 1]⁺.

### Intermediate 50A:

Compound **50A** (56 mg) was prepared by replacing the starting material with compound **50A-1** (65 mg, 0.16 mmol) according to the method for the preparation of compound **1C** from compound **1C-9** in Example 1.

MS (ESI, m/z): 389.2 [M + H]⁺.

### Preparation of compound 50

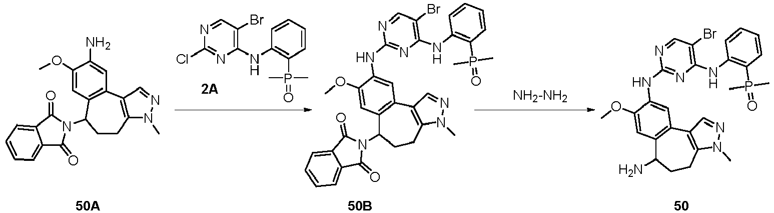

### Compound 50B:

Compound **50B** (38 mg) was prepared by replacing the starting material with compound **50A** (56 mg, 0.14 mmol) and compound **2A** (52 mg, 0.14 mmol) according to the method for the preparation of compound 1 from compound **1B** and compound **1C** in Example 1.

MS (ESI, m/z): 712.1, 714.1 [M + H]⁺.

### Compound 50:

Compound **50B** (38 mg, 0.05 mmol) was dissolved in methanol (2 mL). Subsequently, hydrazine hydrate (27 mg, 0.54 mmol) was added to the reaction liquid, and the reaction liquid was successively stirred at room temperature for 2 h. After the starting material disappeared as detected by LCMS, the reaction liquid was concentrated under reduced pressure. The resulting residue was purified by a C18 reverse phase column. Purification conditions: a chromatography column: 40 g of C18 reverse phase column; mobile phase: water (containing 0.1% formic acid) and acetonitrile; flow rate: 35 mL/min; gradient: acetonitrile elevating from 15% to 55% in 25 min; detection wavelength: 254 nm. The product was collected and lyophilized under reduced pressure to obtain compound 50 (8 mg).

MS (ESI, m/z): 582.1, 584.1 [M + H]⁺.

¹H NMR (300 MHz, CDCl₃) δ 10.53 (s, 1H), 8.55 (s, 1H), 8.40 (s, 1H), 8.23 (s, 1H), 8.22 (s, 1H), 7.50 (s, 1H),7.19 (s, 1H) 7.21 (s, 2H), 7.03 (s, 1H), 6.85 (s, 1H), 4.35 (s, 1H), 3.89 (s, 3H), 3.75 (s, 3H), 2.91 (br s, 2H), 2.22 (br s, 2H), 2.24 (br s, 2H), 1.88 (s, 3H).

### Example 51:

### Preparation of intermediate 51A

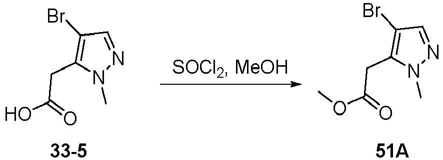

Compound **33-5** (4.9 g, 22.37 mmol) was dissolved in methanol (50 mL). Subsequently, thionyl chloride (3.99 g, 33.56 mmol) was added dropwise to the reaction liquid at 0 °C. The reaction liquid was warmed to room temperature and successively stirred for 16 h. After the starting material disappeared as detected by LCMS, the reaction liquid was concentrated under reduced pressure and added with water (50 mL). The reaction liquid was adjusted to pH = 7 with sodium hydroxide (1 N) at 0 °C. The mixture was extracted with dichloromethane (50 mL × 3), and the organic phases were combined, washed with saturated brine (50 mL × 3) firstly, then dried over anhydrous sodium sulfate, filtered, and concentrated under reduced pressure to obtain compound **51A** (4.4 g).

MS (ESI) M/Z: 233.1, 235.1 [M + H]⁺.

### Preparation of intermediate 51B

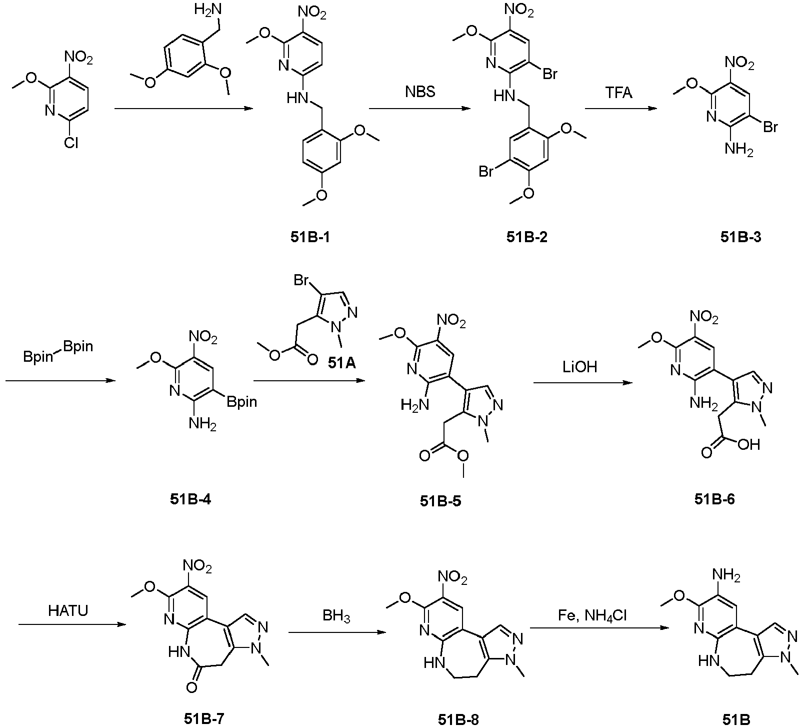

### Compound 51B-1:

The compounds 2-methoxy-3-nitro-6-chloropyridine (5 g, 26.5 mmol) and 2,4-dimethoxybenzylamine (4.43 g, 26.5 mmol) were dissolved in acetonitrile (50 mL) and *N,N-*dimethylformamide (25 mL). Subsequently, triethylamine (2.68 g, 26.5 mmol) was added to the reaction liquid, and the reaction liquid was successively stirred at room temperature for 5 h. After the starting material disappeared as detected by LCMS, the reaction liquid was quenched by addition of water (200 mL). The mixture was extracted with ethyl acetate (100 mL × 3), and the organic phases were combined, washed with saturated brine (80 mL × 3) firstly, then dried over anhydrous sodium sulfate, filtered, and concentrated under reduced pressure. The resulting residue was purified by silica gel column chromatography (eluent: petroleum ether/ethyl acetate = 1/1) to obtain compound **51B-1** (6 g).

MS (ESI) M/Z: 320.1 [M + H]⁺.

### Compound 51B-2:

Compound **51B-1** (6 g, 18.79 mmol) was dissolved in *N*,*N*-dimethylformamide (50 mL). Subsequently, N-bromosuccinimide (6.69 g, 37.58 mmol) was added to the reaction liquid, and the reaction liquid was successively stirred at room temperature for 16 h. After the starting material disappeared as detected by LCMS, the reaction liquid was quenched by addition of water (300 mL). The mixture was extracted with ethyl acetate (300 mL × 3), and the organic phases were combined, washed with saturated brine (200 mL × 3) firstly, then dried over anhydrous sodium sulfate, filtered, and concentrated under reduced pressure. The resulting residue was purified by silica gel column chromatography (eluent: petroleum ether/ethyl acetate = 1/1) to obtain compound **51B-2** (7 g).

MS (ESI) M/Z: 475.9, 477.9, 479.9 [M + H]⁺.

### Compound 51B-3:

Compound **51B-3** (3 g) was prepared by replacing the starting material with compound **51B-2** (6.5 g, 13.63 mmol) according to the method for the preparation of compound **37** from compound **34** in Example 37.

MS (ESI, m/z): 248.1, 250.1 [M + H]⁺.

### Compound 51B-4:

Compound **51B-4** (1.5 g) was prepared by replacing the starting material with compound **51B-3** (3 g, 12.1 mmol) according to the method for the preparation of compound **1A** from compound **1A-1** in Example 1.

MS (ESI, m/z): 296.1 [M + H]⁺.

### Compound 51B-5:

Compound **51B-4** (1 g, 3.39 mmol), compound 51A (658 mg, 2.82 mmol), methanesulfonato(tri-*tert*-butylphosphino)(2'-amino-1,1'-biphenyl-2-yl)palladium(II) (162 mg, 0.28 mmol), *tri-tert-*butylphosphine tetrafluoroborate (82 mg, 0.28 mmol) and potassium phosphate (1199 mg, 5.65 mmol) were dissolved in a mixed solvent of dioxane (20 mL) and water (0.2 mL) under nitrogen atmosphere. The reaction system was heated to 60 °C and successively stirred for 16 h. After the starting material disappeared as detected by LCMS, the reaction liquid was concentrated under reduced pressure, and the resulting residue was purified by silica gel column chromatography (eluent: petroleum ether/ethyl acetate = 1/3) to obtain compound **51B-5** (760 mg).

MS (ESI) M/Z: 322.2 [M + H]⁺.

### Compound 51B-6:

Compound **51B-5** (260 mg, 0.81 mmol) was dissolved in a mixed solvent of tetrahydrofuran (4 mL), methanol (2 mL) and water (2 mL). Lithium hydroxide (39 mg, 1.62 mmol) was added to the reaction liquid, and the reaction liquid was successively stirred at room temperature for 2 h. After the starting material disappeared as detected by LCMS, the reaction liquid was concentrated under reduced pressure and adjusted to pH = 3 with 1N hydrochloric acid. The mixture was extracted with ethyl acetate (20 mL × 3), and the organic phases were combined, washed with saturated brine (20 mL × 3) firstly, then dried over anhydrous sodium sulfate, filtered, and concentrated under reduced pressure to obtain crude compound **51B-6** (170 mg).

MS (ESI) M/Z: 308.1 [M + H]⁺.

### Compound 51B-7:

Compound **51B-6** (400 mg, 1.3 mmol) was dissolved in *N*,*N*-dimethylformamide (5 mL). 2-(7-Azabenzotriazole)-*N*,*N*,*N'*,*N'*-tetramethyluronium hexafluorophosphate (594 mg, 1.56 mmol) and *N*,*N*-diisopropylethylamine (505 mg, 3.91 mmol) were added to the reaction liquid, and the reaction liquid was successively stirred at room temperature for 16 h. After the starting material disappeared as detected by LCMS, the reaction liquid was quenched by addition of water (20 mL). The mixture was extracted with ethyl acetate (20 mL × 3), and the organic phases were combined, washed with saturated brine (20 mL × 3) firstly, then dried over anhydrous sodium sulfate, filtered, and concentrated under reduced pressure to obtain crude compound **51B-7** (300 mg).

MS (ESI) M/Z: 290.1 [M + H]⁺.

### Compound 51B-8:

Compound **51B-8** (140 mg) was prepared by replacing the starting material with compound **51B-7** (170 mg, 0.59 mmol) according to the method for the preparation of compound **11A-1** from compound **9A-4** in Example 11.

MS (ESI, m/z): 276.1 [M + H]⁺.

### Compound 51B:

Compound **51B** (53 mg) was prepared by replacing the starting material with compound **51B-8** (140 mg, 0.51 mmol) according to the method for the preparation of compound **1C** from compound **1C-9** in Example 1.

MS (ESI, m/z): 246.1 [M + H]⁺.

### Preparation of compound 51

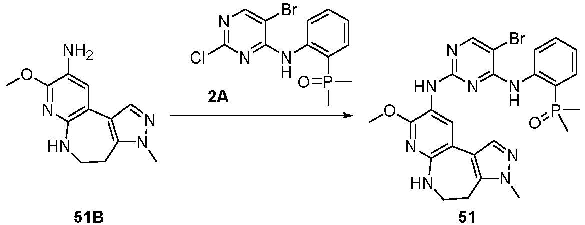

Compound **51** (14 mg) was prepared by replacing the starting material with compound **51B** (57 mg, 0.23 mmol) and compound **2A** (84 mg, 0.23 mmol) according to the method for the preparation of compound **1** from compound **1B** and compound **1C** in Example 1.

MS (ESI, m/z): 569.1, 571.1 [M + H]⁺.

¹H NMR (300 MHz, CDCl₃) δ 10.59 (s, 1H), 8.60 (s, 1H), 8.44 (dd, *J=* 8.4, 4.5 Hz, 1H), 8.23 (s, 1H), 7.39-7.19 (m, 4H), 7.06-7.01 (m, 1H), 3.94 (s, 3H), 3.82 (s, 3H), 3.48 (t, *J*= 5.1 Hz, 2H), 3.02 (t, *J=* 5.1 Hz, 2H), 1.89 (s, 3H), 1.85 (s, 3H).

### Example 52:

### Preparation of compound 52

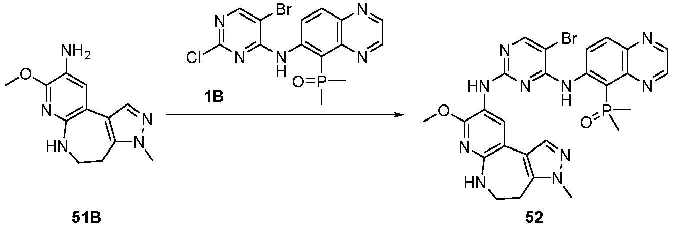

Compound **52** (25 mg) was prepared by replacing the starting material with compound **51B** (80 mg, 0.33 mmol) according to the method for the preparation of compound **1** from compound **1B** and compound **1C** in Example 1.

MS (ESI, m/z): 621.1, 623.1 [M + H]⁺.

¹H NMR (300 MHz, CDCl₃) δ 12.52 (s, 1H), 8.88 (dd, *J* = 9.6, 4.2 Hz, 1H), 8.72-8.69 (m, 2H), 8.53 (s, 1H), 8.30 (s, 1H), 7.76 (s, 1H), 7.07 (s, 2H), 3.95 (s, 3H), 3.74 (s, 3H), 3.51 (t, *J=* 5.1 Hz, 2H), 3.03 (t, *J=* 5.1 Hz, 2H), 2.18 (s, 3H), 2.13 (s, 3H).

### Example 53:

### Preparation of compound 53

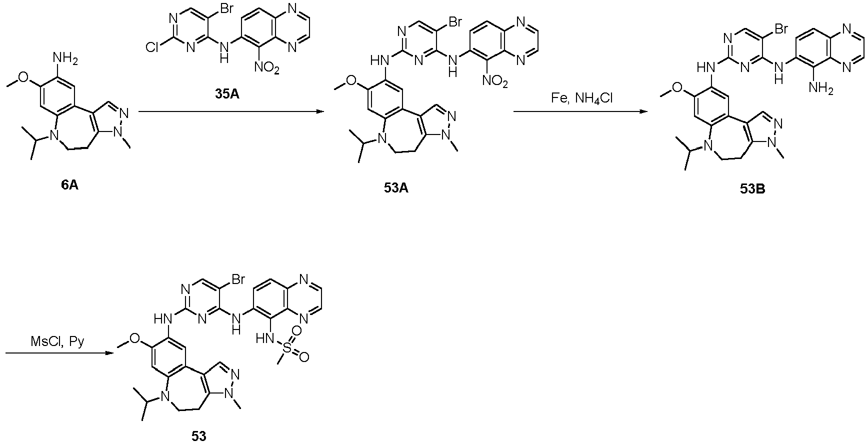

### Compound 53A:

Compound **6A** (2.7 g, 9.43 mmol) and compound **35A** (3.6 g, 9.43 mmol) were dissolved in *N-*methylpyrrolidinone (30 mL). Subsequently, methanesulfonic acid (2.72 g, 28.28 mmol) was added to the above reaction liquid. The reaction system was heated to 95 °C and successively stirred for 3 h. After the starting material disappeared as detected by LCMS, the reaction liquid was cooled to room temperature and purified using a reverse phase C18 column. Purification conditions: a chromatography column: 330 g of C18 reverse phase column; mobile phase: water (containing 0.1% formic acid) and acetonitrile; flow rate: 70 mL/min; gradient: acetonitrile elevating from 10% to 50% in 20 min; detection wavelength: 254 nm. The product was collected and concentrated under reduced pressure to obtain compound **53A** (3.4 g).

MS (ESI, m/z): 631.2, 633.2 [M + H]⁺.

### Compound 53B:

Compound **53A** (3.4 g, 5.38 mmol) was dissolved in a mixed solvent of ethanol (40 mL) and water (8 mL). Subsequently, iron powder (1.50 g, 26.92 mmol) and ammonium chloride (0.86 g, 16.15 mmol) were added to the above reaction liquid, and the reaction system was heated to 80 °C and successively stirred for 2 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound **53B** (2.8 g).

MS (ESI, m/z): 601.2, 603.2 [M + H]⁺.

### Compound 53:

Compound **53B** (2.8 g, 4.66 mmol) was dissolved in pyridine (20 mL) at 0 °C. Subsequently, methanesulfonyl chloride (1.07 g, 9.31 mmol) was added to the reaction liquid. The reaction system was heated to 50 °C and successively stirred for 1 h. After the starting material disappeared as detected by LCMS, the reaction liquid was cooled to room temperature, quenched by addition of water (3 mL), and concentrated under reduced pressure. The resulting residue was purified by silica gel column chromatography (eluent: dichloromethane/methanol = 10/1), and the resulting crude product was purified by a C18 reverse phase column. Purification conditions: a chromatography column: 330 g of C18 reverse phase column; mobile phase: water (containing 0.1% formic acid) and acetonitrile; flow rate: 70 mL/min; gradient: acetonitrile elevating from 5% to 60% in 30 min; detection wavelength: 254 nm. The product was collected and lyophilized under reduced pressure to obtain compound **53** (2.2 g).

MS (ESI, m/z): 679.2, 681.2 [M + H]⁺.

¹H NMR (300 MHz, CDCl₃) δ 9.11 (s, 1H), 8.84-8.79 (m, 2H), 8.60 (d, *J* = 9.3 Hz, 1H), 8.33 (s, 1H), 8.28 (s, 1H), 7.58 (s, 2H), 6.74 (s, 1H), 6.54 (s, 1H), 3.89 (s, 4H), 3.67 (s, 3H), 3.30 (br s, 2H), 2.95 (br s, 5H), 1.31 (d, *J=* 6.3 Hz, 6H).

¹H NMR (300 MHz, DMSO-*d₆*) δ 9.89 (br s, 1H), 8.94 (d, *J* = 1.8 Hz, 1H), 8.85 (d, *J* = 2.1 Hz, 1H), 8.81 (s, 1H), 8.68 (br s, 1H), 8.35 (s, 1H), 8.27 (s, 1H), 7.74 (s, 1H), 7.49 (s, 1H), 7.40 (s, 1H), 6.58 (s, 1H), 3.99-3.91 (m, 1H), 3.76 (s, 3H), 3.71 (s, 3H), 3.22 (t, *J* = 5.4 Hz, 2H), 3.01 (s, 3H), 2.94 (t, *J=* 5.4 Hz, 2H), 1.29 (d, *J=* 6.3 Hz, 6H).

### Example 54:

### Preparation of compound 54

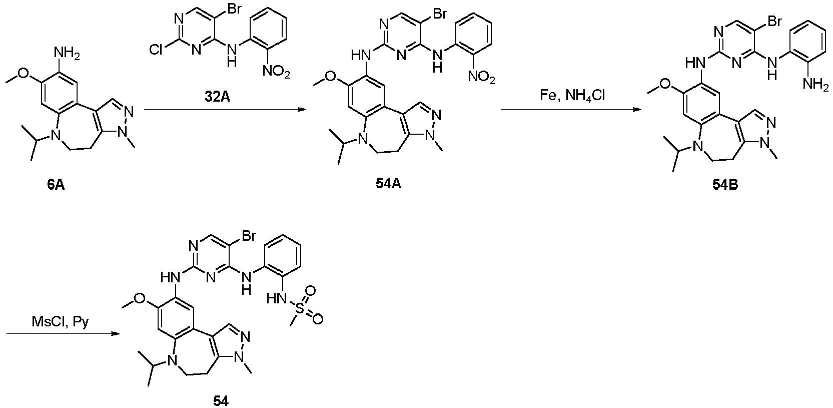

### Compound 54A:

Compound **54A** (110 mg) was prepared by replacing the starting material with compound 6A (130 mg, 0.46 mmol) and compound **32A** (150 mg, 0.46 mmol) according to the method for the preparation of compound **1** from compound **1B** and compound **1C** in Example 1.

MS (ESI, m/z): 579.3, 581.3 [M + H]⁺.

### Compound 54B:

Compound **54B** (89 mg) was prepared by replacing the starting material with compound **54A** (100 mg, 0.17 mmol) according to the method for the preparation of compound **1C** from intermediate **1C-9** in Example 1.

MS (ESI, m/z): 549.3, 551.3 [M + H]⁺.

### Compound 54:

Compound **54** (47 mg) was prepared by replacing the starting material with compound **54B** (80 mg, 0.15 mmol) according to the method for the preparation of compound **32** from intermediate **32C** in Example 32.

MS (ESI, m/z): 627.2, 629.2 [M + H]⁺.

¹H NMR (300 MHz, CDCl₃) δ 8.19 (s, 1H), 8.16 (s, 1H), 7.64 (dd, *J* = 7.5, 1.5 Hz, 1H), 7.45 (s, 1H), 7.38 (d, *J* = 7.5 Hz, 1H), 7.32-7.30 (m, 1H), 7.21-7.12 (m, 4H), 6.53 (s, 1H), 3.92-3.67 (m, 7H), 3.29-3.26 (m, 2H), 2.94-2.90 (m, 5H), 1.31 (d, *J* = 6.6 Hz, 6H).

Example 55:

### Preparation of intermediate 55A

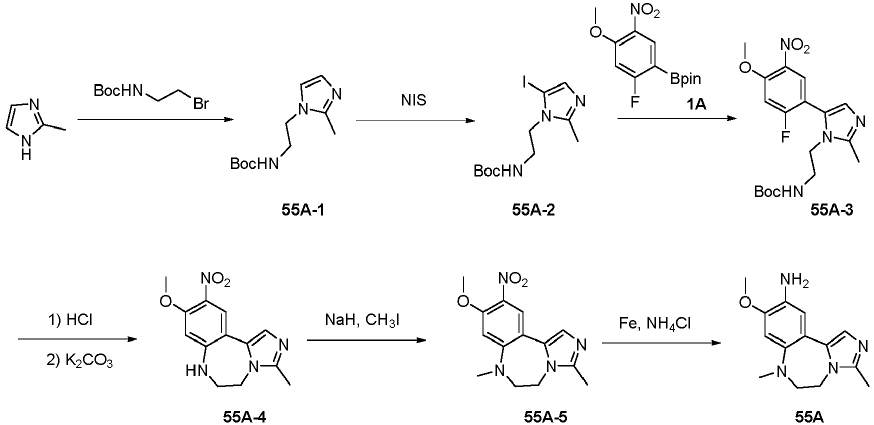

### Compound 55A-1:

2-Methylimidazole (3 g, 36.5 mmol) and anhydrous potassium carbonate (10.1 g, 73.1 mmol) were dissolved in acetonitrile (25 mL). Subsequently, N-tert-Butoxycarbonyl-bromoethylamine (8.2 g, 36.5 mmol) was added to the reaction liquid. The reaction system was heated to 70 °C and successively stirred for 16 h. After the starting material disappeared as detected by LCMS, the reaction liquid was cooled to room temperature and concentrated under reduced pressure. The resulting residue was purified by a reverse phase C18 column. The purification conditions were as follows: a chromatography column: 120 g of C18 reverse phase column; mobile phase: water (containing 10 mM ammonium bicarbonate) and acetonitrile; flow rate: 60 mL/min; gradient: acetonitrile elevating from 30% to 80% in 30 min; detection wavelength: 220 nm. The product was collected and lyophilized under reduced pressure to obtain compound **55A-1** (1.7 g).

MS (ESI) M/Z: 226.3 [M + H]⁺.

### Compound 55A-2:

Compound **55A-1** (2.5 g, 11.1 mmol) was dissolved in acetonitrile (50 mL). Subsequently, N-iodosuccinimide (3.8 g, 16.7 mmol) was added to the reaction liquid. The reaction liquid was heated to 120 °C with a microwave reactor and successively stirred for 4 h. After the starting material disappeared as detected by LCMS, the reaction liquid was cooled to room temperature and concentrated under reduced pressure. The resulting residue was purified by a reverse phase C18 column. The purification conditions were as follows: a chromatography column: 120 g of C18 reverse phase column; mobile phase: water (containing 10 mM ammonium bicarbonate) and acetonitrile; flow rate: 60 mL/min; gradient: acetonitrile elevating from 30% to 80% in 30 min; detection wavelength: 220 nm. The product was collected and lyophilized under reduced pressure to obtain compound **55A-2** (1.1 g).

MS (ESI) M/Z: 352.1 [M + H]⁺.

### Compound 55A-3:

Compound **55A-2** (900 mg, 2.56 mmol), compound **1A** (990 mg, 3.33 mmol) and anhydrous potassium carbonate (708 mg, 5.13 mmol) were dissolved in 1,4-dioxane (8 mL) and water (2 mL). Subsequently, 1,1'-Bis(di-tert-butylphosphino)ferrocene palladium dichloride (167 mg, 0.26 mmol) was added to the reaction liquid. The reaction system was heated to 80 °C and successively stirred for 2 h. After the starting material disappeared as detected by LCMS, the reaction liquid was cooled to room temperature and quenched by addition of water (10 mL). The mixture was extracted with ethyl acetate (30 mL × 3), and the organic phases were combined, washed with saturated brine (30 mL × 3) firstly, then dried over anhydrous sodium sulfate, filtered, and finally concentrated under reduced pressure. The resulting residue was purified by silica gel column chromatography (eluent: petroleum ether/ethyl acetate = 1/1) to obtain compound **55A-3** (300 mg).

MS (ESI) M/Z: 395.2 [M + H]⁺.

### Compound 55A-4:

Compound **55A-4** (130 mg) was prepared by replacing the starting material with compound **55A-3** (270 mg, 0.68 mmol) according to the method for the preparation of compound **42A-7** from compound **42A-6** in Example 42.

MS (ESI, m/z): 274.8 [M + H]⁺.

### Compound 55A-5:

Compound **55A-5** (90 mg) was prepared by replacing the starting material with compound **55A-4** (130 mg, 0.47 mmol) according to the method for the preparation of compound **8A-9** from compound **8A-8** in Example 8.

MS (ESI, m/z): 289.1 [M + H]⁺.

### Intermediate 55A:

Compound **55A** (70 mg) was prepared by replacing the starting material with compound **55A-5** (90 mg, 0.31 mmol) according to the method for the preparation of compound **1C** from compound **1C-9** in Example 1.

MS (ESI, m/z): 259.2 [M + H]⁺.

### Preparation of compound 55

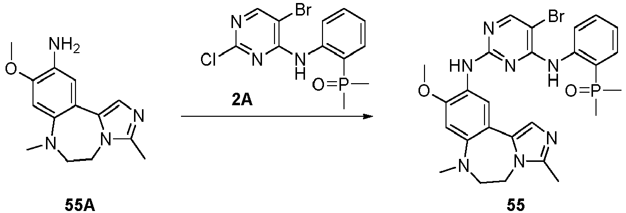

Compound **55** (37 mg) was prepared by replacing the starting material with compound **55A** (72 mg, 0.28 mmol) and compound **2A** (101 mg, 0.28 mmol) according to the method for the preparation of compound **1** from compound **1B** and compound **1C** in Example 1.

MS (ESI, m/z): 582.0, 584.0 [M + H]⁺.

¹H NMR (300 MHz, DMSO-*d*₆) δ 10.91 (s, 1H), 8.34-8.30 (m, 1H), 8.22 (s, 1H), 8.17 (s, 1H), 7.57 (s, 1H), 7.53-7.45 (m, 1H), 7.03-6.97 (m, 2H), 6.77 (s, 1H), 6.66 (s, 1H), 4.03 (t, *J=* 5.4 Hz, 2H), 3.82 (s, 3H), 3.41 (t, *J=* 5.4 Hz, 2H), 2.90 (s, 3H), 2.37 (s, 3H), 1.75 (d, *J=* 13.5 Hz, 6H).

### Example 56:

### Preparation of intermediate 56A

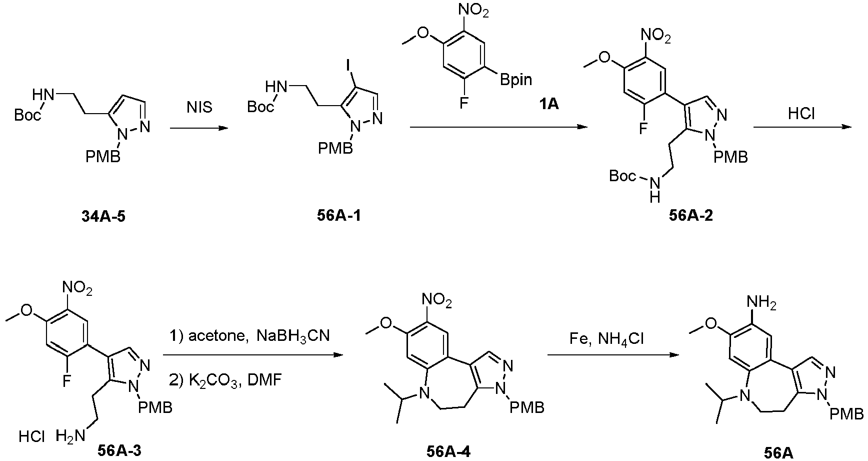

### Compound 56A-1:

Compound **56A-1** (12.5 g) was prepared by replacing the starting material with compound **34A-5** (10 g, 30.2 mmol) according to the method for the preparation of compound **1C-6** from compound **1C-5** in Example 1.

MS (ESI, m/z): 458.1 [M + H]⁺.

### Compound 56A-2:

Compound **56A-2** (10 g) was prepared by replacing the starting material with compound **56A-1** (12.1 g, 26.5 mmol) according to the method for the preparation of compound **1C-7** from compound **1C-6** in Example 1.

MS (ESI, m/z): 501.2 [M + H]⁺.

### Compound 56A-3:

Compound **56A-3** (2.4 g) was prepared by replacing the starting material with compound **56A-2** (3 g, 6.0 mmol) according to the method for the preparation of compound **1C-9** from compound **1C-8** in Example 1.

MS (ESI, m/z): 401.0 [M + H]⁺.

### Compound 56A-4:

Compound **56A-3** (2.4 g, 6.0 mmol) and acetone (0.7 g, 12.0 mmol) were dissolved in methanol (20 mL). Subsequently, anhydrous sodium acetate (1.5 g, 18.0 mmol) was added to the reaction liquid, and the reaction liquid was successively stirred at room temperature for 30 min. Subsequently, glacial acetic acid (0.72 g, 12.0 mmol) and sodium cyanoborohydride (0.75 g, 12.0 mmol) were added to the reaction liquid. The reaction system was successively stirred at room temperature for 2 h. After the starting material disappeared as detected by LCMS, the reaction liquid was concentrated under reduced pressure. The resulting residue was dissolved in *N,N-*dimethylformamide (30 mL); subsequently, anhydrous potassium carbonate (4.2 g, 30.5 mmol) was added to the reaction liquid. The reaction system was heated to 110 °C and successively stirred for 4 h. After the starting material disappeared as detected by LCMS, the reaction liquid was cooled to room temperature and quenched by addition of water (150 mL). The mixture was extracted with ethyl acetate (100 mL × 3), and the organic phases were combined, washed with saturated brine (80 mL × 3) firstly, then dried over anhydrous sodium sulfate, filtered, and finally concentrated under reduced pressure. The resulting residue was purified by silica gel column chromatography (eluent: petroleum ether/ethyl acetate = 1/1) to obtain compound **56A-4** (2.12 g).

MS (ESI) M/Z: 423.2 [M + H]⁺.

### Intermediate 56A:

Compound **56A** (1.67 g) was prepared by replacing the starting material with compound **56A-4** (2.12 g, 5.02 mmol) according to the method for the preparation of compound **1C** from compound **1C-9** in Example 1.

MS (ESI, m/z): 393.3 [M + H]⁺.

### Preparation of compound 56

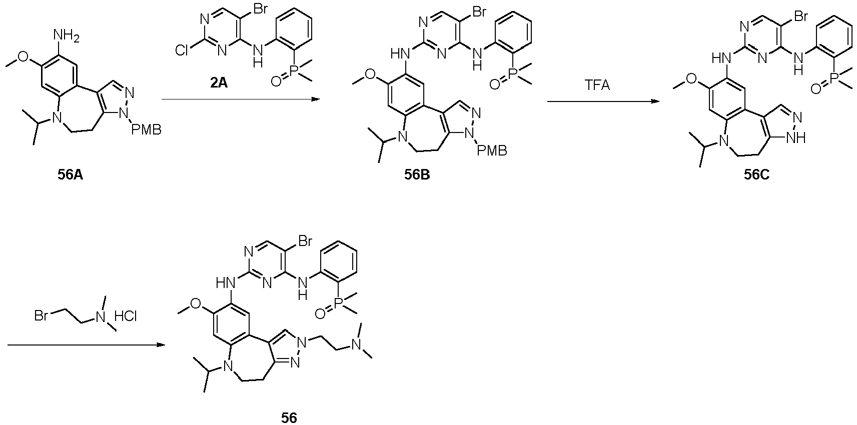

### Compound 56B:

Compound **56B** (2.88 g) was prepared by replacing the starting material with compound **55A** (2.4 g, 6.12 mmol) and compound **2A** (1.98 g, 5.5 mmol) according to the method for the preparation of compound **1** from compound **1B** and compound **1C** in Example 1.

MS (ESI, m/z): 716.3, 718.3 [M + H]⁺.

### Compound 56C:

Compound **56C** (1.2 g) was prepared by replacing the starting material with compound **56B** (2.88 g, 4.02 mmol) according to the method for the preparation of compound **37** from compound **34** in Example 37.

MS (ESI, m/z): 596.2, 598.2 [M + H]⁺.

### Compound 56:

Compound **56C** (100 mg, 0.17 mmol) and anhydrous potassium carbonate (232 mg, 1.68 mmol) were dissolved in *N*,*N*-dimethylformamide (2 mL). Subsequently, the reaction liquid was added with 2-bromo-N,N-dimethylethylamine hydrobromide (195 mg, 0.84 mmol). The reaction system was heated to 120 °C and successively stirred for 2 days. After the starting material disappeared as detected by LCMS, the reaction liquid was cooled to room temperature and prepared under high pressure, wherein preparation conditions were as follows: preparative column: XSelect CSH Prep C18 OBD column, 19 × 250 mm, 5 µm; mobile phase A: water (0.1% formic acid), and mobile phase B: acetonitrile; flow rate: 25 mL/min; gradient: mobile phase B elevating from 25% to 70% in 25 min; detection wavelength: 254/220 nm. The product was collected and lyophilized under reduced pressure to obtain compound **56** (3 mg).

MS (ESI, m/z): 667.2, 669.2 [M + H]⁺.

¹H NMR (300 MHz, CDCl₃) δ 10.58 (s, 1H), 8.51 (s, 1H), 8.42 (s, 1H), 8.23 (s, 1H), 7.45 (s, 1H), 7.29-7.19 (m, 3H), 6.98-6.95 (m, 1H), 6.57 (s, 1H), 4.28-4.26 (m, 2H), 3.93-3.89 (m, 4H), 3.33-3.30 (m, 2H), 3.02-2.96 (m, 4H), 2.41 (s, 6H), 1.86 (d, *J=* 13.2 Hz, 6H), 1.33 (s, 3H), 1.31 (s, 3H).

### Example 57:

### Preparation of compound 57

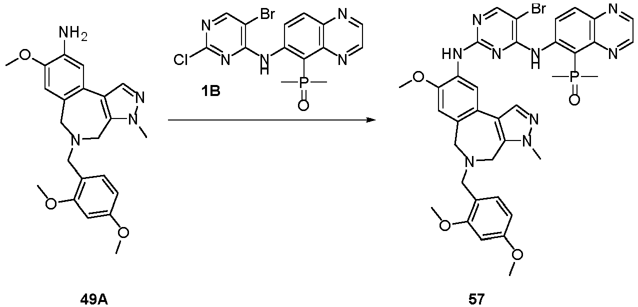

### Compound 57:

Compound **57** (133 mg) was prepared by replacing the starting material with compound **49A** (100 mg, 0.25 mmol) according to the method for the preparation of compound 1 from compound **1B** and compound 1C in Example 1.

MS (ESI, m/z): 770.3, 772.3 [M + H]⁺.

¹H NMR (300 MHz, CDCl₃) δ 12.56 (s, 1H), 8.95 (dd, *J=* 9.6 Hz, 4.2 Hz, 1H), 8.73-8.70 (m, 2H), 8.53 (s, 1H), 8.35 (s, 1H), 7.74 (d, *J* = 9.6 Hz, 1H), 7.49 (s, 1H), 7.25-7.18 (m, 1H), 7.03 (s, 1H), 6.58 (s, 1H), 6.51-6.45 (m, 2H), 4.17 (s, 2H), 4.06 (s, 2H), 3.90 (s, 3H), 3.84 (s, 3H), 3.78 (s, 3H), 3.71 (s, 2H), 3.64 (s, 3H), 2.19 (s, 3H), 2.15 (s, 3H).

### Example 58:

### Preparation of compound 58

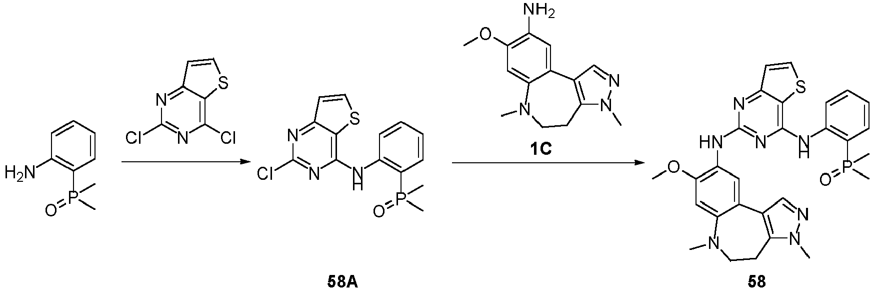

### Compound 58A:

The compounds 2-(dimethylphosphoryl)aniline (1.37 g, 8.1 mmol) and 2,4-dichlorothiophene [3,2-d]pyrimidine (1.99 g, 9.72 mmol) were dissolved in *N*,*N*-dimethylformamide (20 mL), and the reaction solution was added with diisopropylethylamine (2.09 g, 16.2 mmol). The reaction system was heated to 110 °C and successively stirred for 2 h. After the starting material disappeared as detected by LCMS, the reaction liquid was cooled to room temperature and quenched by addition of water (100 mL). The mixture was extracted with ethyl acetate (80 mL × 3), and the organic phases were combined, washed with saturated brine (80 mL × 3) firstly, then dried over anhydrous sodium sulfate, filtered, and finally concentrated under reduced pressure. The resulting residue was purified by silica gel column chromatography (eluent: petroleum ether/ethyl acetate = 2/1) to obtain compound **58A** (1.82 g).

MS (ESI) M/Z: 338.1, 340.1 [M + H]⁺.

### Compound 58:

Compound **58A** (100 mg, 0.3 mmol), compound **1C** (76 mg, 0.3 mmol) and anhydrous potassium carbonate (82 mg, 0.59 mmol) were dissolved *tert*-butanol (3 mL) under nitrogen atmosphere. Subsequently, tris(dibenzylideneacetone)dipalladium (27 mg, 0.03 mmol) and 2-dicyclohexylphosphonium-2',4',6'-triisopropylbiphenyl (21 mg, 0.04 mmol) were added to the reaction liquid. The reaction system was heated to 110 °C and successively stirred for 2 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound 58 (103 mg).

MS (ESI) M/Z: 560.2 [M + H]⁺.

¹H NMR (300 MHz, CDCl₃) δ 11.38 (s, 1H), 8.96-8.87 (m, 1H), 8.69 (s, 1H), 7.71 (m, *J=* 5.4 Hz, 1H), 7.66 (s, 1H), 7.61 (s, 1H), 7.28 (s, 1H), 7.26 (s, 1H), 7.25-7.19 (m, 1H), 7.03-6.97 (m, 1H), 6.59 (s, 1H), 3.93 (s, 3H), 3.85 (s, 3H), 3.32 (t, *J* = 5.4 Hz, 2H), 3.10-3.03 (m, 5H), 1.90 (s, 3H), 1.86 (s, 3H).

### Example 59:

### Preparation of compound 59

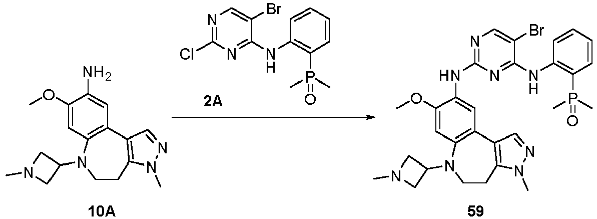

Compound **59** (12 mg) was prepared by replacing the starting material with compound **10A** (71 mg, 0.23 mmol) and compound **2A** (65 mg, 0.18 mmol) according to the method for the preparation of compound **1** from compound **1B** and compound **1C** in Example 1.

MS (ESI, m/z): 637.2, 639.2 [M + H]⁺.

¹H NMR (300 MHz, CDCl₃) δ 10.59 (s, 1H), 8.57 (s, 1H), 8.50-8.41 (m, 2H), 8.26 (s, 1H), 7.39 (s, 1H), 7.37-7.31 (m, 1H), 7.27-7.20 (m, 1H), 7.08-6.99 (m, 1H), 6.23 (s, 1H), 4.54 (t, *J* = 7.2 Hz, 1H), 4.33 (t, *J* = 8.1 Hz, 2H), 3.89 (s, 3H), 3.84 (s, 3H), 3.45 (t, *J=* 8.1 Hz, 2H), 3.28 (t, *J* = 6.0 Hz, 2H), 3.02 (t, *J=* 6.0 Hz, 2H), 2.65 (s, 3H), 1.89 (s, 3H), 1.85 (s, 3H).

### Example 60:

### Preparation of intermediate 60A

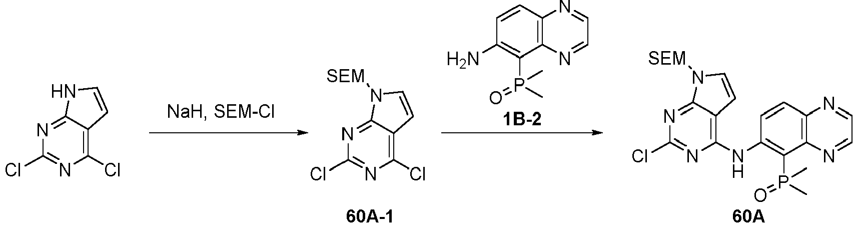

### Compound 60A-1:

Sodium hydride (60%, 0.69 g, 17.25 mmol) was dissolved in N,N-dimethylformamide (25 mL) under nitrogen atmosphere at 0 °C. Subsequently, 2,4-dichloro-7*H*-pyrrolo[2,3-*d*]pyrimidine (2.5 g, 13.3 mmol) was added to the reaction liquid in batches, and the reaction liquid was successively stirred at that temperature for 10 min. After the reaction liquid was added with 2-(trimethylsilyl)ethoxymethyl chloride (2.88 g, 17.29 mmol) and successively stirred at that temperature for 10 min, the reaction liquid was warmed to room temperature and successively stirred for 1 h. After the starting material disappeared as detected by LCMS, the reaction liquid was quenched by addition of water (100 mL). The mixture was extracted with ethyl acetate (80 mL × 3), and the organic phases were combined, washed with saturated brine (80 mL × 3) firstly, then dried over anhydrous sodium sulfate, filtered, and finally concentrated under reduced pressure. The resulting residue was purified by silica gel column chromatography (eluent: petroleum ether/ethyl acetate = 5/1) to obtain compound **60A-1** (3.9 g).

MS (ESI) M/Z: 318.1, 320.1 [M + H]⁺.

### Intermediate 60A:

Compound **60A** (3 g) was prepared by replacing the starting material with compound **60A-1** (5.76 g, 18.08 mmol) according to the method for the preparation of compound **1B** from compound **1B-2** in Example 1.

MS (ESI, m/z): 503.3, 505.3 [M + H]⁺.

### Preparation of compound 60

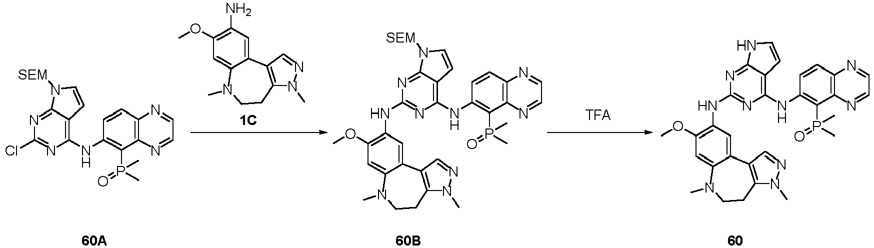

### Compound 60B:

Compound **60B** (70 mg) was prepared by replacing the starting material with compound **60A** (80 mg, 0.16 mmol) according to the method for the preparation of compound **58** from compound **58A** and compound **1C** in Example 58.

MS (ESI, m/z): 725.4 [M + H]⁺.

### Compound 60:

Compound **60** (21 mg) was prepared by replacing the starting material with compound 60B (148 mg, 0.2 mmol) according to the method for the preparation of compound **36** from compound **36B** in Example 36.

MS (ESI, m/z): 595.2 [M + H]⁺.

¹H NMR (300 MHz, CDCl₃) δ 13.04 (s, 1H), 9.77 (dd, *J=* 9.6, 4.2 Hz, 1H), 8.68 (m, 4H), 7.87 (d, *J* = 9.6 Hz, 1H), 7.64 (s, 1H), 7.16 (s, 1H), 6.87 (dd, *J* = 3.6, 2.1 Hz, 1H), 6.74 (dd, *J* = 3.6, 2.1 Hz, 1H), 6.61 (s, 1H), 3.96 (s, 3H), 3.83 (s, 3H), 3.34 (t, *J* = 5.4 Hz, 2H), 3.11-3.06 (m, 5H), 2.18 (s, 3H), 2.13 (s, 3H).

### Example 61:

### Preparation of compound 61

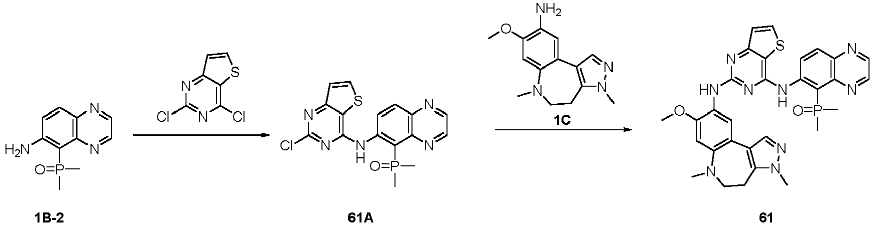

### Compound 61A:

Compound **61A** (1 g) was prepared by replacing the starting material with 2,4-dichlorothiophene[3,2-*d*]pyrimidine (1.85 g, 9.04 mmol) according to the method for the preparation of compound **1B** from compound **1B-2** in Example 1.

MS (ESI, m/z): 390.0, 392.0 [M + H]⁺.

### Compound 61:

Compound **61** (46 mg) was prepared by replacing the starting material with compound **61A** (100 mg, 0.26 mmol) according to the method for the preparation of compound **58** from compound **58A** and compound **1C** in Example 58.

MS (ESI, m/z): 612.2 [M + H]⁺.

¹H NMR (300 MHz, CDCl₃) δ 13.22 (s, 1H), 9.54 (dd, *J=* 9.6 Hz, 4.2 Hz, 1H), 8.72-8.70 (m, 2H), 8.65 (s, 1H), 7.83-7.76 (m, 2H), 7.57-7.51 (m, 2H), 7.30 (s, 1H), 6.62 (s, 1H), 3.95 (s, 3H), 3.82 (s, 3H), 3.36 (t, *J=* 5.4 Hz, 2H), 3.13-3.07 (m, 5H), 2.19 (s, 3H), 2.14 (s, 3H).

### Example 62:

### Preparation of compound 62

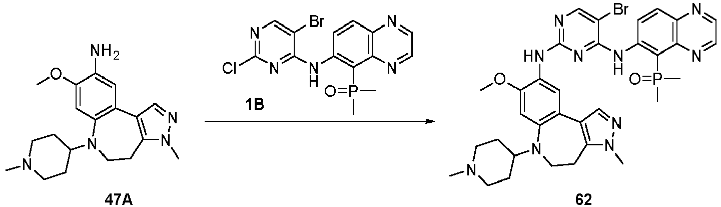

### Compound 62:

Compound **62** (42 mg) was prepared by replacing the starting material with compound **47A** (100 mg, 0.29 mmol) and compound **1B** (100 mg, 0.24 mmol) according to the method for the preparation of compound 1 from compound **1B** and compound **1C** in Example 1.

MS (ESI, m/z): 717.3, 719.3 [M + H]⁺.

¹H NMR (300 MHz, CDCl₃) δ 12.63 (s, 1H), 8.86-8.78 (m, 3H), 8.26 (s, 1H), 8.20 (s, 1H), 7.74 (s, 1H), 7.55 (s, 1H), 7.45 (s, 1H), 6.58 (s, 1H), 3.76 (s, 3H), 3.70 (s, 3H), 3.28-3.26 (m, 2H), 2.96-2.86 (m, 4H), 2.23 (s, 3H), 2.18-2.06 (m, 3H), 2.04 (s, 3H), 1.99 (s, 3H), 1.90-1.80 (m, 4H).

### Example 63:

### Preparation of intermediate 63A

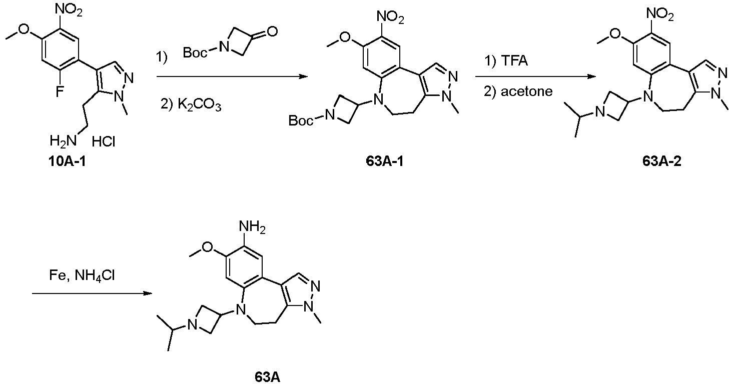

### Compound 63A-1:

Compound **63A-1** (0.9 g) was prepared by replacing the starting material with *tert*-butyl 3-oxoazetidine-1-carboxylate (2.2 g, 12.9 mmol) according to the method for the preparation of compound **47A-1** from compound **10A-1** in Example 47.

MS (ESI, m/z): 430.2 [M + H]⁺.

### Compound 63A-2:

Compound **63A-1** (300 mg, 0.7 mmol) was dissolved in dichloromethane (5 mL); subsequently, trifluoroacetic acid (1 mL) was added to the reaction liquid, and the reaction liquid was successively stirred at room temperature for 2 h. After the starting material disappeared as detected by LCMS, the reaction liquid was concentrated under reduced pressure. The resulting residue was dissolved in methanol (5 mL). Subsequently, acetone (203 mg, 3.49 mmol) was added to the reaction liquid, and the reaction liquid was successively stirred at room temperature for 30 min. Sodium cyanoborohydride (88 mg, 1.4 mmol) was added to the above reaction liquid, and the reaction liquid was successively stirred at room temperature for 2 h. After the starting material disappeared as detected by LCMS, the reaction liquid was concentrated under reduced pressure. The resulting residue was purified by silica gel column chromatography (eluent: dichloromethane/methanol = 10/1) to obtain compound **63A-2** (150 mg).

MS (ESI) M/Z: 372.2 [M + H]⁺.

### Intermediate 63:

Compound **63A** (87 g) was prepared by replacing the starting material with compound **63A-2** (150 mg, 0.4 mmol) according to the method for the preparation of compound **1C** from compound **1C-9** in Example 1.

MS (ESI, m/z): 342.2 [M + H]⁺.

### Preparation of compound 63

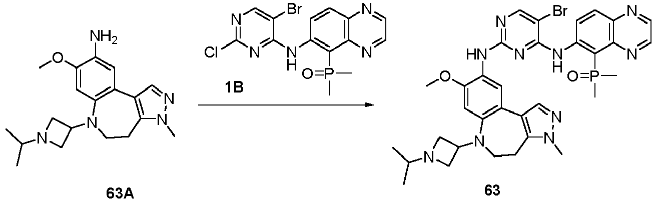

Compound **63** (64 mg) was prepared by replacing the starting material with compound **63A** (87 mg, 0.26 mmol) according to the method for the preparation of compound 1 from compound **1B** and compound **1C** in Example 1.

MS (ESI, m/z): 717.2, 719.2 [M + H]⁺.

¹H NMR (400 MHz, CDCl₃) δ 12.50 (s, 1H), 8.87 (dd, *J* = 9.2 Hz, 4.4 Hz, 1H), 8.71 (d, *J*= 1.6 Hz, 1H), 8.68 (d, *J=* 1.6 Hz, 1H), 8.53 (s, 1H), 8.3 (s, 1H), 7.63 (s, 1H), 7.32 (s, 1H), 6.99 (s, 1H), 6.27 (s, 1H), 4.53-4.44 (m, 1H), 4.16 (t, *J=* 8.0 Hz, 2H), 3.88 (s, 3H), 3.72 (s, 3H), 3.30-3.20 (m, 4H), 3.00 (t, *J=* 6.0 Hz, 2H), 2.79-2.71 (m, 1H), 2.16 (s, 3H), 2.12 (s, 3H), 1.11 (s, 3H), 1.09 (s, 3H).

### Example 64:

### Preparation of compound 64

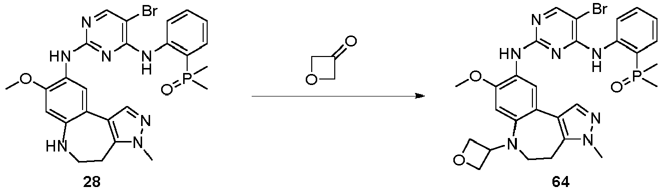

Compound **28** (100 mg, 0.18 mmol) and 3-oxetanone (63 mg, 0.88 mmol) were dissolved in methanol (3 mL), and the reaction liquid was stirred at room temperature for 30 min. The reaction liquid was added with acetic acid (21 mg, 0.35 mmol) and sodium cyanoborohydride (22 mg, 0.35 mmol). The reaction system was successively stirred at room temperature for 2 h. After the starting material disappeared as detected by LCMS, the reaction liquid was quenched by addition of water (10 mL). The mixture was extracted with ethyl acetate (30 mL × 3), and the organic phases were combined, washed with saturated brine (30 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **64A** (64 mg).

MS (ESI, m/z): 624.2, 626.2 [M + H]⁺.

¹H NMR (300 MHz, CDCl₃) δ 10.67 (s, 1H), 8.50-8.40 (m, 2H), 8.23 (s, 1H), 7.39 (s, 1H), 7.36-7.30 (m, 1H), 7.27-7.15 (m, 2H), 7.06-6.98 (m, 1H), 6.01 (s, 1H), 4.96 (t, *J=* 6.3 Hz, 2H), 4.86-4.76 (m, 1H), 4.71 (t, *J=* 6.0 Hz, 2H), 3.86 (s, 3H), 3.84 (s, 3H), 3.37 (t, *J=* 6.0 Hz, 2H), 3.07 (t, *J=* 6.0 Hz, 2H), 1.89 (s, 3H), 1.85 (s, 3H).

### Example 65:

### Preparation of intermediate 65A

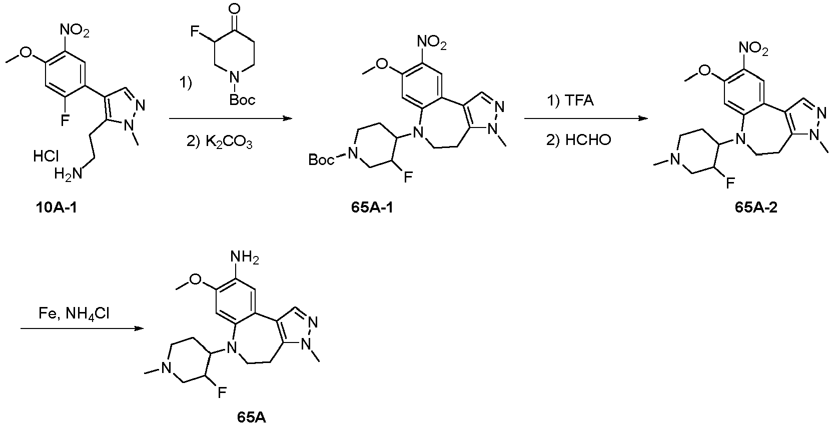

### Compound 65A-1:

Compound **65A-1** (50 mg) was prepared by replacing the starting material with tert-butyl 3-fluoro-4-oxopiperidine-1-carboxylate (828 mg, 3.81 mmol) according to the method for the preparation of compound **47A-1** from compound **10A-1** in Example 47.

MS (ESI, m/z): 476.2 [M + H]⁺.

### Compound 65A-2:

Compound **65A-2** (200 mg) was prepared by replacing the starting material with aqueous formaldehyde solution (40%, 200 mg, 2.67 mmol) according to the method for the preparation of compound **63A-2** from compound **63A-1** in Example 63.

MS (ESI, m/z): 390.2 [M + H]⁺.

### Intermediate 65A:

Compound **65A** (64 mg) was prepared by replacing the starting material with compound **65A-2** (110 mg, 0.28 mmol) according to the method for the preparation of compound **1C** from compound **1C-9** in Example 1.

MS (ESI, m/z): 360.2 [M + H]⁺.

### Preparation of compound 65

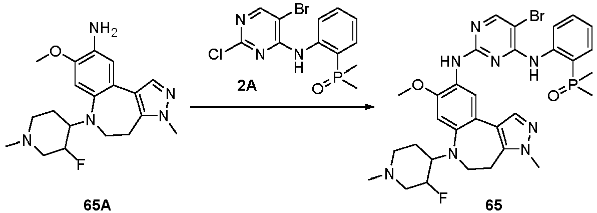

Compound **65** (11 mg) was prepared by replacing the starting material with compound **65A** (30 mg, 0.08 mmol) and compound **2A** (30 mg, 0.08 mmol) according to the method for the preparation of compound **1** from compound **1B** and compound **1C** in Example 1.

MS (ESI, m/z): 683.2, 685.2 [M + H]⁺.

¹H NMR (300 MHz, CDCl₃) δ 10.53 (s, 1H), 8.48-8.42 (m, 1H), 8.24 (s, 1H), 8.21 (s, 1H), 7.45 (s, 1H), 7.36-7.29 (m, 1H), 7.26-7.14 (m, 2H), 7.04-7.97 (m, 1H), 6.59 (s, 1H), 4.87 (d, *J=* 48.0 Hz, 1H), 3.88 (s, 3H), 3.83 (s, 3H), 3.73-3.71 (m, 1H), 3.55-3.05 (m, 5H), 2.96-2.92 (m, 1H), 2.53-2.29 (m, 6H), 1.90 (s, 3H), 1.85 (s, 3H), 1.83-1.76 (m, 1H).

¹⁹F NMR (282 MHz, CDCl₃) δ -196.73.

### Example 66:

### Preparation of compounds 66A and 66B

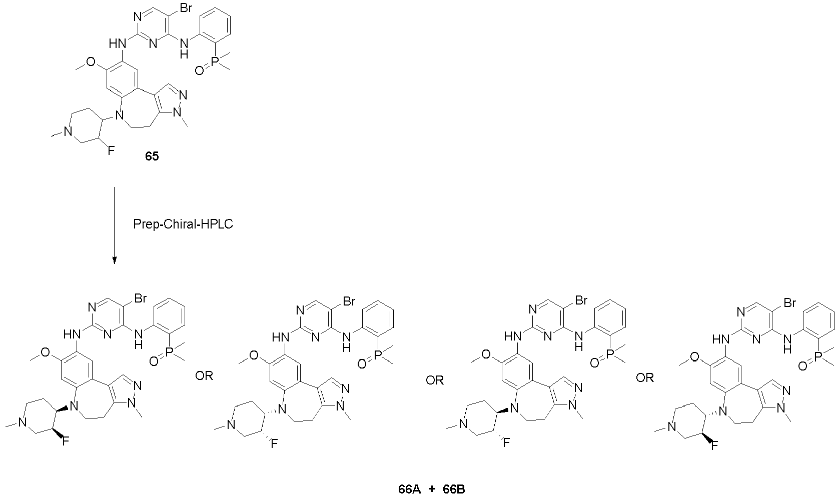

Compound **65** (168 mg, 0.25 mmol) was prepared and separated by a chiral column, wherein preparation conditions were as follows: chiral column: Lux 5 □m Cellulose-4, 2.12 × 25 cm, 5 µm; mobile phase A: n-hexane (10 mM a solution of ammonia in methanol), and mobile phase B: ethanol; flow rate: 20 mL/min; elution: mobile phase B elevating 50% in 51 min; detection wavelength: 205/260 nm; RTi: 22 min; RT₂: 34.5 min. The product was collected and lyophilized under reduced pressure to obtain compound **66A** (15 mg, 100% ee) with a retention time RT₁ of 22 min and compound **66B** (12 mg, 99.7% ee) with a retention time RT₂ of 34.5 min.

The compound 66A and compound 66B were enantiomers of each other, the absolute configuration was not determined.

**66A:** MS (ESI, m/z): 683.3, 685.3 [M + H]⁺.

**66B:** MS (ESI, m/z): 683.2, 685.2 [M + H]⁺.

**66A:** ¹H NMR (300 MHz, CDCl₃) δ 10.83 (s, 1H), 8.56-8.47 (m, 2H), 8.30 (s, 1H), 7.75 (s, 1H), 7.43-7.36 (m, 1H), 7.27 (t, *J* = 8.1 Hz, 1H), 7.14-7.06 (m, 1H), 6.69 (s, 1H), 5.01 (d, *J=* 48.0 Hz, 1H), 3.96 (s, 3H), 3.89 (s, 3H), 3.84-3.31 (m, 6H), 3.23-3.93 (m, 3H), 2.88-2.64 (m, 5H), 1.96 (s, 3H), 1.91 (s, 3H).

¹⁹F NMR (282 MHz, CDCl₃) δ -196.86.

**66B:** ¹H NMR (300 MHz, CDCl₃) δ 10.72 (s, 1H), 8.58-8.51 (m, 2H), 8.31 (s, 1H), 7.45 (s, 1H), 7.42-7.36 (m, 1H), 7.28 (t, *J* = 8.1 Hz, 1H), 7.12-7.05 (m, 1H), 6.68 (s, 1H), 5.01 (d, *J=* 48.0 Hz, 1H), 3.95 (s, 3H), 3.89 (s, 3H), 3.84-3.31 (m, 6H), 3.23-3.93 (m, 3H), 2.88-2.64 (m, 5H), 1.96 (s, 3H), 1.91 (s, 3H).

¹⁹F NMR (282 MHz, CDCl₃) δ -196.86.

### Example 67:

### Preparation of compound 67

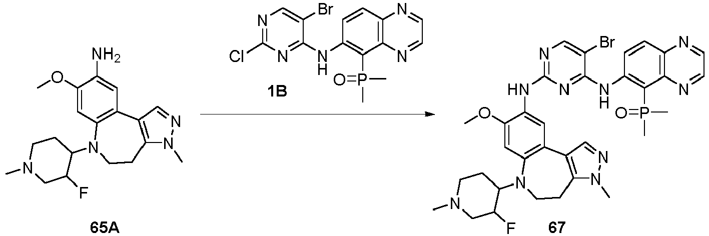

Compound **67** (19 mg) was prepared by replacing the starting material with compound **65A** (30 mg, 0.08 mmol) according to the method for the preparation of compound 1 from compound **1B** and compound **1C** in Example 1.

MS (ESI, m/z): 735.2, 737.2 [M + H]⁺.

¹H NMR (300 MHz, CDCl₃) δ 12.48 (s, 1H), 8.88 (dd, *J=* 8.4 Hz, 1.8 Hz, 1H), 8.73-8.69 (m, 2H), 8.30 (s, 1H), 8.18 (s, 1H), 7.66-7.61 (m, 1H), 7.44 (s, 1H), 7.01 (s, 1H), 6.66 (s, 1H), 4.89 (d, *J=* 48.3 Hz, 1H), 3.89 (s, 3H), 3.77-3.75 (m, 1H), 3.72 (s, 3H), 3.55-3.04 (m, 5H), 3.00-2.96 (m, 1H), 2.54-2.28 (m, 6H), 2.18 (d, *J=* 4.8 Hz, 3H), 2.14 (d, *J=* 4.8 Hz, 3H), 1.81 (d, J= 12.9 Hz, 1H).

¹⁹F NMR (282 MHz, CDCl₃) δ -196.72.

### Example 68:

### Preparation of compound 68

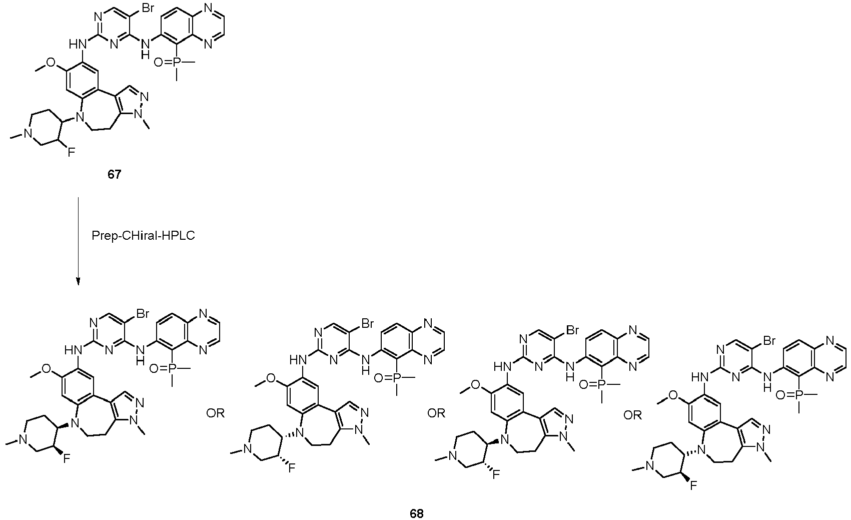

Compound **67** (50 mg, 0.07 mmol) was prepared by a chiral column, wherein preparation conditions were as follows: chiral column: CHIRALPAK IC, 2 × 25 cm, 5 µm; mobile phase A: *n-*hexane:methyl tert-butyl ether =1:1 (0.5% 2 M a solution of ammonia in methanol), and mobile phase B: ethanol; flow rate: 20 mL/min; elution: mobile phase B elevating 30% in 25 min; detection wavelength: 260/210 nm; RT: 23.47 min. The product was collected and lyophilized under reduced pressure to obtain compound **68** (11 mg, i.e., one of the four possible enantiomers separated; 100% ee) with a retention time RT of 23.47 min.

MS (ESI, m/z): 735.2, 737.2 [M + H]⁺.

¹H NMR (300 MHz, CDCl₃) δ 12.51 (s, 1H), 8.89 (dd, *J=* 9.3 Hz, 5.1 Hz, 1H), 8.73-8.69 (m, 2H), 8.43 (s, 1H), 8.31 (s, 1H), 7.69-7.61 (m, 1H), 7.35 (s, 1H), 7.03 (s, 1H), 6.60 (s, 1H), 4.89 (d, *J* = 49.5 Hz, 1H), 3.89 (s, 3H), 3.85-3.75 (m, 1H), 3.73 (s, 3H), 3.55-3.35 (m, 1H), 3.34-3.22 (m, 2H), 3.20-3.05 (m, 2H), 2.99-2.90 (m, 1H), 2.55-2.26 (m, 6H), 2.18 (d, *J=* 4.8 Hz, 3H), 2.14 (d, *J=* 4.8 Hz, 3H), 1.86-1.85 (m, 1H).

### Example 69:

### Preparation of intermediate 69A

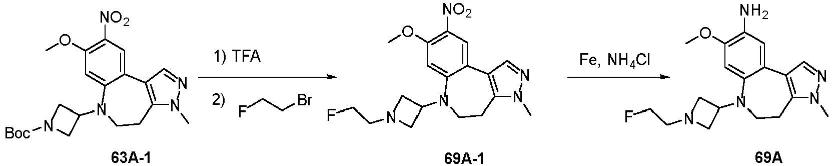

### Compound 69A-1:

Compound **63A-1** (250 mg, 0.58 mmol) was dissolved in dichloromethane (3 mL). Subsequently, trifluoroacetic acid (0.5 mL) was added to the above reaction liquid, and the reaction liquid was successively stirred at room temperature for 2 h. After the starting material disappeared as detected by LCMS, the reaction liquid was concentrated under reduced pressure. The resulting residue was dissolved in *N*,*N*-dimethylformamide (3 mL), followed by addition of anhydrous potassium carbonate (482 mg, 3.5 mmol) and 1-bromo-2-fluoroethane (370 mg, 2.9 mmol) to the reaction liquid. The reaction system was heated to 80 °C and successively stirred for 2 h. After the starting material disappeared as detected by LCMS, the reaction liquid was cooled to room temperature and quenched by addition of water (20 mL). The mixture was extracted with ethyl acetate (30 mL × 3), and the organic phases were combined, washed with saturated brine (30 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **69A-1** (100 mg).

MS (ESI, m/z): 376.2 [M + H]⁺.

### Intermediate 69A:

Compound **69A** (60 mg) was prepared by replacing the starting material with compound **69A-1** (100 mg, 0.27 mmol) according to the method for the preparation of compound **1C** from compound **1C-9** in Example 1.

MS (ESI, m/z): 346.2 [M + H]⁺.

### Preparation of compound 69

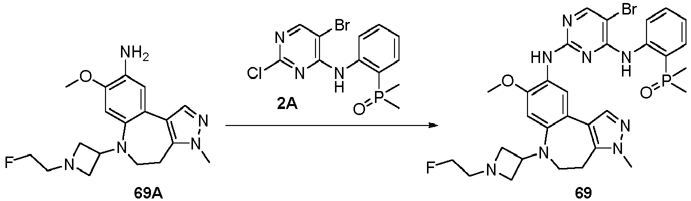

Compound **69** (20 mg) was prepared by replacing the starting material with compound **69A** (60 mg, 0.17 mmol) and compound 2A (52 mg, 0.15 mmol) according to the method for the preparation of compound **1** from compound **1B** and compound **1C** in Example 1.

MS (ESI, m/z): 669.2, 671.2 [M + H]⁺.

¹H NMR (300 MHz, CDCl₃) δ 10.54 (s, 1H), 8.51-8.39 (m, 2H), 8.24 (s, 1H), 7.46 (s, 1H), 8.38-7.17 (m, 3H), 7.06-6.97 (m, 1H), 6.25 (s, 1H), 4.70-4.62 (m, 1H), 4.55-4.44 (m, 2H), 4.19-4.07 (m, 2H), 3.88 (s, 3H), 3.84 (s, 3H), 3.43-3.41 (m, 2H), 3.31-3.21 (m, 2H), 3.11-2.92 (m, 4H), 1.90 (s, 3H), 1.86 (s, 3H).

### Example 70:

### Preparation of intermediate 70A

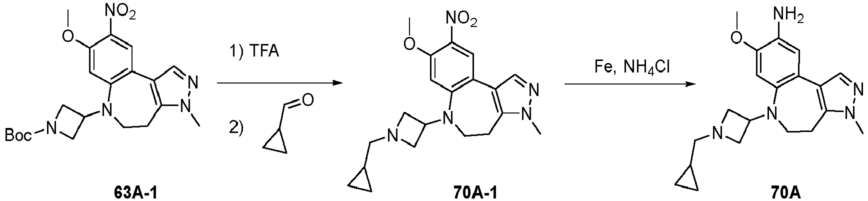

### Compound 70A-1:

Compound **70A-1** (430 mg) was prepared by replacing the starting material with cyclopropanecarboxaldehyde (230 mg, 3.3 mmol) according to the method for the preparation of compound **63A-2** from compound **63A-1** in Example 63.

MS (ESI, m/z): 384.2 [M + H]⁺.

### Intermediate 70A:

Compound **70A** (120 mg) was prepared by replacing the starting material with compound **70A-1** (150 mg, 0.39 mmol) according to the method for the preparation of compound **1C** from compound **1C-9** in Example 1.

MS (ESI, m/z): 354.2 [M + H]⁺.

### Preparation of compound 70

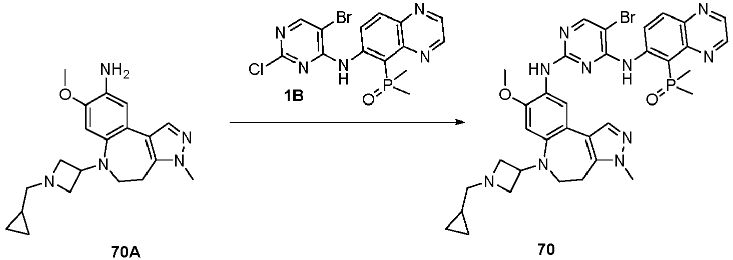

Compound **69** (55 mg) was prepared by replacing the starting material with compound **70A** (120 mg, 0.34 mmol) according to the method for the preparation of compound **1** from compound **1B** and compound **1C** in Example 1.

MS (ESI, m/z): 729.2, 731.2 [M + H]⁺.

¹H NMR (300 MHz, CDCl₃) δ 12.52 (s, 1H), 8.88 (dd, *J=* 9.6 Hz, 4.2 Hz, 1H), 8.73-8.69 (m, 2H), 8.52 (s, 1H), 8.32 (s, 1H), 7.65 (d, *J=* 9.6 Hz, 1H), 7.37 (s, 1H), 7.00 (s, 1 H), 6.27 (s, 1H), 4.65-4.53 (m, 1H), 4.37-4.29 (m, 2H), 3.91 (s, 3H), 3.74 (s, 3H), 3.41 (t, J= 8.1 Hz, 2H), 3.29 (t, J= 5.7 Hz, 2H), 3.02 (t, *J=* 5.7 Hz, 2H), 2.70 (d, *J=* 7.2 Hz, 2H), 2.18 (s, 3H), 2.14 (s, 3H), 0.982-0.840 (m, 1H), 0.625-0.542 (m, 2H), 0.267-0.194 (m, 2H).

### Example 71:

### Preparation of compound 71

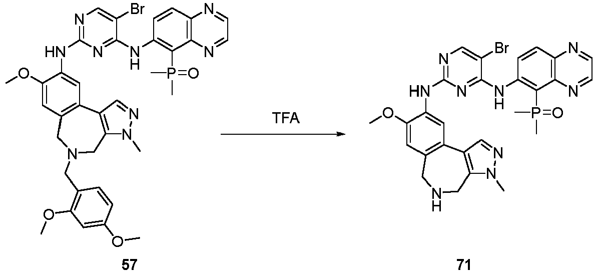

### Compound 71:

Compound **71** (12 mg) was prepared by replacing the starting material with compound **57** (133 mg, 0.17 mmol) according to the method for the preparation of compound **37** from compound **34** in Example 37.

MS (ESI, m/z): 620.1.1, 622.1 [M + H]⁺.

¹H NMR (300 MHz, CDCl₃) δ 12.53 (s, 1H), 8.88-8.83 (m, 1H), 8.74-8.70 (m, 2H), 8.52 (s, 1H), 8.33 (s, 1H), 7.68-7.65 (m, 1H), 7.54 (s, 1H), 6.99 (s, 1H), 6.77 (s, 1H), 4.36 (s, 2H), 4.04 (s, 2H), 3.93 (s, 3H), 3.75 (s, 3H), 2.18 (s, 3H), 2.13 (s, 3H).

### Example 72:

### Preparation of compound 72

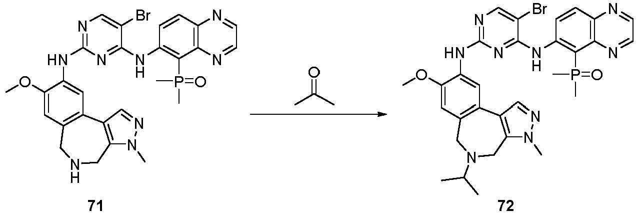

Compound **71** (146 mg, 0.24 mmol), glacial acetic acid (71 mg, 1.18 mmol) and acetone (137 mg, 2.35 mmol) were dissolved in dichloromethane (5 mL), and the reaction liquid was successively stirred at room temperature for 30 min. Subsequently, sodium cyanoborohydride (249 mg, 1.18 mmol) was added to the above reaction liquid, and the reaction liquid was successively stirred at room temperature for 2 h. After the starting material disappeared as detected by LCMS, the reaction liquid was quenched by addition of water (10 mL). The mixture was extracted with ethyl acetate (30 mL × 3), and the organic phases were combined, washed with saturated brine (30 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **72** (98 mg).

MS (ESI, m/z): 662.2, 664.2 [M + H]⁺.

¹H NMR (300 MHz, CDCl₃) δ 12.56 (s, 1H), 8.95 (dd, *J=* 9.6 Hz, 4.2 Hz, 1H), 8.73-8.70 (m, 2H), 8.49 (s, 1H), 8.34 (s, 1H), 7.68 (d, *J=* 9.9 Hz, 1H), 7.51 (s, 1H), 6.99 (s, 1H), 6.73 (s, 1H), 4.23 (s, 2H), 3.95-3.92 (m, 5H), 3.76 (s, 3H), 3.23-3.12 (m, 1H), 2.19 (s, 3H), 2.14 (s, 3H), 1.28 (s, 3H), 1.26 (s, 3H).

### Example 73:

### Preparation of intermediate 73A

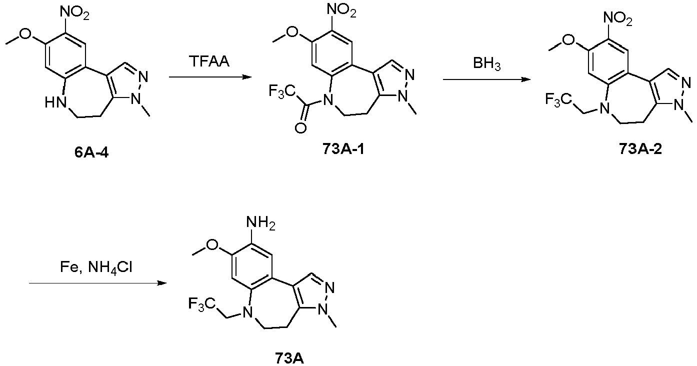

### Compound 73A-1:

Compound **6A-4** (200 mg, 0.73 mmol) was dissolved in dichloromethane (5 mL); subsequently, trifluoroacetic anhydride (766 mg, 3.65 mmol) was added to the reaction liquid, and the reaction liquid was successively stirred at room temperature for 1 h. After the starting material disappeared as detected by LCMS, the reaction liquid was concentrated under reduced pressure. The resulting residue was purified by silica gel column chromatography (eluent: petroleum ether/ethyl acetate = 1/1) to obtain compound **73A-1** (250 mg).

MS (ESI, m/z): 371.2 [M + H]⁺.

### Compound 73A-2:

Compound **73A-2** (93 mg) was prepared by replacing the starting material with compound 73A-1 (168 mg, 0.45 mmol) according to the method for the preparation of compound **11A-1** from compound **9A-4** in Example 11.

MS (ESI, m/z): 357.1 [M + H]⁺.

### Intermediate 73A:

Compound **73A** (70 mg) was prepared by replacing the starting material with compound **73A-2** (93 mg, 0.26 mmol) according to the method for the preparation of compound **1C** from compound **1C-9** in Example 1.

MS (ESI, m/z): 327.2 [M + H]⁺.

### Preparation of compound 73

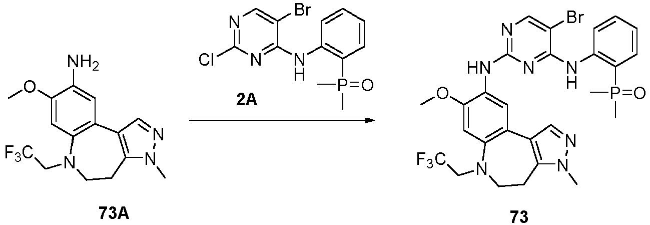

Compound **73** (43 mg) was prepared by replacing the starting material with compound **73A** (70 mg, 0.21 mmol) and compound **2A** (77 mg, 0.21 mmol) according to the method for the preparation of compound **1** from compound **1B** and compound **1C** in Example 1.

MS (ESI, m/z): 650.2, 652.2 [M + H]⁺.

¹H NMR (300 MHz, CDCl₃) δ 10.61 (s, 1H), 8.52-8.44 (m, 2H), 8.25 (s, 1H), 7.38-7.19 (m, 3H), 7.06-6.98 (m, 1H), 6.67 (s, 1H), 3.90 (s, 3H), 3.84 (s, 3H), 3.83-3.75 (m, 2H), 3.49 (t, *J* = 5.7 Hz, 2H), 3.05 (t, *J* = 5.7 Hz, 2H), 1.89 (s, 3H), 1.85 (s, 3H).

¹⁹F NMR (282 MHz, CDCl₃) δ -70.35.

### Example 74:

### Preparation of intermediate 74A

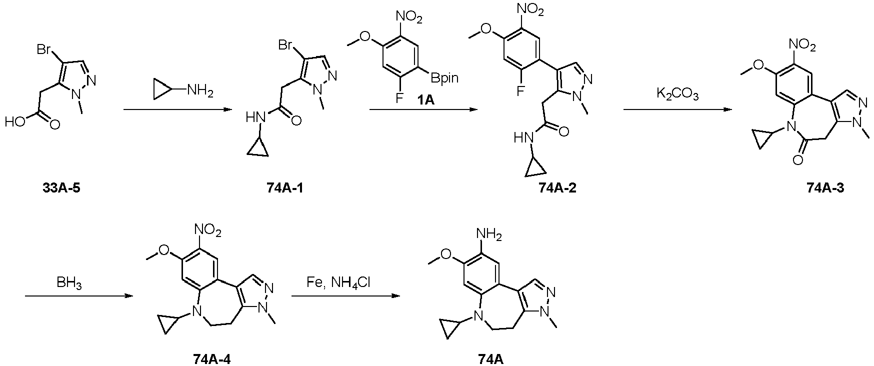

### Compound 74A-1:

Compound **33A-5** (500 mg, 2.28 mmol) was dissolved in N,N-dimethylformamide (5 mL); subsequently, the reaction liquid was added with 2-(7-azabenzotriazole)-N,N,N,N-tetramethyluronium hexafluorophosphate (1042 mg, 2.74 mmol), and successively stirred at room temperature for 30 min. Cyclopropylamine (196 mg, 3.42 mmol) and diisopropylethylamine (738 mg, 5.71 mmol) were added to the reaction liquid, and the reaction liquid was successively stirred at room temperature for 3 h. After the starting material disappeared as detected by LCMS, the reaction liquid was quenched by addition of water (30 mL). The mixture was extracted with ethyl acetate (30 mL × 3), and the organic phases were combined, washed with saturated brine (30 mL × 3) firstly, then dried over anhydrous sodium sulfate, filtered, and concentrated under reduced pressure. The resulting residue was purified by silica gel column chromatography (eluent: dichloromethane/methanol = 15/1) to obtain compound **74A-1** (500 mg).

MS (ESI, m/z): 258.0, 260.0 [M + H]⁺.

### Compound 74A-2:

Compound **74A-1** (700 mg, 2.71 mmol), compound **1A** (967 mg, 3.25 mmol) and anhydrous potassium phosphate (1.1 g, 5.42 mmol) were dissolved in dioxane (10 mL) and water (1 mL) under nitrogen atmosphere, followed by addition of methanesulfonato(tri-*tert*-butylphosphino)(2'-amino-1,1'-biphenyl-2-yl)palladium(II) (155 mg, 0.27 mmol) and tri-*tert*-butylphosphine tetrafluoroborate (79 mg, 0.27 mmol) to the reaction liquid. The reaction system was heated to 50 °C and successively stirred for 4 h. After the starting material disappeared as detected by LCMS, the reaction liquid was cooled to room temperature and concentrated under reduced pressure. The resulting residue was purified by silica gel column chromatography (eluent: dichloromethane/methanol = 15/1) to obtain compound **74A-2** (700 mg).

MS (ESI, m/z): 349.2 [M + H]⁺.

### Compound 74A-3:

Compound **74A-2** (680 mg, 1.95 mmol) was dissolved in *N*,*N*-dimethylformamide (10 mL); subsequently, anhydrous potassium carbonate (1079 mg, 7.81 mmol) was added to the reaction liquid. The reaction system was heated to 100 °C and successively stirred for 3 h. After the starting material disappeared as detected by LCMS, the reaction liquid was cooled to room temperature and quenched by addition of water (50 mL). The mixture was extracted with ethyl acetate (30 mL × 3), and the organic phases were combined, washed with saturated brine (30 mL × 3) firstly, then dried over anhydrous sodium sulfate, filtered, and concentrated under reduced pressure. The resulting residue was purified by silica gel column chromatography (eluent: dichloromethane/methanol = 15/1) to obtain compound **74A-3** (230 mg).

MS (ESI, m/z): 329.2 [M + H]⁺.

### Compound 74A-4:

Compound **74A-4** (110 mg) was prepared by replacing the starting material with compound **74A-3** (240 mg, 0.73 mmol) according to the method for the preparation of compound **11A-1** from compound **9A-4** in Example 11.

MS (ESI, m/z): 315.2 [M + H]⁺.

### Compound 74A:

Compound **74A** (80 mg) was prepared by replacing the starting material with compound **74A-4** (110 mg, 0.35 mmol) according to the method for the preparation of compound **1C** from compound **1C-9** in Example 1.

MS (ESI, m/z): 285.3 [M + H]⁺.

### Preparation of compound 74

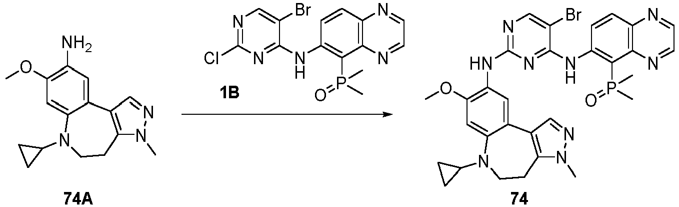

Compound **74** (15 mg) was prepared by replacing the starting material with compound **74A** (70 mg, 0.25 mmol) according to the method for the preparation of compound 1 from compound **1B** and compound **1C** in Example 1.

MS (ESI, m/z): 660.2, 662.2 [M + H]⁺.

¹H NMR (300 MHz, CDCl₃) δ 12.58 (s, 1H), 8.95 (dd, *J=* 9.6 Hz, 4.2 Hz, 1H), 8.72-8.68 (m, 2H), 8.33 (s, 1H), 8.27 (s, 1H), 7.70-7.58 (m, 1H), 7.45 (s, 1H), 7.00 (s, 1H), 6.90 (s, 1H), 3.93 (s, 3H), 3.71 (s, 3H), 3.53 (t, *J* = 5.7 Hz, 2H), 3.00 (t, *J* = 5.7 Hz, 2H), 2.80-2.72 (m, 1H), 2.17 (s, 3H), 2.13 (s, 3H), 0.84-0.77 (m, 2H), 0.61-0.55 (m, 2H).

### Example 75:

### Preparation of compound 75

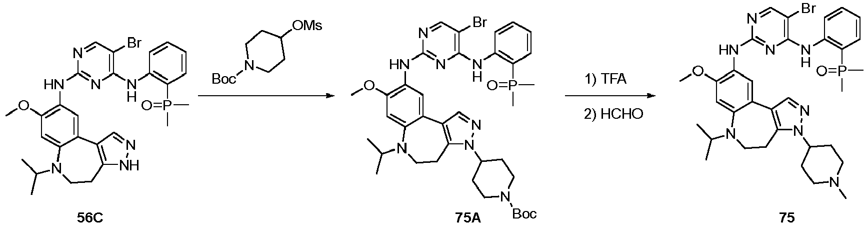

### Compound 75A:

Compound **75A** (250 mg) was prepared by replacing the starting material with *tert*-butyl 4-methanesulfonyloxypiperidine-1-carboxylate (281 mg, 1.01 mmol) according to the method for the preparation of compound **56** from compound **56C** in Example 56.

MS (ESI, m/z): 779.4, 781.4 [M + H]⁺.

### Compound 75:

Compound **75** (7 mg) was prepared by replacing the starting material with aqueous formaldehyde solution (37%, 177 mg, 2.18 mmol) according to the method for the preparation of compound **63A-2** from compound **63A-1** in Example 63.

MS (ESI, m/z): 693.4, 695.4 [M + H]⁺.

¹H NMR (300 MHz, CDCl₃) δ 10.59 (s, 1H), 8.54 (s, 1H), 8.28 (s, 1H), 7.46 (s, 1H), 7.38-7.28 (m, 2H), 7.28-7.15 (m, 2H), 7.04-6.95 (m, 1H), 6.63 (s, 1H), 4.13 (s, 1H), 4.04-3.91 (m, 4H), 3.37 (t, *J* = 5.7 Hz, 2H), 3.32-3.15 (m, 3H), 3.03 (t, *J=* 5.7 Hz, 2H), 2.60-2.31 (m, 6H), 2.22-2.02 (m, 2H), 1.95 (s, 3H), 1.90 (s, 3H), 1.40 (s, 3H), 1.38 (s, 3H).

### Example 76:

### Preparation of intermediate 76A

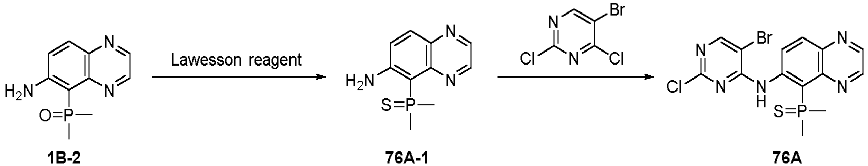

### Compound 76A-1:

Compound **1B-2** (5 g, 22.81 mmol) was dissolved in toluene (125 mL) under nitrogen atmosphere; subsequently, Lawesson reagent (18.45 g, 45.62 mmol) was added to the reaction liquid. The reaction system was heated to 95 °C and successively stirred for 16 h. After the starting material disappeared as detected by LCMS, the reaction liquid was cooled to room temperature and concentrated under reduced pressure. The resulting residue was purified by silica gel column chromatography (eluent: petroleum ether/ethyl acetate = 2/1) to obtain compound **76A-1** (3 g).

MS (ESI, m/z): 238.2 [M + H]⁺.

### Compound 76A:

Compound **76A** (2 g) was prepared by replacing the starting material with compound **76A-1** (3 g, 12.64 mmol) according to the method for the preparation of compound **1B** from compound **1B-2** in Example 1.

MS (ESI, m/z): 428.0, 430.0 [M + H]⁺.

### Preparation of compound 76

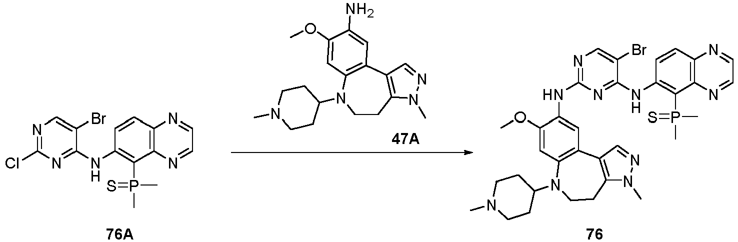

Compound **76** (2 mg) was prepared by replacing the starting material with compound **76A** (109 mg, 0.25 mmol) and compound **47A** (87 mg, 0.25 mmol) according to the method for the preparation of compound 1 from compound **1B** and compound **1C** in Example 1.

MS (ESI, m/z): 733.2, 735.2 [M + H]⁺.

¹H NMR (300 MHz, CDCl₃) δ 11.72 (s, 1H), 8.78 (d, *J =* 1.8 Hz, 1H), 8.71 (d, *J =* 1.8 Hz, 1H), 8.42 (s, 1H), 8.35 (s, 1H), 8.23 (dd, *J=* 9.6 Hz, 4.2 Hz, 1H), 7.65 (d, *J=* 9.3 Hz, 1H), 7.48 (s, 1H), 6.59-6.49 (m, 1H), 6.46 (s, 1H), 3.88 (s, 3H), 3.61 (s, 3H), 3.55-3.44 (m, 3H), 3.39 (t, *J =* 5.7 Hz, 2H), 2.89 (t, *J =* 5.7 Hz, 2H), 2.73-2.60 (m, 5H), 2.51 (s, 3H), 2.46 (s, 3H), 2.37-2.21 (m, 2H), 2.03-1.93 (m, 2H).

### Example 77:

### Preparation of compounds 77A and 77B

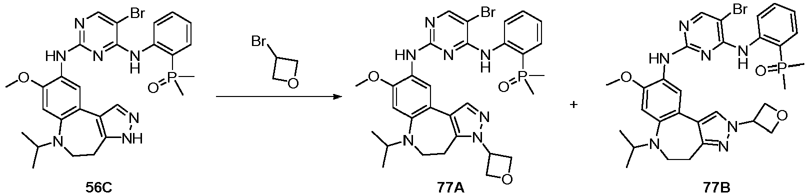

Compounds **77A** (6 mg) and **77B** (4 mg) was prepared by replacing the starting material with 3-bromooxetane (103 mg, 0.75 mmol) according to the method for the preparation of compound **56** from compound **56C** in Example 56.

**77A:** MS (ESI, m/z): 652.2, 654.2 [M + H]⁺.

**77B:** MS (ESI, m/z): 652.2, 654.2 [M + H]⁺.

**77A:** ¹H NMR (400 MHz, CDCl₃) δ 10.56 (s, 1H), 8.50-8.42 (m, 2H), 8.21 (s, 1H), 7.47 (s, 1H), 7.31-7.25 (m, 1H), 7.21 (s, 1H), 7.15 (s, 1H), 6.93 (t, *J=* 7.6 Hz, 1H), 6.54 (s, 1H), 5.43-5.34 (m, 1H), 5.23 (t, *J =* 6.4 Hz, 2H), 5.00 (t, *J =* 6.4 Hz, 2H), 3.93-3.84 (m, 4H), 3.26 (t, *J =* 5.6 Hz, 2H), 2.86 (t, *J=* 5.6 Hz, 2H), 1.87 (s, 3H), 1.84 (s, 3H), 1.30 (s, 3H), 1.28 (s, 3H).

**77B:** ¹H NMR (400 MHz, CDCl₃) δ 10.77 (s, 1H), 8.66 (dd, *J=* 8.4 Hz, 4.4 Hz, 1H), 8.34 (s, 1H), 8.22 (s, 1H), 7.43 (s, 1H), 7.26-7.18 (m, 2H), 7.06 (s, 1H), 6.90 (t, *J =* 8.0 Hz, 1H), 6.56 (s, 1H), 5.27-5.17 (m, 1H), 4.96-4.86 (m, 4H), 3.94-3.86 (m, 4H), 3.31 (t, *J=* 5.2 Hz, 2H), 3.10 (t, *J* = 5.2 Hz, 2H), 1.87 (s, 3H), 1.84 (s, 3H), 1.30 (s, 3H), 1.29 (s, 3H).

### Example 78:

### Preparation of compound 78

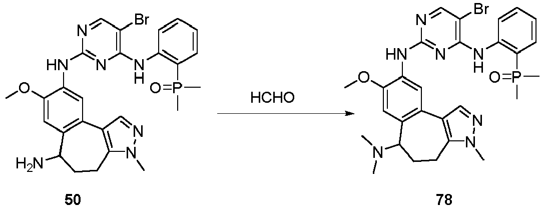

Compound **78** (17 mg) was prepared by replacing the starting material with compound **50** (40 mg, 0.07 mmol) and paraformaldehyde (31 mg, 0.35 mmol) according to the method for the preparation of compound **72** from compound **71** in Example 72.

MS (ESI, m/z): 610.2, 612.2 [M + H]⁺.

¹H NMR (400 MHz, CDCl₃) δ 10.57 (s, 1H), 8.39 (dd, *J=* 8.8 Hz, 4.4 Hz, 1H), 8.26 (s, 1H), 8.23 (s, 1H), 7.55 (s, 1H), 7.36-7.29 (m, 1H), 7.22 (t, *J =* 7.6 Hz, 1H), 7.14 (s, 1H), 7.04 (t, *J =* 7.6 Hz, 1H), 6.78 (s, 1H), 3.97-3.90 (m, 4H), 3.79 (s, 3H), 3.18-3.05 (m, 1H), 3.04-3.94 (m, 1H), 2.69-2.58 (m, 1H), 2.52 (s, 6H), 2.42-2.31 (m, 1H), 1.88 (s, 3H), 1.85 (s, 3H).

### Example 79:

### Preparation of compound 79

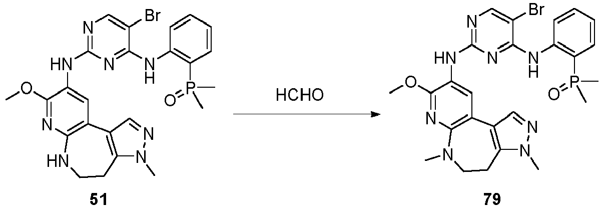

Compound **79** (35 mg) was prepared by replacing the starting material with compound **51** (50 mg, 0.09 mmol) and aqueous formaldehyde solution (40%, 13 mg, 0.18 mmol) according to the method for the preparation of compound **72** from compound **71** in Example 72.

MS (ESI, m/z): 583.2, 585.2 [M + H]⁺.

¹H NMR (300 MHz, CDCl₃) δ 10.69 (s, 1H), 8.58 (s, 1H), 8.45 (dd, *J* = 8.7 Hz, 4.5 Hz, 1H), 8.21 (s, 1H), 7.35-7.29 (m, 1H), 7.27-7.14 (m, 3H), 7.06-6.98 (m, 1H), 3.99 (s, 3H), 3.83 (s, 3H), 3.38 (t, *J=* 5.4 Hz, 2H), 3.16 (s, 3H), 3.03 (t, *J=* 5.4 Hz, 2H), 1.88 (s, 3H), 1.84 (s, 3H).

### Example 80:

### Preparation of compound 80

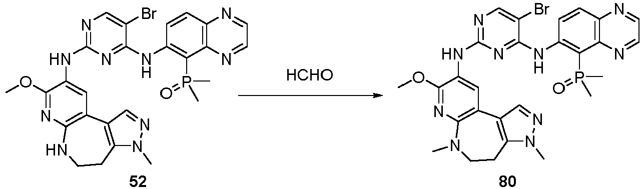

Compound 80 (33 mg) was prepared by replacing the starting material with compound 52 (100 mg, 0.16 mmol) and aqueous formaldehyde solution (40%, 24 mg, 0.32 mmol) according to the method for the preparation of compound 72 from compound 71 in Example 72.

MS (ESI, m/z): 635.2, 637.2 [M + H]⁺.

¹H NMR (300 MHz, DMSO-d₆) δ 8.84-8.77 (m, 2H), 8.72 (s, 1H), 8.53 (s, 1H), 8.25 (s, 1H), 8.00 (s, 1H), 7.57 (s, 1H), 3.85 (s, 3H), 3.71 (s, 3H), 3.33 (t, *J* = 4.2 Hz, 2H), 3.15 (s, 3H), 3.03 (t, *J* = 4.2 Hz, 2H), 2.03 (s, 3H), 1.98 (s, 3H).

### Example 81:

### Preparation of intermediate 81A

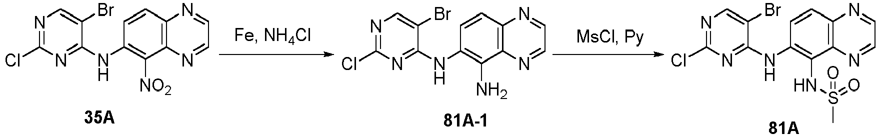

### Compound 81A-1:

Compound **81A-1** (2 g) was prepared by replacing the starting material with compound **35A** (2.4 g, 6.3 mmol) according to the method for the preparation of compound **1C** from compound **1C-9** in Example 1.

MS (ESI, m/z): 351.1, 353.1 [M + H]⁺.

### Compound 81A:

Compound **81A** (300 mg) was prepared by replacing the starting material with compound **81A-1** (590 mg, 1.68 mmol) according to the method for the preparation of compound **32** from compound **32C** in Example 32.

MS (ESI, m/z): 429.0, 431.0 [M + H]⁺.

### Preparation of compound 81

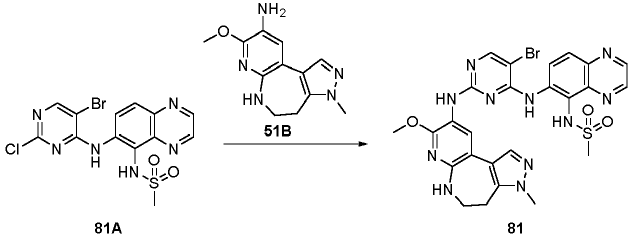

Compound **81A** (88 mg, 0.2 mmol), compound **51B** (50 mg, 0.2 mmol) and ammonium chloride (109 mg, 2 mmol) were dissolved in ethanol (2 mL). The reaction system was heated to 85°C and successively stirred for 16 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound **81** (40 mg).

MS (ESI, m/z): 638.1, 640.1 [M + H]⁺.

¹H NMR (300 MHz, CDCl₃) δ 9.14 (s, 1H), 8.86-8.79 (m, 2H), 8.56 (d, *J* = 9.6 Hz, 1H), 8.43 (s, 1H), 8.28 (s, 1H), 7.68 (s, 1H), 7.58 (s, 1H), 7.06 (s, 1H), 3.94 (s, 3H), 3.70 (s, 3H), 3.48 (t, *J=* 4.8 Hz, 2H), 2.99 (t, *J=* 4.8 Hz, 2H), 2.95 (s, 3H).

### Example 82:

### Preparation of compound 82

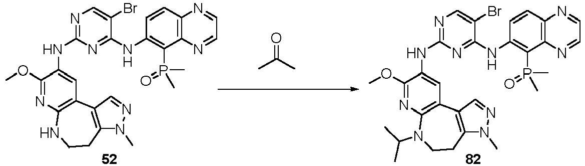

Compound **82** (20 mg) was prepared by replacing the starting material with compound **52** (85 mg, 0.14 mmol) according to the method for the preparation of compound **72** from compound **71** in Example 72.

MS (ESI, m/z): 663.2, 665.2 [M + H]⁺.

¹H NMR (400 MHz, DMSO-d₆) δ 12.63 (s, 1H), 8.84-8.77 (m, 2H), 8.74 (s, 1H), 8.51 (s, 1H), 8.25 (s, 1H), 8.02 (s, 1H), 7.58 (s, 1H), 7.53 (s, 1H), 4.59-4.43 (m, 1H), 3.83 (s, 3H), 3.72 (s, 3H), 3.27 (t, *J=* 5.2 Hz, 2H), 2.95 (t, *J=* 5.2 Hz, 2H), 2.02 (s, 3H), 1.99 (s, 3H), 1.31 (s, 3H), 1.29 (s, 3H).

### Example 83:

### Preparation of compound 83

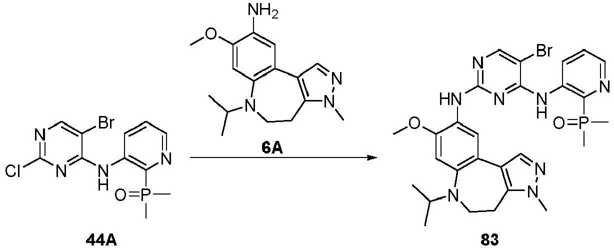

Compound **83** (33 mg) was prepared by replacing the starting material with compound **44A** (126 mg, 0.35 mmol) and compound **6A** (100 mg, 0.35 mmol) according to the method for the preparation of compound **1** from compound **1B** and compound **1C** in Example 1.

MS (ESI, m/z): 611.2, 613.2 [M + H]⁺.

¹HNMR (300 MHz, CDCl₃) δ 11.03 (s, 1H), 9.04-8.96 (m, 1H), 8.30 (s, 1H), 8.24 (s, 1H), 8.18 (d, *J=* 4.5 Hz, 1H), 7.50 (s, 1H), 7.12 (s, 1H), 6.99-6.91 (m, 1H), 6.58 (s, 1H), 3.98-3.90 (m, 1H), 3.88 (s, 3H), 3.84 (s, 3H), 3.32 (t, *J* = 4.8 Hz, 2H), 2.97 (t, *J* = 4.8 Hz, 2H), 1.90 (s, 3H), 1.86 (s, 3H), 1.35 (s, 3H), 1.32 (s, 3H).

### Example 84:

### Preparation of intermediate 84A

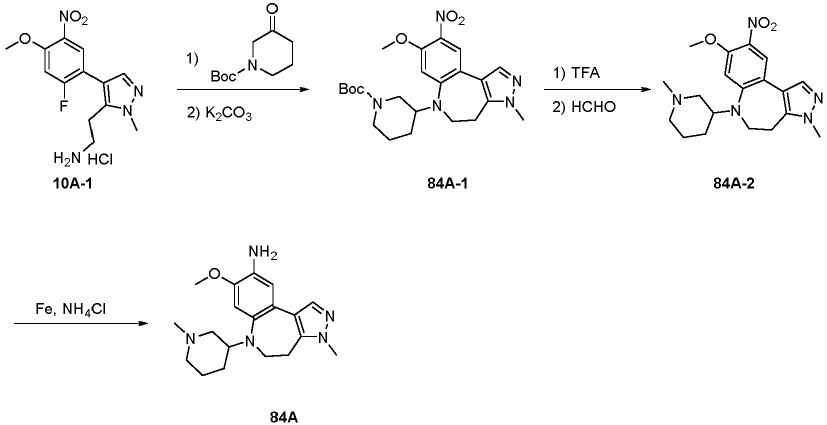

### Compound 84A-1:

Compound **84A-1** (500 mg) was prepared by replacing the starting material with tert-butyl 3-oxopiperidine-1-carboxylate (1693 mg, 8.5 mmol) according to the method for the preparation of compound **47A-1** from compound **10A-1** in Example 47.

MS (ESI, m/z): 458.1 [M + H]⁺.

### Compound 84A-2:

Compound **84A-2** (80 mg) was prepared by replacing the starting material with aqueous formaldehyde solution (40%, 82 mg, 1.1 mmol) according to the method for the preparation of compound **63A-2** from compound **63A-1** in Example 63.

MS (ESI, m/z): 372.2 [M + H]⁺.

### Intermediate 84A:

Compound **84A** (45 mg) was prepared by replacing the starting material with compound **84A-2** (80 mg, 0.22 mmol) according to the method for the preparation of compound **1C** from compound **1C-9** in Example 1.

MS (ESI, m/z): 342.2 [M + H]⁺.

### Preparation of compound 84

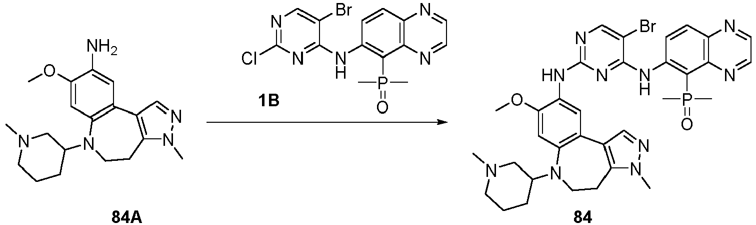

Compound **84** (8 mg) was prepared by replacing the starting material with compound **84A** (70 mg, 0.21 mmol) according to the method for the preparation of compound **1** from compound **1B** and compound **1C** in Example 1.

MS (ESI, m/z): 717.2, 719.2 [M + H]⁺.

¹H NMR (300 MHz, DMSO-d₆) δ 12.62 (s, 1H), 8.86-8.71 (m, 3H), 8.37 (s, 1H), 8.26 (s, 1H), 7.76 (s, 1H), 7.55 (s, 1H), 7.44 (s, 1H), 6.60 (s, 1H), 3.77 (s, 3H), 3.70 (s, 3H), 3.65-3.59 (m, 2H), 3.09-3.00 (m, 2H), 2.97-2.88 (m, 2H), 2.84-2.77 (m, 1H), 2.28 (s, 3H), 2.20 (t, *J =* 10.5 Hz, 1H), 2.04 (s, 3H), 1.99 (s, 3H), 1.96-1.87 (m, 2H), 1.74-1.53 (m, 3H).

### Example 85:

### Preparation of compound 85

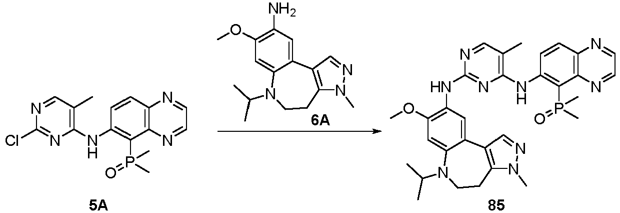

Compound **85** (46 mg) was prepared by replacing the starting material with compound **6A** (100 mg, 0.35 mmol) and compound **5A** (109 mg, 0.31 mmol) according to the method for the preparation of compound **1** from compound **1B** and compound **1C** in Example 1.

MS (ESI, m/z): 598.3 [M + H]⁺.

¹H NMR (400 MHz, CDCl₃) δ 12.36 (s, 1H), 9.20 (dd, *J=* 9.6 Hz, 4.4 Hz, 1H), 8.66-8.64 (m, 2H), 8.38 (s, 1H), 7.95 (s, 1H), 7.76-7.56 (m, 2H), 7.21 (s, 1H), 6.58 (s, 1H), 3.96-3.89 (m, 1H), 3.38 (s, 3H), 3.73 (s, 3H), 3.32 (t, *J =* 5.6 Hz, 2H), 2.97 (t, *J* = 5.6 Hz, 2H), 2.28 (s, 3H), 2.13 (s, 3H), 2.10 (s, 3H), 1.33 (s, 3H), 1.31 (s, 3H).

### Example 86:

### Preparation of intermediate 86A

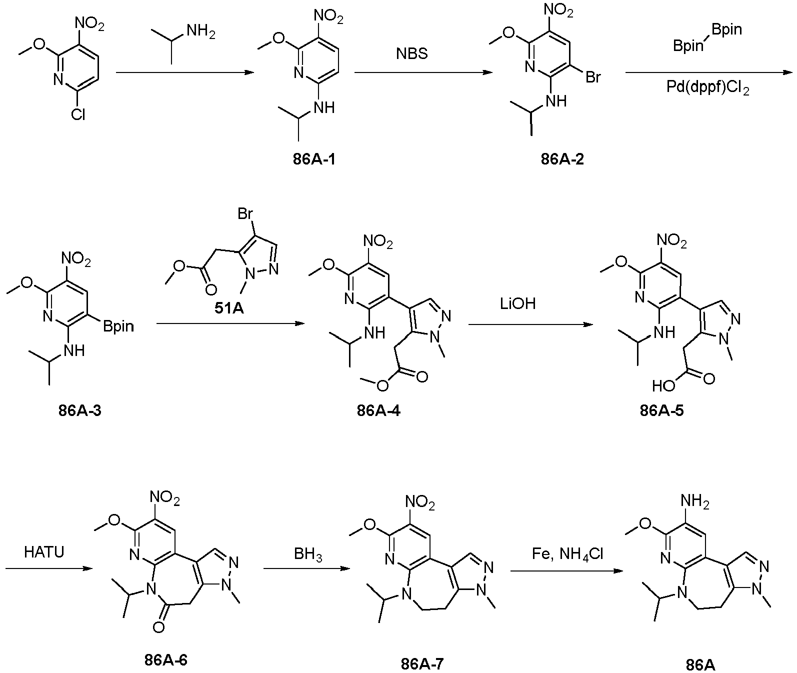

### Compound 86A-1: 91

Compound **86A-1** (3 g) was prepared by replacing the starting material with isopropylamine (1.57 g, 26.5 mmol) according to the method for the preparation of compound **51B-1** from 2-methoxy-3-nitro-6-chloropyridine in Example 51.

MS (ESI, m/z): 212.1 [M + H]⁺.

### Compound 86A-2:

Compound **86A-2** (3.81 g) was prepared by replacing the starting material with compound **86A-1** (3 g, 14.2 mmol) according to the method for the preparation of compound **24B-1** from compound **1C-2** in Example 24.

MS (ESI, m/z): 290.0, 292.0 [M + H]⁺.

### Compound 86A-3:

Compound **86A-3** (3.54 g) was prepared by replacing the starting material with compound **86A-2** (5.3 g, 18.3 mmol) according to the method for the preparation of compound **1A** from compound **1A-1** in Example 1.

MS (ESI, m/z): 338.3 [M + H]⁺.

### Compound 86A-4:

Compound **86A-4** (1.7 g) was prepared by replacing the starting material with compound **86A-3** (3.54 g, 10.5 mmol) according to the method for the preparation of compound **51B-5** from compound **51B-4** in Example 51.

MS (ESI, m/z): 364.1 [M + H]⁺.

### Compound 86A-5:

Compound **86A-5** (1.6 g) was prepared by replacing the starting material with compound **86A-4** (1.7 g, 4.68 mmol) according to the method for the preparation of compound **51B-6** from compound **51B-5** in Example 51.

MS (ESI, m/z): 350.3 [M + H]⁺.

### Compound 86A-6:

Compound **86A-6** (1.4 g) was prepared by replacing the starting material with compound **86A-5** (1.72 g, 4.92 mmol) according to the method for the preparation of compound **51B-7** from compound **51B-6** in Example 51.

MS (ESI, m/z): 332.3 [M + H]⁺.

### Compound 86A-7:

Compound **86A-7** (1.3 g) was prepared by replacing the starting material with compound **86A-6** (1.4 g, 4.23 mmol) according to the method for the preparation of compound **11A-1** from compound **9A-4** in Example 11.

MS (ESI, m/z): 318.1 [M + H]⁺.

### Compound 86A:

Compound **86A** (0.9 g) was prepared by replacing the starting material with compound **86A-7** (1.35 g, 4.25 mmol) according to the method for the preparation of compound **1C** from compound **1C-9** in Example 1.

MS (ESI, m/z): 288.2 [M + H]⁺.

### Preparation of compound 86

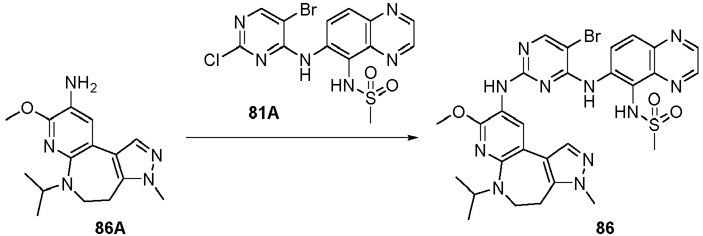

Compound **86** (56 mg) was prepared by replacing the starting material with compound **86A** (75 mg, 0.26 mmol) and compound **81A** (112 mg, 0.26 mmol) according to the method for the preparation of compound 1 from compound **1B** and compound **1C** in Example 1.

MS (ESI, m/z): 680.2, 682.2 [M + H]⁺.

¹H NMR (300 MHz, DMSO-d₆) δ 10.00 (s, 1H), 9.02 (d, *J* = 5.1 Hz, 1H), 8.93 (d, *J* = 5.1 Hz, 1H), 8.86 (s, 1H), 8.70 (s, 1H), 8.58 (s, 1H), 8.34 (s, 1H), 8.08 (s, 1H), 7.66-7.40 (m, 2H), 4.67-4.52 (m, 1H), 3.88 (s, 3H), 3.80 (s, 3H), 3.35 (t, *J=* 5.4 Hz, 2H), 3.08 (s, 3H), 3.03 (t, *J=* 5.4 Hz, 2H), 1.38 (s, 3H), 1.36 (s, 3H).

### Example 87:

### Preparation of intermediate 87A

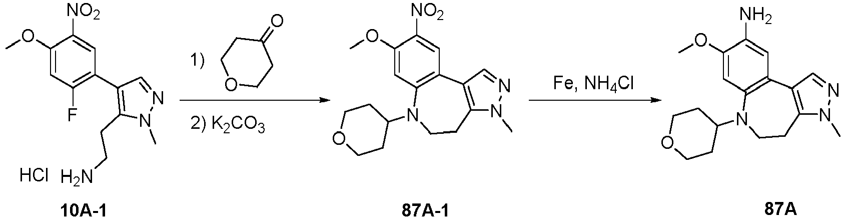

### Compound 87A-1:

Compound **87A-1** (163 mg) was prepared by replacing the starting material with 4-oxacyclohexanone (204 mg, 2.04 mmol) according to the method for the preparation of compound **47A-1** from compound **10A-1** in Example 47.

MS (ESI, m/z): 359.2 [M + H]⁺.

### Intermediate 87A:

Compound **87A** (130 mg) was prepared by replacing the starting material with compound **87A-1** (163 mg, 0.46 mmol) according to the method for the preparation of compound **1C** from compound **1C-9** in Example 1.

MS (ESI, m/z): 329.3 [M + H]⁺.

### Preparation of compound 87

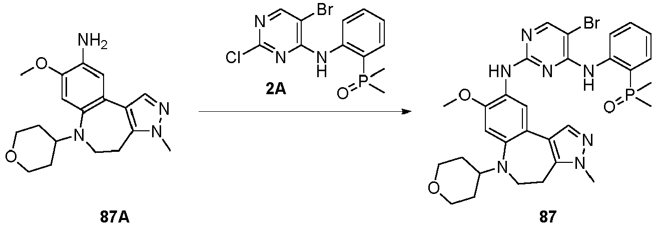

Compound **87** (26 mg) was prepared by replacing the starting material with compound **87A** (50 mg, 0.15 mmol) and compound **2A** (55 mg, 0.15 mmol) according to the method for the preparation of compound **1** from compound **1B** and compound **1C** in Example 1.

MS (ESI, m/z): 652.2, 654.2 [M + H]⁺.

¹H NMR (300 MHz, CDCl₃) δ 10.67 (s, 1H), 8.48 (dd, *J=* 8.1 Hz, 4.2 Hz, 1H), 8.42 (s, 1H), 8.22 (s, 1H), 7.43 (s, 1H), 7.35-7.25 (m, 2H), 7.23-7.15 (m, 1H), 7.04-6.96 (m, 1H), 6.57 (s, 1H), 4.13-4.04 (m, 2H), 3.88 (s, 3H), 3.83 (s, 3H), 3.66-3.36 (m, 5H), 3.00-2.93 (m, 2H), 1.97-1.86 (m, 7H), 1.84 (s, 3H).

### Example 88:

### Preparation of compound 88

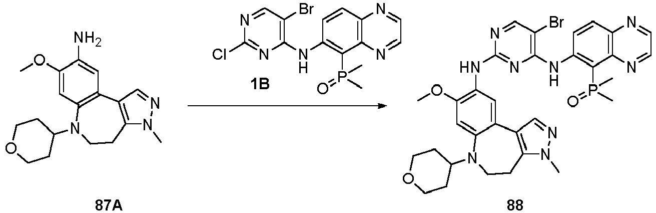

Compound **88** (45 mg) was prepared by replacing the starting material with compound **87A** (50 mg, 0.15 mmol) according to the method for the preparation of compound 1 from compound **1B** and compound **1C** in Example 1.

MS (ESI, m/z): 704.2, 706.2 [M + H]⁺.

¹H NMR (300 MHz, CDCl₃) δ 12.54 (s, 1H), 8.89 (dd, *J=* 9.6 Hz, 4.2 Hz, 1H), 8.73-8.68 (m, 2H), 8.40 (s, 1H), 8.30 (s, 1H), 7.70-7.60 (m, 1H), 7.41 (s, 1H), 7.03 (s, 1H), 6.58 (s, 1H), 4.14-4.03 (m, 2H), 3.89 (s, 3H), 3.72 (m, 3H), 3.67-3.56 (m, 1H), 3.55-3.40 (m, 4H), 3.03-2.93 (m, 2H), 2.18 (s, 3H), 2.13 (s, 3H), 2.00-1.83 (m, 4H).

### Example 89:

### Preparation of compound 89

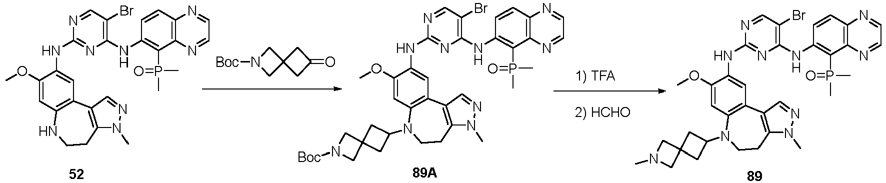

### Compound 89A:

Compound **89A** (88 mg) was prepared by replacing the starting material with compound **52** (150 mg, 0.24 mmol) and tert-butyl 6-oxo-2-azaspiro[3.3]heptane-2-carboxylate (102 mg, 0.48 mmol) according to the method for the preparation of compound **72** from compound **71** in Example 72. MS (ESI, m/z): 815.2, 817.2 [M + H]⁺.

### Compound 89:

Compound **89** (16 mg) was prepared by replacing the starting material with compound **89A** (50 mg, 0.06 mmol) according to the method for the preparation of compound **63A-2** from compound **63A-1** in Example 63.

MS (ESI, m/z): 729.2, 731.2 [M + H]⁺.

¹H NMR (400 MHz, CDCl₃) δ 12.50 (s, 1H), 8.87 (dd, *J=* 9.6 Hz, 4.0 Hz, 1H), 8.72-8.65 (m, 2H), 8.48 (s, 1H), 8.28 (s, 1H), 7.59 (s, 1H), 7.25 (s, 1H), 6.98 (s, 1H), 6.20 (s, 1H), 4.04-3.94 (m, 3H), 3.86 (s, 3H), 3.84-3.79 (m, 2H), 3.72 (s, 3H), 3.20 (t, *J =* 6.0 Hz, 2H), 2.99 (t, *J =* 6.0 Hz, 2H), 2.72-2.64 (m, 5H), 2.30-2.23 (m, 2H), 2.15 (s, 3H), 2.11 (s, 3H).

### Example 90:

### Preparation of compound 90

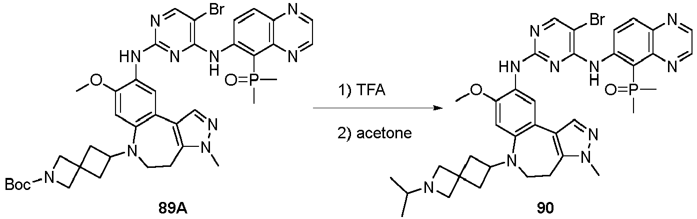

Compound **90** (4 mg) was prepared by replacing the starting material with compound **89A** (50 mg, 0.06 mmol) and acetone (11 mg, 0.14 mmol) according to the method for the preparation of compound **63A-2** from compound **63A-1** in Example 63.

MS (ESI, m/z): 757.2, 759.2 [M + H]⁺.

¹H NMR (300 MHz, CDCl₃) δ 12.49 (s, 1H), 8.88 (dd, *J=* 9.6 Hz, 4.2 Hz, 1H), 8.75-8.64 (m, 2H), 8.53 (s, 1H), 8.38 (s, 1H), 8.30 (s, 1H), 7.67-7.53 (m, 1H), 6.98 (s, 1H), 6.22 (s, 1H), 4.09-3.92 (m, 3H), 3.87 (s, 3H), 3.83-3.77 (m, 2H), 3.73 (s, 3H), 3.26-3.14 (m, 2H), 3.07-3.92 (m, 3H), 2.75-2.67 (m, 2H), 2.30-2.55 (m, 2H), 2.18 (s, 3H), 2.13 (s, 3H), 1.26 (s, 3H), 1.24 (s, 3H).

### Example 91: preparation of intermediate 91A

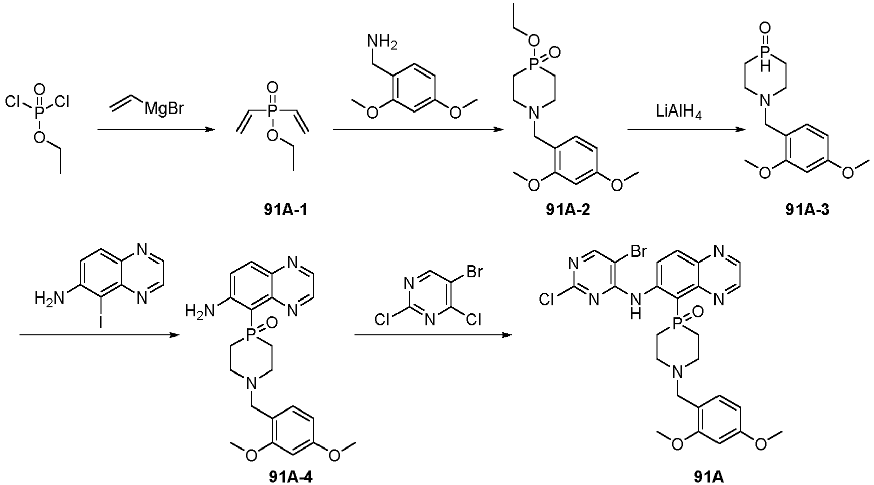

### Compound 90A-1:

Ethyldichlorophosphate (5 g, 30.69 mmol) was dissolved in dichloromethane under nitrogen atmosphere under nitrogen atmosphere, and the reaction liquid was cooled to -78 °C, added with a solution of vinylmagnesium bromide in tetrahydrofuran (1 M, 68 mL, 68 mmol) and successively stirred at that temperature for 1 h. After the starting material disappeared as detected by LCMS, the reaction liquid was quenched by addition of 1 N hydrochloric acid (100 mL). The mixture was extracted with ethyl acetate (150 mL × 3), and the organic phases were combined, washed with saturated brine (150 mL × 3) firstly, then dried over anhydrous sodium sulfate, filtered, and concentrated under reduced pressure. The resulting residue was purified by silica gel column chromatography (eluent: dichloromethane/methanol = 10/1) to obtain compound **90A-1** (1.1 g).

MS (ESI) M/Z: 147.3 [M + H]⁺.

### Compound 91A-2:

Compound **91A-1** (1.4 g, 9.58 mmol) was dissolved in ethanol (10 mL), followed by addition of 2,4-dimethoxybenzylamine (1.92 g, 11.45 mmol) to the reaction liquid. The reaction system was heated to 90 °C and successively stirred for 4 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound **91A-2** (2 g).

MS (ESI) M/Z: 314.1 [M + H]⁺.

### Compound 91A-3:

Compound **91A-2** (1.1 g, 3.51 mmol) was dissolved in anhydrous tetrahydrofuran (15 mL) under nitrogen atmosphere; subsequently, the reaction liquid was added with a solution of lithium aluminum hydride in tetrahydrofuran (1 M, 2.81 mL, 2.81 mmol) at 0 °C and successively stirred at that temperature for 1 h. After the starting material disappeared as detected by LCMS, the reaction liquid was quenched by addition of water (0.1 mL), 15% aqueous sodium hydroxide solution (0.1 mL) and water (0.3 mL). The reaction liquid was successively stirred at room temperature for 1 h and concentrated under reduced pressure. The resulting residue was purified by silica gel column chromatography (eluent: dichloromethane/methanol = 10/1) to obtain compound **91A-3** (0.27 g).

MS (ESI) M/Z: 270.1 [M + H]⁺.

### Compound 91A-4:

Compound **91A-4** (200 mg) was prepared by replacing the starting material with compound **91A-3** (220 mg, 0.82 mmol) according to the method for the preparation of compound **1B-2** from compound **1B-1** in Example 1.

MS (ESI, m/z): 413.1 [M + H]⁺.

### Intermediate 91A:

Compound **91A** (78 mg) was prepared by replacing the starting material with compound **91A-4** (150 mg, 0.36 mmol) according to the method for the preparation of compound **1B** from compound **1B-2** in Example 1.

MS (ESI, m/z): 603.1, 605.1 [M + H]⁺.

### Preparation of compound 91

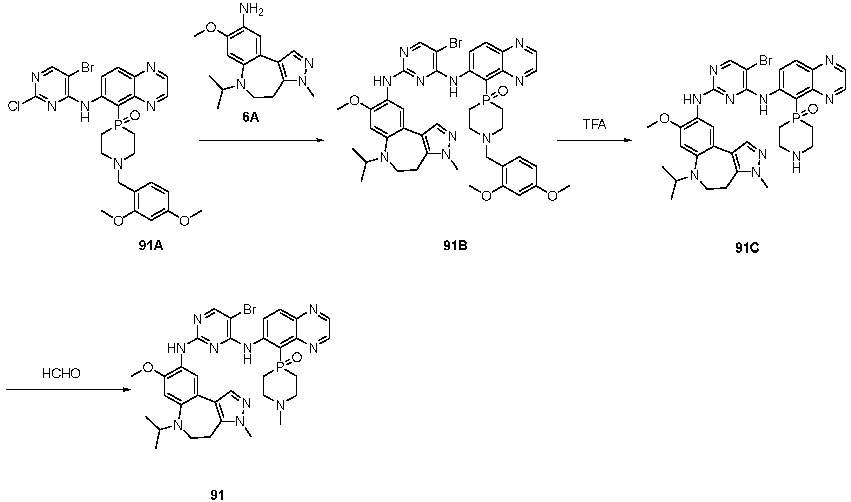

### Compound 91B:

Compound **91B** (50 mg) was prepared by replacing the starting material with compound **91A** (78 mg, 0.13 mmol) and compound **6A** (37 mg, 0.13 mmol) according to the method for the preparation of compound **1** from compound **1B** and compound **1C** in Example 1.

MS (ESI, m/z): 853.4, 855.4 [M + H]⁺.

### Compound 91C:

Compound **91C** (35 mg) was prepared by replacing the starting material with compound **91B** (50 mg, 0.06 mmol) according to the method for the preparation of compound **37** from compound **34** in Example 37.

MS (ESI, m/z): 703.3, 705.3 [M + H]⁺.

### Compound 91:

Compound **91** (10 mg) was prepared by replacing the starting material with compound **91C** (35 mg, 0.05 mmol) and aqueous formaldehyde solution (40%, 19 mg, 0.25 mmol) according to the method for the preparation of compound **72** from compound **71** in Example 72.

MS (ESI, m/z): 717.2, 719.2 [M + H]⁺.

¹H NMR (300 MHz, CDCl₃) δ 11.72 (s, 1H), 9.12 (d, J= 1.8 Hz, 1H), 8.83-8.78 (m, 1H), 8.77 (d, *J=* 1.8 Hz, 1H), 8.34 (s, 1H), 8.31 (s, 1H), 7.76-7.64 (m, 1H), 7.33 (s, 1H), 6.91 (s, 1H), 6.60 (s, 1H), 3.93-3.80 (m, 9H), 3.72-3.65 (m, 4H), 3.38-3.28 (m, 2H), 3.01-2.92 (m, 5H), 2.47-2.30 (m, 2H), 1.34 (s, 3H), 1.32 (s, 3H).

### Example 92:

### Preparation of intermediate 92

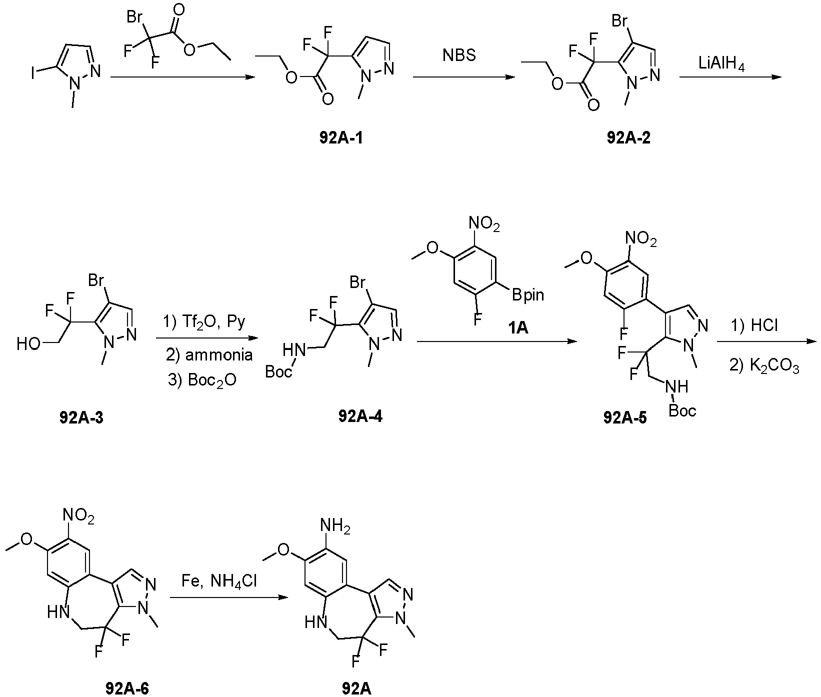

### Compound 92A-1:

5-Iodo-1-methyl-1*H*-pyrazole (9.5 g, 45.67 mmol) and ethyl (bromodifluoro) acetate (13.91 g, 68.51 mmol) were dissolved in dimethyl sulfoxide (100 mL); subsequently, copper powder (5.80 g, 91.35 mmol) was added to the reaction liquid. The reaction system was heated to 80 °C and stirred for 16 h. After the starting material disappeared as detected by LCMS, the reaction liquid was cooled to room temperature and quenched by addition of water (500 mL). The mixture was extracted with ethyl acetate (200 mL × 3), and the organic phases were combined, washed with saturated brine (200 mL × 3) firstly, then dried over anhydrous sodium sulfate, filtered, and concentrated under reduced pressure. The resulting residue was purified by silica gel column chromatography (eluent: petroleum ether/methyl *tert*-butyl ether = 1/1) to obtain compound **92A-1** (6.22 g).

MS (ESI) M/Z: 205.3 [M + H]⁺.

### Compound 92A-2:

Compound **92A-1** (2.95 g, 14.45 mmol) was dissolved in acetonitrile (50 mL); subsequently, N-bromosuccinimide (5.66 g, 31.79 mmol) was added to the reaction liquid. The reaction system was heated to 50 °C and successively stirred for 16 h. After the starting material disappeared as detected by LCMS, the reaction liquid was cooled to room temperature and concentrated under reduced pressure. The resulting residue was purified by silica gel column chromatography (eluent: petroleum ether/methyl tert-butyl ether = 1/1) to obtain compound **92A-2** (3.26 g).

MS (ESI) M/Z: 283.0, 285.0 [M + H]⁺.

### Compound 92A-3:

Compound **92A-3** (1.6 g) was prepared by replacing the starting material with compound **92A-2** (3.76 g, 13.28 mmol) according to the method for the preparation of compound **91A-3** from compound **91A-2** in Example 91.

MS (ESI, m/z): 241.0, 243.0 [M + H]⁺.

### Compound 92A-4:

Compound **92A-3** (1.8 g, 7.47 mmol) and pyridine (1.3 g, 16.430 mmol) were dissolved in acetonitrile (15 mL); subsequently, trifluoromethanesulfonic anhydride (4.21 g, 14.97 mmol) was added dropwise to the reaction liquid at 0 °C. After the reaction liquid was warmed to room temperature and successively stirred for 1 h, the reaction liquid was added with aqueous ammonia (12 mL), and the reaction system was heated to 50 °C and successively stirred for 16 h. After the starting material disappeared as detected by LCMS, the reaction liquid was cooled to room temperature and concentrated under reduced pressure. The resulting residue was dissolved in dichloromethane (30 mL); subsequently, the reaction liquid was added with triethylamine (1.52 g, 15 mmol) and di-tert-butyl dicarbonate (8.18 g, 37.49 mmol) and successively stirred at room temperature for 2 h. The reaction liquid was quenched by addition of water (80 mL). The mixture was extracted with ethyl acetate (100 mL × 2), and the organic phases were combined, washed with saturated brine (100 mL × 3) firstly, then dried over anhydrous sodium sulfate, filtered, and concentrated under reduced pressure. The resulting residue was purified by silica gel column chromatography (eluent: petroleum ether/methyl tert-butyl ether = 1/1) to obtain compound **92A-4** (1.6 g).

MS (ESI) M/Z: 340.0, 342.0 [M + H]⁺.

### Compound 92A-5:

Compound **92A-5** (700 mg) was prepared by replacing the starting material with compound **92A-4** (650 mg, 1.91 mmol) and compound **1A** (681 mg, 2.29 mmol) according to the method for the preparation of compound **51B-5** from compound **51B-4** in Example 51.

MS (ESI, m/z): 431.2 [M + H]⁺.

### Compound 92A-6:

Compound **92A-6** (387 mg) was prepared by replacing the starting material with compound **92A-5** (700 mg, 1.63 mmol) according to the method for the preparation of compound **42A-7** from compound **42A-6** in Example 42.

MS (ESI, m/z): 311.0 [M + H]⁺.

### Intermediate 92A:

Compound **92A** (100 mg) was prepared by replacing the starting material with compound **92A-6** (120 mg, 0.39 mmol) according to the method for the preparation of compound **1C** from compound **1C-9** in Example 1.

MS (ESI, m/z): 281.1 [M + H]⁺.

### Preparation of compound 92

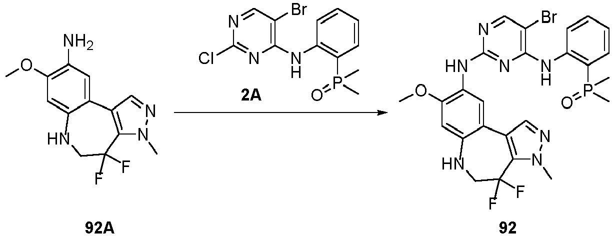

Compound **92** (38 mg) was prepared by replacing the starting material with compound **92A** (100 mg, 0.36 mmol) and compound **2A** (129 mg, 0.36 mmol) according to the method for the preparation of compound **1** from compound **1B** and compound **1C** in Example 1.

MS (ESI, m/z): 604.1, 606.1 [M + H]⁺.

¹H NMR (300 MHz, CDCl₃) δ 10.60 (s, 1H), 8.53 (s, 1H), 8.43 (dd, *J=* 9.6 Hz, 4.2 Hz, 1H), 8.25 (s, 1H), 7.36-7.30 (m, 2H), 7.26-7.15 (m, 2H), 7.04-6.96 (m, 1H), 6.43 (s, 1H), 4.10 (s, 3H), 3.89 (s, 3H), 3.59 (t, *J=* 12.0 Hz, 2H), 1.89 (s, 3H), 1.85 (s, 3H).

¹⁹F NMR (282 MHz, CDCl₃) δ -92.73.

### Example 93:

### Preparation of compound 93

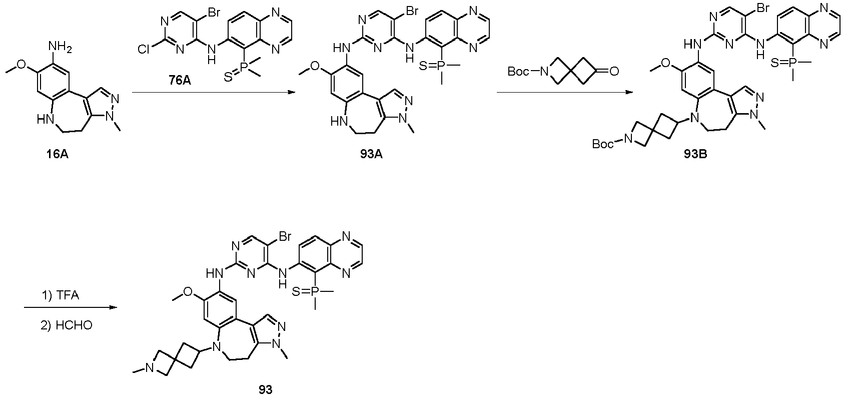

### Compound 93A:

Compound **93A** (50 mg) was prepared by replacing the starting material with compound **16A** (48 mg, 0.19 mmol) and compound **76A** (100 mg, 0.23 mmol) according to the method for the preparation of compound **1** from compound **1B** and compound **1C** in Example 1.

MS (ESI, m/z): 636.2, 638.2 [M + H]⁺.

### Compound 93B:

Compound **93B** (62 mg) was prepared by replacing the starting material with compound **93A** (50 mg, 0.08 mmol) and tert-butyl 6-oxo-2-azaspiro[3.3]heptane-2-carboxylate (33 mg, 0.16 mmol) according to the method for the preparation of compound **72** from compound **71** in Example 72. MS (ESI, m/z): 831.2, 833.2 [M + H]⁺.

### Compound 93:

Compound **93** (7 mg) was prepared by replacing the starting material with compound **93B** (62 mg, 0.08 mmol) according to the method for the preparation of compound **63A-2** from compound **63A-1** in Example 63.

MS (ESI, m/z): 745.2, 747.2 [M + H]⁺.

¹H NMR (400 MHz, CDCl₃) δ 11.74 (s, 1H), 8.77 (d, *J =* 1.6 Hz, 1H), 8.69 (d, *J =* 1.6 Hz, 1H), 8.32-8.29 (m, 2H), 8.21 (dd, *J=* 9.6 Hz, 4.8 Hz, 1H), 7.65-7.58 (m, 1H), 7.39 (s, 1H), 6.52 (s, 1H), 6.14 (s, 1H), 4.49-4.22 (m, 2H), 4.01-3.95 (m, 1H), 3.85 (s, 3H), 3.63 (s, 3H), 3.27-3.05 (m, 2H), 2.99-2.89 (m, 2H), 2.79 (s, 3H), 2.49 (s, 3H), 2.45 (s, 3H), 2.17-2.08 (m, 1H), 2.01-1.95 (m, 1H), 1.29-1.19 (m, 3H), 0.91-0.80 (m, 1H).

### Example 94:

### Preparation of compound 94

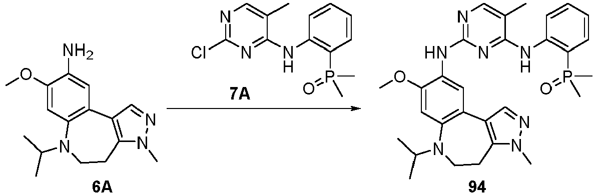

Compound **94** (20 mg) was prepared by replacing the starting material with compound **6A** (70 mg, 0.24 mmol) and compound **7A** (72 mg, 0.24 mmol) according to the method for the preparation of compound **1** from compound **1B** and compound **1C** in Example 1.

MS (ESI, m/z): 546.3 [M + H]⁺.

¹H NMR (300 MHz, CDCl₃) δ 10.94 (s, 1H), 8.66 (dd, *J=* 9.6 Hz, 4.8 Hz, 1H), 8.23 (s, 1H), 7.76 (s, 1H), 7.42 (s, 1H), 7.27-7.18 (m, 1H), 7.11-7.02 (m, 1H), 7.00-6.93 (m, 1H), 6.59 (s, 1H), 3.99-3.90 (m, 1H), 3.88 (s, 3H), 3.83 (s, 3H), 3.32 (t, *J=* 5.4 Hz, 2H), 2.96 (t, *J=* 5.4 Hz, 2H), 2.23 (s, 3H), 1.87 (s, 3H), 1.82 (s, 3H), 1.35 (s, 3H), 1.33 (s, 3H).

### Example 95:

### Preparation of compound 95

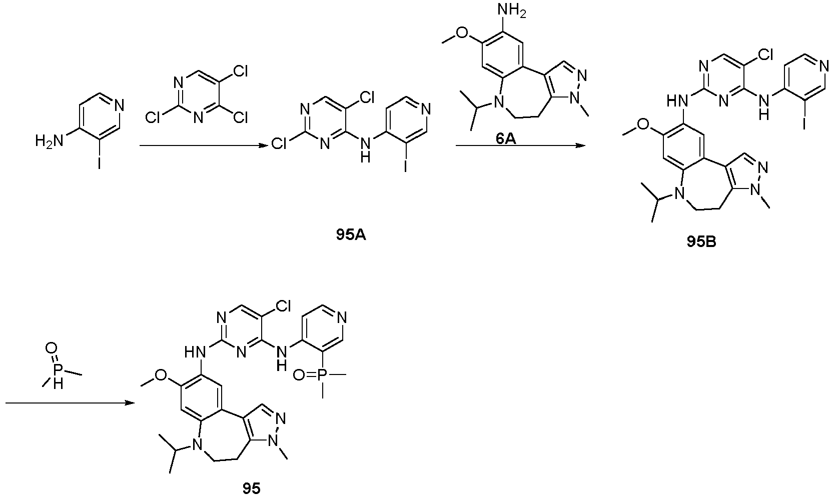

### Compound 95A:

Compound **95A** (290 mg) was prepared by replacing the starting material with 4-amino-3-iodopyridine (500 mg, 2.27 mmol) and 2,4,5-trichloropyrimidine (414 mg, 2.27 mmol) according to the method for the preparation of compound **1B** from compound **1B-2** in Example 1.

MS (ESI, m/z): 366.9, 368.9 [M + H]⁺.

### Compound 95B:

Compound **95B** (65 mg) was prepared by replacing the starting material with compound **6A** (100 mg, 0.35mmol) and compound **95A** (128 mg, 0.35 mmol) according to the method for the preparation of compound 1 from compound **1B** and compound **1C** in Example 1.

MS (ESI, m/z): 617.1, 619.1 [M + H]⁺.

### Compound 95:

Compound **95** (20 mg) was prepared by replacing the starting material with compound **95B** (60 mg, 0.1 mmol) according to the method for the preparation of compound **1B-2** from compound **1B-1** in Example 1.

MS (ESI, m/z): 567.2, 569.2 [M + H]⁺.

¹H NMR (300 MHz, CDCl₃) δ 11.43 (s, 1H), 8.77 (t, *J* = 5.4 Hz, 1H), 8.35 (d, J = 9.0 Hz, 1H), 8.28 (s, 1H), 8.20 (s, 1H), 8.06 (s, 1H), 7.55 (s, 1H), 7.21 (s, 1H), 6.60 (s, 1H), 4.00-3.91 (m, 1H), 8.89 (s, 3H), 8.84 (s, 3H), 3.33 (t, *J* = 5.4 Hz, 2H), 2.98 (t, *J* = 5.4 Hz, 2H), 1.83 (s, 3H), 1.89 (s, 3H), 1.35 (s, 3H), 1.33 (s, 3H).

### Example 96:

### Preparation of intermediate 96A

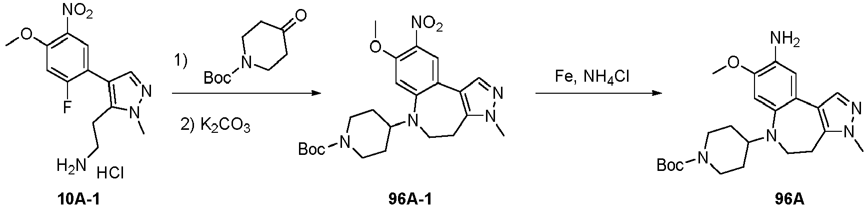

### Compound 96A-1:

Compound **96A-1** (0.9 g) was prepared by replacing the starting material with *N-tert-*butoxycarbonyl-4-piperidone (1.35 g, 6.8 mmol) according to the method for the preparation of compound **47A-1** from compound **10A-1** in Example 47.

MS (ESI, m/z): 458.3 [M + H]⁺.

### Compound 96A:

Compound **96A** (750 mg) was prepared by replacing the starting material with compound **96A-1** (900 mg, 1.97 mmol) according to the method for the preparation of compound **1C** from compound **1C-9** in Example 1.

MS (ESI, m/z): 427.1 [M + H]⁺.

### Preparation of compound 96

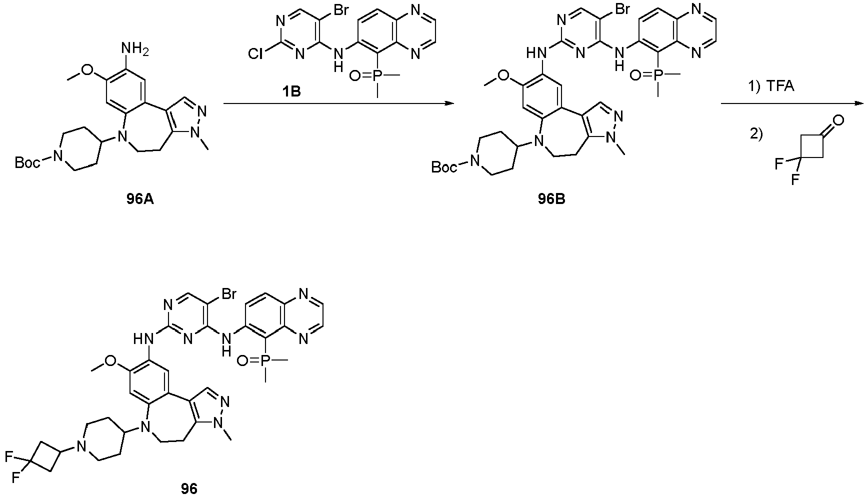

### Compound 96B:

Compound **96B** (65 mg) was prepared by replacing the starting material with compound **96A** (100 mg, 0.23 mmol) and compound **1B** (106 mg, 0.26 mmol) according to the method for the preparation of compound **81** from compound **81A** and compound **51B** in Example 81.

MS (ESI, m/z): 803.4, 805.4 [M + H]⁺.

### Compound 96:

Compound **96** (3 mg) was prepared by replacing the starting material with compound **96B** (65 mg, 0.08 mmol) and 3,3-difluorocyclobutanone (46 mg, 0.43 mmol) according to the method for the preparation of compound **63A-2** from compound **63A-1** in Example 63.

MS (ESI, m/z): 793.2, 795.2 [M + H]⁺.

¹H NMR (300 MHz, CDCl₃) δ 12.48 (s, 1H), 8.89 (dd, *J* = 9.6 Hz, 4.8 Hz, 1H), 8.73-8.68 (m, 2H), 8.40 (s, 1H), 8.30 (s, 1H), 7.70-7.60 (m, 1H), 7.37 (s, 1H), 7.00 (s, 1H), 6.54 (s, 1H), 3.88 (s, 3H), 7.72 (s, 3H), 3.49-3.39 (m, 3H), 3.12-3.01 (m, 2H), 2.97 (t, *J* = 5.4 Hz, 2H), 2.81-2.66 (m, 3H), 2.62-2.44 (m, 2H), 2.18 (s, 3H), 2.13 (s, 3H), 2.04-1.92 (m, 3H), 1.34-1.24 (m, 2H), 0.95-0.83 (m, 1H).

### Compound 97:

### Preparation of compound 97

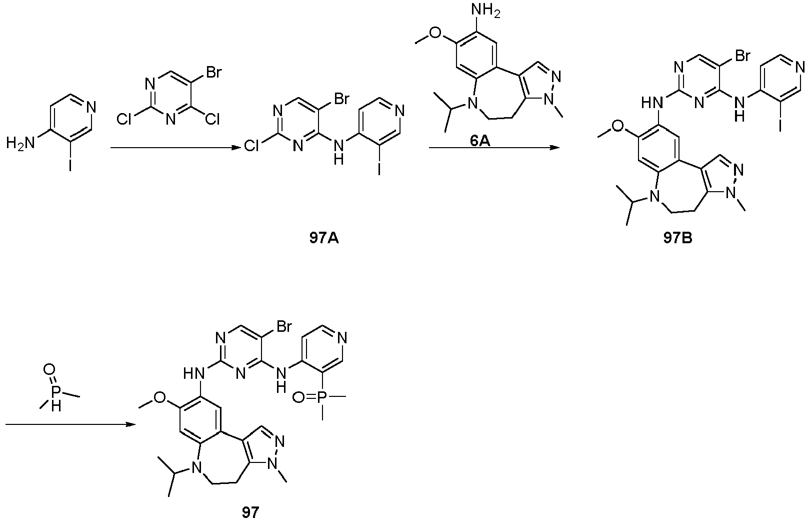

### Compound 97A:

Compound **97A** (130 mg) was prepared by replacing the starting material with 4-amino-3-iodopyridine (500 mg, 2.27 mmol) according to the method for the preparation of compound **1B** from compound **1B-2** in Example 1.

MS (ESI, m/z): 410.8, 412.8 [M + H]⁺.

### Compound 97B:

Compound **97B** (71 mg) was prepared by replacing the starting material with compound **6A** (91 mg, 0.32 mmol) and compound **97A** (130 mg, 0.32 mmol) according to the method for the preparation of compound **1** from compound **1B** and compound **1C** in Example 1.

MS (ESI, m/z): 661.0, 663.0 [M + H]⁺.

### Compound 97:

Compound **97** (23 mg) was prepared by replacing the starting material with compound **97B** (70 mg, 0.11 mmol) according to the method for the preparation of compound **1B-2** from compound **1B-1** in Example 1.

MS (ESI, m/z): 611.2, 613.2 [M + H]⁺.

¹H NMR (300 MHz, CDCl₃) δ 11.23 (s, 1H), 8.73-8.66 (m, 1H), 8.35 (d, *J =* 8.4 Hz, 1H), 8.32-8.25 (m, 2H), 8.07 (s, 1H), 7.51 (s, 1H), 7.17 (s, 1H), 6.60 (s, 1H), 4.00-3.91 (m, 1H), 3.89 (s, 3H), 3.83 (s, 3H), 3.33 (t, *J =* 5.4 Hz, 2H), 2.98 (t, *J =* 5.4 Hz, 2H), 1.93 (s, 3H), 1.89 (s, 3H), 1.35 (s, 3H), 1.32 (s, 3H).

### Example 98:

### Preparation of compound 98

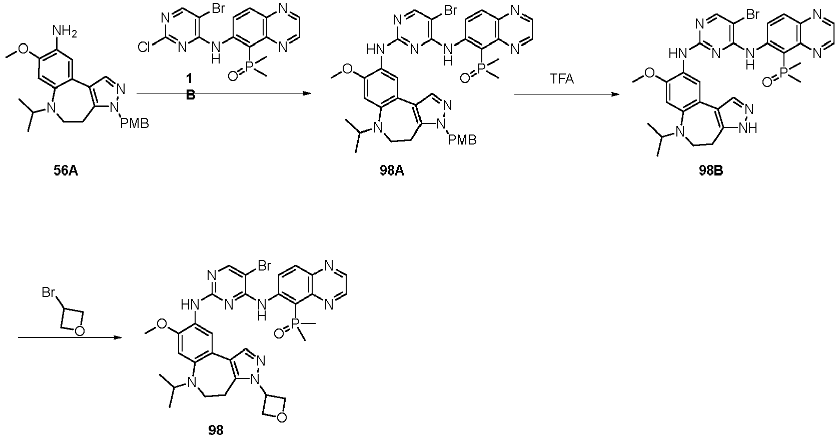

### Compound 98A:

Compound **98A** (336 mg) was prepared by replacing the starting material with compound **56A** (300 mg, 0.76 mmol) according to the method for the preparation of compound **1** from compound **1B** and compound **1C** in Example 1.

MS (ESI, m/z): 768.2, 770.2 [M + H]⁺.

### Compound 98B:

Compound **98B** (265 mg) was prepared by replacing the starting material with compound **98A** (326 mg, 0.42 mmol) according to the method for the preparation of compound **37** from compound **34** in Example 37.

MS (ESI, m/z): 648.0, 650.0 [M + H]⁺.

### Compound 98:

Compound **98** (50 mg) was prepared by replacing the starting material with compound **98B** (260 mg, 0.4 mmol) and 3-bromooxetane (165 mg, 1.2 mmol) according to the method for the preparation of compound **56** from compound **56C** in Example 56.

MS (ESI, m/z): 704.2, 706.2 [M + H]⁺.

¹H NMR (300 MHz, CDCl₃) δ 12.57 (s, 1H), 9.02 (dd, *J* = 9.3 Hz, 5.2 Hz, 1H), 8.71 (d, *J* = 2.1 Hz, 1H), 8.67 (d, *J* = 2.1 Hz, 1H), 8.46 (s, 1H), 8.29 (s, 1H), 7.69-7.59 (m, 1H), 7.36 (s, 1H), 7.18 (s, 1H), 6.58 (s, 1H), 5.41-5.29 (m, 1H), 5.14 (t, *J* = 6.6 Hz, 2H), 4.98 (t, *J* = 6.6 Hz, 2H), 3.98-3.86 (m, 4H), 3.31 (t, *J* = 5.4 Hz, 2H), 2.88 (t, *J* = 5.4 Hz, 2H), 2.19 (s, 3H), 2.14 (s, 3H), 1.33 (s, 3H), 1.31 (s, 3H).

### Compound 99:

### Preparation of intermediate 99A

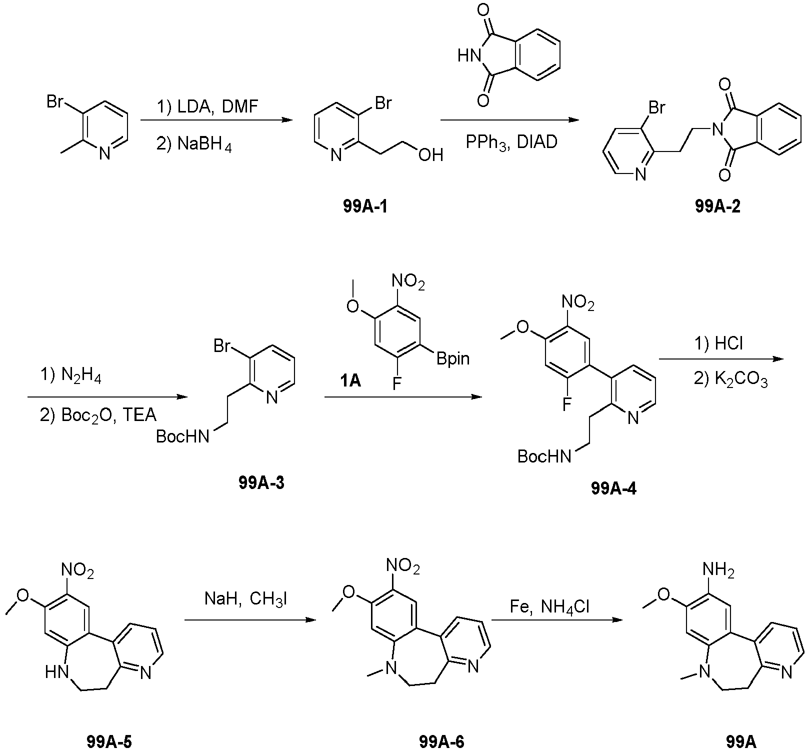

### Compound 99A-1:

2-Methyl-3-bromopyridine (5 g, 28.9 mmol) was dissolved in tetrahydrofuran (80 mL) under nitrogen atmosphere; subsequently, the reaction liquid was slowly added dropwise with a solution of lithium diisopropylamide in tetrahydrofuran (2 M, 14.5 mmol, 29 mmol) at-78 °C and successively stirred at that temperature for 1 h. The reaction liquid was added dropwise with *N,N-*dimethylformamide (2.11 g, 28.9 mmol) and successively stirred at that temperature for 30 min. After the starting material disappeared as detected by LCMS, the reaction liquid was quenched by addition of methanol (30 mL). Subsequently, the reaction liquid was added with glacial acetic acid (1.74 g, 28.9 mmol) and sodium borohydride (1.31 g, 34.68 mmol), warmed to room temperature and successively stirred for 10 min, and quenched by the addition of saturated aqueous sodium bicarbonate solution (100 mL). The mixture was extracted with ethyl acetate (100 mL × 3), and the organic phases were combined, washed with saturated brine (100 mL × 3) firstly, then dried over anhydrous sodium sulfate, filtered, and finally concentrated under reduced pressure. The resulting residue was purified by silica gel column chromatography (eluent: petroleum ether/ethyl acetate = 1/1) to obtain compound **99A-1** (4.5 g).

MS (ESI, m/z): 201.8, 203.8 [M + H]⁺.

### Compound 99A-2:

Compound **99A-2** (2.5 g) was prepared by replacing the starting material with compound **99A-1** (2 g, 9.89 mmol) according to the method for the preparation of compound **51A-1** from compound **40A-2** in Example 51.

MS (ESI, m/z): 330.9, 332.9 [M + H]⁺.

### Compound 99A-3:

Compound **99A-2** (2.5 g, 7.55 mmol) was dissolved in ethanol (30 mL), followed by addition of hydrazine hydrate (1.51 g, 30.19 mmol) to the reaction liquid. The reaction system was heated to 80 °C and successively stirred for 16 h. After the starting material disappeared as detected by LCMS, the reaction liquid was cooled to room temperature., and the precipitated solid was filtered and dried. The filter cake was dissolved in dichloromethane (30 mL), and the reaction liquid was added with triethylamine (1.81 g, 17.88 mmol) and di-tert-butyl carbonate (2.93 g, 13.43 mmol) and successively stirred at room temperature for 16 h. The reaction liquid was quenched by addition of water (80 mL). The mixture was extracted with ethyl acetate (80 mL × 3), and the organic phases were combined, washed with saturated brine (80 mL × 3) firstly, then dried over anhydrous sodium sulfate, filtered, and finally concentrated under reduced pressure. The resulting residue was purified by silica gel column chromatography (eluent: petroleum ether/ethyl acetate = 1/1) to obtain compound **99A-3** (2.5 g).

MS (ESI, m/z): 300.9, 302.9 [M + H]⁺.

### Compound 99A-4:

Compound **99A-4** (470 mg) was prepared by replacing the starting material with compound **99A-3** (800 mg, 2.65 mmol) according to the method for the preparation of compound **1C-7** from compound **1C-6** in Example 1.

MS (ESI, m/z): 392.1 [M + H]⁺.

### Compound 99A-5:

Compound **99A-5** (400 mg) was prepared by replacing the starting material with compound **99A-4** (700 mg, 1.78 mmol) according to the method for the preparation of compound **42A-7** from compound **42A-6** in Example 42.

MS (ESI, m/z): 272.2 [M + H]⁺.

### Compound 99A-6:

Compound **99A-6** (230 mg) was prepared by replacing the starting material with compound **99A-5** (400 mg, 1.66 mmol) according to the method for the preparation of compound **8A-9** from compound **8A-8** in Example 8.

MS (ESI, m/z): 286.3 [M + H]⁺.

### Intermediate 99A:

Compound **99A** (160 mg) was prepared by replacing the starting material with compound **99A-6** (230 mg, 0.81 mmol) according to the method for the preparation of compound **1C** from compound **1C-9** in Example 1.

MS (ESI, m/z): 256.3 [M + H]⁺.

### Preparation of compound 99

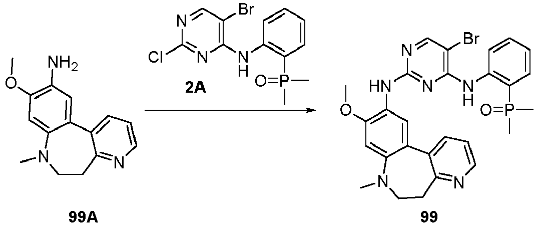

Compound **99** (20 mg) was prepared by replacing the starting material with compound **99A** (35 mg, 0.14 mmol) and compound **2A** (50 mg, 0.14 mmol) according to the method for the preparation of compound **1** from compound **1B** and compound **1C** in Example 1.

MS (ESI, m/z): 579.1, 581.1 [M + H]⁺.

¹H NMR (400 MHz, CDCl₃) δ 10.55 (s, 1H), 8.46-8.34 (m, 1H), 8.33-8.26 (m, 1H), 8.22 (s, 1H), 8.20 (s, 1H), 7.49-7.35 (m, 2H), 7.24-7.15 (m, 1H), 7.11 (dd, *J =* 6.4, 6.8 Hz, 1H), 6.87-6.76 (m, 2H), 6.57 (s, 1H), 3.96 (s, 3H), 3.59 (t, *J=* 6.4 Hz, 2H), 3.03 (t, *J=* 6.4 Hz, 2H), 2.79 (s, 3H), 1.83 (s, 3H), 1.80 (s, 3H).

### Example 100:

### Preparation of compound 100

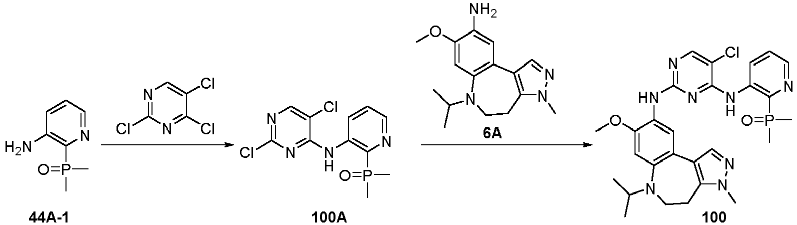

### Compound 100A:

Compound **100A** (75 mg) was prepared by replacing the starting material with compound **44A-1** (1 g, 5.88 mmol) and 2,4,5-trichloropyrimidine (2.16 g, 11.75 mmol) according to the method for the preparation of compound **2A** in Example 2.

MS (ESI, m/z): 317.2, 319.2 [M + H]⁺.

### Compound 100:

Compound **100** (20 mg) was prepared by replacing the starting material with compound **100A** (70 mg, 0.22 mmol) and compound **6A** (63 mg, 0.22 mmol) according to the method for the preparation of compound **1** from compound **1B** and compound **1C** in Example 1.

MS (ESI, m/z): 567.2, 569.2 [M + H]⁺.

¹H NMR (400 MHz, CDCl₃) δ 11.20 (s, 1H), 9.09-9.04 (m, 1H), 8.28 (s, 1H), 8.16 (d, *J* = 4.4 Hz, 1H), 8.12 (s, 1H), 7.52 (s, 1H), 7.15 (s, 1H), 6.93 (s, 1H), 6.57 (s, 1H), 3.96-3.88 (m, 1H), 3.87 (s, 3H), 3.83 (s, 3H), 3.30 (t, *J* = 5.6 Hz, 2H), 2.96 (t, *J* = 5.6 Hz, 2H), 1.88 (s, 3H), 1.85 (s, 3H), 1.32 (s, 3H), 1.31 (s, 3H).

### Example 101:

### Preparation of intermediate 101A

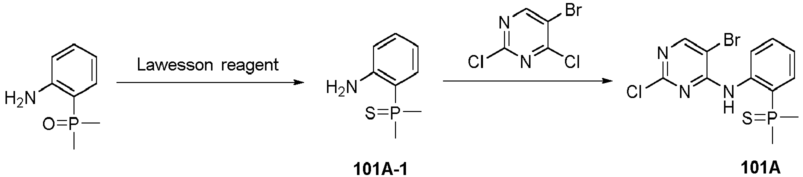

### Compound 101A-1:

Compound **101A-1** (770 mg) was prepared by replacing the starting material with 2-(dimethylphosphoryl)aniline (5 g, 29.6 mmol) according to the method for the preparation of compound **76A-1** from compound **1B-2** in Example 76.

MS (ESI, m/z): 186.2 [M + H]⁺.

### Intermediate 101A:

Compound **101A** (330 g) was prepared by replacing the starting material with compound **101A-1** (770 mg, 4.16 mmol) according to the method for the preparation of compound **2A** from 2-(dimethylphosphinoxy)aniline in Example 2.

MS (ESI, m/z): 376.0, 378.0 [M + H]⁺.

### Preparation of compound 101

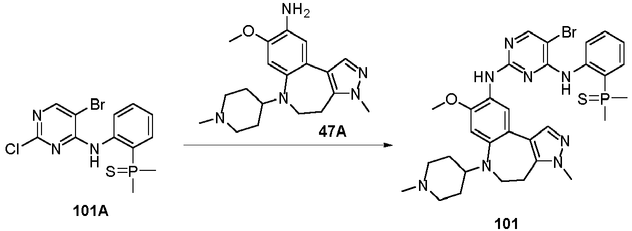

Compound 101 (88 mg) was prepared by replacing the starting material with compound **101A** (80 mg, 0.22 mmol) and compound **47A** (76 mg, 0.22 mmol) according to the method for the preparation of compound **1** from compound **1B** and compound **1C** in Example 1.

MS (ESI, m/z): 681.2, 683.2 [M + H]⁺.

¹H NMR (300 MHz, CD₃OD) δ 8.25 (s, 1H), 7.80-7.60 (m, 3H), 7.44-7.13 (m, 3H), 6.65 (s, 1H), 3.89 (s, 3H), 3.87-3.78 (m, 4H), 3.63 (d, *J* = 12.6 Hz, 2H), 3.38 (t, *J* = 5.1 Hz, 2H), 3.28-3.16 (m, 2H), 3.03 (t, *J* = 5.1 Hz, 2H), 2.92 (s, 3H), 2.24-2.13 (m, 4H), 2.11 (s, 3H), 2.06 (s, 3H).

### Example 102:

### Preparation of compound 102

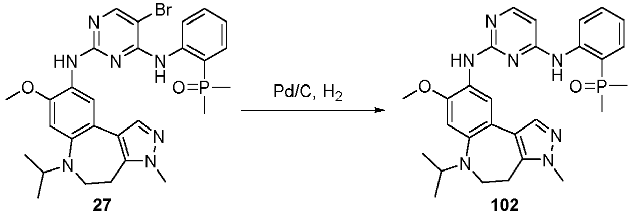

Compound **102** (30 mg) was prepared by replacing the starting material with compound **27** (120 mg, 0.2 mmol) according to the method for the preparation of compound **22** from compound **2** in Example 22.

MS (ESI, m/z): 532.3 [M + H]⁺.

¹H NMR (400 MHz, CDCl₃) δ 11.02 (s, 1H), 9.09 (s, 1H), 8.54 (dd, *J* = 8.4 Hz, 4.4 Hz, 1H), 8.15 (s, 1H), 7.88 (d, *J* = 6.0 Hz, 1H), 7.51 (s, 1H), 7.22-7.14 (m, 1H), 7.06-6.98 (m, 1H), 6.96-6.90 (m, 1H), 6.58 (s, 1H), 6.12 (d, *J* = 6.4 Hz, 1H), 3.98-3.88 (m, 1H), 3.85 (s, 3H), 3.81 (s, 3H), 3.30 (t, *J* = 5.6 Hz, 2H), 2.95 (t, *J* = 5.6 Hz, 2H), 1.83 (s, 3H), 1.80(s, 3H), 1.33 (s, 3H), 1.31 (s, 3H).

### Example 103:

### Preparation of intermediate 103A

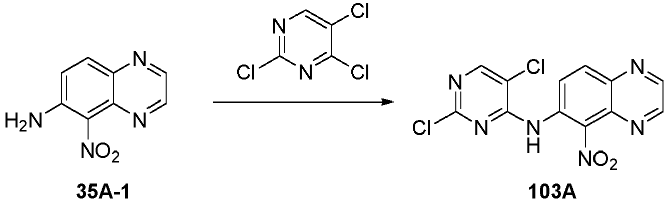

Compound **103A** (720 mg) was prepared by replacing the starting material with compound **35A-1** (700 mg, 3.68 mmol) and 2,4,5-trichloropyrimidine (1350 mg, 7.36 mmol) according to the method for the **1B** preparation of compound **1B-2** in Example 1.

MS (ESI, m/z): 337.0, 339.0 [M + H]⁺.

### Preparation of compound 103

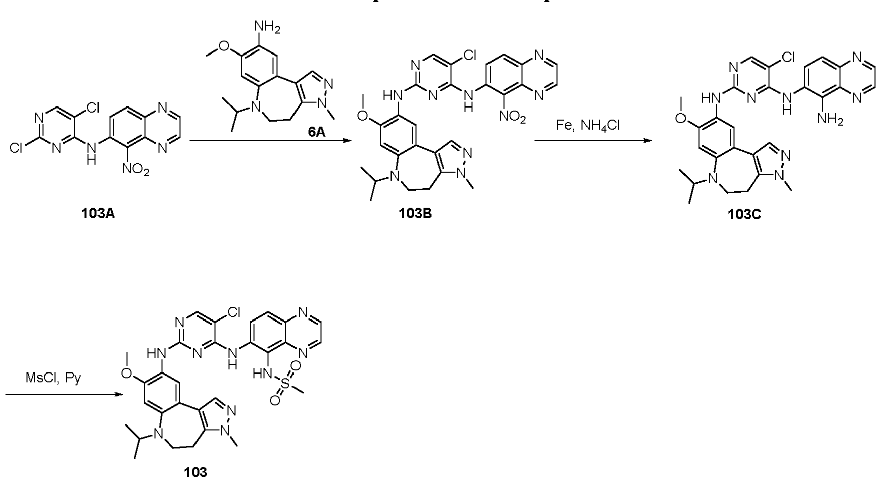

### Compound 103B:

Compound **103B** (100 mg) was prepared by replacing the starting material with compound **103A** (150 mg, 0.46 mmol) and compound **6A** (127 mg, 0.46 mmol) according to the method for the preparation of compound **1** from compound **1B** and compound **1C** in Example 1.

MS (ESI, m/z): 587.0, 589.0 [M + H]⁺.

### Compound 103C:

Compound **103C** (80 mg) was prepared by replacing the starting material with compound **103B** (100 mg, 0.17 mmol) according to the method for the preparation of compound **1C** from intermediate **1C-9** in Example 1.

MS (ESI, m/z): 557.3, 559.3 [M + H]⁺.

### Compound 103:

Compound **103** (20 mg) was prepared by replacing the starting material with compound **103C** (50 mg, 0.09 mmol) according to the method for the preparation of compound **32** from intermediate **32C** in Example 32.

MS (ESI, m/z): 635.3, 637.3 [M + H]⁺.

¹H NMR (400 MHz, CDCl₃) δ 9.16 (s, 1H), 8.81 (d, J= 2.0 Hz, 1H), 8.78 (d, J = 2.0 Hz, 1H), 8.63 (d, *J =* 9.2Hz, 1H), 8.32 (s, 1H), 8.17 (s, 1H), 7.63-7.51 (m, 2H), 7.30 (s, 1H), 6.77 (s, 1H), 6.53 (s, 1H), 3.93-3.80 (m, 4H), 3.66 (s, 3H), 3.33-3.24 (m, 2H), 2.96-2.86 (m, 5H), 1.30 (s, 3H), 1.28 (s, 3H).

### Example 104:

### Preparation of intermediate 104A

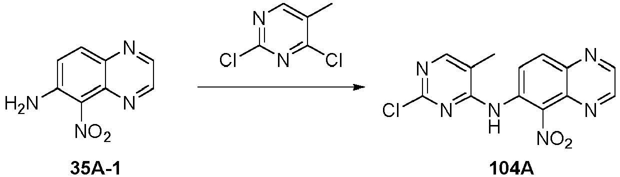

Compound **104A** (510 mg) was prepared by replacing the starting material with compound **35A-1** (500 mg, 2.63 mmol) and 2,4-dichloro-5-methylpyrimidine (857 mg, 5.26 mmol) according to the method for preparation of compound **1B** from compound **1B-2** in Example 1.

MS (ESI, m/z): 317.2, 319.2 [M + H]⁺.

### Preparation of compound 104

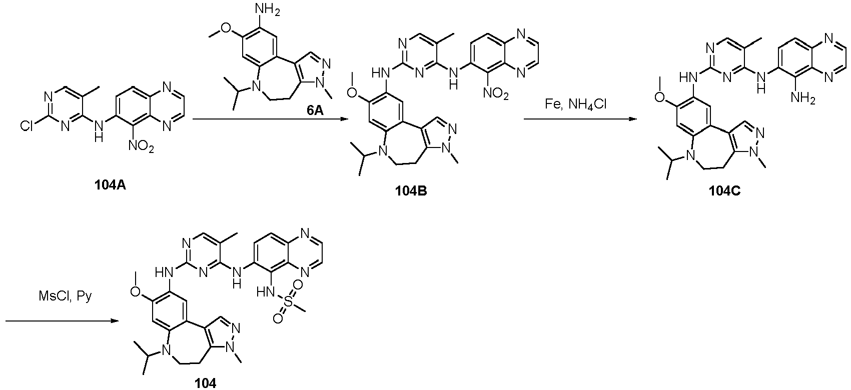

### Compound 104B:

Compound **104B** (80 mg) was prepared by replacing the starting material with compound **104A** (100 mg, 0.32 mmol) and compound **6A** (90 mg, 0.32 mmol) according to the method for the preparation of compound 1 from compound **1B** and compound **1C** in Example 1.

MS (ESI, m/z): 567.3 [M + H]⁺.

### Compound 104C:

Compound **104C** (65 mg) was prepared by replacing the starting material with compound **104B** (75 mg, 0.13 mmol) according to the method for the preparation of compound **1C** from intermediate **1C-9** in Example 1.

MS (ESI, m/z): 536.8 [M + H]⁺.

### Compound 104:

Compound **104** (20 mg) was prepared by replacing the starting material with compound **104C** (50 mg, 0.09 mmol) according to the method for the preparation of compound **32** from intermediate **32C** in Example 32.

MS (ESI, m/z): 615.4 [M + H]⁺.

¹H NMR (400 MHz, CDCl₃) δ 8.81-8.77 (m, 2H), 8.75 (d, *J =* 1.6 Hz, 1H), 8.69 (d, *J =* 9.6 Hz, 1H), 8.29 (s, 1H), 7.95 (s, 1H), 7.86 (s, 1H), 7.51 (d, *J =* 9.2 Hz, 1H), 6.84 (s, 1H), 6.53 (s, 1H), 3.92-3.84 (m, 4H), 3.66 (s, 3H), 3.29 (t, *J=* 5.6 Hz, 2H), 2.95-2.87 (m, 5H), 2.25 (s, 3H), 1.30 (s, 3H), 1.28 (s, 3H).

### Example 105:

### Preparation of compound 105

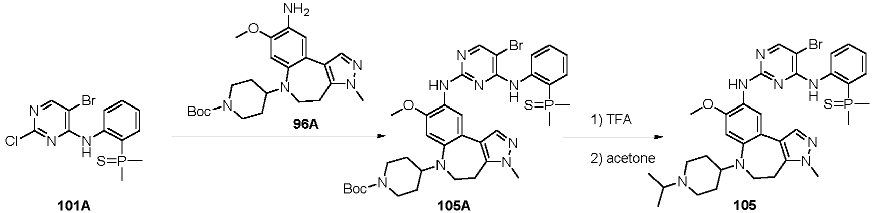

### Compound 105A:

Compound **105A** (146 mg) was prepared by replacing the starting material with compound **96A** (227 mg, 0.53 mmol) and compound **101A** (200 mg, 0.53 mmol) according to the method for the preparation of compound **81** from compound **81A** and compound **51B** in Example 81.

MS (ESI, m/z): 767.2, 769.2 [M + H]⁺.

### Compound 105:

Compound **105** (8 mg) was prepared by replacing the starting material with compound **105A** (70 mg, 0.09 mmol) according to the method for the preparation of compound **63A-2** from compound **63A-1** in Example 63.

MS (ESI, m/z): 709.3, 711.3 [M + H]⁺.

¹H NMR (400 MHz, CDCl₃) δ 9.32 (s, 1H), 8.38 (s, 1H), 8.25 (s, 1H), 7.95 (dd, *J=* 8.4 Hz, 3.2 Hz, 1H), 7.53-7.46 (m, 1H), 7.32 (s, 1H), 7.26-7.21 (m, 1H), 7.13-7.05 (m, 1H), 6.90 (s, 1H), 6.48 (s, 1H), 3.85 (s, 3H), 3.78 (s, 3H), 3.51-3.23 (m, 6H), 2.94 (t, *J =* 5.6 Hz, 2H), 2.61 (t, *J=* 12.8 Hz, 2H), 2.40-2.32 (m, 2H), 2.10 (s, 3H), 2.06 (s, 3H), 2.03-1.95 (m, 2H), 1.28 (s, 3H), 1.26 (s, 3H).

### Example 106:

### Preparation of compound 106

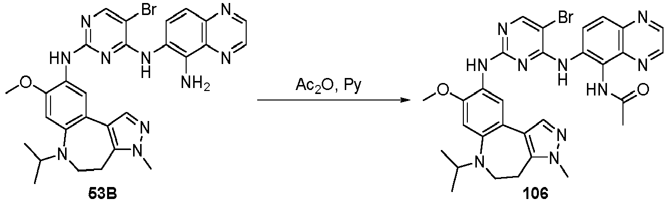

Compound **106** (63 mg) was prepared by replacing the starting material with compound **53B** (100 mg, 0.17 mmol) and acetic anhydride (34 mg, 0.33 mmol) according to the method for the preparation of compound **32** from intermediate **32C** in Example 32.

MS (ESI, m/z): 643.2, 645.2 [M + H]⁺.

¹H NMR (300 MHz, CDCl₃) δ 9.36 (s, 1H), 9.19 (s, 1H), 8.80 (d, *J =* 2.1 Hz, 1H), 8.76 (d, *J=* 2.1 Hz, 1H), 8.31 (s, 1H), 8.22 (s, 1H), 8.19 (d, *J =* 6.3 Hz, 1H), 7.50 (d, *J =* 9.6 Hz, 1H), 7.38 (s, 1H), 6.62-6.31 (m, 2H), 3.94-3.78 (m, 4H), 3.62 (s, 3H), 3.26 (t, *J =* 5.4 Hz, 2H), 2.87 (t, *J =* 5.4 Hz, 2H), 2.48 (s, 3H), 1.30 (s, 3H), 1.27 (s, 3H).

### Example 107:

### Preparation of compound 107

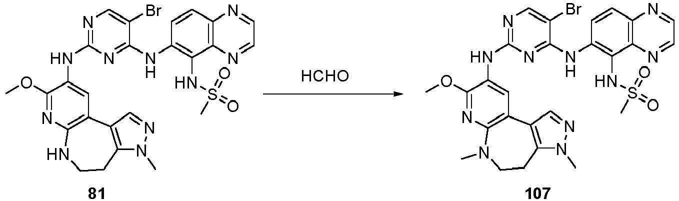

Compound **107** (10 mg) was prepared by replacing the starting material with compound **81** (40 mg, 0.06 mmol) and aqueous formaldehyde solution (40%, 10 mg, 0.13 mmol) according to the method for the preparation of compound **72** from compound **71** in Example 72.

MS (ESI, m/z): 652.1, 654.1 [M + H]⁺.

¹H NMR (300 MHz, CDCl₃) δ 9.16 (s, 1H), 8.82 (m, 2H), 8.52 (d, *J =* 9.4 Hz, 1H), 8.38 (s, 1H), 8.26 (s, 1H), 7.61 (s, 2H), 7.24 (s, 1H), 6.74 (s, 1H), 3.99 (s, 3H), 3.71 (s, 3H), 3.44-3.34 (m, 2H), 3.16 (s, 3H), 3.00 (t, *J=* 5.1 Hz, 2H), 2.95 (s, 3H).

### Example 108:

### Preparation of compound 108

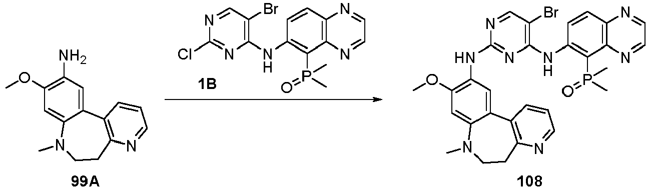

Compound **108** (40 mg) was prepared by replacing the starting material with compound **99A** (80 mg, 0.31 mmol) according to the method for the preparation of compound **1** from compound **1B** and compound **1C** in Example 1.

MS (ESI, m/z): 631.1, 633.1 [M + H]⁺.

¹H NMR (300 MHz, CDCl₃) δ 12.48 (s, 1H), 8.83 (dd, *J =* 8.4, 5.2 Hz, 1H), 8.76 (d, *J =* 2.1 Hz, 1H), 8.71 (d, *J =* 1.8 Hz, 1H), 8.31-8.27 (m, 3H), 7.70 (d, *J =* 9.3 Hz, 1H), 7.43 (s, 1H), 7.32 (s, 1H), 6.75 (s, 1H), 6.67 (s, 1H), 3.99 (s, 3H), 3.60 (t, *J=* 6.6 Hz, 2H), 3.02 (t, *J=* 6.6 Hz, 2H), 2.81 (s, 3H), 2.16 (s, 3H), 2.11 (s, 3H).

### Example 109:

### Preparation of intermediate 109A

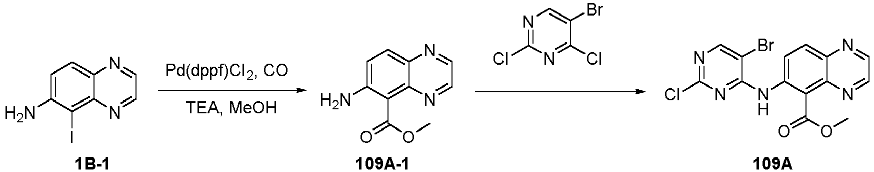

### Compound 109A-1:

Compound **1B-1** (6.9 g, 25.46 mmol) and triethylamine (12.88 g, 127.28 mmol) were dissolved in methanol (100 mL), followed by addition of [1,1'-bis(diphenylphosphino)ferrocene]dichloropalladium(II) (1.86 g, 2.55 mmol) to the reaction liquid. The reaction liquid was charged with carbon monoxide (50 atm) and heated to 70 °C and successively stirred for 16 h. After the starting material disappeared as detected by LCMS, the reaction liquid was cooled to room temperature and vented under reduced pressure, then concentrated under reduced pressure. The resulting residue was purified by silica gel column chromatography (eluent: petroleum ether/ethyl acetate = 1/10) to obtain compound **109A-1** (1.5 g). MS (ESI) M/Z: 204.3 [M + H]⁺.

### Intermediate 109A:

Compound **109A** (0.5 g) was prepared by replacing the starting material with compound **109A-1** (1.5 g, 7.38 mmol) according to the method for the preparation of compound **1B** from compound **1B-2** in Example 1.

MS (ESI, m/z): 394.0, 396.0 [M + H]⁺.

### Preparation of compound 109

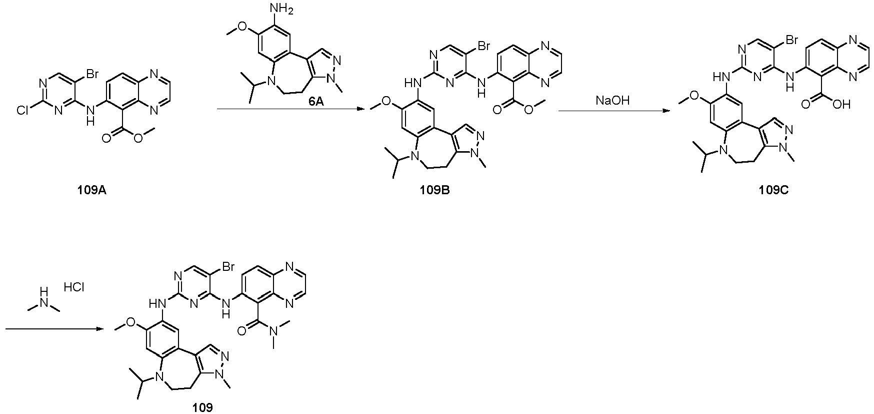

### Compound 109B:

Compound **109B** (400 mg) was prepared by replacing the starting material with compound **109A** (350 mg, 0.89 mmol) and compound **6A** (254 mg, 0.89 mmol) according to the method for the preparation of compound **81** from compound **81A** and compound **51B** in Example 81.

MS (ESI, m/z): 644.2, 646.2 [M + H]⁺.

### Compound 109C:

Compound **109C** (150 mg) was prepared by replacing the starting material with compound **109B** (400 mg, 0.62 mmol) according to the method for the preparation of compound **1C-3** from compound **1C-2** in Example 1.

MS (ESI, m/z): 630.1, 632.1 [M + H]⁺.

### Compound 109:

Compound **109** (7 mg) was prepared by replacing the starting material with compound **109C** (50 mg, 0.08 mmol) according to the method for the preparation of compound **48A-5** from compound **48A-4** in Example 48.

MS (ESI, m/z): 657.2, 659.2 [M + H]⁺.

¹H NMR (300 MHz, CDCl₃) δ 8.84 (d, *J* = 1.8 Hz, 1H), 8.74 (d, *J* = 1.8 Hz, 1H), 8.65 (d, *J* = 9.3 Hz, 1H), 8.50 (s, 1H), 8.42 (s, 1H), 8.25 (s, 1H), 7.65 (s, 1H), 7.35 (s, 1H), 6.90 (s, 1H), 6.58 (s, 1H), 3.99-3.91 (m, 4H), 3.68 (s, 3H), 3.53-3.48 (m, 1H), 3.35 (s, 3H), 3.18-3.11 (m, 1H), 3.04-2.99 (m, 1H), 2.93 (s, 3H), 2.89-2.83 (m, 1H), 1.36-1.29 (m, 6H).

### Example 110:

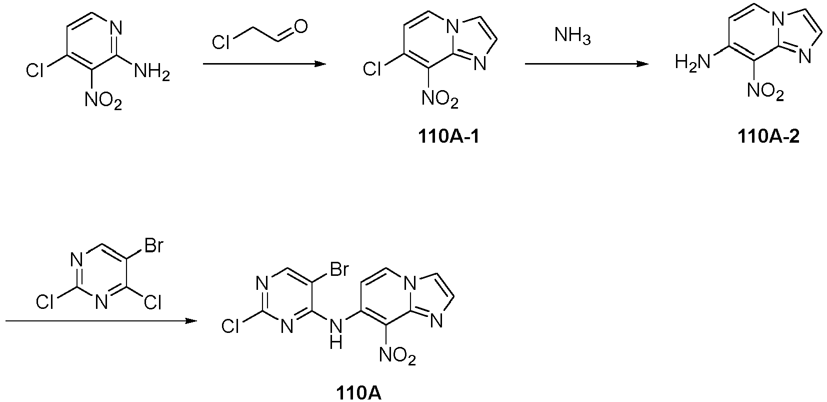

### Compound 110A-1:

2-Amino-3-nitro-4-chloropyridine (3 g, 17.29 mmol) and an aqueous chloroacetaldehyde solution (40%, 5.09 g, 25.928 mmol) were dissolved in 1,4-dioxane (10 mL), and the reaction liquid was successively stirred at room temperature for 2 h. After the starting material disappeared as detected by LCMS, the precipitated solid was filtered and the filter cake was washed with diethyl ether (5 mL × 3). The filter cake was dried to obtain compound **110A-1** (2.8 g).

MS (ESI, m/z): 198.3, 200.3 [M + H]⁺.

### Compound 110A-2:

Compound **110A-1** (800 mg, 4.05 mmol) was dissolved in methanolic ammonia (7 M, 20 mL), and the reaction liquid was successively stirred at room temperature for 2 h. After the starting material disappeared as detected by LCMS, the reaction liquid was concentrated under reduced pressure. The resulting residue was purified by silica gel column chromatography (eluent: dichloromethane/methanol = 10/1) to obtain compound **110A-2** (100 mg).

MS (ESI, m/z): 179.2 [M + H]⁺.

### Intermediate 110A:

Compound **110A** (480 mg) was prepared by replacing the starting material with compound **110A-2** (500 mg, 2.81 mmol) according to the method for the preparation of compound **1B** from compound **1B-2** in Example 1.

MS (ESI, m/z): 368.9, 370.9 [M + H]⁺.

### Preparation of compound 110

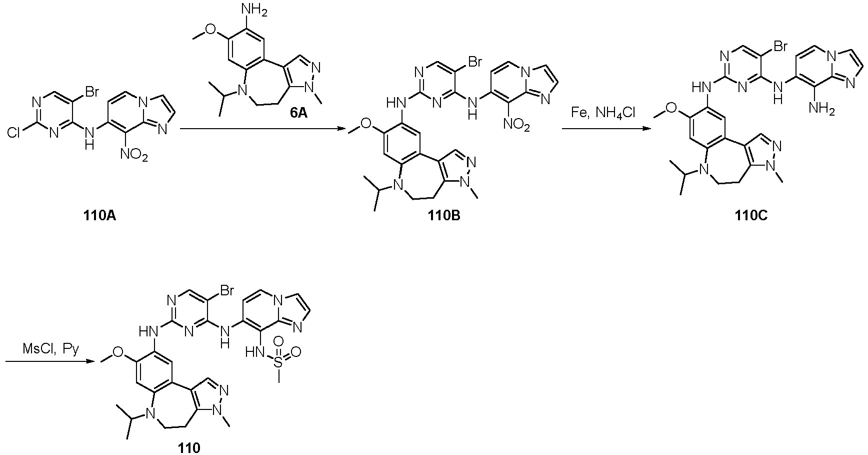

### Compound 110B:

Compound **110B** (160 mg) was prepared by replacing the starting material with compound **110A** (290 mg, 0.79 mmol) and compound **6A** (150 mg, 0.52 mmol) according to the method for the preparation of compound **1** from compound **1B** and compound **1C** in Example 1.

MS (ESI, m/z): 619.1, 621.1 [M + H]⁺.

### Compound 110C:

Compound **110C** (130 mg) was prepared by replacing the starting material with compound **110B** (150 mg, 0.24 mmol) according to the method for the preparation of compound **1C** from intermediate **1C-9** in Example 1.

MS (ESI, m/z): 589.3, 591.3 [M + H]⁺.

### Compound 110:

Compound **110** (16 mg) was prepared by replacing the starting material with compound **110C** (150 mg, 0.25 mmol) according to the method for the preparation of compound **32** from intermediate **32C** in Example 32.

MS (ESI, m/z): 667.2, 669.2 [M + H]⁺.

¹H NMR (300 MHz, CDCl₃) δ 8.90 (s, 1H), 8.31 (s, 1H), 8.23 (s, 1H), 8.03 (d, *J =* 5.4 Hz, 1H), 7.52 (s, 2H), 7.30 (s, 2H), 7.23 (s, 1H), 6.55 (s, 1H), 3.86 (s, 4H), 3.78 (s, 3H), 3.32-3.23 (m, 2H), 3.17 (s, 3H), 2.97-2.90 (m, 2H), 1.31 (s, 3H), 1.30 (s, 3H).

### Example 111:

### Preparation of intermediate 111A

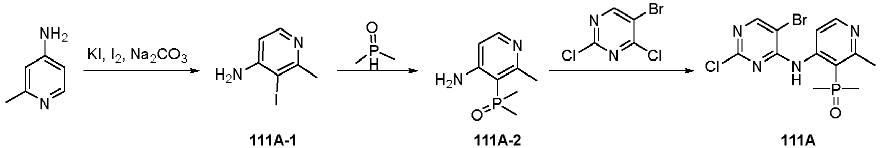

### Compound 111A-1:

2-Methyl-4-aminopyridine (6 g, 55.48 mmol) and sodium carbonate (4.12 g, 38.84 mmol) were dissolved in water (30 mL), followed by the addition of potassium iodide (11.97 g, 72.13 mmol) and iodine (11.27 g, 44.39 mmol) to the reaction liquid in batches. The reaction system was heated to 100 °C and successively stirred for 5 h. After the starting material disappeared as detected by LCMS, the reaction liquid was cooled to room temperature. The mixture was extracted with ethyl acetate (50 mL × 3), and the organic phases were combined, washed with saturated aqueous sodium thiosulfate solution (50 mL × 3) firstly, then dried over anhydrous sodium sulfate, filtered, and finally concentrated under reduced pressure. The resulting residue was purified by silica gel column chromatography (eluent: petroleum ether/ethyl acetate = 1/1) to obtain compound **111A-1** (0.83 g). MS (ESI, m/z): 235.0 [M + H]⁺.

### Compound 111A-2:

Compound **111A-2** (238 mg) was prepared by replacing the starting material with compound **111A-1** (400 mg, 1.71 mmol) according to the method for the preparation of compound **1B-2** from compound **1B-1** in Example 1.

MS (ESI, m/z): 185.3 [M + H]⁺.

### Intermediate 111A:

Compound **111A** (27 mg) was prepared by replacing the starting material with compound **111A-2** (238 mg, 1.29 mmol) according to the method for the preparation of compound **1B** from compound **1B-2** in Example 1.

MS (ESI, m/z): 375.0, 377.0 [M + H]⁺.

### Preparation of compound 111

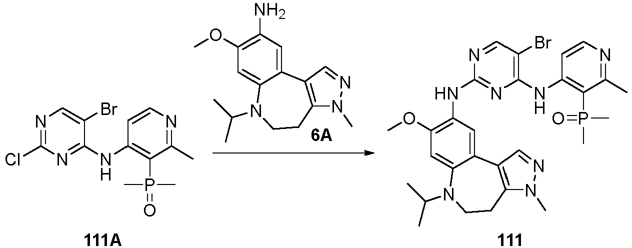

Compound **111** (6 mg) was prepared by replacing the starting material with compound **111A** (22 mg, 0.06 mmol) and compound **6A** (17 mg, 0.06 mmol) according to the method for the preparation of compound **1** from compound **1B** and compound **1C** in Example 1.

MS (ESI, m/z): 625.2, 627.2 [M + H]⁺.

¹H NMR (400 MHz, CDCl₃) δ 12.33 (s, 1H), 8.51 (dd, *J=* 6.0, 3.2 Hz, 1H), 8.27 (s, 2H), 7.94 (s, 1H), 7.44 (s, 1H), 7.11 (s, 1H), 6.56 (s, 1H), 3.93-3.86 (m, 1H), 3.86 (s, 3H), 3.81 (s, 3H), 3.29 (t, *J* = 5.2 Hz, 2H), 2.95 (t, *J =* 5.6 Hz, 2H), 2.59 (s, 3H), 2.01 (d, *J =* 13.2 Hz, 6H), 1.31 (d, *J =* 6.4 Hz, 6H).

### Example 112:

### Preparation of intermediate 112A

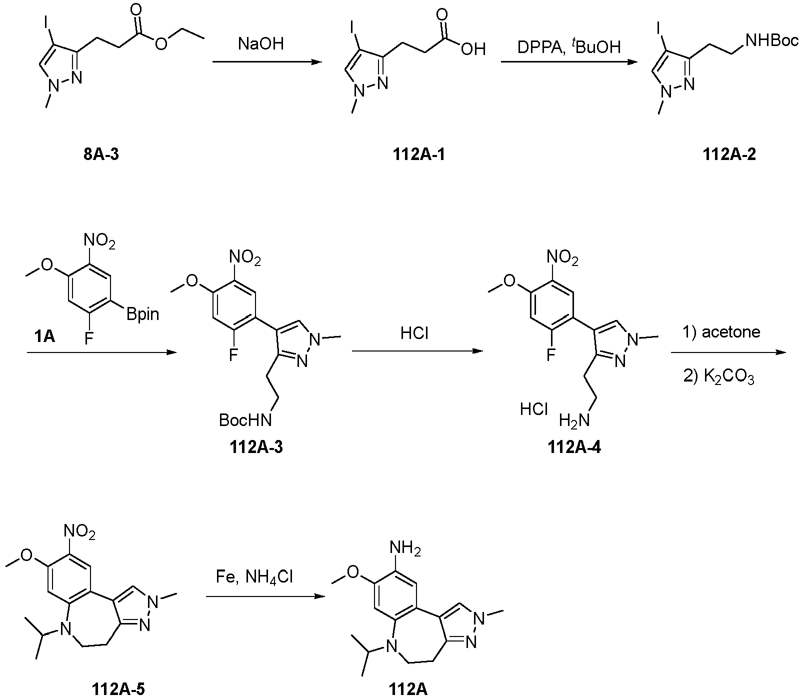

### Compound 112A-1:

Compound **112A-1** (1.19 g) was prepared by replacing the starting material with compound **8A-3** (2.05 g, 6.65 mmol) according to the method for the preparation of compound **1C-3** from compound **1C-2** in Example 1.

MS (ESI, m/z): 280.9 [M + H]⁺.

### Compound 112A-2:

Compound **112A-2** (952 mg) was prepared by replacing the starting material with compound **112A-1** (1.2 g, 4.29 mmol) according to the method for the preparation of compound **1C-4** from compound **1C-3** in Example 1.

MS (ESI, m/z): 352.1 [M + H]⁺.

### Compound 112A-3:

Compound **112A-3** (885 mg) was prepared by replacing the starting material with compound **112A-2** (930 mg, 2.65 mmol) according to the method for the preparation of compound **1C-7** from compound **1C-6** in Example 1.

MS (ESI, m/z): 395.2 [M + H]⁺.

### Compound 112A-4:

Compound **112A-4** (645 mg) was prepared by replacing the starting material with compound **112A-3** (885 mg, 2.24 mmol) according to the method for the preparation of compound **1C-8** from compound **1C-7** in Example 1.

MS (ESI, m/z): 295.1 [M + H]⁺.

### Compound 112A-5:

Compound **112A-5** (260 mg) was prepared by replacing the starting material with compound **112A-4** (330 mg, 1.12 mmol) according to the method for the preparation of compound **56A-4** from compound **56A-3** in Example 56.

MS (ESI, m/z): 317.3 [M + H]⁺.

### Intermediate 112A:

Compound **112A** (160 mg) was prepared by replacing the starting material with compound **112A-5** (260 mg, 0.82 mmol) according to the method for the preparation of compound **1C** from intermediate **1C-9** in Example 1.

MS (ESI, m/z): 287.3 [M + H]⁺.

### Preparation of compound 112

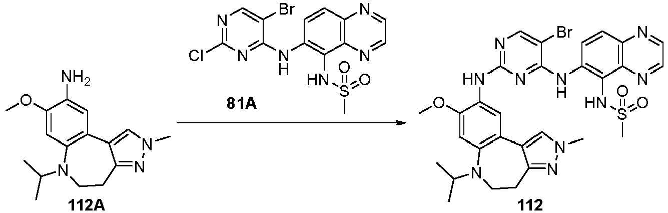

Compound **112** (36 mg) was prepared by replacing the starting material with compound **112A** (57 mg, 0.2 mmol) and compound **81A** (86 mg, 0.2 mmol) according to the method for the preparation of compound **1** from compound **1B** and compound **1C** in Example 1.

MS (ESI, m/z): 679.2, 681.2 [M + H]⁺.

¹H NMR (400 MHz, CDCl₃) δ 9.08 (s, 1H), 8.83 (d, *J=* 2.0 Hz, 1H), 8.79 (d, *J=* 2.0 Hz,1H), 8.73 (d, *J=* 9.6 Hz, 1H), 8.26 (s, 1H), 8.12 (s, 1H), 7.72 (s, 1H), 7.52 (s, 1H), 7.23 (s, 1H), 7.04 (s, 1H), 6.53 (s, 1H), 3.86 (s, 4H), 3.49 (s, 3H), 3.33-3.27 (m, 2H), 3.10-3.02 (s, 2H), 2.93 (s, 3H), 1.30 (d, *J* = 6.8 Hz, 6H).

### Example 113:

### Preparation of compound 113

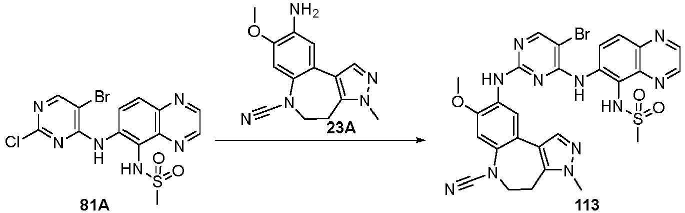

Compound **113** (13 mg) was prepared by replacing the starting material with compound **81A** (80 mg, 0.19 mmol) and compound **23A** (50 mg, 0.19 mmol) according to the method for the preparation of compound **1** from compound **1B** and compound **1C** in Example 1.

MS (ESI, m/z): 662.1, 664.1 [M + H]⁺.

¹H NMR (300 MHz, CDCl₃) δ 9.21 (s, 1H), 8.89-8.83 (m, 2H), 8.49-8.46 (m, 2H), 8.32 (s, 1H), 7.66-7.62 (m, 3H), 6.88 (s, 1H), 6.61 (s, 1H), 3.93 (s, 3H), 3.82 (t, *J =* 5.4 Hz, 2H), 3.68 (s, 3H), 3.18 (t, *J=* 5.7 Hz, 2H), 2.98 (s, 3H).

### Example 114:

### Preparation of compound 114

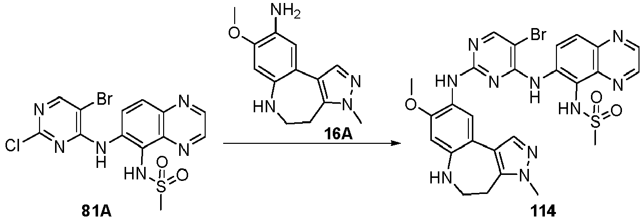

Compound **114** (3 mg) was prepared by replacing the starting material with compound **81A** (123 mg, 0.29 mmol) and compound **16A** (70 mg, 0.29 mmol) according to the method for the preparation of compound **1** from compound **1B** and compound **1C** in Example 1.

MS (ESI, m/z): 637.2, 639.2 [M + H]⁺.

¹H NMR (400 MHz, CDCl₃) δ 9.10 (s, 1H), 8.82-8.76 (m, 2H), 8.55 (d, *J=* 9.2 Hz, 1H), 8.23-8.17 (m, 2H), 7.71 (s, 1H), 7.57 (s, 1H), 7.37 (s, 1H), 6.67 (s, 1H), 6.25 (s, 1H), 3.82 (s, 3H), 3.64 (s, 3H), 3.46-3.37 (m, 2H), 2.98-2.94 (m, 5H).

### Example 115:

### Preparation of compound 115

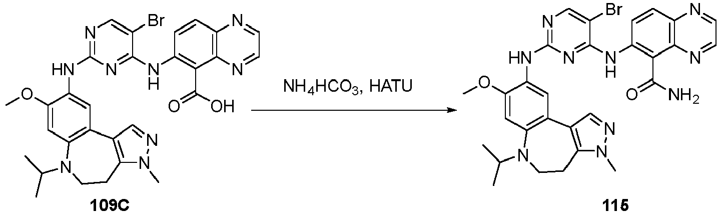

Compound **115** (5 mg) was prepared by replacing the starting material with compound **109C** (110 mg, 0.17 mmol) and ammonium bicarbonate (28 mg, 0.35 mmol) according to the method for the preparation of compound **48A-5** from compound **48A-4** in Example 48.

MS (ESI, m/z): 629.1, 631.1 [M + H]⁺.

¹H NMR (300 MHz, CDCl₃) δ 13.45 (s, 1H), 10.97 (s, 1H), 9.24 (d, *J* = 9.6 Hz, 1H), 8.78-8.76 (m, 2H), 8.41 (s, 1H), 8.33 (s, 1H), 7.69-7.65 (m, 1H), 7.36 (s, 1H), 7.12 (s, 1H), 6.60 (s, 1H), 6.17 (s, 1H), 3.91 (s, 4H), 3.74 (s, 3H), 3.36-3.30 (m, 2H), 3.02-2.94 (m, 2H), 1.35 (s, 3H), 1.33 (s, 3H).

### Example 116:

### Preparation of intermediate 116A

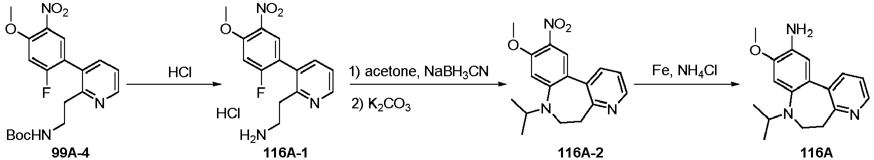

### Compound 116A-1:

Compound **116A-1** (750 mg) was prepared by replacing the starting material with compound **99A-4** (1 g, 2.56 mmol) according to the method for the preparation of compound **1C-8** from compound **1C-7** in Example 1.

MS (ESI, m/z): 291.1 [M + H]⁺.

### Compound 116A-2:

Compound **116A-2** (370 mg) was prepared by replacing the starting material with compound **116A-1** (500 mg, 1.53 mmol) according to the method for the preparation of compound **56A-4** from compound **56A-3** in Example 56.

MS (ESI, m/z): 314.3 [M + H]⁺.

### Intermediate 116A:

Compound **116A** (55 mg) was prepared by replacing the starting material with compound **116A-2** (70 mg, 0.22 mmol) according to the method for the preparation of compound **1C** from compound **1C-9** in Example 1.

MS (ESI, m/z): 284.1 [M + H]⁺.

### Preparation of compound 116

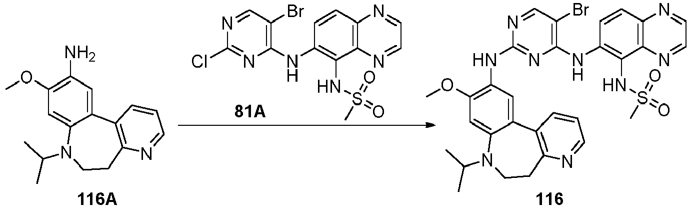

Compound **116** (10 mg) was prepared by replacing the starting material with compound **116A** (50 mg, 0.18 mmol) and compound **81A** (76 mg, 0.18 mmol) according to the method for the preparation of compound **1** from compound **1B** and compound **1C** in Example 1.

MS (ESI, m/z): 676.3, 678.3 [M + H]⁺.

¹H NMR (300 MHz, CDCl₃) δ 9.13 (s, 1H), 8.89 (d, *J* = 2.1 Hz, 1H), 8.81 (d, *J* = 2.1 Hz, 1H), 8.58 (d, *J* = 9.3 Hz, 1H), 8.29 (s, 1H), 8.25 (d, *J* = 4.8 Hz, 1H), 8.09 (s, 1H), 7.57 (d, *J* = 10.5 Hz, 1H), 7.48 (s, 1H), 7.43 (s, 1H), 7.24 (d, *J* = 8.1 Hz, 1H), 6.76 (s, 2H), 3.94 (s, 3H), 3.66-3.53 (m, 3H), 3.04 (t, *J* = 6.6 Hz, 2H), 2.93 (s, 3H), 1.15 (s, 3H), 1.13 (s, 3H).

### Example 117:

### Preparation of compounds 117A and 117B

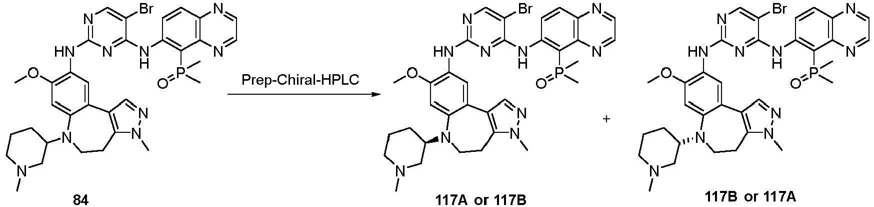

Compound **84** (60 mg, 0.25 mmol) was prepared by a chiral column, wherein preparation conditions were as follows: CHIRAL ART Cellulose-SB, 2 × 25 cm, 5 µm; mobile phase A: *n-*hexane (10 mM a solution of ammonia in methanol), and mobile phase B: ethanol; flow rate: 20 mL/min; elution: mobile phase B elevating 20% in 16 min; detection wavelength: 262 nm; RT₁: 10.7 min; RT₂: 13.2 min. The product was collected and lyophilized under reduced pressure to obtain compound **117A** (18 mg, 100% ee) with a retention time of 10.7 min and compound **117B** (18 mg, 98.4% ee) with a retention time of 13.2 min.

**117A:** MS (ESI, m/z): 717.2, 719.2 [M + H]⁺.

**117B:** MS (ESI, m/z): 717.2, 719.2 [M + H]⁺.

**117A:** ¹H NMR (300 MHz, CDCl₃) δ 12.49 (s, 1H), 8.88 (dd, *J* = 9.0 Hz, 4.8 Hz, 1H), 8.72 (d, *J* = 1.8 Hz, 1H), 8.69 (d, *J* = 1.8 Hz, 1H), 8.44 (s, 1H), 8.31 (s, 1H), 7.66 (d, *J* = 9.9 Hz, 1H), 7.38 (s, 1H), 6.99 (s, 1H), 6.67 (s, 1H), 4.04-3.88 (m, 4H), 3.73 (s, 3H), 3.50-3.38 (m, 3H), 3.31-3.23 (m, 1H), 3.02 (t, *J* = 5.4 Hz, 2H), 2.59 (s, 3H), 2.37-2.27 (m, 2H), 2.18 (s, 3H), 2.14 (s, 3H), 2.09-2.00 (m, 2H), 1.95-1.85 (m, 1H), 1.61-1.44 (m, 1H).

117B:¹H NMR (300 MHz, CDCl₃) δ 12.52 (s, 1H), 8.89 (dd, *J* = 9.0 Hz, 4.8 Hz, 1H), 8.73 (d, *J* = 1.8 Hz, 1H), 8.70 (d, *J* = 1.8 Hz, 1H), 8.42 (s, 1H), 8.31 (s, 1H), 7.64 (d, *J* = 9.9 Hz, 1H), 7.36 (s, 1H), 6.98 (s, 1H), 6.69 (s, 1H), 4.00-3.86 (m, 4H), 3.73 (s, 3H), 3.51-3.39 (m, 3H), 3.30-3.21 (m, 1H), 3.04 (t, *J* = 5.4 Hz, 2H), 2.56 (s, 3H), 2.37-2.25 (m, 2H), 2.16 (s, 3H), 2.14 (s, 3H), 2.09-2.02 (m, 2H), 1.92-1.83 (m, 1H), 1.60-1.43 (m, 1H).

### Example 118:

### Preparation of intermediate 118A

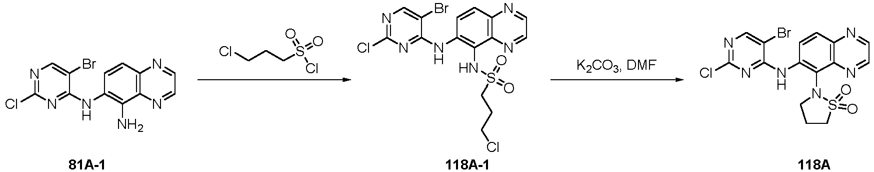

### Compound 118A-1:

Compound **81A-1** (600 mg, 1.71 mmol) was dissolved in pyridine (25 mL). Subsequently, 3-chloropropylsulfonyl chloride (453 mg, 2.56 mmol) was added to the reaction liquid at 0 °C. The reaction system was heated to 40 °C and successively stirred for 16 h. After the starting material disappeared as detected by LCMS, the reaction liquid was cooled to room temperature and distilled under reduced pressure. The resulting residue was purified by silica gel column chromatography (eluent: dichloromethane/methanol = 10/1) to obtain compound **118A-1** (456 mg).

MS (ESI, m/z): 490.9, 492.9, 494.9 [M + H]⁺.

### Compound 118A:

Compound **118A-1** (200 mg, 0.41 mmol) and anhydrous potassium carbonate (112 mg, 0.81 mmol) were dissolved in *N*,*N*-dimethylformamide (3 mL). The reaction system was heated to 50 °C and successively stirred for 1 h. After the starting material disappeared as detected by LCMS, the reaction liquid was cooled to room temperature and quenched by addition of water (50 mL). The mixture was extracted with ethyl acetate (50 mL × 3), the organic phases were combined, washed with saturated brine (50 mL × 3) firstly, then dried over anhydrous sodium sulfate, filtered, and finally concentrated under reduced pressure, and the resulting residue was purified by silica gel column chromatography (eluent: dichloromethane/methanol = 10/1) to obtain compound **118A** (150 mg).

MS (ESI) M/Z: 454.9, 456.9 [M + H]⁺.

### Preparation of compound 118

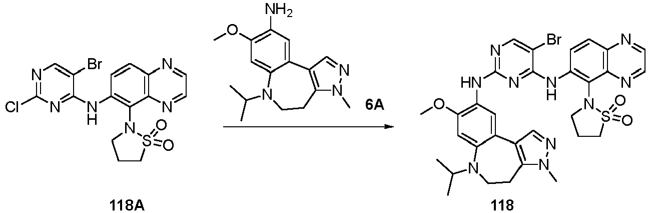

Compound **118** (25 mg) was prepared by replacing the starting material with compound **118A** (42 mg, 0.09 mmol) and compound **6A** (32 mg, 0.11 mmol) according to the method for the preparation of compound **1** from compound **1B** and compound **1C** in Example 1.

MS (ESI, m/z): 705.2, 707.2 [M + H]⁺.

¹H NMR (300 MHz, CDCl₃) δ 8.94 (s, 1H), 8.90-8.79 (m, 2H), 8.73 (d, *J* = 1.8 Hz, 1H), 8.28 (s, 2H), 7.63 (s, 1H), 7.41-7.34 (m, 1H), 7.11 (s, 1H), 6.59 (s, 1H), 4.39-4.27 (m, 1H), 3.90 (s, 6H), 3.74 (s, 3H), 3.55-3.46 (m, 2H), 3.22-3.93 (m, 3H), 2.81-2.64 (m, 2H), 1.33 (s, 6H).

### Example 119:

### Preparation of intermediate 119A

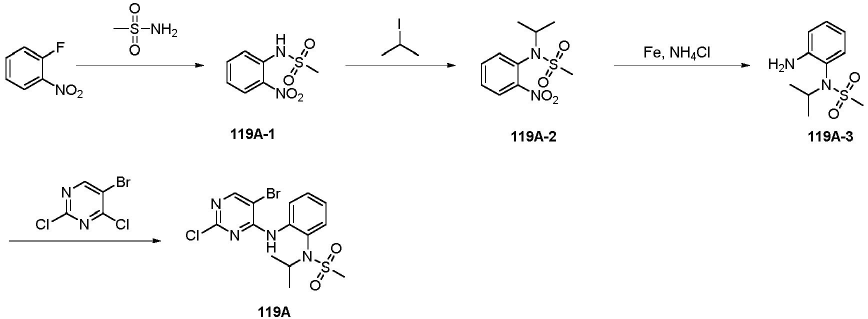

### Compound 119A-1:

o-nitrofluorobenzene (4 g, 28.35 mmol) and methylsulfonamide (4.04 g, 42.52 mmol) were dissolved in N,N-dimethylformamide (20 mL) at room temperature under nitrogen atmosphere. Subsequently, potassium *tert*-butoxide (6.36 g, 56.7 mmol) was added in batches to the reaction liquid, and the reaction system was heated to 80 °C and successively stirred for 16 h. After the starting material disappeared as detected by LCMS, the reaction liquid was cooled to room temperature and quenched by addition of water (100 mL). The mixture was extracted with ethyl acetate (100 mL × 3), and the organic phases were combined, washed with saturated brine (100 mL × 3) firstly, then dried over anhydrous sodium sulfate, filtered, and finally concentrated under reduced pressure; the resulting residue was purified by silica gel column chromatography (eluent: petroleum ether/ethyl acetate = 1/1) to obtain compound **119A-1** (3 g).

MS (ESI) M/Z: 215.1 [M - H]⁺.

¹H NMR (300 MHz, CDCl₃) δ 9.79 (s, 1H), 8.29 (dd, *J* = 8.4, 1.5 Hz, 1H), 7.91 (dd, *J* = 8.4, 1.5 Hz, 1H), 7.74-7.68 (m, 1H), 7.29-7.23 (m, 1H), 3.18 (s, 3H).

### Compound 119A-2:

Compound **119A-1** (3 g, 13.88 mmol) and 2-iodopropane (7.08 g, 41.63 mmol) were dissolved in N,N-dimethylformamide (20 mL). Subsequently, cesium carbonate (13.56 g, 41.63 mmol) was added to the reaction liquid, and the reaction system was heated to 80 °C and successively stirred for 5 h. After the starting material disappeared as detected by LCMS, the reaction liquid was cooled to room temperature and quenched by addition of water (100 mL). The mixture was extracted with ethyl acetate (100 mL × 3), and the organic phases were combined, washed with saturated brine (100 mL × 3) firstly, then dried over anhydrous sodium sulfate, filtered, and finally concentrated under reduced pressure; the resulting residue was purified by silica gel column chromatography (eluent: petroleum ether/ethyl acetate = 1/1) to obtain compound **119A-2** (2.5 g).

¹H NMR (300 MHz, CDCl₃) δ 7.90 (dd, *J* = 7.8, 1.8 Hz, 1H), 7.65 (dd, *J* = 7.8, 1.8 Hz, 1H), 7.58 (dd, *J* = 7.8, 1.8 Hz, 1H), 7.51 (dd, *J=* 7.8, 1.5 Hz, 1H), 4.36-4.27 (m, 1H), 3.15 (s, 3H), 1.32 (d, *J* = 6.6 Hz, 3H), 1.09 (d, *J* = 6.6 Hz, 3H).

### Compound 119A-3:

Compound **119A-3** (1.85 g) was prepared by replacing the starting material with compound **119A-2** (2.5 g, 8.96 mmol) according to the method for the preparation of compound 1C from intermediate **1C-9** in Example 1.

MS (ESI, m/z): 229.2 [M + H]⁺.

### Intermediate 119A:

Compound **119A** (200 mg) was prepared by replacing the starting material with compound **119A-3** (1.5 g, 6.57 mmol) according to the method for the preparation of compound **1B** from compound **1B-2** in Example 1.

MS (ESI, m/z): 418.9, 420.9 [M + H]⁺.

### Preparation of compound 119

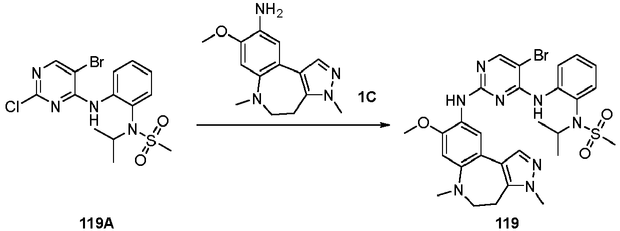

Compound **119** (5 mg) was prepared by replacing the starting material with compound **119A** (40 mg, 0.095 mmol) according to the method for the preparation of compound **1** from compound **1B** and compound **1C** in Example 1.

MS (ESI, m/z): 641.2, 643.2 [M + H]⁺.

¹H NMR (400 MHz, CDCl₃) δ 8.38 (s, 1H), 8.34-8.31 (m, 2H), 8.15 (s, 1H), 7.68-7.44 (m, 2H), 7.27 (s, 1H), 7.10-7.02 (m, 2H), 6.56 (s, 1H), 4.69-4.62 (m, 1H), 3.89 (s, 3H), 3.83 (s, 3H), 3.33-3.25 (m, 2H), 3.08-2.98 (m, 8H), 1.32 (d, *J=* 6.8 Hz, 3H), 1.06 (d, *J=* 6.8 Hz, 3H).

### Example 120:

### Preparation of intermediate 120A

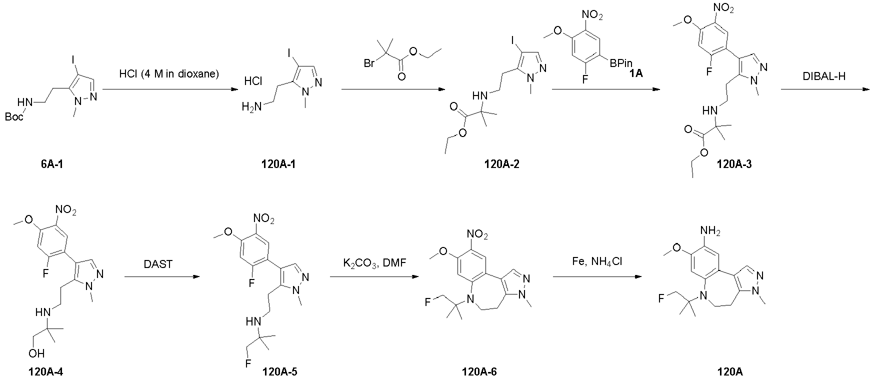

### Compound 120A-1:

Compound **6A-1** (5 g, 14.24 mmol) was dissolved in dichloromethane (40 mL), and a solution of hydrogen chloride in dioxane (4 M, 40 mL) was added to the reaction liquid. The reaction system was successively stirred at room temperature for 1.5 h. After the starting material disappeared as detected by LCMS, the reaction liquid was concentrated under reduced pressure to obtain compound **120A-1** (3.6 g).

MS (ESI, m/z): 252.2 [M + H]⁺.

### Compound 120A-2:

Compound **120A-1** (500 mg, 1.74 mmol) was dissolved in acetonitrile (5 mL). Subsequently, anhydrous potassium carbonate (719 mg, 5.2 mmol), sodium iodide (131 mg, 0.87 mmol), and ethyl 2-bromoisobutanoate (679 mg, 3.48 mmol) were added to the reaction liquid. The reaction system was heated to 80 °C and successively stirred for 16 h. After the starting material disappeared as detected by LCMS, the reaction liquid was cooled to room temperature and concentrated under reduced pressure. The resulting residue was purified by silica gel column chromatography (eluent: dichloromethane/ethyl acetate = 1/1) to obtain compound **120A-2** (381 mg).

MS (ESI, m/z): 366.1 [M + H]⁺.

### Compound 120A-3:

Compound **120A-2** (500 mg, 1.68 mmol) and compound **1A** (827.47 mg, 2.36 mmol) and potassium carbonate (465 mg, 3.37 mmol) were dissolved in a mixed solvent of 1,4-dioxane (5 mL) and water (1 mL) under nitrogen atmosphere. Subsequently, butyldi-1-adamantylphosphine (60 mg, 0.17 mmol) and chloro[(di(1-adamantyl)-N-butylphosphine)-2-(2-aminobiphenyl)]palladium(II) (113 mg, 0.17 mmol) were added to the reaction liquid. The reaction system was heated to 80 °C and successively stirred for 3 h. After the starting material disappeared as detected by LCMS, the reaction liquid was cooled to room temperature and concentrated under reduced pressure. The resulting residue was purified by silica gel column chromatography (eluent: dichloromethane/ethyl acetate = 1/1) to obtain compound **120A-3** (460 mg).

MS (ESI, m/z): 409.2 [M + H]⁺.

### Compound 120A-4:

Compound **120A-3** (2 g, 4.9 mmol) was dissolved in dichloromethane (30 mL) under nitrogen atmosphere. A solution of diisobutylaluminum hydride in toluene (1.5 M, 8.17 mL, 12.25 mmol) was added to the reaction liquid at -78 °C, and the reaction liquid was successively stirred at that temperature for 30 h. The reaction system was slowly warmed to room temperature and successively stirred for 1 h. After the starting material disappeared as detected by LCMS, the reaction liquid was quenched by addition of saturated aqueous ammonium chloride (100 mL). The mixture was extracted with ethyl acetate (100 mL × 3), the organic phases were combined, washed with saturated brine (100 mL × 3) firstly, then dried over anhydrous sodium sulfate, filtered, and finally concentrated under reduced pressure, and the resulting residue was purified by silica gel column chromatography (eluent: dichloromethane/methanol = 10/1) to obtain compound **120A-4** (0.75 g).

MS (ESI, m/z): 367.1 [M + H]⁺.

### Compound 120A-5:

Compound **120A-4** (700 mg, 1.91 mmol) was dissolved in dichloromethane (7 mL) under nitrogen atmosphere. Diethylaminosulfur trifluoride (3080 mg, 19.11 mmol) was slowly added dropwise to the reaction liquid at -78 °C, and the reaction liquid was successively stirred at that temperature for 30 min. The reaction system was slowly warmed to room temperature and successively stirred for 16 h. After the starting material disappeared as detected by LCMS, the reaction liquid was quenched by addition of saturated aqueous sodium bicarbonate solution (20 mL). The mixture was extracted with ethyl acetate (20 mL × 3), the organic phases were combined, washed with saturated brine (20 mL × 3) firstly, then dried over anhydrous sodium sulfate, filtered, and finally concentrated under reduced pressure, and the resulting residue was purified by silica gel column chromatography (eluent: dichloromethane/methanol = 10/1) to obtain compound **120A-5** (164 mg).

MS (ESI, m/z): 369.4 [M + H]⁺.

### Compound 120A-6:

Compound **120A-6** (104 mg) was prepared by replacing the starting material with compound **120A-5** (150 mg, 0.41 mmol) according to the method for the preparation of compound **1C-9** from compound **1C-8** in Example 1.

MS (ESI, m/z): 349.1 [M + H]⁺.

### Intermediate 120A:

Compound **120A** (86 mg) was prepared by replacing the starting material with compound **120A-6** (104 mg, 0.3 mmol) according to the method for the preparation of compound **1C** from compound **1C-9** in Example 1.

MS (ESI, m/z): 319.1 [M + H]⁺.

### Preparation of compound 120

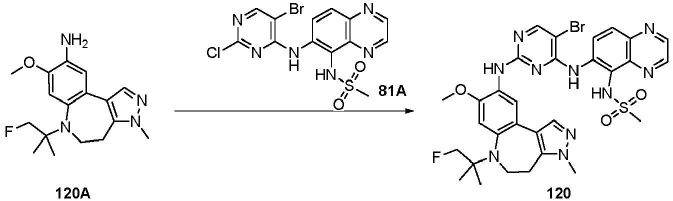

Compound **120A** (60 mg, 0.19 mmol) and compound **81A** (73 mg, 0.17 mmol) were dissolved in ethanol (1.5 mL). The reaction liquid was heated to 150 °C with a microwave reactor and successively stirred for 40 min. After the starting material disappeared as detected by LCMS, the reaction liquid was cooled to room temperature and concentrated under reduced pressure. The resulting residue was purified by silica gel column chromatography (eluent: dichloromethane/methanol = 20/1) to obtain compound **120** (26 mg).

MS (ESI) M/Z: 711.2, 713.2 [M + H]⁺.

¹H NMR (300 MHz, CDCl₃) δ 9.14 (s, 1H), 8.84-8.81 (m, 2H), 8.60 (d, *J =* 9.3 Hz, 1H), 8.28 (s, 2H), 7.60 (s, 1H), 7.55 (s, *J =* 9.3 Hz, 1H), 7.40 (s, 1H), 6.82 (s, 1H), 6.59 (s, 1H), 3.88 (s, 3H), 3.69 (s, 3H), 3.53 (s, 1H), 3.46-3.42 (m, 3H), 3.10-3.02 (m, 2H), 2.95 (s, 3H), 1.35 (s, 3H), 1.28 (s, 3H).

¹⁹F NMR (282 MHz, CDCl₃) δ -140.58.

### Example 121:

### Preparation of intermediate 121A

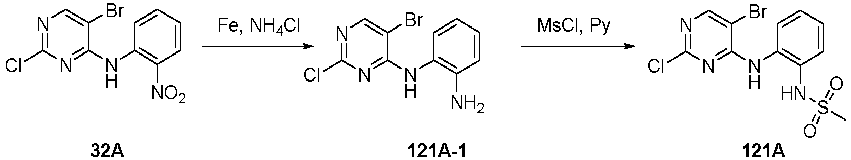

### Compound 121A-1:

Compound **121A-1** (380 mg) was prepared by replacing the starting material with compound **32A** (500 mg, 1.52 mmol) according to the method for the preparation of compound **1C** from compound **1C-9** in Example 1.

MS (ESI, m/z): 298.9, 300.9 [M + H]⁺.

### Compound 121A:

Compound **121A** (150 mg) was prepared by replacing the starting material with compound **121A-1** (200 mg, 0.67 mmol) according to the method for the preparation of compound **32** from compound **32C** in Example 32.

MS (ESI, m/z): 376.9, 378.9 [M + H]⁺.

### Preparation of compound 121

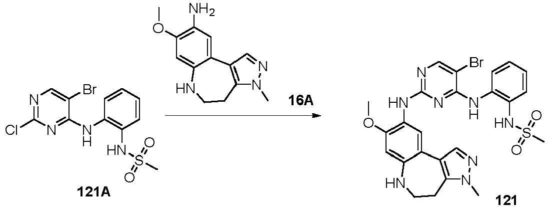

Compound **121** (22 mg) was prepared by replacing the starting material with compound **121A** (70 mg, 0.19 mmol) and compound **16A** (54 mg, 0.22 mmol) according to the method for the preparation of compound **1** from compound **1B** and compound **1C** in Example 1.

MS (ESI, m/z): 585.1, 587.1 [M + H]⁺.

¹H NMR (300 MHz, DMSO-*d₆*) δ 10.02 (br s, 1H), 8.34 (s, 1H), 8.16-8.12 (m, 3H), 7.65 (s, 1H), 7.56 (s, 1H), 7.28 (dd, *J* = 7.8, 1.8 Hz, 1H), 7.03-6.92 (m, 2H), 6.51 (s, 1H), 5.85 (s, 1H), 3.73 (s, 3H), 3.67 (s, 3H), 3.27-3.21 (m, 2H), 2.98 (s, 3H), 2.96-2.94 (m, 2H).

### Example 122:

### Preparation of compound 122

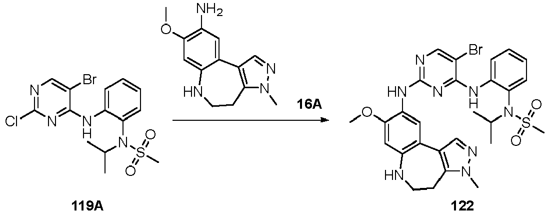

Compound **122** (15 mg) was prepared by replacing the starting material with compound **119A** (60 mg, 0.14 mmol) and compound **16A** (35 mg, 0.14 mmol) according to the method for the preparation of compound **1** from compound **1B** and compound **1C** in Example 1.

MS (ESI, m/z): 627.2, 629.2 [M + H]⁺.

¹H NMR (300 MHz, DMSO-*d₆*) δ 8.34 (s, 1H), 8.25 (s, 1H), 8.18 (s, 1H), 8.07 (s, 1H), 7.67 (s, 1H), 7.52 (s, 1H), 7.41-7.38 (m, 1H), 7.09-7.02 (m, 2H), 6.54 (s, 1H), 5.88 (dd, *J* = 4.2 Hz, 1H), 4.49-4.40 (m, 1H), 3.73 (s, 3H), 3.68 (s, 3H), 3.29-3.24 (m, 2H), 3.14 (s, 3H), 2.99-2.95 (m, 2H), 1.18 (d, *J* = 6.9 Hz, 3H), 0.98 (d, *J* = 6.9 Hz, 3H).

### Example 123:

### Preparation of compound 123

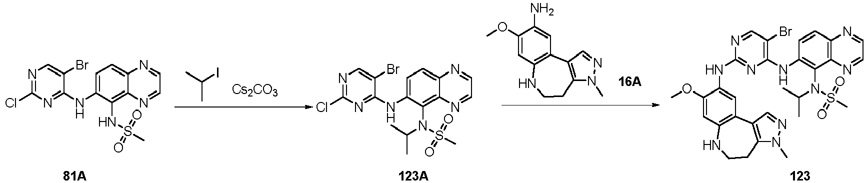

### Compound 123A:

Compound **81A** (200 mg, 0.47 mmol) and 2-iodopropane (237 mg, 1.39 mmol) were dissolved in dimethyl sulfoxide (3 mL). Subsequently, cesium carbonate (455 mg, 1.4 mmol) was added to the reaction liquid, and the reaction system was heated to 80 °C and successively stirred for 1 h. After the starting material disappeared as detected by LCMS, the reaction liquid was cooled to room temperature and purified directly using a reverse phase C18 column. Purification conditions were as follows: a chromatography column: 40 g of C18 reverse phase column; mobile phase: water (containing 0.1% formic acid) and acetonitrile; flow rate: 35 mL/min; gradient: acetonitrile elevating from 10% to 60% in 30 min; detection wavelength 254 nm. The product was collected and lyophilized under reduced pressure to obtain compound **123A** (70 mg).

MS (ESI) M/Z: 470.9, 472.9 [M + H]⁺.

### Compound 123:

Compound **123** (20 mg) was prepared by replacing the starting material with compound **123A** (70 mg, 0.15 mmol) and compound **16A** (36 mg, 0.15 mmol) according to the method for the preparation of compound **1** from compound **1B** and compound **1C** in Example 1.

MS (ESI, m/z): 679.2, 681.2 [M + H]⁺.

¹H NMR (300 MHz, CDCl₃) δ 8.82-8.74 (s, 3H), 8.64 (d, *J* = 6.9 Hz, 1H), 8.22 (s, 2H), 7.83-7.63 (m, 2H), 6.86 (s, 1H), 6.31 (s, 1H), 4.54-4.47 (m, 1H), 3.84 (s, 3H), 3.68 (s, 3H), 3.57-3.49 (m, 1H), 3.42-3.35 (m, 1H), 3.23 (s, 3H), 3.06-2.97 (m, 2H), 1.35 (d, *J* = 4.8 Hz, 3H), 1.26 (d, *J* = 4.8 Hz, 3H).

### Example 124:

### Preparation of compound 124

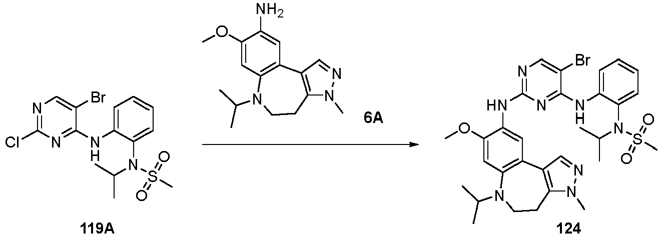

Compound **124** (2 mg) was prepared by replacing the starting material with compound **119A** (60 mg, 0.14 mmol) and compound **6A** (41 mg, 0.14 mmol) according to the method for the preparation of compound **1** from compound **1B** and compound **1C** in Example 1.

MS (ESI, m/z): 669.3, 671.3 [M + H]⁺.

¹H NMR (300 MHz, DMSO-*d₆*) δ 8.44-8.38 (m, 1H), 8.35-8.30 (m, 1H), 8.28 (s, 1H), 8.19 (s, 1H), 7.75 (s, 1H), 7.46 (s, 1H), 7.41-7.378 (m, 1H), 7.07-7.01 (m, 2H), 6.58 (s, 1H), 4.49-4.43 (m, 1H), 3.98-3.92 (m, 1H), 3.75 (s, 6H), 3.24-3.19 (m, 2H), 3.13 (s, 3H), 2.96-2.93 (m, 2H), 1.29 (d, *J* = 6.6 Hz, 6H), 1.19 (d, *J* = 6.6 Hz, 3H), 0.98 (d, *J* = 6.6 Hz, 3H).

### Example 125:

### Preparation of compound 125

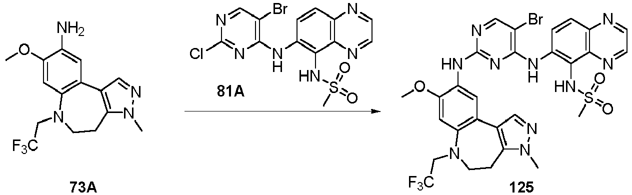

Compound **125** (3 mg) was prepared by replacing the starting material with compound **73A** (32 mg, 0.1 mmol) and compound **81A** (42 mg, 0.1 mmol) according to the method for the preparation of compound **1** from compound **1B** and compound **1C** in Example 1.

MS (ESI, m/z): 719.1, 721.1 [M + H]⁺.

¹H NMR (400 MHz, DMSO-*d₆*) δ 9.98 (s, 1H), 8.95 (s, 1H), 8.86-8.82 (m, 2H), 8.72-8.59 (s, 1H), 8.37 (s, 1H), 8.29 (s, 1H), 7.80 (s, 1H), 7.57-7.46 (s, 1H), 7.38 (s, 1H), 6.88 (s, 1H), 4.34-4.27 (m, 2H), 3.79 (s, 3H), 3.71 (s, 3H), 3.42-3.40 (m, 2H), 3.03-3.01 (m, 5H).

¹⁹F NMR (377 MHz, DMSO-*d₆*) δ -69.58.

### Example 126:

### Preparation of intermediate 126A

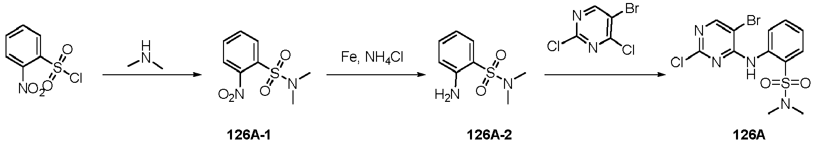

### Compound 126A-1:

Anhydrous sodium carbonate (3.59 g, 33.87 mmol) was added to a solution of dimethylamine in tetrahydrofuran (2 M, 13.5 mL). Subsequently, o-nitrobenzenesulfonyl chloride (5 g, 22.56 mmol) was added in batches to the reaction liquid, and the reaction liquid was successively stirred at room temperature for 16 h. After the starting material disappeared as detected by LCMS, the reaction liquid was quenched by addition of water (100 mL). The mixture was extracted with ethyl acetate (100 mL × 3), and the organic phases were combined, washed with saturated brine (100 mL × 3) firstly, then dried over anhydrous sodium sulfate, filtered, and finally concentrated under reduced pressure to obtain compound **126A-1** (4.6 g).

MS (ESI, m/z): 231.1 [M + H]⁺.

### Compound 126A-2:

Compound **126A-2** (3.5 g) was prepared by replacing the starting material with compound **126A-1** (4.6 g, 19.98 mmol) according to the method for the preparation of compound **1C** from compound **1C-9** in Example 1.

MS (ESI, m/z): 201.1 [M + H]⁺.

### Intermediate 126A:

Compound **126A** (100 mg) was prepared by replacing the starting material with compound **126A-2** (500 mg, 2.5 mmol) according to the method for the preparation of compound **1B** from compound **1B-2** in Example 1.

MS (ESI, m/z): 391.0, 393.0 [M + H]⁺.

### Preparation of compound 126

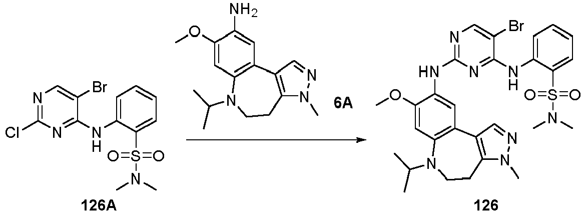

Compound **126** (43 mg) was prepared by replacing the starting material with compound **126A** (70 mg, 0.18 mmol) and compound **6A** (56 mg, 0.18 mmol) according to the method for the preparation of compound **1** from compound **1B** and compound **1C** in Example 1.

MS (ESI, m/z): 641.3, 643.3 [M + H]⁺.

¹H NMR (400 MHz, CDCl₃) δ 9.33 (s, 1H), 8.44-8.22 (m, 3H), 7.84 (s, 1H), 7.54-7.32 (m, 1H), 7.25-7.07 (m, 3H), 6.55 (s, 1H), 3.97-3.63 (m, 7H), 3.39-3.15 (m, 2H), 3.00-2.86 (m, 2H), 2.76 (s, 6H), 1.30 (s, 6H).

### Example 127:

### Preparation of intermediate 127A

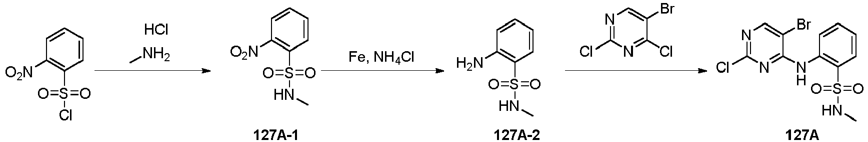

### Compound 127A-1:

Triethylamine (11.42 g, 112.81 mmol) was added to methylamine hydrochloride (4.57 g, 67.69 mmol) in dichloromethane (100 mL). Subsequently, o-nitrobenzenesulfonyl chloride (5 g, 22.56 mmol) was added in batches to the reaction liquid, and the reaction liquid was successively stirred at room temperature for 2 h. After the starting material disappeared as detected by LCMS, the reaction liquid was quenched by addition of water (100 mL). The mixture was extracted with ethyl acetate (100 mL × 3), and the organic phases were combined, washed with saturated brine (100 mL × 3) firstly, then dried over anhydrous sodium sulfate, filtered, and finally concentrated under reduced pressure to obtain compound **127A-1** (4.5 g).

MS (ESI, m/z): 217.1 [M + H]⁺.

### Compound 127A-2:

Compound **127A-2** (2.2 g) was prepared by replacing the starting material with compound 127A-**1 (2.7** g, 12.49 mmol) according to the method for the preparation of compound **1C** from compound **1C-9** in Example 1.

MS (ESI, m/z): 187.1 [M + H]⁺.

### Compound 127A:

5-Bromo-2,4-dichloropyrimidine (1224 mg, 5.37 mmol) and compound **127A-2** (500 mg, 2.69 mmol) were dissolved in *N*,*N*-dimethylformamide (5 mL); subsequently, anhydrous potassium carbonate (1113 mg, 8.05 mmol) was added to the reaction liquid. The reaction liquid was heated to 45 °C and successively stirred for 2 h. After the starting material disappeared as detected by LCMS, the reaction liquid was cooled to room temperature and quenched by addition of water (50 mL). The mixture was extracted with ethyl acetate (50 mL × 3), the organic phases were combined, washed with saturated brine (50 mL × 3), then dried over anhydrous sodium sulfate, filtered, and concentrated under reduced pressure, and the resulting residue was purified by silica gel column chromatography (eluent: ethyl acetate) to obtain compound **127A** (450 mg).

MS (ESI) M/Z: 377.0, 379.0 [M + H]⁺.

### Preparation of compound 127

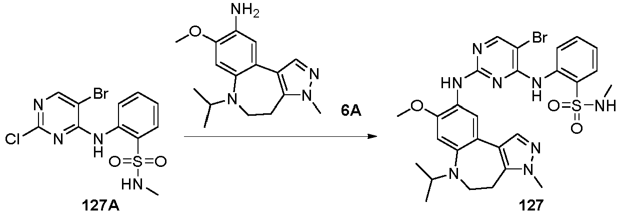

Compound **127** (8 mg) was prepared by replacing the starting material with compound **127A** (100 mg, 0.27 mmol) and compound **6A** (114 mg, 0.4 mmol) according to the method for the preparation of compound **1** from compound **1B** and compound **1C** in Example 1.

MS (ESI, m/z): 627.3, 629.3 [M + H]⁺.

¹H NMR (400 MHz, CDCl₃) δ 9.03 (s, 1H), 8.39 (d, *J* = 8.4 Hz, 1H), 8.15 (s, 1H), 7.92 (dd, *J* = 8.0, 1.6 Hz, 1H), 7.23 (s, 1H), 7.19 (s, 1H), 7.04-7.00 (m, 2H), 6.55 (s, 1H), 5.45 (s, 1H), 4.72 (s, 1H), 3.94-3.89 (m, 1H), 3.87 (s, 3H), 3.79 (s, 3H), 3.28-3.25 (m, 2H), 2.93-2.90 (m, 2H), 2.64 (d, *J* = 4.2 Hz, 3H), 1.30 (d, *J* = 6.4 Hz, 6H).

### Example 128:

### Preparation of intermediate 128A

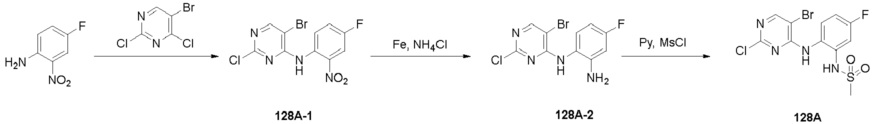

### Compound 128A-1:

Compound **128A-1** (8.5 g) was prepared by replacing the starting material with 2-nitro-4-fluoroaniline (5 g, 32.05 mmol) according to the method for the preparation of compound **1B** from compound **1B-2** in Example 1.

MS (ESI, m/z): 347.0, 349.0 [M + H]⁺.

### Compound 128-2:

Compound **128A-2** (6.2 g) was prepared by replacing the starting material with compound **128A-1** (8.5 g, 24.4 mmol) according to the method for the preparation of compound **1C** from compound **1C-9** in Example 1.

MS (ESI, m/z): 317.0, 319.0 [M + H]⁺.

### Compound 128A:

Compound **128A** (360 mg) was prepared by replacing the starting material with compound **128A-2** (400 mg, 1.27 mmol) according to the method for the preparation of compound **32** from compound **32C** in Example 32.

MS (ESI, m/z): 395.0, 397.0 [M + H]⁺.

### Preparation of compound 128

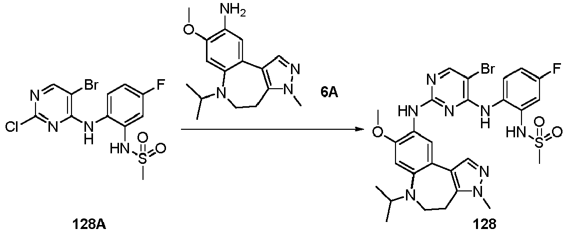

Compound **128** (40 mg) was prepared by replacing the starting material with compound **128A** (70 mg, 0.18 mmol) and compound **6A** (51 mg, 0.18 mmol) according to the method for the preparation of compound **1** from compound **1B** and compound **1C** in Example 1.

MS (ESI, m/z): 645.1, 647.1 [M + H]⁺.

¹H NMR (300 MHz, DMSO-*d₆*) δ 10.03 (br s, 1H), 8.28 (s, 1H), 8.13 (s, 1H), 8.00 (s, 1H), 7.89-7.84 (m, 1H), 7.72 (s, 1H), 7.45 (s, 1H), 7.13 (dd, *J* = 9.9, 3.0 Hz, 1H), 6.62 (s, 1H), 6.52 (s, 1H), 3.95-3.86 (m, 1H), 3.75 (s, 3H), 3.73 (s, 3H), 3.20-3.17 (m, 2H), 2.97 (s, 3H), 2.94-2.91 (m, 2H), 1.27 (d, *J* = 6.3 Hz, 6H).

¹⁹F NMR (282 MHz, DMSO-*d₆*) δ -117.35.

### Example 129:

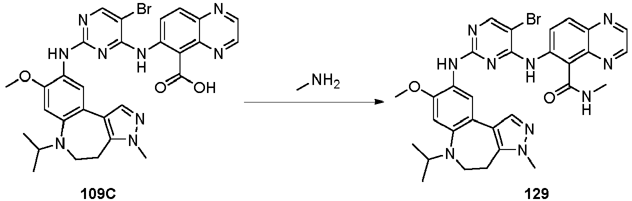

Compound **129** (9 mg) was prepared by replacing the starting material with compound **109C** (100 mg, 0.16 mmol) according to the method for the preparation of compound **48A-5** from compound **48A-4** in Example 48.

MS (ESI, m/z): 643.2, 645.2 [M + H]⁺.

¹H NMR (400 MHz, DMSO-*d₆*) δ 12.05 (s, 1H), 10.14 (d, *J* = 4.8 Hz, 1H), 9.01-8.87 (m, 3H), 8.29 (s, 1H), 7.73-7.49 (m, 3H), 6.80 (s, 1H), 6.60 (s, 1H), 3.99-3.92 (m, 1H), 3.77 (s, 3H), 3.71 (s, 3H), 3.23-3.20 (m, 2H), 2.95-2.93 (m, 5H), 1.29 (d, *J* = 6.4 Hz, 6H).

### Example 130:

### Preparation of compound 130

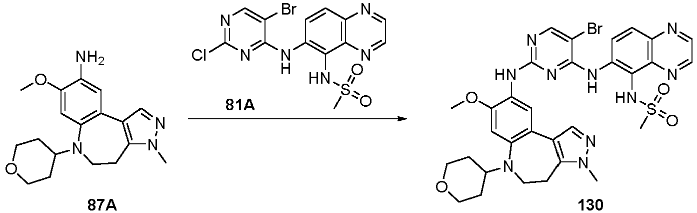

Compound **130** (42 mg) was prepared by replacing the starting material with compound **87A** (100 mg, 0.31 mmol) and compound **81A** (81 mg, 0.19 mmol) according to the method for the preparation of compound **1** from compound **1B** and compound **1C** in Example 1.

MS (ESI, m/z): 721.2, 723.2 [M + H]⁺.

¹H NMR (300 MHz, CDCl₃) δ 9.25 (s, 1H), 8.86-8.82 (m, 2H), 8.54 (d, *J=* 9.0 Hz, 1H), 8.36-8.19 (m, 2H), 7.85-7.50 (m, 3H), 6.74 (s, 1H), 6.52 (s, 1H), 4.39-4.03 (m, 3H), 3.90 (m, 3H), 3.67 (s, 3H), 3.53-3.34 (m, 5H), 3.09-2.80 (m, 4H), 2.15-1.78 (m, 4H).

### Example 131:

### Preparation of intermediate 131A

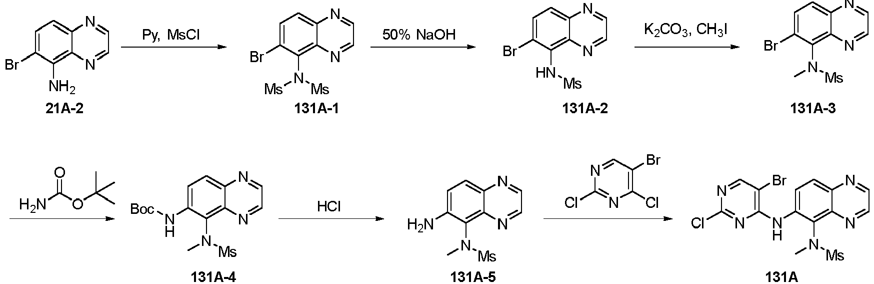

### Compound 131A-1:

Compound **21A-2** (5.8 g, 25.89 mmol) was dissolved in pyridine (60 mL). Subsequently, methanesulfonyl chloride (14.82 g, 129.43 mmol) was added to the reaction liquid, and the reaction system was heated to 50 °C and successively stirred for 5 h. After the starting material disappeared as detected by LCMS, the reaction liquid was cooled to room temperature and concentrated under reduced pressure. The resulting residue was purified by silica gel column chromatography (eluent: dichloromethane/methanol = 10/1) to obtain compound **131A-1** (5.1 g).

¹H NMR (300 MHz, DMSO-*d₆*) δ 9.13-9.09 (m, 2H), 8.28 (d, *J* = 9.0 Hz, 1H), 8.22 (d, *J* = 9.0 Hz, 1H), 3.71-3.67 (m, 6H).

### Compound 131A-2:

Compound **131A-1** (8 g, 21 mmol) was dissolved in a mixed solvent of methanol (80 mL) and tetrahydrofuran (80 mL). Subsequently, 50% aqueous sodium hydroxide solution (33.6 g, 420 mmol) was added to the above reaction liquid at 0 °C. The reaction system was warmed to room temperature and successively stirred for 1 h. After the starting material disappeared as detected by LCMS, the reaction liquid was added water (200 mL) for dilution and adjusted to pH = 6 with concentrated hydrochloric acid. The mixture was extracted with a mixed solvent of chloroform/isopropanol of 3:1 (80 mL ×3). The organic phases were combined, washed with saturated brine (300 mL × 2) firstly, then dried over anhydrous sodium sulfate, filtered, and finally concentrated under reduced pressure, and the resulting residue was purified by silica gel column chromatography (eluent: dichloromethane/methanol = 5/1) to obtain compound **131A-2** (6 g).

¹H NMR (300 MHz, DMSO-*d₆*) δ 9.83 (br s, 1H), 9.09-9.01 (m, 2H), 8.15 (d, *J* = 9.0 Hz, 1H), 8.01 (d, *J* = 9.0 Hz, 1H), 3.31 (s, 3H).

### Compound 131A-3:

Compound **131A-3** (3 g) was prepared by replacing the starting material with compound **131A-2** (3 g, 9.9 mmol) according to the method for the preparation of compound **21A-5** from compound **21A-4** in Example 21.

¹H NMR (300 MHz, CDCl₃) δ 8.92-8.90 (m, 2H), 8.06-8.02 (m, 2H), 3.41 (s, 3H), 3.30 (s, 3H).

### Compound 131A-4:

Compound **131A-4** (4 g) was prepared by replacing the starting material with compound **131A-3** (2.8 g, 8.8 mmol) according to the method for the preparation of compound **21A-6** from compound **21A-5** in Example 21.

¹H NMR (300 MHz, CDCl₃) δ 8.82 (d, *J* = 9.6 Hz, 1H), 8.79-8.77 (m, 2H), 8.10 (d, *J* = 9.6 Hz, 1H), 7.87 (br s, 1H), 3.45 (s, 3H), 3.14 (s, 3H), 1.45 (s, 9H).

### Compound 131A-5:

Compound **131A-5** (0.68 g) was prepared by replacing the starting material with compound **131A-4** (1 g, 2.8 mmol) according to the method for the preparation of compound **21A-7** from compound **21A-6** in Example 21.

MS (ESI, m/z): 253.2 [M + H]⁺.

### Intermediate 131A:

Compound **131A** (1 g) was prepared by replacing the starting material with compound **131A-5** (650 mg, 2.6 mmol) according to the method for the preparation of compound **1B** from compound **1B-2** in Example 1.

¹H NMR (300 MHz, DMSO-*d₆*) δ 9.11 (s, 1H), 9.05-8.97 (m, 2H), 8.66 (s, 1H), 8.56 (d, *J* = 9.6 Hz, 1H), 8.25 (d, *J* = 9.3 Hz, 1H), 3.41 (s, 3H), 3.19 (s, 3H).

### Preparation of compound 131

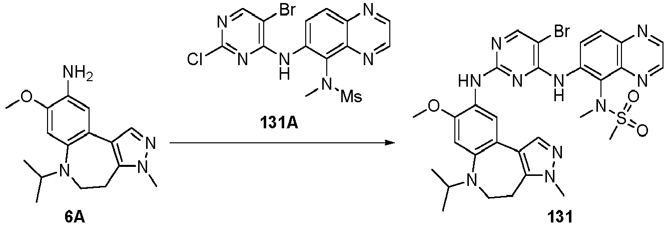

Compound **131** (42 mg) was prepared by replacing the starting material with compound **131A** (80 mg, 0.18 mmol) and compound **6A** (54 mg, 0.19 mmol) according to the method for the preparation of compound **1** from compound **1B** and compound **1C** in Example 1.

MS (ESI, m/z): 693.2, 695.2 [M + H]⁺.

¹H NMR (400 MHz, DMSO-*d₆*) δ 8.93 (d, *J* = 2.0 Hz, 1H), 8.86 (d, *J* = 2.0 Hz, 1H), 8.78-8.70 (m, 1H), 8.51 (s, 1H), 8.46 (s, 1H), 8.29 (s, 1H), 7.73 (s, 1H), 7.61-7.50 (m, 1H), 7.38 (s, 1H), 6.60 (s, 1H), 3.99-3.93 (m, 1H), 3.77 (s, 3H), 3.69 (s, 3H), 3.38 (s, 3H), 3.23-3.21 (m, 5H), 2.94-2.91 (m, 2H), 1.31-1.29 (m, 6H).

### Example 132:

### Preparation of intermediate 132A

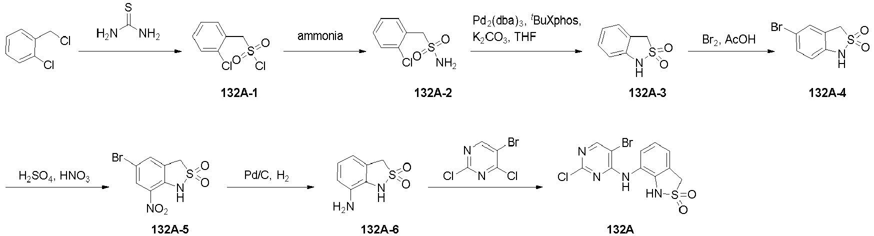

### Compound 132A-1:

Thiourea (7.09 g, 93.15 mmol) was dissolved in ethanol (75 mL). Subsequently, (2-chlorophenyl) methanesulfonyl chloride (15 g, 93.15 mmol) was added to the reaction liquid, and the reaction liquid was heated to 80 °C and successively stirred for 1 h. The reaction liquid was cooled to room temperature and concentrated under reduced pressure, and the residue was added to acetonitrile (120 mL) containing a solution of N-chlorosuccinimide (49.75 g, 372.6 mmol) and hydrogen chloride in ethanol (2 M, 40 mL, 700.7 mmol). The reaction liquid was successively stirred at room temperature for 1 h. After the starting material disappeared as detected by LCMS, the reaction liquid was concentrated under reduced pressure. The resulting residue was purified by silica gel column chromatography (eluent: petroleum ether/ethyl acetate = 3/1) to obtain compound **132A-1** (20 g).

¹H NMR (300 MHz, CDCl₃) δ 7.64-7.61 (m, 1H), 7.55-7.52 (m, 1H), 7.48-7.39 (m, 2H), 5.14 (s, 2H).

### Compound 132A-2:

Compound **132A-1** (20 g, 88.86 mmol) was dissolved in acetone (150 mL); subsequently, aqueous ammonia (100 mL) was added to the reaction liquid at 0 °C. The reaction system was warmed to room temperature and successively stirred for 2 h. After the starting material disappeared as detected by TLC, the reaction liquid was concentrated under reduced pressure. The reaction liquid was adjusted to pH = 2 with concentrated hydrochloric acid, and the mixture was extracted with a mixed solvent of ethyl acetate (300 mL ×3). The organic phases were combined, washed with saturated brine (300 mL × 2) firstly, then dried over anhydrous sodium sulfate, filtered, and finally concentrated under reduced pressure to obtain compound **132A-2** (14.5 g).

¹H NMR (300 MHz, DMSO-*d₆*) δ 7.54-7.47 (m, 2H), 7.41-7.36 (m, 2H), 7.04 (s, 2H), 4.46 (s, 2H).

### Compound 132A-3:

Under nitrogen atmosphere, compound **132-2** (7.5 g, 36.47 mmol), anhydrous potassium carbonate (10.08 g, 72.94 mmol), tris(dibenzylideneacetone)dipalladium (3.34 g, 3.65 mmol), and 2-di*-tert-*butylphosphino-2',4',6'-triisopropylbiphenyl (3.1 g, 7.29 mmol) were dissolved in tetrahydrofuran (100 mL). The reaction system was heated to 80 °C and successively stirred for 16 h. After the starting material disappeared as detected by LCMS, the reaction liquid was cooled to room temperature and quenched by addition of water (100 mL). The mixture was extracted with a mixed solvent of ethyl acetate (150 mL × 3). The organic phases were combined, washed with saturated brine (100 mL × 2) firstly, then dried over anhydrous sodium sulfate, filtered, and finally concentrated under reduced pressure. The resulting residue was purified by silica gel column chromatography (eluent: petroleum ether/ethyl acetate = 2/1) to obtain compound **132A-3** (4.5 g). ¹H NMR (300 MHz, CDCl₃) δ 7.33-7.26 (m, 2H), 7.11-7.06 (m, 1H), 6.93-6.90 (m, 1H), 6.58 (s, 1H), 4.41 (s, 2H).

### Compound 132A-4:

Compound **132A-3** (500 mg, 2.96 mmol) was dissolved in glacial acetic acid (5 mL). Bromine (496 mg, 3.1 mmol) was added to the reaction liquid at 0 °C. The reaction system was warmed to room temperature and successively stirred for 2 h. After the starting material disappeared as detected by TLC, the reaction liquid was directly purified using a reverse phase C18 column. Purification conditions were as follows: a chromatography column: 40 g of C18 reverse phase column; mobile phase: water (containing 0.1% formic acid) and acetonitrile; flow rate: 35 mL/min; gradient: acetonitrile elevating from 40% to 60% in 30 min; detection wavelength 254 nm. The product was collected and lyophilized under reduced pressure to obtain compound **132A-4** (450 mg).

MS (ESI) M/Z: 245.9, 247.9 [M - H]⁺.

### Compound 132A-5:

Compound **132-4** (350 mg, 1.41 mmol) was dissolved in concentrated sulfuric acid (7 mL) at 0 °C; subsequently, the reaction liquid was added with potassium nitrate (157 mg, 1.55 mmol) and successively stirred at that temperature for 30 min. After the starting material disappeared as detected by LCMS, the reaction liquid was poured into ice water (50 g). The mixture was extracted with a mixed solvent of ethyl acetate (15 mL × 3). The organic phases were combined, washed with saturated brine (10 mL × 2) firstly, then dried over anhydrous sodium sulfate, filtered, and finally concentrated under reduced pressure. The resulting residue was purified by silica gel column chromatography (eluent: dichloromethane/methanol = 15/1) to obtain compound **132A-5** (270 mg). MS (ESI) M/Z: 290.8, 292.8 [M - H]⁺.

### Compound 132A-6:

Compound **132A-5** (140 mg, 0.48 mmol) was dissolved in ethanol (2 mL). Subsequently, wet palladium on carbon (10%, 56 mg) was added to the reaction liquid. After the reaction system was purged with hydrogen 3 times, the reaction liquid was stirred at room temperature under hydrogen atmosphere for 2 h. After the starting material disappeared as detected by LCMS, the reaction liquid was filtered through celite, the filter cake was washed with ethyl acetate (10 mL × 3). The filtrate was concentrated under reduced pressure. The resulting residue was purified by silica gel column chromatography (eluent: petroleum ether/ethyl acetate = 1/1) to obtain compound **132A-6** (570 mg).

¹H NMR (300 MHz, DMSO-*d₆*) δ 9.47 (s, 1H), 6.78 (dd, *J* = 7.8, 7.8 Hz, 1H), 6.61 (dd, *J* = 8.1, 1.2 Hz, 1H), 6.61 (dd, *J* = 7.5, 1.2 Hz, 1H), 4.85 (s, 2H), 4.44 (s, 2H).

### Intermediate 132A:

Compound **132A** (65 mg) was prepared by replacing the starting material with compound **132A-6** (60 mg, 0.33 mmol) according to the method for the preparation of compound **1B** from compound **1B-2** in Example 1.

MS (ESI) M/Z: 375.0, 377.0 [M + H]⁺.

### Preparation of compound 132

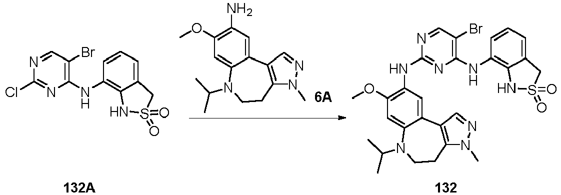

Compound **132** (10 mg) was prepared by replacing the starting material with compound **132A** (57 mg, 0.15 mmol) and compound **6A** (43 mg, 0.15 mmol) according to the method for the preparation of compound **1** from compound **1B** and compound **1C** in Example 1.

MS (ESI, m/z): 625.1, 627.1 [M + H]⁺.

¹H NMR (400 MHz, DMSO-*d₆*) δ 9.94 (s, 1H), 8.14 (d, *J* = 3.2 Hz, 1H), 8.02 (s, 1H), 7.83 (s, 2H), 7.55-7.52 (m, 1H), 7.36 (s, 1H), 6.97-6.95 (m, 1H), 6.72 (s, 1H), 6.51 (d, *J* = 2.8 Hz, 1H), 4.48 (s, 2H), 3.90-3.85 (m, 1H), 3.73 (s, 6H), 3.17-3.15 (m, 2H), 2.92-2.89 (m, 2H), 1.26-1.23 (m, 6H).

### Example 133:

### Preparation of compound 133

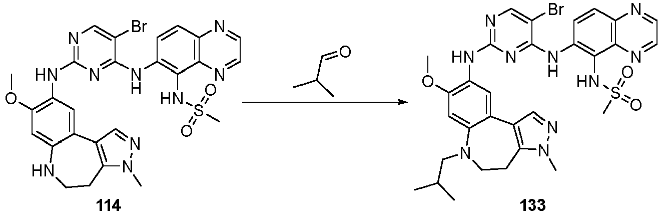

Compound **114** (100 mg, 0.16 mmol) and isobutyraldehyde (57 mg, 0.79 mmol) were dissolved in methanol (2 mL) and stirred for 10 min, followed by addition of acetic acid (19 mg, 0.31 mmol) and sodium cyanoborohydride (20 mg, 0.31 mmol) to the reaction liquid. The reaction system was successively stirred at room temperature for 1 h. After the starting material disappeared as detected by LCMS, the reaction liquid was directly purified using a reverse phase C18 column. Purification conditions were as follows: a chromatography column: 40 g of C18 reverse phase column; mobile phase: water (containing 0.1% formic acid) and acetonitrile; flow rate: 35 mL/min; gradient: acetonitrile elevating from 0% to 50% in 35 min; detection wavelength 254 nm. The product was collected and lyophilized under reduced pressure to obtain compound **133** (26 mg).

MS (ESI) M/Z: 693.2, 695.2 [M - H]⁺.

¹H NMR (300 MHz, CDCl₃) δ 9.14 (s, 1H), 8.86-8.77 (m, 2H), 8.61 (d, *J* = 9.3 Hz, 1H), 8.26 (s, 2H), 7.62 (s, 1H), 7.59-7.55 (m, 1H), 7.36 (s, 1H), 6.81 (s, 1H), 6.54 (s, 1H), 3.89 (s, 3H), 3.70 (s, 3H), 3.35-3.31 (m, 2H), 3.08 (d, *J* = 7.2 Hz, 2H), 2.98-2.95 (m, 5H), 1.92-1.88 (m, 1H), 0.91 (d, *J* = 6.6 Hz, 6H).

### Example 134:

### Preparation of compound 134

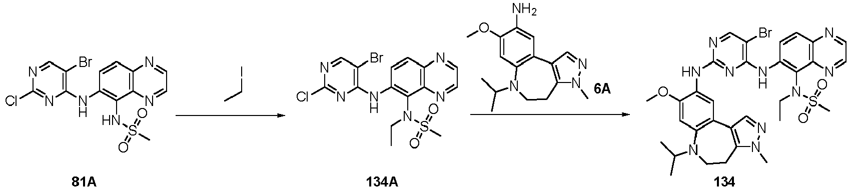

### Compound 134A:

Compound **134A** (120 mg) was prepared by replacing the starting material with iodothane (218 mg, 1.4 mmol) according to the method for the preparation of compound **123A** from compound **81A** in Example 123.

MS (ESI, m/z): 456.9, 458.9 [M + H]⁺.

### Compound 134:

Compound **134** (43 mg) was prepared by replacing the starting material with compound **134A** (60 mg, 0.13 mmol) and compound **6A** (38 mg, 0.13 mmol) according to the method for the preparation of compound **1** from compound **1B** and compound **1C** in Example 1.

MS (ESI, m/z): 707.3, 709.3 [M + H]⁺.

¹H NMR (400 MHz, CDCl₃) δ 8.79-8.73 (m, 4H), 8.33 (s, 1H), 8.25 (s, 1H), 7.62 (s, 1H), 7.31-7.29 (m, 1H), 6.90 (s, 1H), 6.55 (s, 1H), 4.23-4.14 (m, 1H), 3.88 (s, 3H), 3.85-3.80 (m, 2H), 3.68 (s, 3H), 3.47-3.44 (m, 1H), 3.20-3.16 (m, 1H), 3.13 (s, 3H), 2.96-2.92 (m, 2H), 1.33-1.28 (m, 6H), 1.14 (t, *J* = 7.2 Hz, 3H).

### Example 135:

### Preparation of compound 135

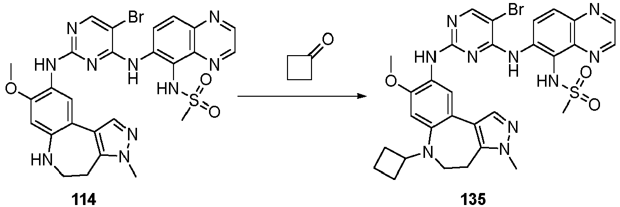

Compound **135** (21 mg) was prepared by replacing the starting material with cyclobutanone (38 mg, 0.55 mmol) according to the method for the preparation of compound **133** from compound **114** in Example 133.

MS (ESI, m/z): 691.2, 693.2 [M + H]⁺.

¹H NMR (300 MHz, CDCl₃) δ 9.15 (s, 1H), 8.88-8.78 (m, 2H), 8.55 (d, *J* = 9.3 Hz, 1H), 8.27-8.25 (m, 2H), 7.73 (s, 1H), 7.56 (d, *J* = 9.3 Hz, 1H), 7.44 (s, 1H), 6.70 (s, 1H), 6.44 (s, 1H), 4.14-4.03 (m, 1H), 3.89 (s, 3H), 3.68 (s, 3H), 3.36-3.26 (m, 2H), 3.06-2.99 (m, 2H), 2.96 (s, 3H), 2.34-2.24 (m, 2H), 2.17-2.05 (m, 2H), 1.86-1.76 (m, 2H).

### Example 136:

### Preparation of intermediate 136A

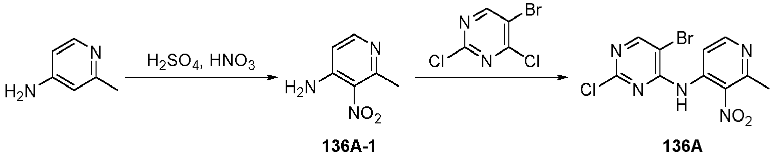

### Compound 136A-1:

The compound 2-methyl-4-aminopyridine (3 g, 27.74 mmol) was dissolved in concentrated sulfuric acid (6 mL) at 0 °C; subsequently, the reaction liquid was added dropwise with nitric acid (2.6 g, 41.26 mmol). The reaction system was heated to 65 °C and successively stirred for 2 h. After the starting material disappeared as detected by LCMS, the reaction liquid was cooled to 0 °C and adjusted to pH = 9 with aqueous sodium hydroxide solution (1 M). The mixture was extracted with a mixed solvent of ethyl acetate (50 mL × 3). The organic phases were combined, washed with saturated brine (50 mL × 2) firstly, then dried over anhydrous sodium sulfate, filtered, and finally concentrated under reduced pressure. The resulting residue was purified by silica gel column chromatography (eluent: dichloromethane/ethyl acetate = 1/1) to obtain compound **136A-1** (1.8 g). MS (ESI) M/Z: 154.1 [M + H]⁺.

### Intermediate 136A:

Compound **136A** (1.2 g) was prepared by replacing the starting material with compound **136A-1** (600 mg, 3.92 mmol) according to the method for the preparation of compound **1B** from compound **1B-2** in Example 1.

MS (ESI) M/Z: 343.9, 345.9 [M + H]⁺.

### Preparation of compound 136

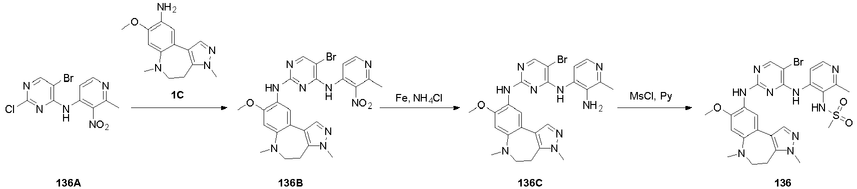

### Compound 136B:

Compound **136B** (40 mg) was prepared by replacing the starting material with compound **136A** (200 mg, 0.58 mmol) according to the method for the preparation of compound **1** from compound **1B** and compound **1C** in Example 1.

MS (ESI, m/z): 566.1, 568.1 [M + H]⁺.

### Compound 136C:

Compound **136C** (20 mg) was prepared by replacing the starting material with compound **136B** (40 mg, 0.07 mmol) according to the method for the preparation of compound **1C** from compound **1C-9** in Example 1.

MS (ESI, m/z): 536.4, 538.4 [M + H]⁺.

### Compound 136:

Compound **136** (6 mg) was prepared by replacing the starting material with compound **136C** (20 mg, 0.04 mmol) according to the method for the preparation of compound **32** from compound **32C** in Example 32.

MS (ESI, m/z): 614.1, 616.1 [M + H]⁺.

¹H NMR (400 MHz, CDCl₃) δ 8.95 (s, 1H), 8.57 (s, 1H), 8.28 (s, 1H), 8.13-8.00 (m, 1H), 7.61-7.41 (m, 2H), 7.15-7.00 (m, 1H), 6.57 (s, 1H), 3.89 (s, 6H), 3.67-3.60 (m, 2H), 3.29 (s, 3H), 3.15-2.96 (m, 5H), 2.80 (s, 3H).

### Example 137:

### Preparation of intermediate 137A

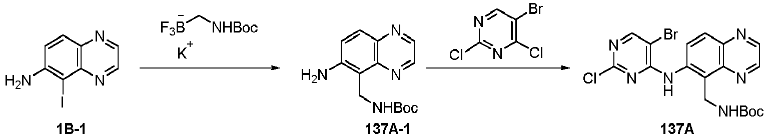

### Compound 137A-1:

Compound **137A-1** (1 g) was prepared by replacing the starting material with compound **1B-1** (1.5 g, 5.53 mmol) and potassium (((tert-butoxycarbonyl)amino)methyl)trifluoroborate (1.44 g, 6.09 mmol) according to the method for the preparation of compound **120A-3** from compound **120A-2** in Example 120.

MS (ESI, m/z): 275.2 [M + H]⁺.

### Intermediate 137A:

Compound **137A** (800 mg) was prepared by replacing the starting material with compound **137A-1 (520** mg, 1.9 mmol) according to the method for the preparation of compound **1B** from compound **1B-2** in Example 1.

MS (ESI) M/Z: 465.1, 467.1 [M + H]⁺.

### Preparation of compound 137

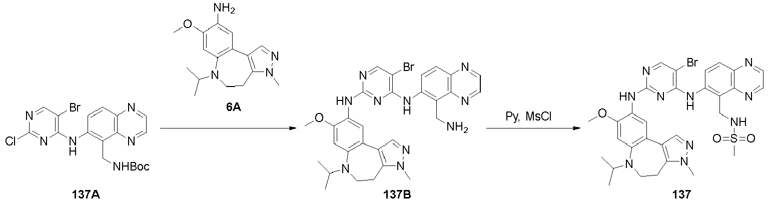

### Compound 137B:

Compound **137B** (20 mg) was prepared by replacing the starting material with compound **137A** (250 mg, 0.54 mmol) and compound **6A** (154 mg, 0.54 mmol) according to the method for the preparation of compound **1** from compound **1B** and compound **1C** in Example 1.

MS (ESI, m/z): 615.2, 617.2 [M + H]⁺.

### Compound 137:

Compound **137** (2 mg) was prepared by replacing the starting material with compound **137B** (20 mg, 0.03 mmol) according to the method for the preparation of compound **32** from compound **32C** in Example 32.

MS (ESI, m/z): 693.1, 695.1 [M + H]⁺.

¹H NMR (300 MHz, CDCl₃) δ 8.89-8.78 (m, 2H), 8.43 (s, 1H), 8.25 (s, 2H), 8.11 (d, *J* = 9.3 Hz, 1H), 7.65 (d, *J* = 9.3 Hz, 1H), 7.57-7.42 (m, 1H), 6.51 (s, 2H), 5.58 (t, *J* = 6.6 Hz, 1H), 4.96 (d, *J* = 6.3 Hz, 2H), 3.88 (s, 4H), 3.64 (s, 3H), 3.33-3.17 (m, 2H), 2.94 (s, 3H), 2.91-2.83 (m, 2H), 1.29 (s, 6H).

### Example 138:

### Preparation of compound 138

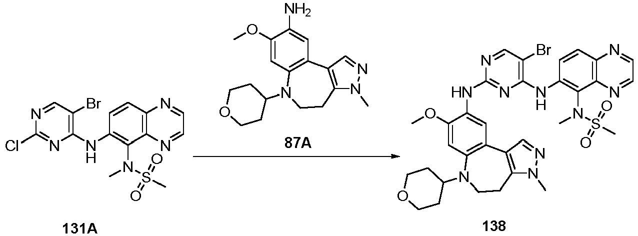

Compound **138** (14 mg) was prepared by replacing the starting material with compound **131A** (70 mg, 0.16 mmol) and compound **87A** (52 mg, 0.16 mmol) according to the method for the preparation of compound **1** from compound **1B** and compound **1C** in Example 1.

MS (ESI, m/z): 735.1, 737.1 [M + H]⁺.

¹H NMR (300 MHz, DMSO-*d₆*) δ 8.93 (d, *J* = 2.1 Hz, 1H), 8.87 (d, *J* = 2.1 Hz, 1H), 8.80-8.72 (m, 1H), 8.51 (s, 1H), 8.46 (s, 1H), 8.30 (s, 1H), 7.76 (s, 1H), 7.62-7.50 (m, 1H), 7.38 (s, 1H), 6.64 (s, 1H), 3.97-3.93 (m, 2H), 3.77 (s, 3H), 3.73-3.71 (m, 1H), 3.69 (s, 3H), 3.53-3.45 (m, 2H), 3.39 (s, 3H), 3.30-3.27 (m, 2H), 3.22 (s, 3H), 2.95-2.92 (m, 2H), 1.88-1.82 (m, 4H).

### Example 139:

### Preparation of compound 139

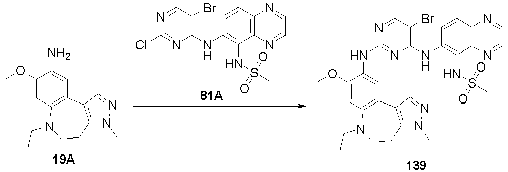

Compound **139** (4 mg) was prepared by replacing the starting material with compound **81A** (99 mg, 0.23 mmol) and compound **19A** (63 mg, 0.23 mmol) according to the method for the preparation of compound **1** from compound **1B** and compound **1C** in Example 1.

MS (ESI, m/z): 665.2, 667.2 [M + H]⁺.

¹H NMR (400 MHz, CDCl₃) δ 9.10 (s, 1H), 8.82-8.78 (m, 2H), 8.58 (d, *J* = 9.2 Hz, 1H), 8.32-8.26 (m, 2H), 7.56 (s, 2H), 7.33 (s, 1H), 6.73 (s, 1H), 6.51 (s, 1H), 3.87 (s, 3H), 3.66 (s, 3H), 3.38-3.25 (m, 4H), 3.00-2.93 (m, 5H), 1.28 (t, *J* = 6.8Hz, 3H).

### Biological Assay Evaluation:

### (I) In vitro enzymatic experiment for EGFR WT and EGFR L858R/T790M/C797S

In the assay, the inhibition effect of the compound on the kinase activity of EGFR WT (wild type EGFR) and EGFR L858R/T790M/C797S was tested by adopting a time-resolved fluorescence resonance energy transfer (TR-FRET) method, and half maximal inhibitory concentration IC₅₀ of the compound on the kinase activity of EGFR was obtained.

### 1. Materials

EGFR recombinase and EGFR L858R/T790M/C797S recombinase, purchased from Signalchem. HTRF KinEASE-TK kit, purchased from Cisbio.
DTT, MnCl₂, and MgCl₂, purchased from Sigma.
ATP, purchased from Promega.
Brigatinib, purchased from Selleck.

### 2. Method

1) 1× working solution was prepared: 5 mM MgCl₂; 1 mM DTT; 1 mM MnCl₂ and 1 × kinase buffer (in kit).
2) 10 µL of gradiently-diluted compound was transferred to a 384-well plate using Echo 550 (Labcyte).
3) 5 µL of 2× EGFR WT or EGFR L858R/T790M/C797S recombinase solution was added to the 384-well plate, and the plate was incubated at room temperature for 10 min.
4) 5 µL of 2× TK-substrate-biotin substrate solution (containing ATP) was added to the 384-well plate, and the plate was incubated at room temperature for 40 min.
5) 5 µL of assay solution containing Sa-XL 665 HTRF and 5 µL of TK-antibody-Cryptatewas were added to the plate, and the plate was incubated at room temperature for 1 h.
6) The fluorescence signal values at 615 nm and 665 nm were measured for each well using an Envision microplate reader (PerkinElmer).
7) The ratio of fluorescence signal values at 665 nm and 615 nm was calculated for each well.
8) Data analysis was performed using GraphPad Prism software to give the IC₅₀ for compounds. The results of inhibition on kinase activity of wild-type EGFR and L858R/T790M/C797S EGFR triple-mutation are shown in Table 1.

The IC₅₀ data of the compounds disclosed herein for inhibiting the enzymatic activity of each EGFR are shown in Table 1. In above compounds, a compound with IC₅₀ below 1 nM was identified with A; a compound with IC₅₀ between 1-10 nM was identified with B; a compound with IC₅₀ between 10-50 nM was identified with C; a compound with IC₅₀ between 50-100 nM was identified with D; a compound with IC₅₀ between 100-1000 nM was identified with E; a compound with IC₅₀ greater than 1000 nM was identified with F.

As can be seen from Table 1, the compounds disclosed herein have good inhibition effect on kinase activity of L858R/T790M/C797S EGFR triple-mutation, and weak inhibition effect on kinase activity of wild type EGFR (EGFR (WT)), which indicates that the compounds disclosed herein have good kinase activity and selectivity.

**Table 1 Results of enzymatic inhibition**

| **Compound** | **EGFR (WT) IC₅₀ (nM)** | **EGFR (L858R/T790M/C797S) IC₅₀ (nM)** |
|---|---|---|
| 1 | A | A |
| 2 | B | A |
| 3 | B | A |
| 4 | B | A |
| 5 | A | A |
| 6 | B | A |
| 7 | B | A |
| 8 | B | A |
| 9 | C | A |
| 10 | A | A |
| 11 | B | A |
| 12 | B | A |
| 13 | A | A |
| 14 | B | A |
| 15 | C | A |
| 16 | B | A |
| 17 | B | B |
| 18 | D | B |
| 19 | B | A |
| 20 | B | A |
| 21 | B | A |
| 22 | C | B |
| 23 | C | B |
| 24 | B | A |
| 25 | B | A |
| 26 | C | A |
| 27 | B | A |
| 28 | B | A |
| 29 | C | A |
| 30 | C | A |
| 31 | B | A |
| 32 | A | A |
| 33 | B | A |
| 34 | D | B |
| 35 | A | A |
| 36 | B | A |
| 37 | B | A |
| 38 | C | A |
| 39 | C | A |
| 40 | C | A |
| 41 | B | A |
| 42 | B | A |
| 43 | B | A |
| 44 | B | A |
| 45 | B | A |
| 46 | C | A |
| 47 | B | A |
| 48 | C | A |
| 49 | C | A |
| 50 | A | A |
| 51 | C | A |
| 52 | B | A |
| 53 | A | A |
| 54 | B | A |
| 55 | C | A |
| 56 | B | A |
| 57 | D | B |
| 58 | C | A |
| 59 | A | A |
| 60 | B | A |
| 61 | B | A |
| 62 | A | A |
| 63 | A | A |
| 64 | A | A |
| 65 | B | A |
| 66A | B | A |
| 66B | B | A |
| 67 | A | A |
| 68 | A | A |
| 69 | A | A |
| 70 | A | A |
| 71 | B | A |
| 72 | B | A |
| 73 | C | A |
| 74 | A | A |
| 75 | B | A |
| 76 | A | A |
| 77A | B | A |
| 77B | E | B |
| 78 | B | A |
| 79 | C | A |
| 80 | B | A |
| 81 | B | A |
| 82 | B | B |
| 83 | B | A |
| 84 | A | A |
| 85 | A | A |
| 86 | B | B |
| 87 | B | A |
| 88 | A | A |
| 89 | A | A |
| 90 | A | A |
| 91 | A | A |
| 92 | B | A |
| 93 | A | A |
| 94 | B | A |
| 95 | B | A |
| 96 | B | A |
| 97 | B | A |
| 98 | B | A |
| 99 | D | B |
| 100 | B | A |
| 101 | B | A |
| 102 | C | A |
| 103 | B | A |
| 104 | B | A |
| 105 | B | A |
| 106 | B | A |
| 107 | A | A |
| 108 | C | A |
| 109 | B | A |
| 110 | B | A |
| 111 | B | A |
| 112 | B | A |
| 113 | B | A |
| 114 | A | A |
| 115 | F | B |
| 116 | D | B |
| 117A | A | A |
| 117B | A | A |
| 118 | C | B |
| 119 | C | B |
| 120 | C | B |
| 121 | E | B |
| 122 | D | B |
| 123 | B | A |
| 124 | F | F |
| 125 | F | E |
| 126 | B | A |
| 127 | F | F |
| 128 | B | A |
| 129 | B | A |
| 130 | B | A |
| 131 | B | A |
| 132 | C | A |
| 133 | A | A |
| 134 | C | B |
| 135 | B | B |
| 136 | B | A |
| 137 | B | A |
| 138 | B | A |
| 139 | A | A |

### (II) Cell proliferation inhibition assay

### A431 cell proliferation inhibition assay

In the assay, the inhibition effect of the compounds on the proliferation of A431 cells was tested by adopting a CellTiter-Glo method, and the concentration IC₅₀ of the compound for inhibiting half maximal growth of cells was obtained.

### 1. Materials

A431 cells, purchased from ATCC.
DMEM medium, fetal bovine serum (FBS), and Penicillin-Streptomycin, purchased from GIBCO. Brigatinib, purchased from Selleck.
CellTiter-Glo reagent, purchased from Promega.

### 2. Method

1) A431 cells were seeded in a 384-well plate at a density of 800 cells/well with 30 µL/well, and the plate was cultured in a cell incubator for 24 h (37 °C, 5% CO₂).
2) Day 0: 30 µL of gradiently-diluted compounds to be tested with a final concentration of DMSO of 0.1% was added to plate using Echo, and the plate was incubated in a cell incubator for 72 h (37 °C, 5% CO₂). 30 µL of DMSO was added into each well in blank control group.
3) Day 3: 30 µL Cell Titer-Glo reagent was added to each well, and the plate was in the dark under room temperature for 30 min.
4) The chemiluminescent signal was detected by an Envision plate reader (PerkinElmer).
5) Data analysis was performed using GraphPad Prism software to give the IC₅₀ for compounds.

### Ba/F3_L858R/T790M/C797S cell proliferation inhibition assay

In the assay, the inhibition effect of the compounds on the proliferation of Ba/F3_L858R/T790M/C797S cells was tested by adopting a CellTiter-Glo method, and the concentration IC₅₀ of the compound for inhibiting half maximal growth of cells was obtained.

### 1. Materials

Ba/F3_L858R/T790M/C797S cells were constructed by Pharmaron Beijing Co., Ltd.
1640 medium, fetal bovine serum (FBS), Penicillin-Streptomyces, and GlutaMAX-I
Supplement, purchased from GIBCO.
Brigatinib, purchased from Selleck.
CellTiter-Glo reagent, purchased from Promega.

### 2. Method

1) Ba/F3_L858R/T790M/C797S cells were seeded in a 384-well plate at a density of 700 cells/well with 30 µL/well.
2) Day 0: 30 µL of gradiently-diluted compounds to be tested with a final concentration of DMSO of 0.1% was added to plate using Echo, and the plate was incubated in a cell incubator for 72 h (37 °C, 5% CO₂). 30 µL of DMSO was added into each well in blank control group.
3) Day 3: 30 µL Cell Titer-Glo reagent was added to each well, and the plate was in the dark under room temperature for 30 min.
4) The chemiluminescent signal was detected by an Envision plate reader (PerkinElmer).
5) Data analysis was performed using GraphPad Prism software to give the IC₅₀ for compounds.

### Ba/F3_Del19/T790M/C797S cell proliferation inhibition assay

In the assay, the inhibition effect of the compounds on the proliferation of Ba/F3_Del19/T790M/C797S cells was tested by adopting a CellTiter-Glo method, and the concentration IC₅₀ of the compound for inhibiting half maximal growth of cells was obtained.

### 1. Materials

Ba/F3_Del19/T790M/C797S, purchased from Kyinno Biotechnology Co., Ltd.
1640 medium, fetal bovine serum (FBS), Penicillin-Streptomyces, and GlutaMAX-I Supplement, purchased from GIBCO.
Brigatinib, purchased from Selleck.
CellTiter-Glo reagent, purchased from Promega.

### 2. Method

1) Ba/F3_Del19/T790M/C797S cells were seeded in a 384-well plate at a density of 700 cells/well with 30 µL/well.
2) Day 0: 30 µL of gradiently-diluted compounds to be tested with a final concentration of DMSO of 0.1% was added to plate using Echo, and the plate was incubated in a cell incubator for 72 h (37 °C, 5% CO₂). 30 µL of DMSO was added into each well in blank control group.
3) Day 3: 30 µL Cell Titer-Glo reagent was added to each well, and the plate was in the dark under room temperature for 30 min.
4) The chemiluminescent signal was detected by an Envision plate reader (PerkinElmer).

Data analysis was performed using GraphPad Prism 6 software to give the IC₅₀ for compounds.

The IC₅₀ data of the compounds disclosed herein for inhibiting the cell viability of each EGFR are shown in Table 2. In above compounds, a compound with IC₅₀ below 10 nM was identified with A; a compound with IC₅₀ between 10-50 nM was identified with B; a compound with IC₅₀ between 50-100 nM was identified with C; a compound with IC₅₀ between 100-1000 nM was identified with D; a compound with IC₅₀ greater than 1000 nM was identified with E.

The results of the inhibition of cell viability are shown in Table 2.

As can be seen from the experimental results in Table 2, compared with control example Brigatinib, the compounds disclosed herein have better inhibition effect on cell proliferation of a Ba/F3 Del19/T790M/C797S EGFR triple-mutation cell line and a Ba/F3L 858R/T790M/C797S EGFR triple-mutation cell line, and weaker inhibition effect on EGFR wild type (EGFR WT) cell line A431, which indicates that the compounds disclosed herein have better cell viability and selectivity.

**Table 2 Results of cell proliferation inhibition assay data**

| **Compound** | **A431 EGFR WT IC₅₀(nM)** | **Ba/F3 (Del19/T790M/C797S) IC₅₀(nM)** | **Ba/F3 (L858R/T790M/C797S) IC₅₀(nM)** |
|---|---|---|---|
| 1 | C | A | A |
| 2 | C | A | A |
| 3 | D | A | A |
| 4 | D | A | A |
| 5 | C | A | A |
| 6 | D | A | A |
| 7 | D | A | A |
| 8 | C | A | A |
| 9 | D | B | B |
| 10 | B | A | A |
| 11 | D | A | A |
| 12 | D | A | A |
| 13 | C | A | A |
| 14 | D | A | A |
| 15 | D | A | B |
| 16 | C | A | A |
| 17 | D | B | C |
| 18 | E | C | D |
| 19 | D | A | B |
| 20 | D | A | A |
| 21 | D | A | A |
| 22 | E | B | D |
| 23 | D | B | C |
| 24 | D | A | A |
| 25 | D | A | B |
| 26 | D | A | A |
| 27 | D | A | A |
| 28 | D | A | A |
| 29 | D | B | B |
| 30 | D | B | C |
| 31 | D | A | B |
| 32 | D | A | A |
| 33 | C | A | A |
| 34 | E | B | B |
| 35 | D | A | B |
| 36 | D | A | A |
| 37 | D | A | B |
| 38 | D | B | C |
| 39 | D | B | B |
| 40 | D | A | B |
| 41 | D | A | A |
| 42 | D | A | A |
| 43 | C | A | A |
| 44 | D | A | B |
| 45 | D | A | B |
| 46 | D | B | B |
| 47 | C | A | B |
| 48 | D | A | B |
| 49 | D | B | B |
| 50 | D | B | B |
| 51 | D | A | B |
| 52 | D | A | A |
| 53 | D | A | A |
| 54 | D | A | B |
| 55 | D | B | D |
| 56 | D | A | B |
| 57 | D | B | C |
| 58 | E | B | B |
| 59 | D | B | B |
| 60 | B | A | A |
| 61 | B | A | A |
| 62 | B | A | A |
| 63 | B | A | A |
| 64 | D | A | B |
| 65 | D | A | B |
| 66A | D | A | B |
| 66B | D | A | B |
| 67 | C | A | A |
| 68 | C | A | A |
| 69 | D | A | A |
| 70 | B | A | A |
| 71 | C | A | A |
| 72 | C | A | B |
| 73 | D | B | B |
| 74 | D | A | A |
| 75 | D | B | C |
| 76 | C | A | A |
| 77A | D | A | B |
| 77B | D | C | C |
| 78 | D | A | B |
| 79 | D | B | B |
| 80 | D | A | A |
| 81 | D | A | A |
| 82 | E | B | B |
| 83 | D | A | B |
| 84 | B | A | A |
| 85 | D | A | A |
| 86 | D | A | B |
| 87 | D | A | A |
| 88 | C | A | A |
| 89 | B | B | B |
| 90 | B | A | A |
| 91 | D | A | B |
| 92 | D | A | B |
| 93 | B | A | B |
| 94 | D | B | B |
| 95 | D | B | C |
| 96 | D | A | A |
| 97 | D | A | B |
| 98 | D | B | B |
| 99 | D | B | B |
| 100 | D | B | B |
| 101 | D | A | A |
| 102 | E | C | D |
| 103 | D | A | A |
| 104 | D | A | A |
| 105 | D | A | B |
| 106 | D | A | B |
| 107 | D | A | A |
| 108 | D | A | B |
| 109 | D | B | B |
| 110 | D | B | C |
| 111 | D | A | B |
| 112 | D | A | B |
| 113 | D | A | B |
| 114 | D | A | A |
| 115 | E | A | B |
| 116 | D | B | C |
| 117A | B | A | A |
| 117B | C | A | B |
| 118 | D | B | B |
| 119 | D | B | B |
| 120 | D | B | B |
| 121 | D | A | A |
| 122 | D | B | B |
| 123 | D | A | A |
| 124 | E | B | B |
| 125 | D | A | B |
| 126 | E | D | D |
| 127 | D | D | D |
| 128 | E | A | A |
| 129 | D | B | B |
| 130 | D | A | A |
| 131 | E | B | B |
| 132 | E | B | B |
| 133 | D | A | A |
| 134 | D | A | B |
| 135 | D | A | A |
| 136 | E | D | D |
| 137 | D | B | B |
| 138 | D | A | A |
| 139 | D | A | A |

### (III) In vivo efficacy study experiment

### 1. Purpose

The anti-tumor activity and toxic and side effects of compound 53 and compound 27 administered orally for 21 consecutive days on PC9 (Del19/T790M/C797S) were evaluated.

### 2. Materials

SPF-grade and female BALB/c-nu mice, purchased from Beijing Vital River Laboratory Animal Technology Co., Ltd.

PC9 (Del19/T790M/C797S) cells, self-constructed by Qilu Pharmaceutical Co.,Ltd.

### 3. Procedures

### 3.1 Cell culturing

PC9 (Del19/T790M/C797S) cells were cultured in RPMI 1640 medium containing 10% FBS at 37 °C in a 5% carbon dioxide incubator; and the cells in exponential growth phase were collected for inoculation.

### 3.2 Cell inoculation

Under sterile conditions, a cell suspension of *in vitro* cultured PC9 (Del19/T790M/C797S) was taken, centrifuged to adjust the cell concentration to 3 × 10⁷ cells/mL, and inoculated in mice at the right side armpit (0.1 mL/mouse), and the day of inoculation was defined as day 0.

### 3.3 Tumor grouping, administration and measurement

a. When the mean tumor volume was about 150 mm³, 35 mice with moderate tumor volume were selected for enrollment and randomized into 5 groups according to tumor volume: G1: vehicle control group, G2: compound 53 (15 mg/kg), G3: compound 53 (60 mg/kg), G4: compound 27 (15 mg/kg) and G5: compound 27 (35 mg/kg), 7 mice/group.
b. The animals were grouped and then started to be administrated, and the volume for oral administration (po) was 10 mL/kg; the weighing was performed once daily with consecutively 21-day administration; the tumor diameters were measured 2 times a week.
c. Tumor volume (TV): tumor volumes were measured 2 times a week to observe tumor mass volume changes and growth rate. Tumor volume V = 1/2 × a × b², where a and b represent tumor long diameter and short diameter, respectively. The growth inhibition effect of the compounds on tumor tissues is evaluated by using a tumor growth inhibition TGI (%). TGI (%) = [1- (average tumor volume of a certain administration group - average tumor volume at the day of grouping in the administration group)/(average tumor volume of negative control group - average tumor volume on the day of grouping in the negative control group)] × 100%. The data of the administration group and the negative control group were obtained on the same day.
d. The mice's living status, including appearance signs, general behavioral activities, mental status, food consumption, respiratory status, fecal and urinary traits, injection site conditions and other toxic manifestations, was closely observed during the course of the experiment.
e. After the endpoint was reached, the mice were euthanized, and animal carcasses were frozen and stored in a freezer and transferred to qualified medical waste treatment organization for disposal.

### 4 Results

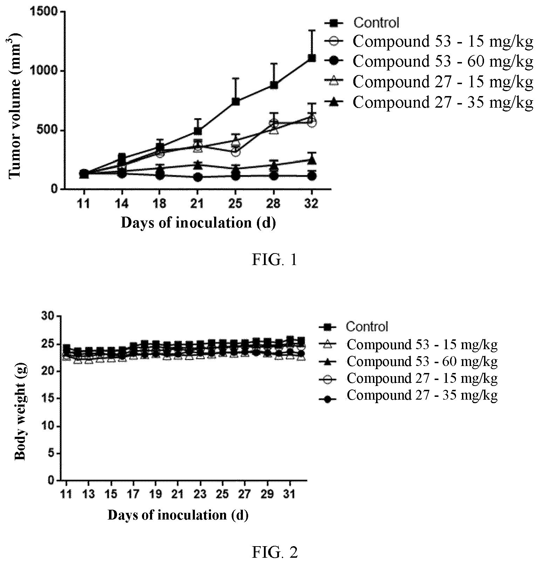

The results are shown in Table 3 and FIGs. 1 and 2.

**Table 3 Results of in vivo efficacy study**

| **Grouping** | **Dose (mg/kg)** | **Tumor volume ^{a} (mm³)** | **TGI (%)** | **Body weight change (%)** |
|---|---|---|---|---|
| G1: vehicle control group | - | 1110±235.7 | - | 5.3 |
| G2: compound 53 | 15 | 565.8±82.0 | 55.9 | 0 |
| G3: compound 53 | 60 | 117.0±16.1^{∗∗} | 102.0 | 6.8 |
| G4: compound 27 | 15 | 617.6±110.7 | 50.6 | 7.0 |
| G5: compound 27 | 35 | 253.0±58.5^{∗∗} | 88.1 | -1.7 |

| | | | | |
|---|---|---|---|---|
| a, mean ± SEM (standard error); b, p value was statistically analyzed based on tumor volume, and compared with group G1, ^{∗}P < 0.05 and ^{∗∗}P < 0.01 in the above other groups. | | | | |

### 5. Conclusion

From the above results, it can be seen that both compound 53 and compound 27 can significantly inhibit tumor growth, and show a significant dose-effect relationship, and the mice are well tolerated.

## Claims

1. A compound represented by formula (I‴), 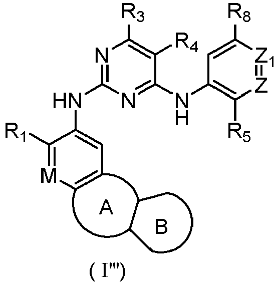
or a stereoisomer, a tautomer or a pharmaceutically acceptable salt, a prodrug, a hydrate, a solvate and an isotopically labeled derivative thereof,
wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₂₋₆ alkynyloxy, C₁₋₆ alkylamino, C₁₋₆ haloalkylamino, C₃₋₆ cycloalkylamino, 3-6 membered heterocycloalkylamino, C₂₋₆ alkenylamino, and C₂₋₆ alkynylamino;
M is selected from N and CRₐ;
Z is selected from N and CR₆;
Z₁ is selected from N and CR₇;
Rₐ is H, halogen, C₁₋₆ alkyl, C₃₋₆ cycloalkyl, C₁₋₆ heteroalkyl, or C₁₋₆ haloalkyl;
or, Rₐ cyclizes with R₁ to form a substituted or unsubstituted 5-8 membered heterocyclyl or 5-8 membered carbocyclyl;
ring A is selected from the substituted or unsubstituted 5-8 membered heterocyclyl or 5-8 membered carbocyclyl;
ring B is absent or selected from aryl or 5-6 membered heteroaryl, and 4-8 membered heterocycloalkyl or C₄₋₈ cycloalkyl, which are optionally substituted with one or more R₂;
R₂ is each independently selected from H, halogen, -CN, -C(=O)R_{b}, -C(=O)NR_{b}R_{c}, -S(=O)₂R_{b}, - S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₅₋₆ aryl, C₅₋₆ arylalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, C₂₋₆ alkenylamino, and -(CH₂)ᵣNR_{c}R_{d}, r is optionally selected from 0, 1, 2, and 3; wherein the C₃₋₆ cycloalkyl, the 3-6 membered heterocycloalkyl, the C₅₋₆ aryl, the C₅₋₆ arylalkyl, the C₃₋₆ cycloalkyloxy, the 3-6 membered heterocycloalkyloxy, the C₂₋₆ alkenyloxy, the C₁₋₆ alkylamino, the C₁₋₆ haloalkylamino, the C₃₋₆ cycloalkylamino, the 3-6 membered heterocycloalkylamino or the C₂₋₆ alkenylamino in R₂ is optionally substituted with one or more C₁₋₃ alkyl or C₁₋₃ alkoxy;
R₃ and R₄ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl, 3-8 membered heterocycloalkyl, C₁₋₆ alkylamino, C₁₋₆ haloalkylamino, C₃₋₆ cycloalkylamino, 3-6 membered heterocycloalkylamino, and C₂₋₆ alkenylamino;
or, R₃ cyclizes with R₄ to form aryl, C₄₋₇ cycloalkyl, 5-7 membered heterocycloalkyl, or 5-6 membered heteroaryl;
R₅ is selected from substituted or unsubstituted -NH₂, -C(=O)NR_{b}R_{c}, -S(=O)₂R_{b}, -P(=O)R_{b}R_{c}, - P(=O)R_{b}NR_{c}R_{d}, -P(=O)R_{b}OR_{c}, -P(=O)OR_{b}OR_{c}, -P(=S)R_{b}R_{c}, -P(=S)R_{b}NR_{c}R_{d}, -P(=S)R_{b}OR_{c}, - P(=S)OR_{b}OR_{c}, -S(=O)₂NR_{b}R_{c}, R_{b}S(=O)₂NR_{c}-, -N=S(=O)R_{b}R_{c} or R_{b}N=S(=O)R_{c}-, -NR_{b}C(O)R_{c}, and R_{c}S(=NR_{b})(=O)NR_{d}-;
R_{b}, R_{c} and R_{d} are each independently selected from H, -CN, C₁₋₃ alkyl, C₁₋₃ haloalkyl, C₃₋₆ cycloalkyl, C₄₋₆ heterocycloalkyl, C₅₋₁₀ aryl, and 5-10 membered heteroaryl;
or, R_{b} and R_{c} cyclizes with atoms to which they are both attached to form 5-6 membered heterocycloalkyl unsubstituted or optionally substituted with one or more C₁₋₃ alkyl or C₁₋₃ alkoxy;
R₆, R₇ and R₈ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ haloalkylamino, C₃₋₆ cycloalkylamino, 3-6 membered heterocycloalkylamino, and C₂₋₆ alkenylamino;
or, R₅ cyclizes with R₆ to form a 4-7 membered ring containing -P(=O)(R_{b})-, -P(=S)(R_{b})-, - N(R_{b})S(=O)₂-, -S(=O)₂N(R_{b})-, or -S(=O)₂;
or, R₆ cyclizes with R₇ to form C₄₋₆ cycloalkyl, 4-6 membered heterocycloalkyl, aryl, and 5-6 membered heteroaryl;
or, R₇ cyclizes with R₈ to form C₄₋₆ cycloalkyl, 4-6 membered heterocycloalkyl, aryl, and 5-6 membered heteroaryl.

2. The compound represented by formula (I‴) as claimed in claim 1, wherein R₁ is selected from H, halogen, -CN, C₁₋₆ alkyl, C₁₋₆ alkoxy, C₁₋₆ haloalkyl, and C₁₋₆ haloalkoxy; preferably, R₁ is selected from H, halogen, -CN, C₁₋₆ alkyl, and C₁₋₆ alkoxy.

3. The compound represented by formula (I‴) as claimed in claim 1, wherein M is selected from N or CRₐ, wherein Rₐ is H, halogen, C₁₋₃ alkyl, C₃₋₆ cycloalkyl or C₁₋₃ haloalkyl; preferably, M is selected from N and CH.

4. The compound represented by formula (I‴) as claimed in claim 1, wherein ring A is selected from a substituted or unsubstituted 5-8 membered carbocyclyl and a 5-8 membered heterocyclyl containing 1 or 2 heteroatoms selected from O, S and N;
preferably, ring A may comprise a double bond; or
preferably, 1 or 2 ring atoms on ring A may be optionally replaced with -C(=O), -N(=O), -S(=O), and -S(=O)₂, ring A may also be optionally substituted with one or more Rₓ groups, wherein the Rₓ is selected from H, -OH, -CN, -NH₂, halogen, C₁₋₆ alkylcarbonyl, C₁₋₆ alkyl, C₁₋₆ haloalkyl, C₃₋₈ cycloalkyl, 3-8 membered heterocycloalkyl, C₃₋₈ cycloalkyl-C₁₋₆ alkyl-, 3-8 membered heterocycloalkyl-C₁₋₆ alkyl-, aryl-C₁₋₆ alkyl-, C₅₋₁₃ spirocyclyl, and 5-13 membered spiroheterocyclyl; wherein the C₃₋₈ cycloalkyl, the 3-8 membered heterocycloalkyl, the C₃₋₈ cycloalkyl-C₁₋₆ alkyl-, the 3-8 membered heterocycloalkyl-C₁₋₆ alkyl-, the aryl-C₁₋₆ alkyl-, C₅₋₁₃ spirocyclyl, and the 5-13 membered spirocyclyl membered spiroheterocyclyl are optionally substituted with one or more R_{y}; wherein, the R_{y} is selected from H, halogen, C₁₋₆ alkyl, C₁₋₆ haloalkyl, C₁₋₆ alkoxy, C₃₋₈ cycloalkyl, 4-8 membered heterocycloalkyl, C₃₋₈ cycloalkyl-C₁₋₆ alkyl-, 4-8 membered heterocycloalkyl-C₁₋₆ alkyl-, 5-10 membered aryl, and 5-10 membered heteroaryl.

5. The compound represented by formula (I‴) as claimed in claim 1, wherein ring B is aryl or 5-6 membered heteroaryl, optionally substituted with one or more R₂; the aryl and the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, pyridazinyl, or triazinyl.

6. The compound represented by formula (I‴) as claimed in claim 1, wherein R₂ is each independently selected from H, halogen, -CN, -C(=O)R_{b}, -S(=O)₂R_{b}, -S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₅₋₆ aryl, C₅₋₆ arylalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, C₂₋₆ alkenylamino, and -(CH₂)ᵣNR_{c}R_{d}, wherein r is optionally selected from 0, 1, 2, and 3; wherein the 3-6 membered heterocycloalkyl and the C₅₋₆ arylalkyl in R₂ is optionally substituted with one or more C₁₋₃ alkyl or C₁₋₃ alkoxy.

7. The compound represented by formula (I‴) as claimed in claim 1, wherein R₃ and R₄ are each independently selected from H, halogen, -CN, C₁₋₆ alkyl, C₁₋₆ alkoxy, C₁₋₆ haloalkyl, C₁₋₆ haloalkoxy, and C₃₋₆ cycloalkyl;
or, R₃ cyclizes with R₄ to form phenyl, C₄₋₇ cycloalkyl, and 5-7 membered heterocycloalkyl or 5-6 membered heteroaryl containing 1 or 2 heteroatoms selected from O, S, and N; preferably, the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, pyridazinyl, or triazinyl.

8. The compound represented by formula (I‴) as claimed in claim 1, wherein R₅ is selected from substituted or unsubstituted -NH₂, -C(=O)NR_{b}R_{c}, -S(=O)₂R_{b}, -P(=O)R_{b}R_{c}, -P(=O)R_{b}NR_{c}R_{d}, - P(=O)R_{b}OR_{c}, -P(=O)OR_{b}OR_{c}, -P(=S)R_{b}R_{c}, -P(=S)R_{b}NR_{c}R_{d}, -P(=S)R_{b}OR_{c}, -P(=S)OR_{b}OR_{c}, - S(=O)₂NR_{b}R_{c}, R_{b}S(=O)₂NR_{c}-, -N=S(=O)R_{b}R_{c} or R_{b}N=S(=O)(R_{c})-, and -NR_{b}C(O)R_{c};
or, R₅ cyclizes with R₆ to form a 4-7 membered ring containing -P(=O)(R_{b})-, -P(=S)(R_{b})-, - N(R_{b})S(=O)₂-, -S(=O)₂N(R_{b})-, or -S(=O)₂.

9. The compound represented by formula (I‴) as claimed in claim 1, wherein R_{b}, R_{c}, and R_{d} are each independently selected from H, C₁₋₃ alkyl, C₁₋₃ haloalkyl, and C₃₋₆ cycloalkyl;
or, R_{b} and R_{c} cyclizes with atoms to which they are both attached to form 5-6 membered heterocycloalkyl unsubstituted or optionally substituted with one or more C₁₋₃ alkyl or C₁₋₃ alkoxy.

10. The compound represented by formula (I‴) as claimed in claim 1, wherein R₆ and R₇ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ alkylamino, C₁₋₆ haloalkylamino, C₃₋₆ cycloalkylamino, 3-6 membered heterocycloalkylamino, and C₂₋₆ alkenylamino;
or, R₆ cyclizes with R₇ to form C₄₋₆ cycloalkyl, 4-6 membered heterocycloalkyl, phenyl, and 5-6 membered heteroaryl; preferably, the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, pyridazinyl, or triazinyl.

11. The compound represented by formula (I‴) as claimed in claim 1, wherein R₈ is H.

12. A compound represented by formula (I"), 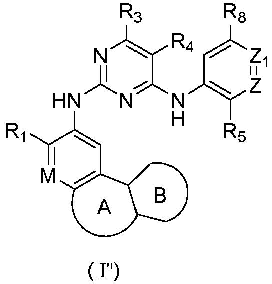
or a stereoisomer, a tautomer or a pharmaceutically acceptable salt, a prodrug, a hydrate, a solvate and an isotopically labeled derivative thereof,
wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₂₋₆ alkynyloxy, C₁₋₆ alkylamino, C₁₋₆ haloalkylamino, C₃₋₆ cycloalkylamino, 3-6 membered heterocycloalkylamino, C₂₋₆ alkenylamino, and C₂₋₆ alkynylamino;
M is selected from N and CRₐ; Rₐ is H, halogen, C₁₋₃ alkyl, C₃₋₆ cycloalkyl or C₁₋₃ haloalkyl;
Z is selected from N and CR₆;
Z₁ is selected from N and CR₇;
or, Rₐ cyclizes with R₁ to form a substituted or unsubstituted 5-8 membered heterocyclyl;
ring A is selected from the substituted or unsubstituted 5-8 membered heterocyclyl or 5-8 membered carbocyclyl;
ring B is aryl or 5-6 membered heteroaryl, optionally substituted with one or more R₂; the aryl and the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, pyridazinyl, or triazinyl;
R₂ is each independently selected from H, halogen, -CN, -C(=O)R_{b}, -S(=O)₂R_{b}, -S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₅₋₆ aryl, C₅₋₆ arylalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, C₂₋₆ alkenylamino, and -(CH₂)ᵣNR_{c}R_{d}, wherein r is optionally selected from 0, 1, 2, and 3;
R₃ and R₄ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl, 3-8 membered heterocycloalkyl, C₁₋₆ alkylamino, C₁₋₆ haloalkylamino, C₃₋₆ cycloalkylamino, 3-6 membered heterocycloalkylamino, and C₂₋₆ alkenylamino;
or, R₃ cyclizes with R₄ to form aryl, C₄₋₇ cycloalkyl, 5-7 membered heterocycloalkyl, or 5-6 membered heteroaryl;
R₅ is selected from substituted or unsubstituted -NH₂, -C(=O)NR_{b}R_{c}, -S(=O)₂R_{b}, -P(=O)R_{b}R_{c}, - P(=O)R_{b}NR_{c}R_{d}, -P(=O)R_{b}OR_{c}, -P(=O)OR_{b}OR_{c}, -P(=S)R_{b}R_{c}, -P(=S)R_{b}NR_{c}R_{d}, -P(=S)R_{b}OR_{c}, - P(=S)OR_{b}OR_{c}, -S(=O)₂NR_{b}R_{c}, R_{b}S(=O)₂NR_{c}-, -N=S(=O)R_{b}R_{c} or R_{b}N=S(=O)(R_{c})-, and - NR_{b}C(O)R_{c};
R_{b}, R_{c} and R_{d} are each independently selected from H, -CN, C₁₋₃ alkyl, C₁₋₃ haloalkyl, C₃₋₆ cycloalkyl, C₄₋₆ heterocycloalkyl, C₅₋₁₀ aryl, and 5-10 membered heteroaryl;
or, R_{b} and R_{c} cyclizes with atoms to which they are both attached to form 5-6 membered heterocycloalkyl;
R₆, R₇ and R₈ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ alkylamino, C₁₋₆ haloalkylamino, C₃₋₆ cycloalkylamino, 3-6 membered heterocycloalkylamino, and C₂₋₆ alkenylamino;
or, R₅ cyclizes with R₆ to form a 4-7 membered ring containing -P(=O)(R_{b})-, -P(=S)(R_{b})-, - N(R_{b})S(=O)₂-, -S(=O)₂N(R_{b})-, or -S(=O)₂;
or, R₆ cyclizes with R₇ to form C₄₋₆ cycloalkyl, 4-6 membered heterocycloalkyl, aryl, and 5-6 membered heteroaryl;
or, R₇ cyclizes with R₈ to form C₄₋₆ cycloalkyl, 4-6 membered heterocycloalkyl, aryl, and 5-6 membered heteroaryl.

13. A compound represented by formula (I'), 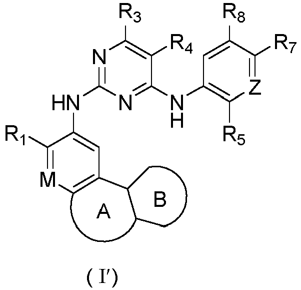
or a stereoisomer, a tautomer or a pharmaceutically acceptable salt, a prodrug, a hydrate, a solvate and an isotopically labeled derivative thereof,
wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₂₋₆ alkynyloxy, C₁₋₆ alkylamino, C₁₋₆ haloalkylamino, C₃₋₆ cycloalkylamino, 3-6 membered heterocycloalkylamino, C₂₋₆ alkenylamino, and C₂₋₆ alkynylamino;
M is selected from N and CRₐ;
Z is selected from N and CR₆;
Rₐ is H, halogen, C₁₋₃ alkyl, C₃₋₆ cycloalkyl or C₁₋₃ haloalkyl;
or, Rₐ cyclizes with R₁ to form a substituted or unsubstituted 5-8 membered heterocyclyl;
ring A is selected from the substituted or unsubstituted 5-8 membered heterocyclyl or 5-8 membered carbocyclyl;
ring B is aryl or 5-6 membered heteroaryl, optionally substituted with one or more R₂; the aryl and the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl;
R₂ each is independently selected from H, halogen, -CN, -C(=O)R_{b}, -S(=O)₂R_{b}, -S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₅₋₆ aryl, C₅₋₆ arylalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, and C₂₋₆ alkenylamino;
R₃ and R₄ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl, 3-8 membered heterocycloalkyl, C₁₋₆ alkylamino, C₁₋₆ haloalkylamino, C₃₋₆ cycloalkylamino, 3-6 membered heterocycloalkylamino, and C₂₋₆ alkenylamino;
or, R₃ cyclizes with R₄ to form aryl, C₄₋₇ cycloalkyl, 5-7 membered heterocycloalkyl, or 5-6 membered heteroaryl;
R₅ is selected from substituted or unsubstituted -NH₂, -C(=O)NR_{b}R_{c}, -S(=O)₂R_{b}, -P(=O)R_{b}R_{c}, - P(=O)R_{b}NR_{c}R_{d}, -P(=O)R_{b}OR_{c}, -P(=O)OR_{b}OR_{c}, -P(=S)R_{b}R_{c}, -P(=S)R_{b}NR_{c}R_{d}, -P(=S)R_{b}OR_{c}, - P(=S)OR_{b}OR_{c}, -S(=O)₂NR_{b}R_{c}, R_{b}S(=O)₂NR_{c}-, -N=S(=O)R_{b}R_{c} and R_{b}N=S(=O)R_{c}-;
R_{b}, R_{c} and R_{d} are each independently selected from H, -CN, C₁₋₃ alkyl, C₁₋₃ haloalkyl, C₃₋₆ cycloalkyl, C₄₋₆ heterocycloalkyl, C₅₋₁₀ aryl, and 5-10 membered heteroaryl;
R₆, R₇ and R₈ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ haloalkylamino, C₃₋₆ cycloalkylamino, 3-6 membered heterocycloalkylamino, and C₂₋₆ alkenylamino;
or, R₅ cyclizes with R₆ to form a 4-7 membered ring containing P(=O)R_{b};
or, R₆ cyclizes with R₇ to form C₄₋₆ cycloalkyl, 4-6 membered heterocycloalkyl, aryl, and 5-6 membered heteroaryl;
or, R₇ cyclizes with R₈ to form C₄₋₆ cycloalkyl, 4-6 membered heterocycloalkyl, aryl, and 5-6 membered heteroaryl.

14. A compound represented by formula (I), 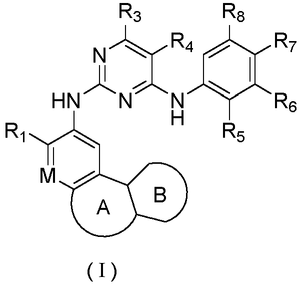
or a stereoisomer, a tautomer or a pharmaceutically acceptable salt, a prodrug, a hydrate, a solvate and an isotopically labeled derivative thereof,
wherein,
R₁ is selected from H, halogen, -CN, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy, C₂₋₆ alkenyloxy, C₁₋₆ alkylamino, C₁₋₆ haloalkylamino, C₃₋₆ cycloalkylamino, 3-6 membered heterocycloalkylamino, and C₂₋₆ alkenylamino;
M is selected from N and CRₐ;
Rₐ is H, halogen, C₁₋₃ alkyl, C₃₋₆ cycloalkyl or C₁₋₃ haloalkyl;
or, Rₐ cyclizes with R₁ to form a substituted or unsubstituted 5-8 membered heterocyclyl;
ring A is selected from substituted or unsubstituted 4-8 membered heterocyclyl and 5-8 membered carbocyclyl;
ring B is aryl or 5-6 membered heteroaryl, optionally substituted with one or more R₂; the aryl and the 5-6 membered heteroaryl may be pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl;
R₂ is each independently selected from H, halogen, -CN, -C(=O)R_{b}, -S(=O)₂R_{b}, -S(=O)(=NR_{c})R_{b}, -NH₂, -OH, -SH, C₁₋₆ alkyl, C₁₋₆ alkoxy, C₁₋₆ haloalkyl, C₁₋₆ haloalkoxy, C₃₋₆ cycloalkyl, 3-6 membered heterocycloalkyl, C₃₋₆ cycloalkyloxy, 3-6 membered heterocycloalkyloxy and C₂₋₆ alkenyloxy, C₁₋₆ alkylamino, C₁₋₆ haloalkylamino, C₃₋₆ cycloalkylamino, 3-6 membered heterocycloalkylamino, and C₂₋₆ alkenylamino;
R₃ and R₄ are each independently selected from H, halogen, -CN, C₁₋₆ alkyl, C₁₋₆ alkoxy, C₁₋₆ haloalkyl, C₁₋₆ haloalkoxy, C₃₋₈ cycloalkyl, 3-8 membered heterocycloalkyl, C₁₋₆ alkylamino, C₁₋₆ haloalkylamino, C₃₋₆ cycloalkylamino, 3-6 membered heterocycloalkylamino, and C₂₋₆ alkenylamino;
or, R₃ cyclizes with R₄ to form aryl, C₄₋₇ cycloalkyl, 5-7 membered heterocycloalkyl, or 5-6 membered heteroaryl;
R₅ is selected from substituted or unsubstituted -NH₂, -C(=O)NR_{b}R_{c}, -S(=O)₂R_{b}, -P(=O)R_{b}R_{c}, - P(=O)R_{b}NR_{c}R_{d}, -S(=O)₂NR_{b}R_{c}, R_{b}S(=O)₂NR_{c}-, -N=S(=O)R_{b}R_{c}, and R_{b}N=S(=O)R_{c}-;
R_{b}, R_{c} and R_{d} are each independently selected from H, -CN, C₁₋₃ alkyl, C₁₋₃ haloalkyl, C₃₋₆ cycloalkyl, C₄₋₆ heterocycloalkyl, C₅₋₁₀ aryl, and 5-10 membered heteroaryl;
R₆, R₇ and R₈ are each independently selected from H, halogen, -CN, -OH, C₁₋₆ alkyl, C₁₋₆ alkoxy, C₁₋₆ haloalkyl, C₁₋₆ haloalkoxy, C₂₋₆ alkenyl, C₂₋₆ alkynyl, C₃₋₆ cycloalkyl, 3-6 membered heterocycloalkyl, 5-6 membered heteroaryl, C₁₋₆ alkylamino, C₁₋₆ haloalkylamino, C₃₋₆ cycloalkylamino, 3-6 membered heterocycloalkylamino, and C₂₋₆ alkenylamino;
or, R₅ cyclizes with R₆ to form a 4-7 membered ring containing P(=O)R_{b};
or, R₆ cyclizes with R₇ to form C₄₋₆ cycloalkyl, 4-6 membered heterocycloalkyl, aryl, and 5-6 membered heteroaryl;
or, R₇ cyclizes with R₈ to form C₄₋₆ cycloalkyl, 4-6 membered heterocycloalkyl, aryl, and 5-6 membered heteroaryl.

15. The compound as claimed in any one of claims 1 to 14,
R₁ is selected from H, halogen, -CN, C₁₋₃ alkyl, C₁₋₃ alkoxy, C₁₋₃ haloalkyl, and C₁₋₃ haloalkoxy;
M is selected from N and CH;
ring A is selected from the substituted or unsubstituted 5-8 membered heterocyclyl or 5-8 membered carbocyclyl;
ring B is 5-6 membered heteroaryl optionally substituted with one or more R₂; the 5-6 membered heteroaryl is pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl;
R₂ is each independently selected from H, C₁₋₄ alkyl, C₁₋₄ haloalkyl, -(CH₂)ᵣNR_{c}R_{d}, 3-6 membered heterocycloalkyl, and C₅₋₆ arylalkyl, wherein r is optionally selected from 0, 1, 2, and 3, and the 3-6 membered heterocycloalkyl and the C₅₋₆ arylalkyl are optionally substituted with one or more C₁₋₃ alkyl or C₁₋₃ alkoxy;
R₃ and R₄ are each independently selected from H, halogen, C₁₋₄ alkyl, C₁₋₄ halogenated alkyl, and C₃₋₆ cycloalkyl;
or, R₃ cyclizes with R₄ to form 5-6 membered heteroaryl; the 5-6 membered heteroaryl is pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl;
R₅ is selected from -C(=O)NR_{b}R_{c}, -P(=O)R_{b}R_{c}, -P(=S)R_{b}R_{c}, -S(=O)₂NR_{b}R_{c}, R_{b}S(=O)₂NR_{c}-, and - NR_{b}C(O)R_{c};
R_{b}, R_{c} and R_{d} are each independently selected from H, C₁₋₃ alkyl, C₁₋₃ alkoxy, and C₃₋₆ cycloalkyl;
or, R_{b} and R_{c} cyclizes with atoms to which they are both attached to form 5-6 membered heterocycloalkyl unsubstituted or optionally substituted with one or more C₁₋₃ alkyl or C₁₋₃ alkoxy; or, R₅ cyclizes with R₆ to form a 4-7 membered ring containing -P(=O)(R_{b})-, -P(=S)(R_{b})-, - N(R_{b})S(=O)₂-, -S(=O)₂N(R_{b})-, or -S(=O)₂;
R₆, R₇ and R₈ are each independently selected from H, halogen, and C₁₋₃ alkyl;
or, R₈ is selected from H, R₆ cyclizes with R₇ to form 5-6 membered heteroaryl; the 5-6 membered heteroaryl is pyrrolyl, furanyl, thienyl, pyrazolyl, thiazolyl, oxazolyl, isothiazolyl, isoxazolyl, imidazolyl, triazolyl, phenyl, pyrimidinyl, pyridyl, pyrazinyl, or pyridazinyl.

16. The compound as claimed in any one of claims 1 to 15, wherein the M is selected from N and CH; preferably, M is CH.

17. The compound as claimed in any one of claims 1 to 16, wherein the R₁ is selected from H, halogen, -CN, C₁₋₃ alkyl, C₁₋₃ alkoxy, C₁₋₃ haloalkyl, and C₁₋₃ haloalkoxy;
preferably, wherein the R₁ is selected from H, methyl, ethyl, n-propyl, isopropyl, n-butyl, methoxy, ethoxy, n-propoxy, isopropoxy, n-butoxy, trifluoromethyl, trifluoromethoxy, trichloromethyl, trichloromethoxy, and 2,2,2-trifluoroethoxy; and
more preferably, wherein the R₁ is selected from methoxy.

18. The compound as claimed in any one of claims 1 to 17, wherein the R₂ is selected from H, methyl, ethyl, isopropyl, difluoromethyl, trifluoromethyl, -CH₂CH₂N(CH₃)CH₃, 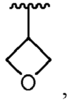 and 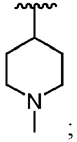 preferably, wherein the R₂ is selected from H, methyl, ethyl, isopropyl, difluoromethyl, and trifluoromethyl; and
more preferably, wherein the R₂ is selected from methyl.

19. The compound as claimed in any one of claims 1 to 18, wherein R₃ and R₄ are each independently selected from H, F, Cl, Br, CN, methyl, ethyl, isopropyl, methoxy, ethoxy, isopropoxy, difluoromethyl, trifluoromethyl, 2,2,2-trifluoroethyl, and cyclopropyl;
preferably, wherein the R₃ is selected from H, and the R₄ is each independently selected from H, F, Cl, Br, methyl, difluoromethyl, trifluoromethyl, and cyclopropyl;
more preferably, wherein the R₃ is selected from H, and the R₄ is independently selected from F, Cl, Br, methyl, ethyl, difluoromethyl, and trifluoromethyl;
more preferably, wherein the R₃ is selected from H, and R₄ is selected from Cl, Br, and methyl; still more preferably, wherein the R₃ is selected from H, and the R₄ is selected from Br;
or, wherein the R₃ cyclizes with the R₄ to form a thiophene ring and a pyrrole ring, wherein the thiophene ring and the pyrrole ring may be optionally substituted with C₁₋₄ alkyl.

20. The compound as claimed in any one of claims 1 to 19, wherein the R₅ is selected from 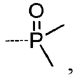 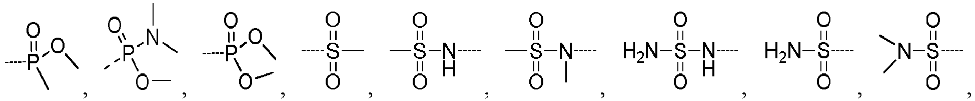 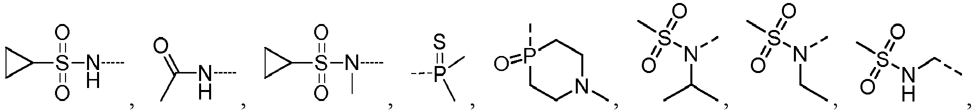 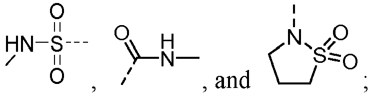 preferably, wherein the R₅ is selected from 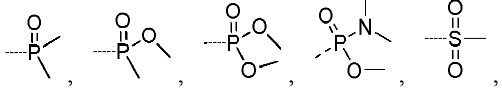 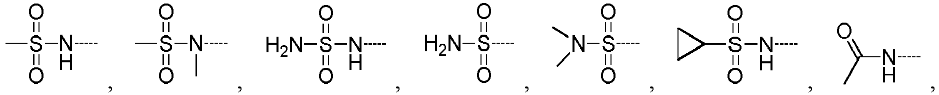 and 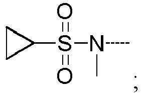 preferably, wherein the R₅ is selected from 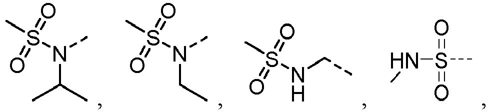 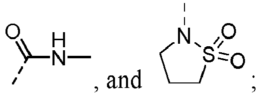 preferably, wherein the R₅ is selected from 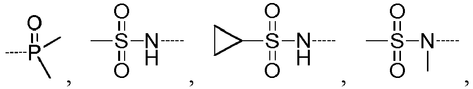 and 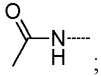 or preferably, wherein the R₅ is selected from 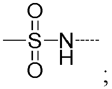 or R₅ cyclizes with R₆ to form

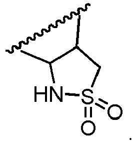

21. The compound as claimed in any one of claims 1 to 20, wherein R₆, R₇, and R₈ are each independently selected from H, methyl, and halogen; or, R₆ and R₈ are selected from H, and R₇ is selected from F; or, R₆, R₇, and R₈ are each independently selected from H and methyl, preferably H;
or, R₆ cyclizes with R₇ independently or R₇ cyclizes with R₈ independently to form cyclobutane, cyclopentane, a tetrahydropyrrole ring, a tetrahydrofuran ring, a tetrahydropyrane ring, a thiophene ring, an imidazole ring, a pyrazole ring, a pyrrole ring, an oxazole ring, a thiazole ring, an isoxazole ring, a piperazine ring, an isothiazole ring, a benzene ring, a pyridine ring, a piperidine ring, a pyrimidine ring, a pyridazine ring, and a pyrazine ring;
preferably, R₆ cyclizes with R₇ independently or R₇ cyclizes with R₈ independently to form a cyclobutane, a pyridine ring, and a pyrazine ring; and
more preferably, R₆ cyclizes with R₇ to form a pyrazine ring.

22. The compound as claimed in any one of claims 1 to 21, wherein the structural unit 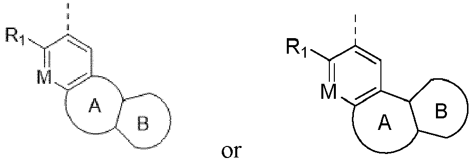 is selected from 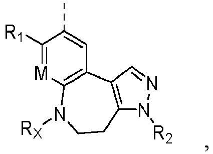 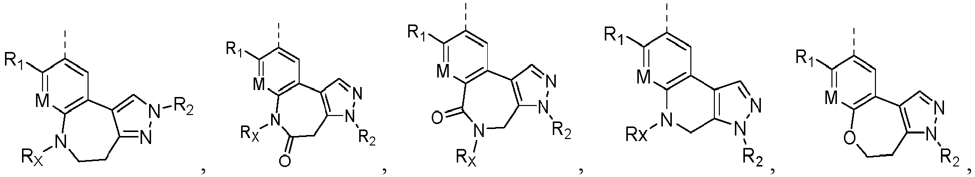 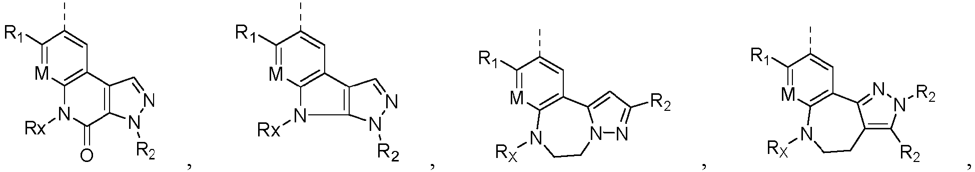 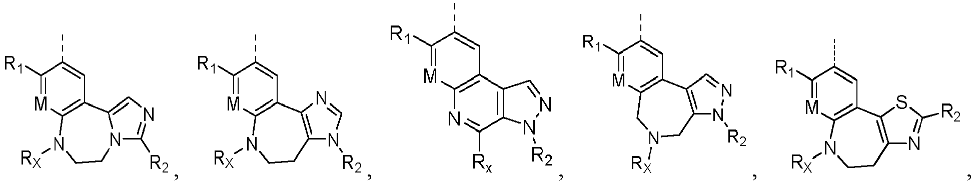 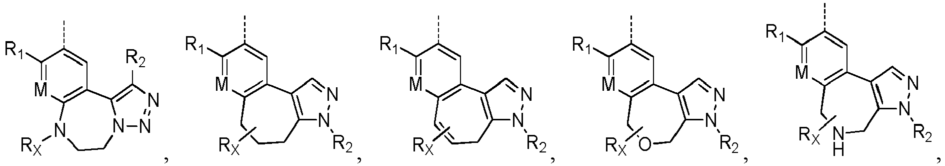 and 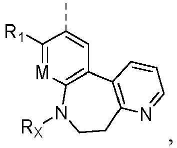
wherein, R₁ is defined as in any one of claims 1 to 21;
M is defined as in any one of claims 1 to 21;
R₂ is defined as in any one of claims 1 to 21;
Rₓ is selected from H, -OH, -CN, -NH₂, halogen, C₁₋₆ alkylcarbonyl, C₁₋₆ alkyl, C₁₋₆ haloalkyl, C₃₋₈ cycloalkyl, 3-8 membered heterocycloalkyl, C₃₋₈ cycloalkyl-C₁₋₆ alkyl-, 3-8 membered heterocycloalkyl-C₁₋₆ alkyl-, aryl-C₁₋₆ alkyl-, C₅₋₁₃ spirocyclyl, and 5-13 membered spiroheterocyclyl; wherein the C₃₋₈ cycloalkyl, the 3-8 membered heterocycloalkyl, the C₃₋₈ cycloalkyl-C₁₋₆ alkyl-, the 3-8 membered heterocycloalkyl-C₁₋₆ alkyl-, the aryl-C₁₋₆ alkyl-, C₅₋₁₃ spirocyclyl, and the 5-13 membered spirocyclyl membered spiroheterocyclyl are optionally substituted with one or more R_{y}; wherein, the R_{y} is selected from H, halogen, C₁₋₆ alkyl, C₁₋₆ haloalkyl, C₁₋₆ alkoxy, C₃₋₈ cycloalkyl, 4-8 membered heterocycloalkyl, C₃₋₈ cycloalkyl-C1-6 alkyl-, 4-8 membered heterocycloalkyl-C₁₋₆ alkyl-, 5-10 membered aryl, and 5-10 membered heteroaryl.

23. The compound as claimed in claim 22, wherein, Rₓ is selected from H, -OH, -CN, -NH₂, halogen, C₁₋₆ alkylcarbonyl, C₁₋₆ alkyl, C₁₋₆ haloalkyl, C₃₋₈ cycloalkyl, 3-8 membered heterocycloalkyl, C₃₋₈ cycloalkyl-C₁₋₆ alkyl-, and 3-8 membered heterocycloalkyl-C₁₋₆ alkyl-; wherein the C₃₋₈ cycloalkyl, 3-8 membered heterocycloalkyl, C₃₋₈ cycloalkyl-C₁₋₆ alkyl-, 3-8 membered heterocycloalkyl-C₁₋₆ alkyl-, and aryl-C₁₋₆ alkyl- are optionally substituted with one or more R_{y};
or, Rₓ is selected from H, -OH, -CN, -NH₂, halogen, C₁₋₆ alkylcarbonyl, C₁₋₆ alkyl, C₁₋₆ haloalkyl, C₃₋₆ cycloalkyl, 3-6 membered heterocycloalkyl, C₃₋₆ cycloalkyl-C₁₋₄ alkyl-, 3-6 membered heterocycloalkyl-C₁₋₄ alkyl-, aryl-C₁₋₆ alkyl-, and 7-11 membered spiroheterocyclyl, wherein the C₃₋₆ cycloalkyl, the 3-6 membered heterocycloalkyl, the C₃₋₆ cycloalkyl-C₁₋₄ alkyl-, the 3-6 membered heterocycloalkyl-C₁₋₄ alkyl-, the aryl-C₁₋₆ alkyl-, and the spiroheterocyclyl are optionally substituted with one or more R_{y};
wherein, the R_{y} is selected from H, halogen, C₁₋₆ alkyl, C₁₋₆ haloalkyl, C₁₋₆ alkoxy, C₃₋₈ cycloalkyl, 4-8 membered heterocycloalkyl, C₃₋₈ cycloalkyl-C₁₋₆ alkyl-, 4-8 membered heterocycloalkyl-C₁₋₆ alkyl-, 5-10 membered aryl, and 5-10 membered heteroaryl; preferably, the R_{y} is selected from H, halogen, C₁₋₄ alkyl, C₁₋₄ haloalkyl, C₁₋₄ alkoxy, and C₃₋₆ cycloalkyl-C₁₋₆ alkyl-; preferably, the R_{y} is selected from H, F, methyl, ethyl, isopropyl, methoxy, 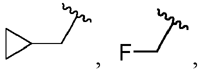 and FCH₂CH₂-;
wherein, when Rₓ is directly attached to N atom, Rₓ is not -OH, -NH₂, and halogen.

24. The compound as claimed in claim 22 or 23, wherein, Rₓ is selected from H, -OH, -CN, -NH₂, F, methyl, ethyl, isopropyl, trifluoroethyl, methylcarbonyl, 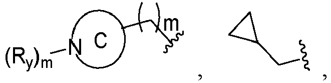 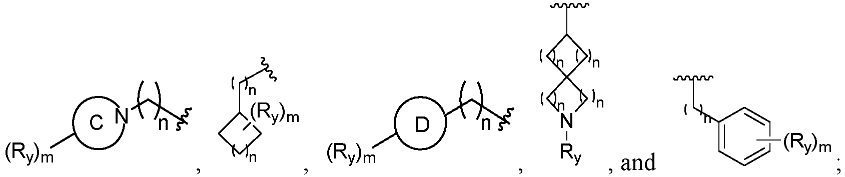 wherein, ring C is 4-8 membered heterocycloalkyl, ring D is 4-8 membered heterocycloalkyl containing oxygen; m and n are independently 0, 1, 2, or 3; R_{y} is defined as in claim 22 or 23; preferably, Rₓ is selected from H, -OH, -CN, -NH₂, methyl, ethyl, isopropyl, methylcarbonyl, 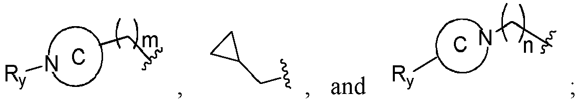 wherein, ring C is 4-8 membered heterocycloalkyl; m and n are independently 0, 1, 2, or 3; R_{y} is defined as in claim 22 or 23; preferably, Rₓ is selected from H, -OH, -CN, -NH₂, F, methyl, ethyl, isopropyl, trifluoroethyl, methylcarbonyl, 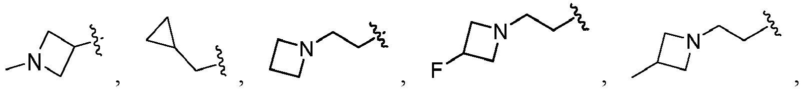 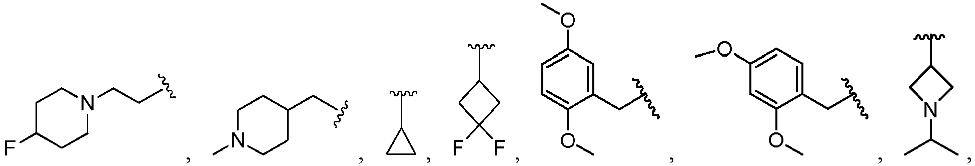 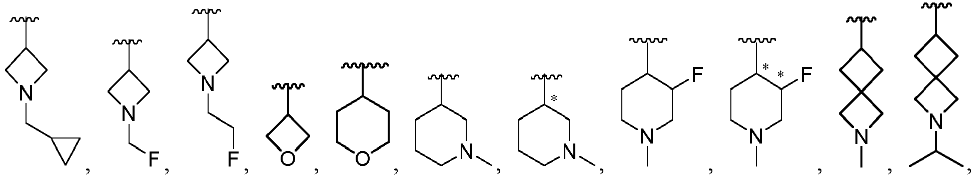 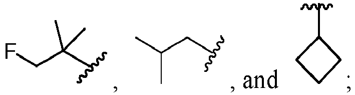 preferably, Rₓ is selected from H, methyl, ethyl, isopropyl, 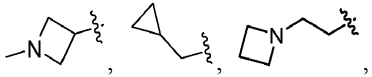 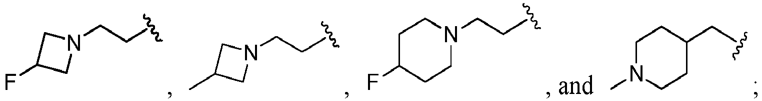 preferably, Rₓ is selected from H, methyl, ethyl, isopropyl, 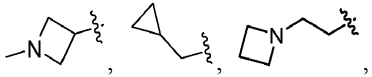 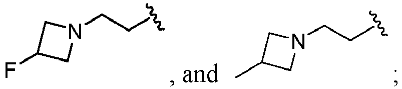 preferably, Rₓ is selected from H, methyl, ethyl, and isopropyl; preferably, Rₓ is selected from methyl and isopropyl.

25. The compound as claimed in claim 22 or 23, wherein the structural unit 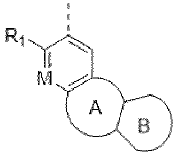 or 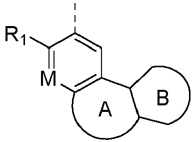 is selected from 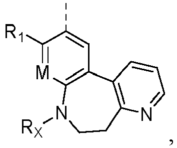 and R₁, M, and Rₓ are defined as in claim 22 or 23; or the structural unit 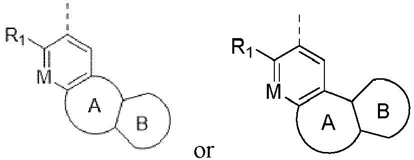 is selected from 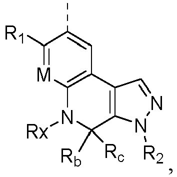 wherein R₁, M, Rₓ, R_{b}, R_{c}, and R₂ are defined as in claim 22 or 23; or the structural unit 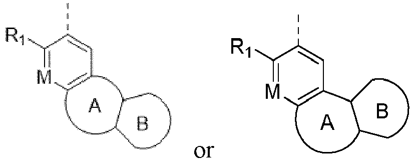 is selected from 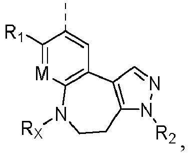 wherein, M, R₁, R₂, and Rₓ are defined as claim 22 or 23.

26. The compound as claimed in any one of claims 1 to 25, which is selected from 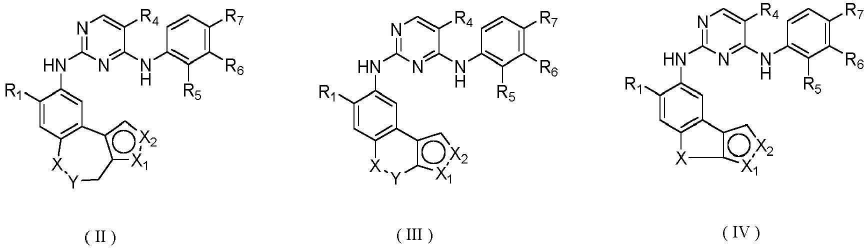 and 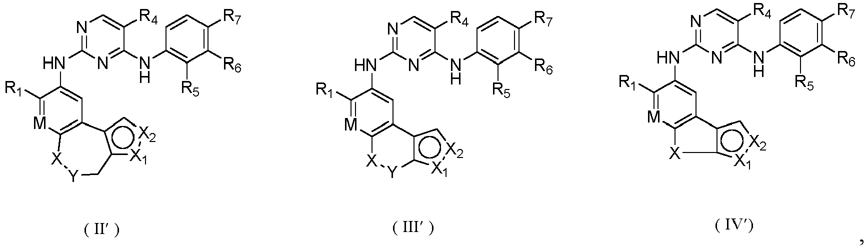 wherein,
X and Y are each independently selected from -C(=O)-, -C=C-, -NRₓ-, -O-, -CR₉R₁₀-, -S(=O)-, and -S(=O)₂-;
X₁ and X₂ are each independently selected from N and NR₂;
R₉ and R₁₀ are selected from H, halogen, C₁₋₆ alkyl, C₁₋₆ alkoxy, C₁₋₆ haloalkyl, C₁₋₆ haloalkoxy,
C₃₋₈ cycloalkyl and 3-8 membered heterocycloalkyl;
R₁ is defined as in any one of claims 1 to 25;
R₂ is defined as in any one of claims 1 to 25;
R₄ is defined as in any one of claims 1 to 25;
R₅ is defined as in any one of claims 1 to 25;
R₆ and R₇ are defined as in any one of claims 1 to 25;
Rₓ is defined as in any one of claims 22 to 25;
M, if present, is defined as in any one of claims 1 to 25.

27. The compound as claimed in claim 26, which is selected from 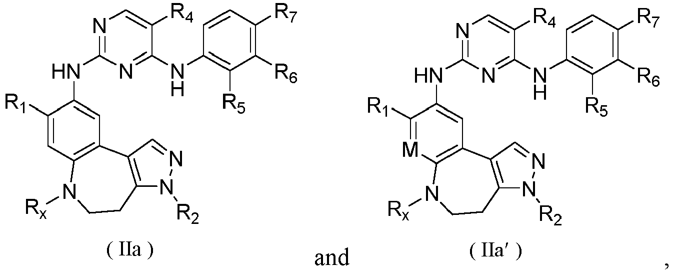 wherein, R₁, R₂, R₄, R₅, R₆, R₇, and Rₓ are defined as in claim 26; M, if present, is defined as in claim 26.

28. The compound as claimed in claim 27, which is selected from 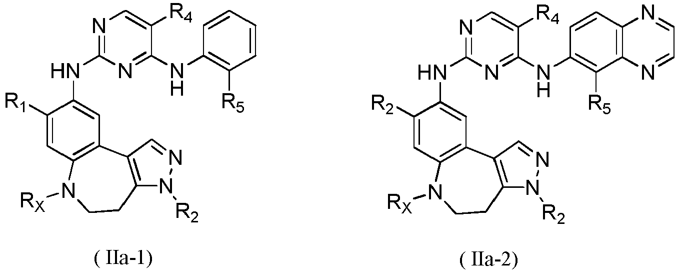 or 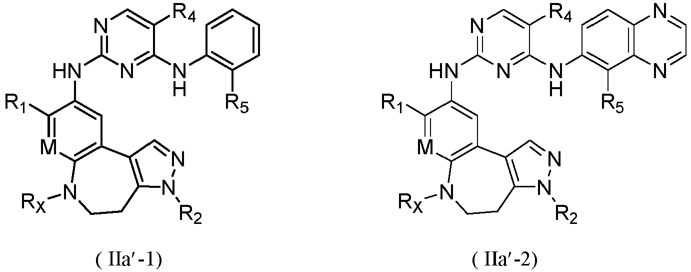 wherein, R₁, R₂, R₄, R₅, and Rₓ are defined as in claim 27; M, if present, is defined as in claim 27.

29. The compound as claimed in claim 28, which is selected from 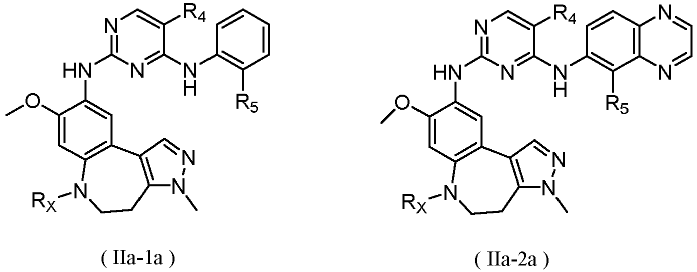 or 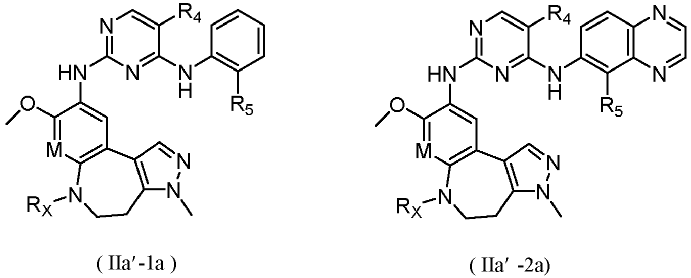 wherein, R₄, R₅, and Rₓ are defined as in claim 28; M, if present, is defined as in claim 28.

30. The compound as claimed in claim 29, which is selected from 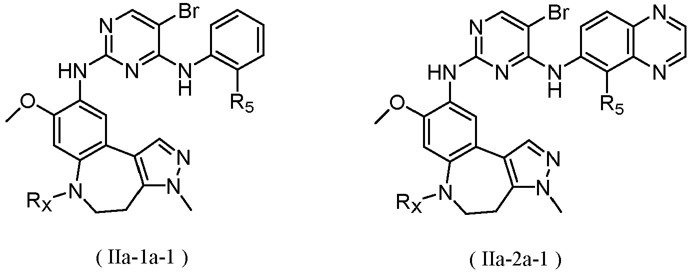 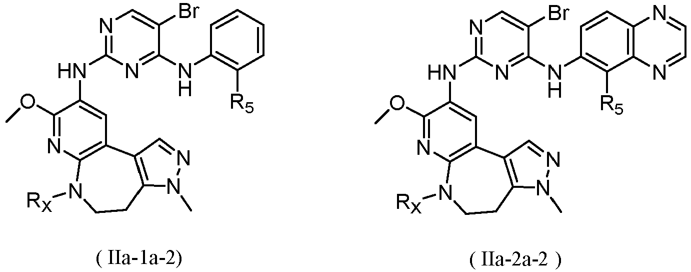 wherein, R₅ and Rₓ are as defined in claim 29.

31. The compound, or the stereoisomer, the tautomer or the pharmaceutically acceptable salt, the prodrug, the hydrate, the solvate and the isotopically labeled derivative thereof as claimed in any one of claims 1 to 30, which is selected from

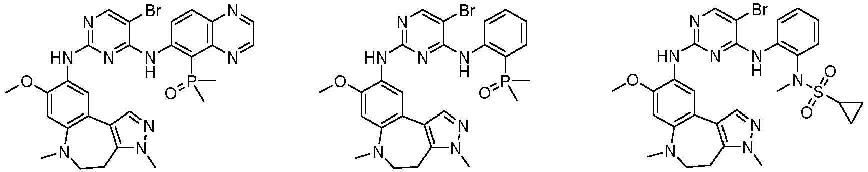

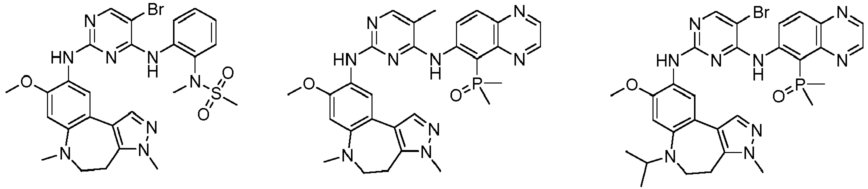

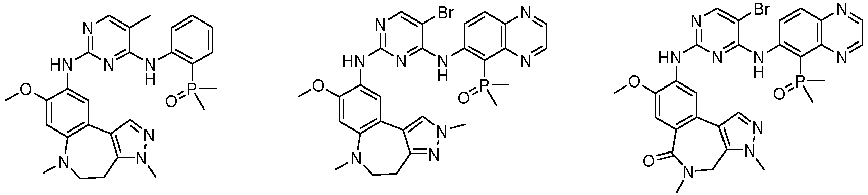

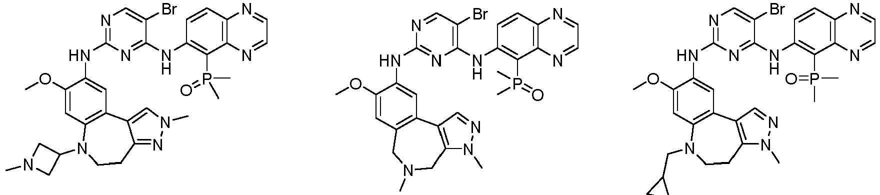

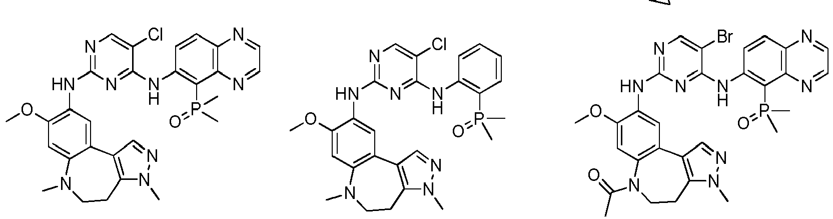

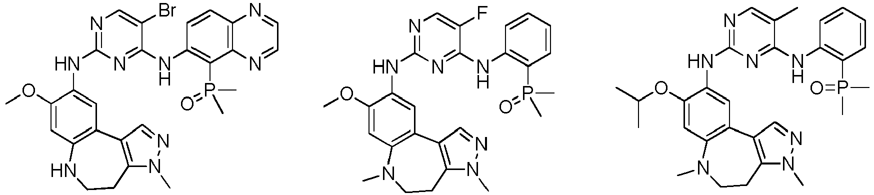

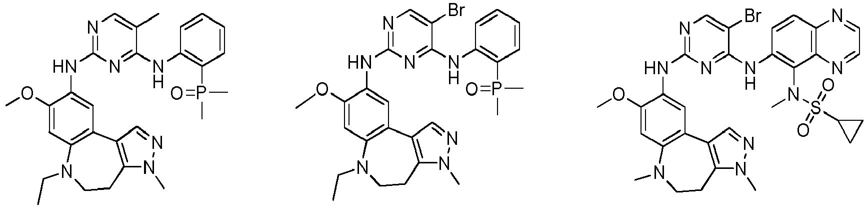

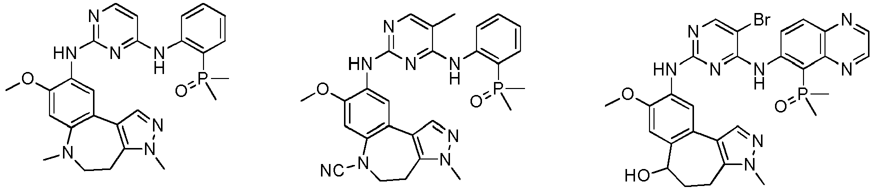

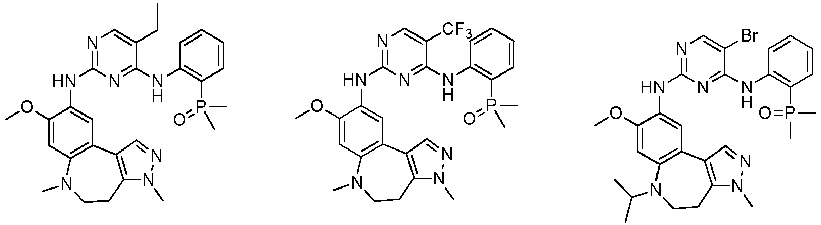

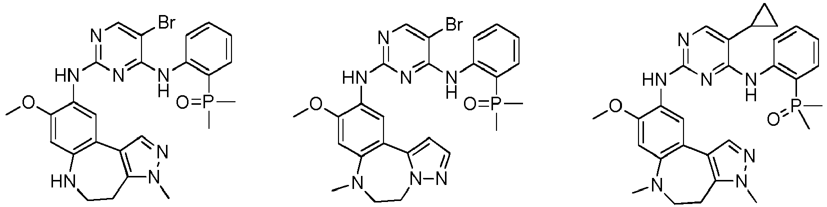

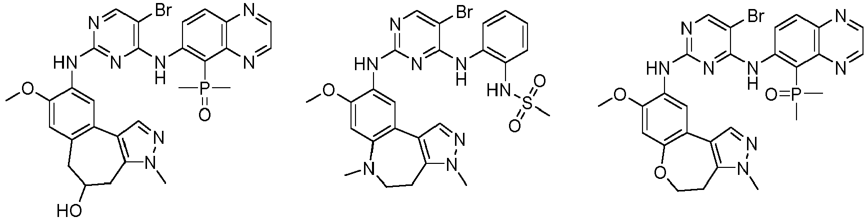

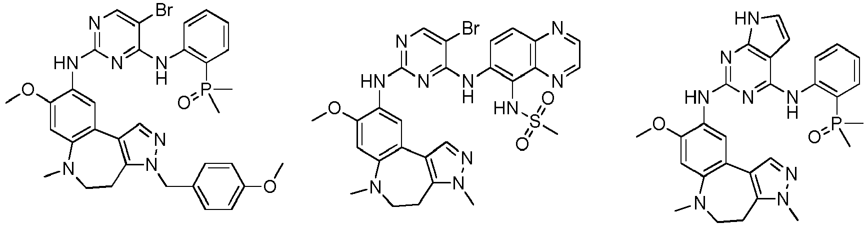

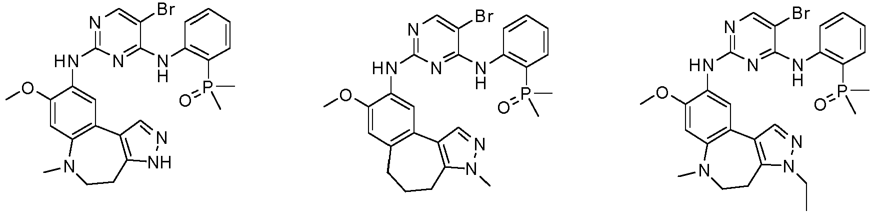

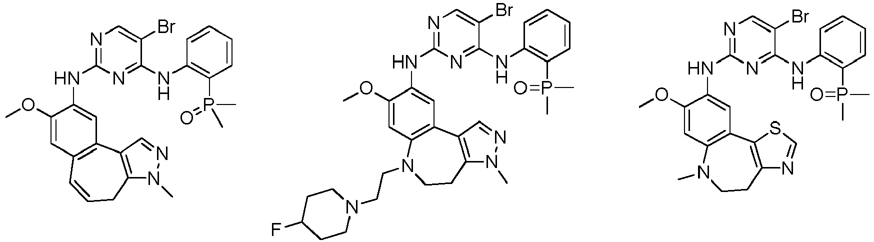

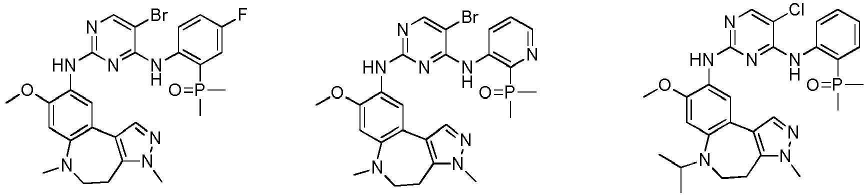

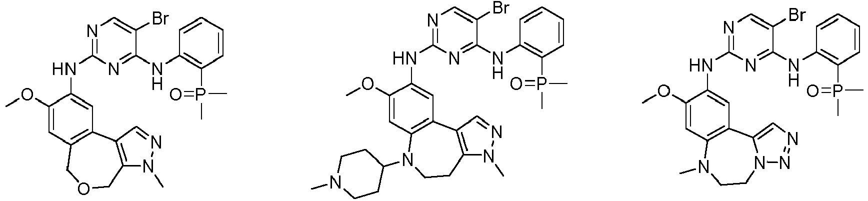

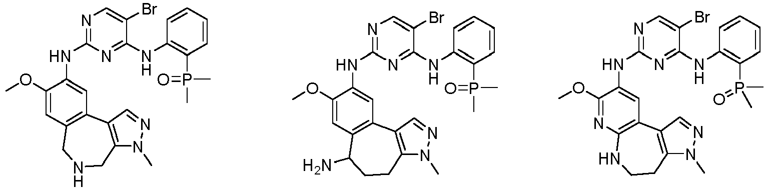

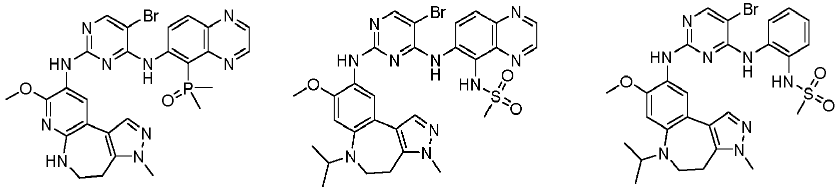

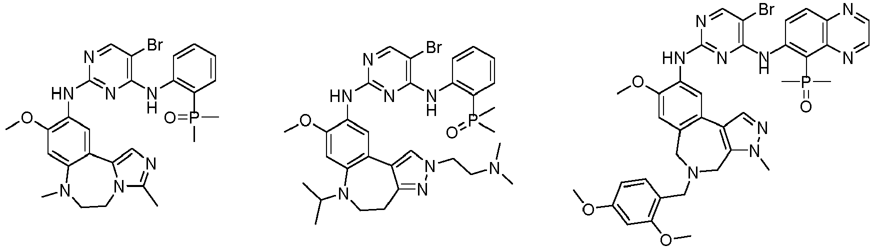

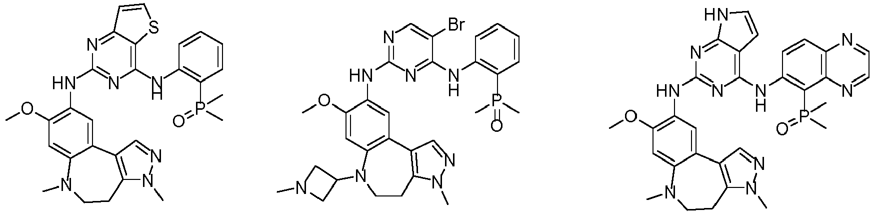

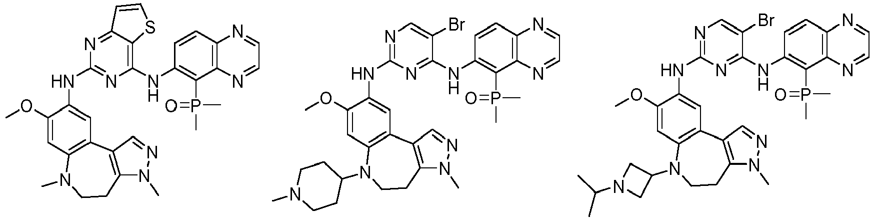

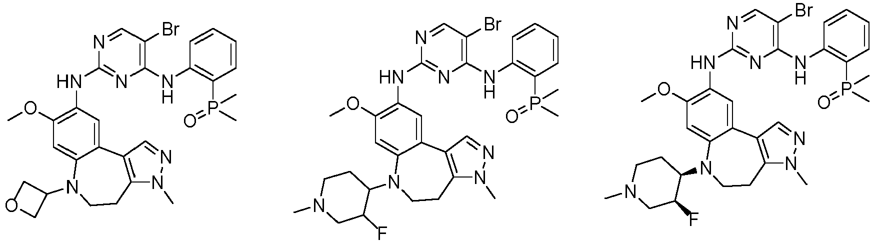

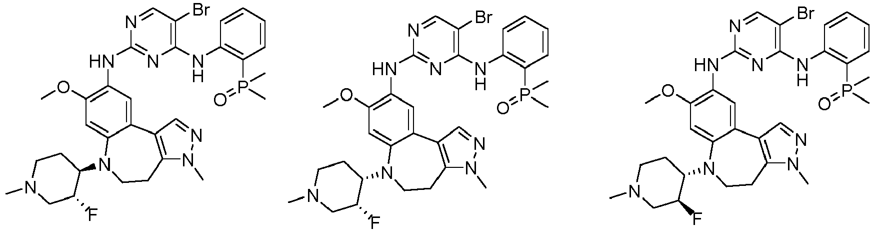

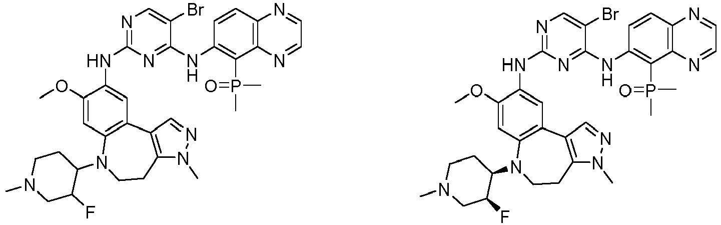

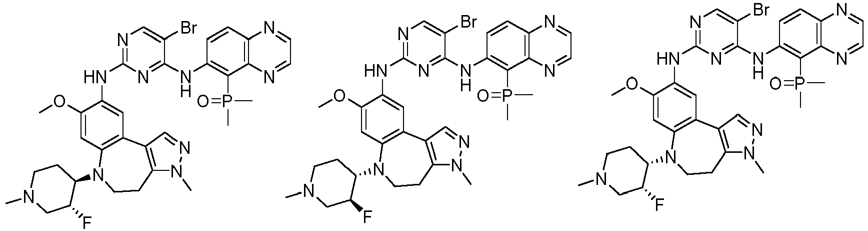

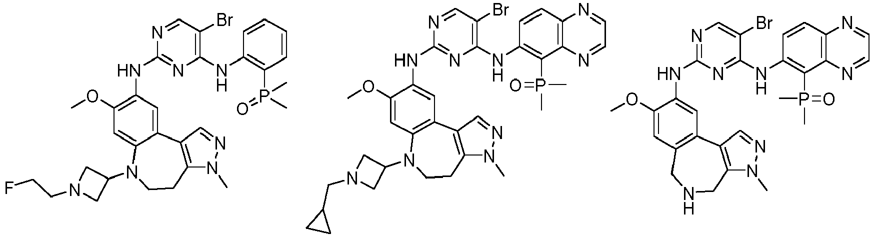

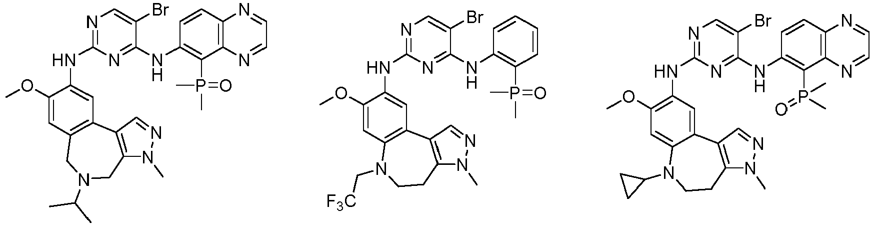

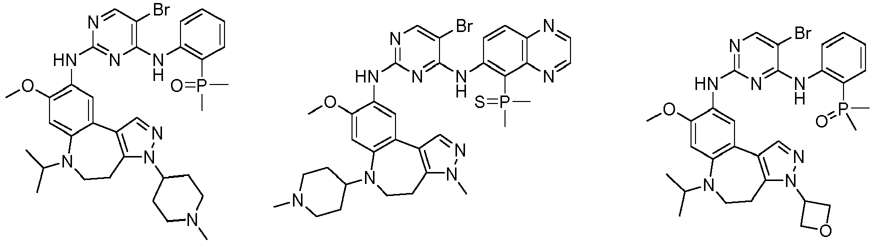

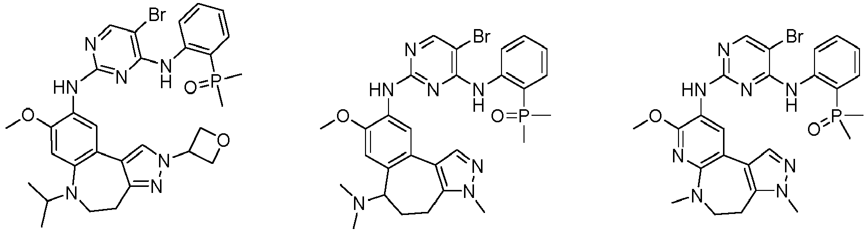

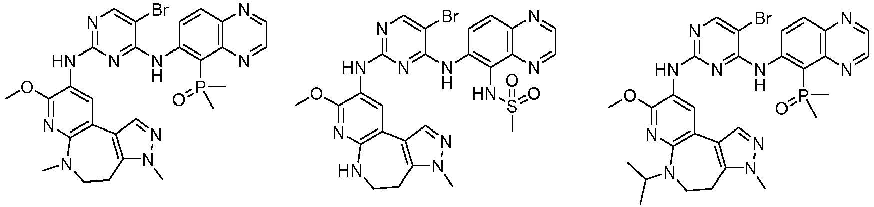

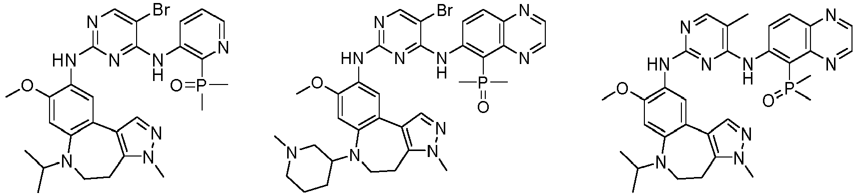

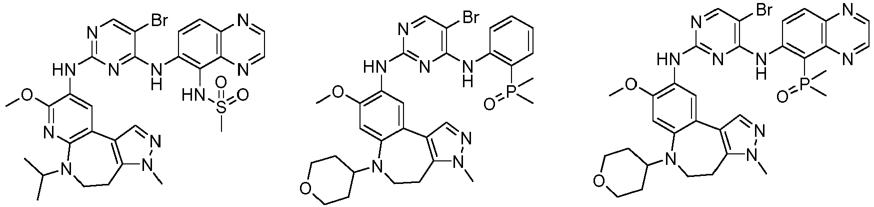

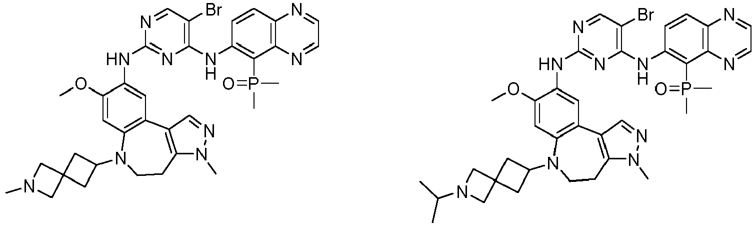

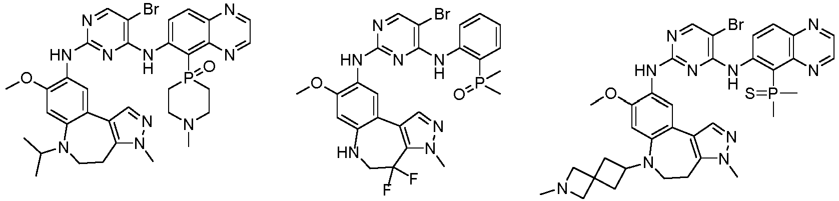

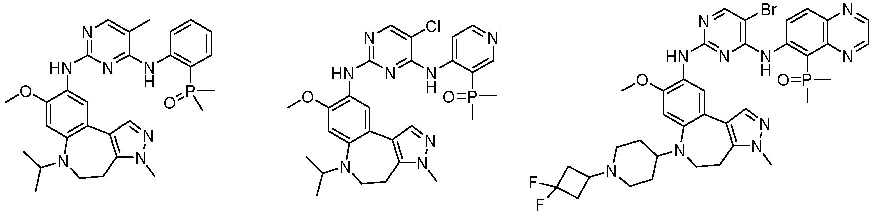

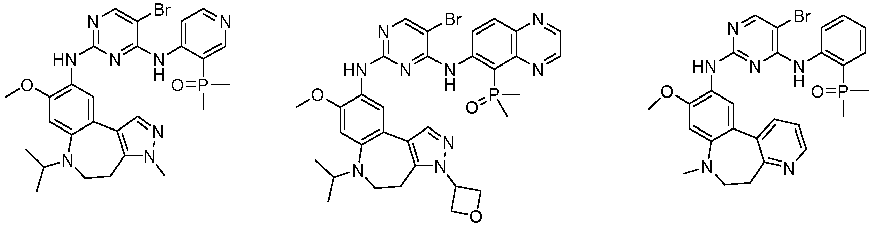

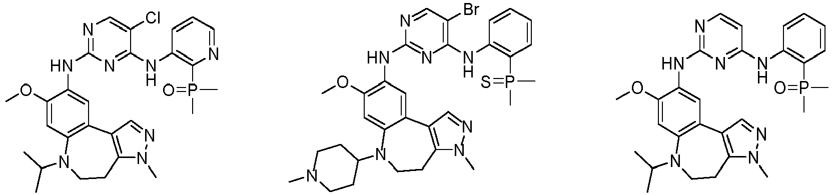

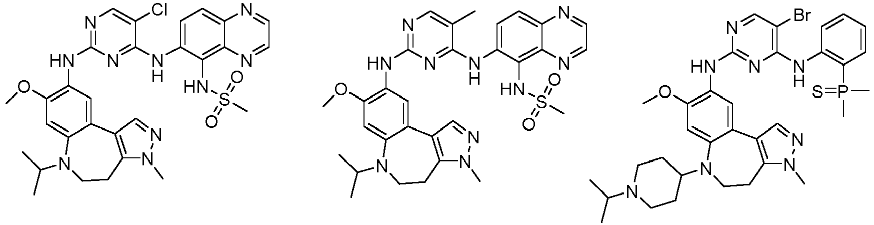

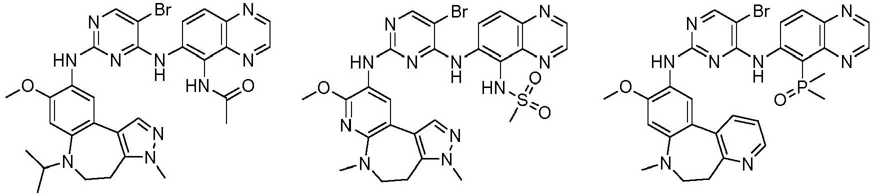

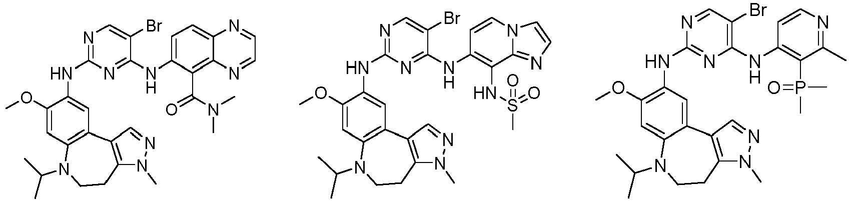

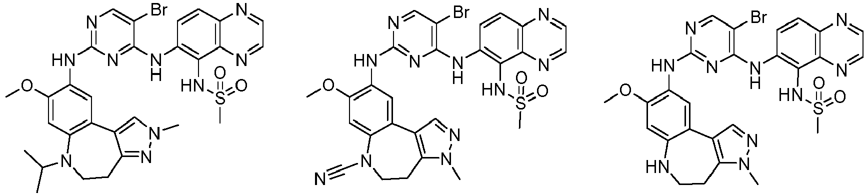

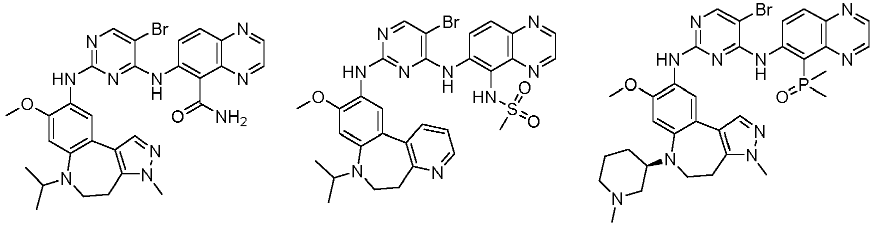

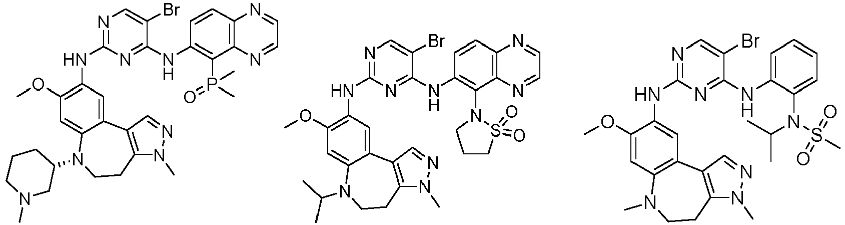

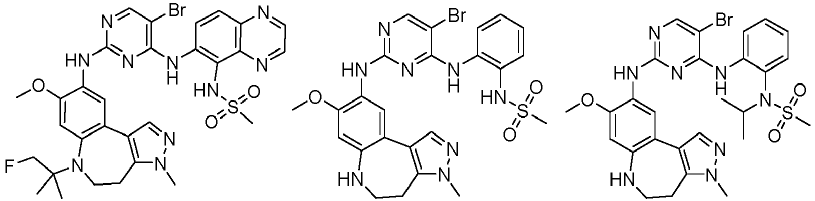

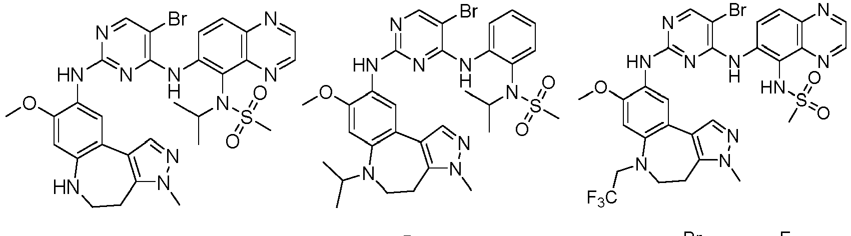

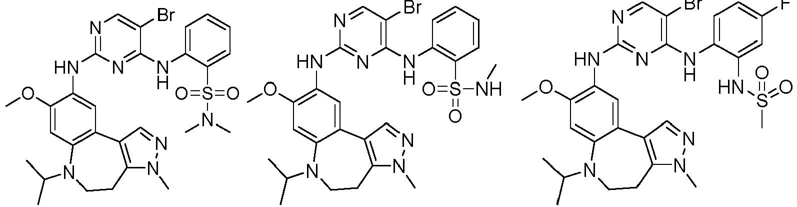

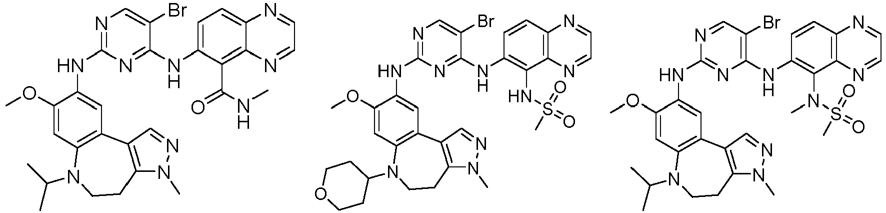

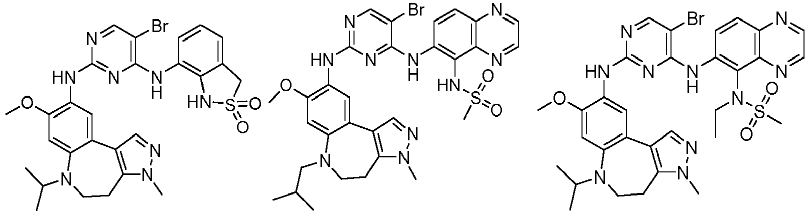

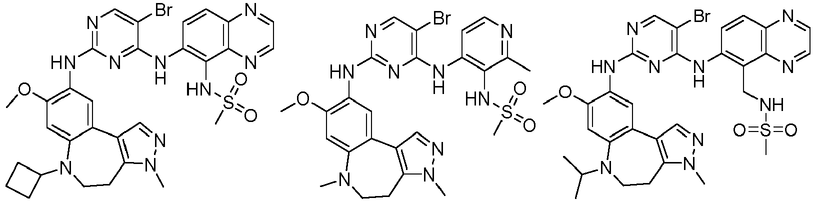

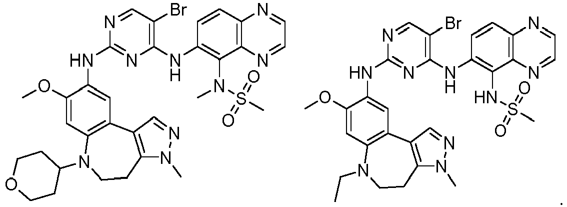

32. A pharmaceutical composition comprising the compound or the pharmaceutically acceptable salt thereof as claimed in any one of claims 1 to 31, and a pharmaceutically acceptable carrier, diluent, or excipient.

33. Use of the compound as claimed in any one of claims 1 to 32 or the pharmaceutically acceptable salt thereof, or the pharmaceutical composition as claimed in claim 32 for the manufacturing of a medicament for the treatment of cancer.

34. The use as claimed in claim 33, wherein the cancer comprises lymphoma, non-Hodgkin's lymphoma, ovarian cancer, cervical cancer, prostate cancer, colorectal cancer, breast cancer, pancreatic cancer, glioma, glioblastoma, melanoma, leukemia, gastric cancer, endometrial cancer, lung cancer, hepatocellular cancer, gastric cancer, gastrointestinal stromal tumor (GIST), acute myelogenous leukemia (AML), cholangiocarcinoma, renal cancer, thyroid cancer, anaplastic large cell lymphoma, mesothelioma, multiple myeloma, and melanoma.

35. The use as claimed in claim 34, wherein the cancer is lung cancer.

36. A compound represented by formula (V) or a stereoisomer thereof, or a pharmaceutically acceptable salt thereof, 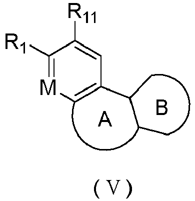
wherein, R₁₁ is -NH₂ or -NO₂;
R₁ is defined as in any one of claims 1 to 31;
ring A and ring B are defined as in any one of claims 1 to 31;
M is defined as in any one of claims 1 to 31;
structural unit 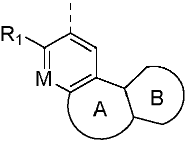 is defined as in any one of claims 22 to 31.

37. The compound or the stereoisomer thereof, or the pharmaceutically acceptable salt thereof as claimed in claim 36, which is selected from 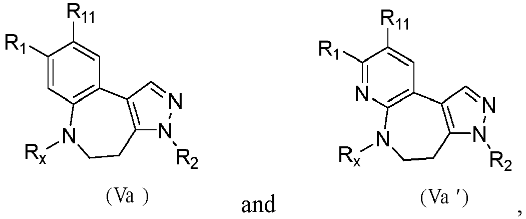 wherein,
R₁ is defined as in any one of claims 1 to 31;
Rₙ is defined as in claim 36;
R₂ is defined as in any one of claims 1 to 31;
Rₓ is defined as in any one of claims 22 to 31.

38. The compound or the stereoisomer thereof, or the pharmaceutically acceptable salt thereof as claimed in claim 37 is selected from, 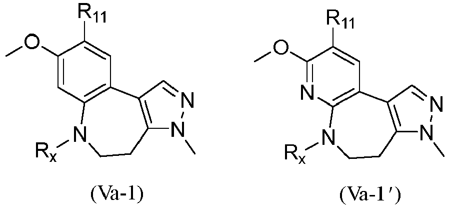 wherein, R₁₁ and Rₓ are as defined in claim 37.

39. Use of the compound or the stereoisomer thereof, or the pharmaceutically acceptable salt thereof as claimed in any one of claims 36 to 38 for preparing the compound, or the stereoisomer, the tautomer or the pharmaceutically acceptable salt, the prodrug, the hydrate, the solvate and the isotopically labeled derivative thereof as claimed in any one of claims 1 to 31.
